# EUROPEAN PATENT APPLICATION

(11) **EP 4 843 714 A1**
(43) Date of publication of application: **25.11.2026**
(21) Application number: 25178039.1
(22) Date of filing: 21.05.2025
(51) Int. Cl.: B25J 9/06, B25J 9/10, B25J 11/00, B25J 15/12, A61B 34/30

(54) **SOFT ROBOT**

(71) Applicant: Max-Planck-Gesellschaft zur Förderung der Wissenschaften e.V., 80539 München (DE)
(72) Inventor: SITTI, Metin, 70506 Stuttgart (DE); BAO, Xianqiang, 70506 Stuttgart (DE); WANG, Fan, 70506 Stuttgart (DE); ZHANG, Jianhua, 70506 Stuttgart (DE)
(74) Representative: Manitz Finsterwald Patent- und Rechtsanwaltspartnerschaft mbB

(57) **Abstract**

A soft robot comprises a body that has an outer part, an inner part, and a magnetic unit, wherein the outer part is flexible and comprises a flexible channel and wherein the inner part is movably arranged within the channel. One of the inner part and the outer part forms a magnetic member comprising the magnetic unit and the other one of the inner part and the outer part forms a flexible target member. The magnetic unit is arranged inside the channel or adjacent to the channel in an outer direction perpendicular to the channel. The outer part, the inner part, and the magnetic unit are configured to be placed within a magnetic field and the magnetic member and the target member are configured to move relative to each other to allow for a change of a magnetization profile of the target member.

## Description

The present disclosure relates to a soft robot, a system comprising the soft robot, an arrangement of a first soft robot and a second soft robot, and a method for operating the soft robot.

Soft robots, characterized by their shape programmability, high compliance, and physical adaptability, are capable of safely and efficiently interacting with complex environments (*1-3*). They have demonstrated significant potential in various application scenarios, including healthcare (*4-7*), industry (*8*, *9*), marine exploration (*10*, *11*), and search and rescue operations (*12*). The morphing and functionality of soft robots primarily rely on their actuation components, with common forms of actuation including light (*13, 14*), heat (*15*, *16*), sound (*17*), magnetic fields (*2*, *18, 19*), fluids (*20, 21*), electric fields (*22-24*), and tendon/cable mechanisms (*25*). Among these, magnetic actuation, which operates through the interaction between an external magnetic field and magnetic particles/units within the soft robots, generating force or torque, offers contactless operation, precise and dexterous control, rapid response, strong adaptability to environments, and ease of miniaturization, making it one of the most promising actuation strategies for soft robots in confined operation spaces (*1*, *2, 26, 27*).

In magnetic soft robots, morphing typically involves altering an external magnetic field. Nevertheless, the morphing range attainable with this method can be limited. Generally, a specific magnetization profile can generate only a single or a specific range of morphing modes, while diverse tasks and multiple functions can demand more drastically different morphing sequences. Even for some morphing modes that can be generated by certain fixed magnetization profiles, relying entirely on external magnetic fields would lead to complex control schemes, bulky and intricate magnetic field generation systems, and challenges in actuating specific regions of soft robots (*2, 28*).

Therefore, it is essential to be able to reprogram the magnetization profile of soft robots during their applications requiring diverse tasks or multiple functions (*29*, *30*). Additionally, many operational tasks demand a high degree of continuity, which places increased demand on reprogramming speed and in-situ operation.

Existing studies have explored reprogramming techniques, which can be broadly divided into phase change of the base materials (*31-34*) and Curie temperature-based reorientation of magnetic domains (*29*). Although these approaches facilitate reprogramming, they limit real-time operations, as methods like laser heating or oven heating require sufficient time for both heating and cooling. Besides, the depth of heating and selection of heating points impose specific requirements on the wall thickness and shape of the soft robot; for example, laser heating is only suitable for two-dimensional structures and thin-walled designs (*29*). Furthermore, the deployment of heating technology is constrained by the operating workspace, consequently limiting in-situ operability to some extent. There are also template-based reprogramming methods (*35, 36*), which require templates for positioning, further limiting both the real-time capability and in-situ operability of reprogramming. The inability to conduct real-time, in-situ magnetization reprogramming to achieve the required magnetization profile adversely impacts the operational continuity of magnetically controlled robots, thereby imposing significant constraints on their applications.

It is therefore an object of the present invention, to provide a soft robot that allows for real-time, in-situ shape changes.

### SUMMARY

The present disclosure provides a soft robot, a system comprising the soft robot, an arrangement of a first soft robot according to the present disclosure and a second soft robot according to the present disclosure, and a method for operating the soft robot. Embodiments are given in the dependent claims, the description, and the drawings.

In one aspect, the present disclosure is directed at a soft robot comprising a body, wherein the body has an outer part, an inner part, and a magnetic unit. The outer part is flexible and comprises a flexible channel and the inner part is movably arranged within the channel. Furthermore, one of the inner part and the outer part forms a magnetic member comprising the magnetic unit and the other one of the inner part and the outer part forms a flexible target member. The magnetic unit is arranged inside the channel or adjacent to the channel in an outer direction perpendicular to the channel and the outer part, the inner part, and the magnetic unit are configured to be placed within a magnetic field so that the magnetic field exerts a magnetic force on the magnetic unit. Additionally, the magnetic member and the target member are configured to move relative to each other to allow for a change of a magnetization profile of the target member and for a change of a response of the target member to the magnetic field.

The soft robot according to the present disclosure allows for real-time, in-situ shape changes in a constant magnetic field. By altering the magnetization profile of the target member, the magnetic forces that are exerted on the target member within the magnetic field change, leading to a deformation or movement of the target member, without the need to change the magnetic field itself. Furthermore, the deformation of the target member may be independent of a deformation of another body or another part of the body within the magnetic field. The soft robot therefore allows operation of multiple parts of the body or multiple bodies independently from each other in the same magnetic field.

The magnetic member may be configured to be moved between a first position with respect to the target member and a second position with respect to the target member, wherein the target member assumes a first shape within the magnetic field if the magnetic member is at the first position and the target member assumes a second shape within the magnetic field if the magnetic member is at the second position, the first shape being different from the second shape.

The target member may be a flexible member. With the outer part being the target member, the magnetic unit may be movably arranged within the channel of the flexible outer part. Movement of the magnetic unit within the channel then changes the magnetization profile of the outer part comprising the channel. With the inner part being the target member, the inner part may comprise an inner flexible part that is guided within the channel. The inner part may follow the channel. The magnetic unit may be fixed with respect to the outer part, for example, the magnetic unit may be attached to the outer part or it may form a section of the outer part. By moving the outer part, which forms the magnetic member, along the inner part, which forms the target member, the position of the magnetic unit with respect to the inner part and therefore also the magnetization profile of the inner part may be changed.

The target member and the magnetic member may be configured to move relative to each other to allow for the change of the magnetization profile of the target member by fixing the target member with respect to an environment of the body and/or a support of the body and by moving the magnetic member relative to the environment and/or the support. Alternatively, the magnetic member may be fixed relative to the environment and/or the support and the target member may move relative to the environment and/or the support. For example, with the inner part forming the target member and the outer part forming the magnetic member, the outer part may remain fixed with respect to the environment of the body or the support of the body and the inner part may be moved relative to the outer part.

For example, the magnetic unit of the magnetic member may be configured to be moved between a first position with respect to the target member and a second position with respect to the target member, wherein the target member assumes a first shape within the magnetic field with the magnetic unit at the first position and the target member assumes a second shape within the magnetic field with the magnetic unit at the second position, the first shape being different from the second shape.

The magnetic member and the target member may be configured to move relative to each other along the channel. For example, a direction of relative movement of the magnetic member and the target member may be parallel to the channel. The magnetic member and the target member may be configured to move relative to each other by at least the inner part and the outer part being configured to move relative to each other.

The outer part may be one of a flexible tube, a two-dimensional sheet, and a three-dimensional box. The outer part may also have any other shape with other embodiments.

The channel may extend from a first end of the outer part to a second end of the outer part. The channel may be open at at least one end, such as at both ends. Alternatively, the channel may also be closed at both ends or only open at one of the ends and closed at the other one of the ends. For example, the channel may be open at an end that is located at a support of the body and it may be closed at an other end of the channel facing away from the support. The other end may be a free end of the first part.

The channel extends in a longitudinal direction. In principle, it may have any cross-sectional shape. For example, the channel may have a circular cross-section or a rectangular cross-section, such as a square cross-section.

The longitudinal direction and the outer direction thereby may be defined relative to the channel so that the longitudinal direction follows the shape of the channel and/or a shape of the outer part. The outer direction then may be orientated perpendicular to the channel at every point along the channel.

For example, the magnetic unit may be arranged adjacent to the channel in the outer direction by being placed in a radial direction from a center of the channel.

With an outer part that is configured as a tube, the magnetic unit may be placed on an outer surface of the tube or it may be integrated in a sidewall of the tube confining the channel. For example, the magnetic unit may be configured as a tubular member, such as a flexible tubular member. With the outer part being configured as a sheet or a box, the magnetic unit may be fixed to or integrated into the sheet or into a sidewall of the box.

The magnetic unit may be placed directly at the channel or it may have a distance from the channel.

The target member may be free of any magnetic unit or it may also comprise a magnetic unit, such as a further magnetic unit as described below.

The magnetic force acting on the magnetic unit within the magnetic field may comprise a force component that is orientated perpendicular to a direction of the movement of the magnetic unit relative to the target member. Additionally or alternatively, the magnetic force may have a component that is orientated perpendicular to the channel.

The magnetic field may be essential homogeneous, such as homogeneous, within an interaction zone in which the magnetic unit interacts with the magnetic field during operation of the soft robot.

According to an embodiment, the movement of the magnetic unit comprises a translation of the magnetic unit with respect to the target member and/or a rotation of the magnetic unit with respect to the target member. For example, it may comprise both a translation and a rotation.

The magnetic unit may be configured to both translate and rotate with respect to the target member. With other embodiments, movement of the magnetic unit may be restricted to one of a translation without rotation and a rotation without translation. For example, movement of the magnetic unit may be restricted to a translation without rotation.

In general, a direction of a magnetic moment of the magnetic unit may be tilted with respect to a direction of the channel. For example, the direction of the magnetic moment of the magnetic unit may be perpendicular to the direction of the channel.

According to an embodiment, a direction of a magnetic moment of the magnetic unit is parallel to the channel. This provides for a deflection of the target member within the magnetic field that is insensitive to a rotation of the target member with respect to the magnetic member. For example, with the magnetic unit being placed within the channel, the magnetic unit may rotate within the channel without changing a deflection of the outer part forming the target member.

With some embodiments, the outer part may form the magnetic member and the inner part may form the target member. The outer part then comprises the magnetic unit and the magnetic unit may be located adjacent to the channel in the outer direction. The outer part may be configured to change a shape of the inner part by deflecting or deforming due to the force exerted on the magnetic unit by the magnetic field and by causing a deflection or deformation of the inner part guided within the channel of the outer part. The inner part and the outer part thereby may be deflected or deformed at a position at which the magnetic unit is located adjacent to the channel. With embodiments, in which the inner part protrudes from the channel of the outer part the deflection or deformation of the inner part due to the deflection or deformation of the outer part may change a position of a free end of the inner part protruding from the channel.

According to an embodiment, the inner part forms the magnetic member comprising the magnetic unit so that the magnetic unit is movably arranged within the channel, wherein the magnetic unit is configured to be actuated through the channel. This mechanically conceals the movement of the magnetic unit from the environment. The magnetic member is prevented from interacting mechanically with the environment during the movement relative to the target member.

The magnetic unit may be actuated through the channel by a mechanical link between the magnetic unit and an actuator. The actuator may be, for example, placed at an end of the channel. The mechanical link may, for example, be formed by a manipulator placed within the channel. The manipulator may be configured as a flexible part, such as a flexible hollow member, such as a tube, or a flexible solid rod. An outer shape of the flexible part may correspond to an inner shape of the channel.

The magnetic unit may also be actuated through the channel and/or connected to the actuator by other means, such as pneumatically by air or a liquid present within the channel.

According to an embodiment, movement of the magnetic unit is limited to an interior of the channel. This prevents a change of the outer dimensions, such as a length, of the outer part due to movement of the inner part comprising the magnetic unit within the channel.

The movement of the magnetic unit may be limited to the interior of the channel at least in one direction. For example, the movement of the magnetic unit may be limited to the interior of the channel in a first direction along the channel and the magnetic unit may be allowed to exit the channel in the second direction along the channel, the second direction being opposite to the first direction. For example, the second direction may face towards an actuator for moving the magnetic unit and /or the second direction may face towards a clamped end of the outer part. The first direction may face towards a free end of the outer part.

According to an embodiment, the inner part comprises a flexible part, wherein the flexible part is movably arranged with respect to the outer part. The flexible part may be configured to transfer a force, for example to the magnetic unit or to a target object manipulated by the soft robot.

According to an embodiment, the flexible part and/or the magnetic unit are configured as solid figures, such as filled cylinders. This provides for a versatile and flexible construction that is easy to manufacture.

According to an embodiment, the flexible part and/or the magnetic unit are hollow,
wherein, for example, the flexible part and/or the magnetic unit are configured as hollow cylinders. Such a construction of the flexible part and/or the magnetic unit allows for a placement of additional components within the inner part, such as the additional inner part described below. With some embodiments, the hollow inner part may house an actor for manipulating a target object. The actor may, for example, be configured as a surgical instrument. The hollow inner part may form a working channel for advancing and retracting the actor.

According to an embodiment, the magnetic unit is fixedly attached to the flexible part, wherein the flexible part forms a manipulator for actuating the magnetic unit.

According to an embodiment, the manipulator mechanically connects the magnetic unit to an actuator, wherein the manipulator is configured to transfer an actuation force from the actuator to the magnetic unit.

According to an embodiment, the magnetic unit is attached to an end of the manipulator. With another embodiment, the magnetic unit may be attached to the manipulator at a location spaced apart from an end of the manipulator.

According to an embodiment, the body comprises a further magnetic unit, wherein the further magnetic unit is configured to be placed within the magnetic field so that the magnetic field exerts a magnetic force on the further magnetic unit. The further magnetic unit is arranged inside the channel or adjacent to the channel in an outer direction perpendicular to the channel and the magnetic unit and the further magnetic unit are configured for being placed at a distance from each other, for example along the channel. Providing the further magnetic unit in addition to the magnetic unit allows for versatile magnetization profiles of the target member.

The distance may be larger than zero and the magnetic unit and the further magnetic unit may be configured for being spaced apart from each other, for example along the channel.

The magnetic unit and the further magnetic unit may be configured for being placed at the distance from each other by being fixed with respect to each other at the distance. Alternatively, the magnetic unit and the further magnetic unit may be configured for being placed at the distance from each other by being movable with respect to each other into at least one state in which the magnetic unit is located at the distance from the further magnetic unit. In general, the distance between the magnetic unit and the further magnetic unit may be fixed or it may be variable.

The magnetic unit may form a first magnetic unit and the further magnetic unit may form a second magnetic unit. With some embodiments, the soft robot may comprise a third magnetic unit. The third magnetic unit may be movably arranged with respect to the outer part. For example, the first magnetic unit, the second magnetic unit and the second connected unit may be movably arranged with respect to the outer part and fixed with respect to each other. The third magnetic unit may be longitudinally fixed with respect to the magnetic unit and the further magnetic unit at respective distances from the magnetic unit and the further magnetic unit. The respective distances may be larger than zero so that the third magnetic unit may be spaced apart from both the magnetic unit and the further magnetic unit.

According to an embodiment, a magnetic moment of the magnetic unit differs from a magnetic moment of the further magnetic unit. Thereby, a direction and/or a magnitude of the magnetic moment of the magnetic unit may differ from the magnetic moment of the further magnetic unit. With other embodiments, the magnetic moment of the magnetic unit may be the same as the magnetic moment of the further magnetic unit.

According to the present disclosure, any magnetic moment may be the magnetic dipole moment.

According to an embodiment, the magnetic unit and the further magnetic unit are configured for being placed at the distance from each other with no additional magnetic unit being placed in between the magnetic unit and the further magnetic unit. The deformation of the target member then entirely depends on the magnetic moment of the magnetic unit and the magnetic moment of the further magnetic unit. This provides a simple construction and predictive behavior.

For example, the direction of the magnetic moment of the further magnetic unit may be opposite to the direction of the magnetic moment of the magnetic unit and the magnetic unit and the further magnetic unit may be spaced apart from each other along the channel. The magnetic unit and the further magnetic unit then experience an opposite torque within a homogeneous magnetic field. As a consequence, the section of the target member in between the magnetic unit and the further magnetic unit may be bent in an arc.

According to an embodiment, the further magnetic unit is movably arranged with respect to the target member to allow for a change of the magnetization profile of the target member within the external magnetic field and a change of a response of the target member to the magnetic field.

According to an embodiment, the further magnetic unit is longitudinally fixed with respect to the magnetic unit. The magnetic member then may also comprise the further magnetic unit. Both the magnetic unit and the further magnetic unit may be movable with respect to the target member.

According to an embodiment, the further magnetic unit is movable with respect to the magnetic unit. Thereby, the further magnetic unit may be fixed with respect to the target member. Alternatively, it may be movable with respect to the target member, for example independently of the magnetic unit.

According to an embodiment, the magnetic unit and the further magnetic unit are configured to be placed adjacent to each other, such as adjoining each other in a longitudinal direction or nested around each other in the outer direction. When being placed adjacent to each other, a resulting magnetic moment generated by the magnetic unit and the further magnetic unit may be a superposition of the magnetic moment of the magnetic unit and the magnetic moment of the further magnetic unit.

According to an embodiment, one of the magnetic unit and the further magnetic unit is placed within the channel and the other one of the magnetic unit and the further magnetic unit is placed at least partially around the channel. This facilitates placing the magnetic unit and the further magnetic unit next to each other. The other one of the magnetic unit and the further magnetic unit may be fixed with respect to the outer part. The other one of the magnetic unit and the further magnetic unit may fully surround the channel. For example, the channel may extend through the other one of the magnetic unit and the further magnetic unit.

According to an embodiment, a direction of a magnetic moment of the further magnetic unit is opposite to a direction of a magnetic moment of the magnetic unit. With embodiments, in which the further magnetic unit may be placed adjacent to the magnetic unit, opposing directions of the magnetic moments allow to reduce or invert the magnetic moment of the magnetic unit by placing the further magnetic unit adjacent to the magnetic unit.

According to an embodiment, a magnitude of the magnetic moment of the further magnetic unit equals a magnitude of the magnetic moment of the magnetic unit.

According to an embodiment, a magnitude of the magnetic moment of the further magnetic unit is essentially twice or half a magnitude of the magnetic moment of the magnetic unit, such as twice or half the magnitude of the magnetic moment of the magnetic unit.

If the magnitude of the magnetic moment of the further magnetic unit is essentially twice or half the magnitude of the magnetic moment of the magnetic unit, the magnitude of the magnetic moment of the further magnetic unit may deviate from the magnitude of the magnetic moment of the magnetic unit by a margin magnetic moment. The margin magnetic moment may be adapted to compensate for a change of a bending amplitude of the target member in the magnetic field due to movement of the further magnetic unit between a position located adjacent to the magnetic unit and a position located away from the magnetic unit. This may result in the bending amplitude of the target member with the further magnetic unit located adjacent to the magnetic unit being the same as with the further magnetic unit located away from the magnetic unit.

According to an embodiment, the magnetic unit is configured to be placed adjacent to the further magnetic unit by placing, in a first configuration, a manipulator connected to one of the magnetic unit and the further magnetic unit within the channel and the magnetic unit is configured to be placed, in a second configuration, at the distance from the further magnetic unit by removing the manipulator from the channel. In the first configuration, the outer part and the manipulator placed within the channel have a first stiffness against transverse deflection or bending and, in the second configuration, the outer part without the manipulator has a second stiffness against transverse deflection or bending. A magnitude of the magnetic moment of the magnetic unit then deviates from twice or half the magnitude of the magnetic moment of the further magnetic unit by a margin magnetic moment, wherein a magnetic force acting on the margin magnetic moment in the magnetic field compensates for a difference between the first stiffness and the second stiffness so that a deflection amplitude or a bending amplitude of the outer part within the magnetic field is essentially equal in the first configuration and in the second configuration.

In general, the magnetic member and the target member may be configured to switch the response of the target member to the magnetic field on or off depending on the relative position between the magnetic member and the target member. For example, movement of the magnetic member and the target member relative to each other may lead to a change of the magnetization profile of the target member between a first magnetization profile, in which the magnetic moment of the target member is essentially zero, such as zero, and a second magnetization profile, in which the target member has a magnetic moment that is non-zero.

According to an embodiment, the magnetic unit may be configured to be moved between a first position, in which it is located adjacent to the further magnetic unit, and a second position, in which it is located away from the further magnetic unit. The direction of the magnetic moment of the magnetic unit may be opposite the direction of the magnetic moment of the further magnetic unit. In one variant of this embodiment, the magnitude of the magnetic moment of the magnetic unit may be equal to the magnitude of the magnetic moment of the further magnetic unit. Thus, the superposition of the magnetic moments of the magnetic unit and the further magnetic unit is zero if the magnetic unit is in the first position and an action of the further magnetic unit is switched off by moving the magnetic unit into the first position. In another variant of this embodiment, the magnitude of the magnetic moment of the magnetic unit may be essentially twice or half, for example twice, or twice or half, such as twice, the magnitude of the magnetic moment of the further magnetic unit. Thus, the superposition of the magnetic moments of the magnetic unit and the further magnetic unit has the same magnitude but a reverse direction as the magnetic moment of the further magnetic unit. The direction of the magnetic moment of the further magnetic unit then may be reversed by placing the magnetic unit adjacent to the further magnetic unit.

With both variants of the foregoing embodiment, the inner part may form the magnetic member and may comprise the magnetic unit and the further magnetic unit may be fixed to the outer part, for example to a free end of the outer part. Moving the magnetic unit to the first position with respect to the outer part then switches the action of the further magnetic unit off in the first variant and reverses the direction of the magnetic moment of the further magnetic unit in the second variant.

According to an embodiment, the soft robot comprises an additional body having an additional outer part, an additional inner part, and an additional magnetic unit, wherein the additional outer part is flexible and comprises a flexible additional channel and the additional inner part is movably arranged within the additional channel. One of the additional inner part and the additional outer part forms an additional magnetic member comprising the additional magnetic unit and the other one of the additional inner part and the additional outer part forms a flexible additional target member. The additional magnetic unit is arranged inside the additional channel or adjacent to the additional channel in an outer direction perpendicular to the additional channel and the additional outer part, the additional inner part, and the additional magnetic unit are configured to be placed within the magnetic field so that the magnetic field exerts a magnetic force on the additional magnetic unit. Furthermore, the additional magnetic member is configured to move relative to the additional target member to allow for a change of a magnetization profile of the additional target member and for a change of a response of the additional target member to the magnetic field. Thereby, the outer part and the additional outer part are deformable independently from each other and the magnetic unit and the additional magnetic unit are movable independently from each other.

With this embodiment, the magnetic field forms a common magnetic field acting on both the body and the additional body. The soft robot according to this embodiment then comprises two target members, such as two outer parts, that may be actuated independently from each other within the common magnetic field by moving their respective magnetic members independently from each other. The body and the additional body may be configured to be actuated independently from each other, for example to cooperate with each other
The additional body may be configured as it is disclosed for the body.

According to an embodiment, the outer part and the additional outer part are fixed to a common support.

According to an embodiment, the outer part and the additional outer part are placed next to each other. For example, the outer part and the additional outer part may be placed next to each other along a bending direction of the outer part and/or the additional outer part. This allows to vary a distance between free ends of the outer part and the additional outer part by bending at least one of the outer part and the additional outer part, such as both the outer part and the additional outer part.

According to an embodiment, the outer part is configured as an actor for moving a target, such as a fluid or a solid object.

According to an embodiment, the body comprises a further inner part, wherein the further inner part is flexible and comprises a further channel and wherein the further inner part is stacked in the outer direction in between the outer part and the inner part so that the further inner part is located within the channel of the outer part and the inner part is located within the further channel of the further inner part. The inner part and the further inner part are movable with respect to each other. Such a construction provides a multitude of degrees of freedom for actuating the soft robot and changing the magnetization profile of the target member.

For example, both the outer part and the further inner part may form tubular parts that are stacked within each other.

According to an embodiment, the further inner part is configured to extend through an end of the channel of the outer part further into the magnetic field. The outer part then may provide a flexible guiding member for directing the extending further inner part.

According to an embodiment, the further inner part comprises the further magnetic unit and the further magnetic unit is fixed to the further inner part. The magnetic unit thereby may be fixed to the inner part or the outer part. For example, the magnetic unit may be fixed to the inner part and both the inner part and the further inner part may be independently movable within the outer part.

According to an embodiment, a stiffness of the outer part is larger than a stiffness of the further inner part. This prevents buckling of the outer part upon movement of the further inner part within the channel of the outer part and facilitates movement of the further inner part within the flexible outer part

According to an embodiment, a stiffness of the inner part is lower than the stiffness of the further inner part and, optionally, than the stiffness of the outer part. This prevents buckling of the further inner part upon movement of the inner part within the channel of the further inner part and facilitates movement of the inner part within the flexible further inner part and the flexible outer part.

According to an embodiment, the soft robot further comprises a set of further inner parts with at least one further inner part, wherein each of the further inner parts is flexible and comprises a further channel. The further inner parts of the set are stacked one into the other in between the outer part and the inner part. Furthermore, at least one of the further inner parts, such as a multitude of the further inner parts, like all further inner parts, may each comprise a respective further magnetic unit attached thereon. Such a construction provides a large number of degrees of freedom for changing the magnetization profile of the target member.

According to an embodiment, a stiffness of the inner part, respective stiffnesses of the further inner parts, and a stiffness of the outer part decrease in the outer direction from the inner part to the outer part. This prevents, upon movement of one of the parts, buckling of the other parts located radially outside the respective one of the parts.

According to an embodiment, the inner part, the further inner parts and the outer part are configured to be axially arranged with respect to each other with respective ends of the further inner parts and of the outer part being sequentially located along a length of the channel in a set of working zones so that each one of the working zones only comprises one of the respective ends. At least one of the working zones is filled with the magnetic field and the working zones of the set do not overlap with each other. Thereby, the inner part, the further inner parts and the outer part are configured to axially translate with respect to each other with each of the respective ends remaining within its respective working zone during actuation of the soft robot. Such a configuration facilitates actuation of the soft robot since changes in the stiffness of the body within each working zone is limited to a change of the stiffness caused by the presence or absence of the respective one of the further inner part and the outer part located within the respective working zone. Therefore, the change of the stiffness of the body is easily predictable.

According to an embodiment, at least two of the working zones are filled with a respective magnetic field, wherein the magnetic fields of the at least two of the working zones differ from each other.

For example, each of the working zones may be filled with a respective magnetic field and the magnetic fields of at least two of the working zones may differ from each other.

A working zone filled with a magnetic field may form a magnetic interaction zone.

According to an embodiment, the soft robot further includes a set of magnetic units, the magnetic unit being part of the set, wherein to at least some, such as to each one, of the further inner parts a respective one of the magnetic units of the set is attached. Furthermore, respective magnetic fields within the individual interaction zones and magnetic moments of respective magnetic units located within the individual interaction zones are configured to generate magnetic forces on the individual magnetic units that increase with a number of parts of the body present in the interaction zone comprising the respective magnetic unit. The increase of the magnetic forces thereby may compensate an increase of the stiffness of the body due to the larger number of parts. Thereby, the magnetic fields within the individual interaction zones may increase and/or the magnetic moments of the individual magnetic units may increase.

According to an embodiment, the inner part comprises a first inner part section that is configured to be located next to the magnetic unit and a second inner part section adjoining the first inner part section, the second inner part section being configured to be placed away from the magnetic unit. The second inner part section is configured to connect the first inner part section to an actuator to actuate a movement of the inner part relative to the outer part. The first inner part section is made from a first inner part material and the second inner part section is made from a second inner part material, wherein the second inner part material has a different stiffness than the first inner part material, such as a higher stiffness than the first inner part material. This allows to adapt the stiffness of the inner part for optimizing moveability of the inner part within the outer part. For example, the stiffness of the second inner part section may be adapted to prevent buckling of the inner part within the channel of the outer part which may block movement of the inner part.

With embodiments, in which the inner part forms the magnetic member comprising the magnetic unit, the first inner part section may adjoin the magnetic unit and the magnetic unit may be fixed to the first inner part section. With embodiments, in which the outer part forms the magnetic member comprising the magnetic unit, the first inner part section may be located along the channel at a position of the magnetic unit.

The second inner part section may serve as a connector between the first inner part section and an end of the inner part fixed to an actuator for actuating movement of the inner part. For example, the second inner part section may serve as a connector between a magnetic interaction zone and an actuation zone of the inner part. The body may be configured to be connected to an actuator in the actuation zone.

The first inner part section may be configured to be located at least partly within the magnetic field and the second inner part section may be configured to be located at least partly outside the magnetic field.

According to an embodiment, the second inner part section is configured to be directly connected to an actuator, such as a mechanical actuator. The second inner part section may be directly connected to the actuator According to an embodiment, the flexible part of the inner part forms the first inner part section.

According to an embodiment, the second inner part section is at least partially located within a feeding zone, wherein the feeding zone is located in between an interaction zone and an actuation zone for actuation of the relative movement between the magnetic member and the target member, wherein the magnetic unit is configured to interact with the magnetic field within the interaction zone, such as only within the interaction zone.

According to an embodiment, the second inner part section is located outside a bending zone of the channel for all positions that the magnetic member is configured to be located at relative to the target member during operation of the soft robot, wherein the bending zone comprises a region in which the shape of the channel changes due to the magnetic force on the magnetic unit.

According to an embodiment, the outer part comprises a first outer part section that is configured to be located next to the magnetic unit and a second outer part section adjoining the first outer part section, the second outer part section being configured to be placed away from the magnetic unit. The second outer part section is configured to connect the first outer part section to an actuator to actuate a movement of the inner part relative to the outer part. The first outer part section is made from a first outer part material and the second outer part section is made from a second outer part material, wherein the second outer part material has a different stiffness, such as a higher stiffness, than the first outer part material. This allows to adapt the stiffness of the outer part for optimizing moveability of the inner part within the outer part. For example, the stiffness of the second outer part section may be adapted to prevent buckling of the outer part when the inner part moves within the channel of the outer part which may block movement of the inner part.

With embodiments, in which the inner part forms the magnetic member comprising the magnetic unit, the first outer part section may be located along the channel at a position of the magnetic unit. With embodiments, in which the outer part forms the magnetic member comprising the magnetic unit, the first outer part section may adjoin the magnetic unit and the magnetic unit may be fixed to the first outer part section.

The first inner part section may be located within the first outer part section and the second inner part section may be located within the second outer part section.

According to an embodiment, the magnetic unit forms a part of a magnetic element, wherein the magnetic element extends in a longitudinal direction from a first end of the magnetic element to a second end of the magnetic element. A magnetic moment of the magnetic element, such as a direction of the magnetic moment of the magnetic element, continuously changes from a first magnetic moment at the first end to a second magnetic moment at the second end, the second magnetic moment being different from the first magnetic moment. This allows for complex deformation of the target member due to individual sections of the magnetic unit in between the first end and the second end bending in different directions within the magnetic field.

The magnetic unit may be placed at the first end of the magnetic element.

The further magnetic unit may form a part of the magnetic element. For example, the further magnetic unit may be placed at the second end of the magnetic element.

Alternatively, the further magnetic unit may form a part of a further magnetic element, wherein the further magnetic element extends in a longitudinal direction from a first end to a second end. A magnetic moment of the further magnetic element, such as a direction of the magnetic moment of the further magnetic element, continuously changes from a first further magnetic moment at the first end to a second further magnetic moment at the second end, the first further magnetic moment being different from the second further magnetic moment.

According to an embodiment, the magnetic element is flexible. For example, the magnetic element may comprise a flexible substrate material and a multitude of magnetic units embedded into the substrate material. The multitude of magnetic units may comprise the magnetic unit and/or the further magnetic unit. The individual magnetic units of the multitude of magnetic units each may be formed by a magnetic particle.

According to an embodiment, the magnetic element is configured as an elongated cylinder, such as an elongated hollow cylinder or an elongated solid cylinder.

According to an embodiment, a projection of the direction of the magnetic moment of the magnetic element onto a plane perpendicular to the channel or to the longitudinal direction continuously rotates from a first direction of the first magnetic moment to a second direction of the second magnetic moment. This provides for a continuous deformation of the magnetic element within the magnetic field. For example, it may provide for a circular deformation or a helical of the magnetic element within the magnetic field.

According to an embodiment, the magnetic moment of the magnetic element comprises a non-zero component in a direction along the channel or parallel to the longitudinal direction. This may provide for a deformation of the magnetic element in the magnetic field in which the magnetic element extends in the longitudinal direction.

According to an embodiment, a material of the channel and/or a material of the inner part comprises a surface tension diffuser, such as Slide STD-1, Kaupo, DE.

According to an embodiment, the soft robot comprises a lubricant in between the channel and the inner part, such as talcum powder, an oil-based lubricant. The lubricant may comprise glycerol and/or water.

According to an embodiment, the soft robot further comprises an actuator, wherein the actuator is configured to generate an actuation force that induces a movement of the magnetic unit relative to the target member.

According to an embodiment, the actuator is configured to deliver the actuation force to the magnetic unit via the channel, wherein the actuator, for example, delivers the force on the magnetic unit by a mechanical link or a medium, such as a gas or a liquid, present within the channel. The mechanical link may be formed, for example, by the flexible part of the inner part. The mechanical link may comprise the second inner part section.

According to an embodiment, the magnetic unit is configured to remain longitudinally stationary with respect to a surrounding of the soft robot while the target member translates longitudinally with respect to the magnetic unit. This allows to deform the target member at a fixed position with respect to the surrounding while the target member is moving through the surrounding. For example, the magnetic unit may be placed within a vicinity of an obstacle and a deformation of the target member due to a torque exerted on the magnetic unit by the magnetic field may deflect the target member away from the obstacle.

With the inner part forming the magnetic member comprising the magnetic unit and the outer part forming the target member, the inner part may be configured to remain longitudinally stationary within the channel with respect to the surrounding and the outer part may be configured to translate with respect to the surrounding along the inner part.

With embodiments comprising the further magnetic unit, the further magnetic unit may remain longitudinally stationary with respect to the surrounding while the target member translates longitudinally with respect to the further magnetic unit and the surrounding.

According to an embodiment, the outer part is configured as an elongated cylinder, for example as a flexible tube or a flexible rod. This provides a simple construction of the outer part.

The body of the soft robot may be configured as a continuum robot, such as a cylindrical continuum robot. For example, the outer part may form a backbone of the continuum robot. Likewise, the additional body of the soft robot may be configured as an additional continuum robot, such as a cylindrical continuum robot. For example, the additional outer part may form a backbone of the additional continuum robot.

According to an embodiment, the body forms part of a medical instrument, such as a catheter for medical use. The outer part then may form an outer sleeve of the medical instrument and the inner part may be movably arranged within the outer sleeve. The magnetic unit then is located within the outer sleeve or at the outer sleeve next to the channel so that the outer sleeve is deformed by the force exerted by the magnetic field on the magnetic unit. This differentiates the medical instrument according to the present disclosure from medical instruments, such as catheters, that have a flexible magnetic guidewire protruding from their outer sleeve, for which the magnetic unit is not located at the outer sleeve and an external magnetic field only deforms the guidewire but not the outer sleeve.

According to an embodiment, the soft robot comprises a support and the outer part extends in a longitudinal direction between a first end and a second end, wherein the second end is fixed to the support and the first end is configured as a free end. The channel runs from the second end to the first end and the outer part forms the target member and the inner part forms the magnetic member. This allows to use the outer part, for example, as an actuator for moving a target, such as a liquid or a solid object.

According to an embodiment, the soft robot is configured as an artificial cilia array, wherein the body forms an artificial cilium placed in the artificial cilia array and the artificial cilia array comprises a multitude of other bodies forming other artificial cilia. The other artificial cilia each comprise an outer part, an inner part, and a magnetic unit, wherein the outer part of each of the other artificial cilia is flexible and comprises a flexible channel and the inner part of each of the other artificial cilia is movably arranged within the channel of the respective one of the other artificial cilia. The inner part of each of the other artificial cilia forms a magnetic member comprising the magnetic unit of the respective one of the other artificial cilia and the outer part of each of the other artificial cilia forms a flexible target member of the respective one of the other artificial cilia. The magnetic unit of each of the other artificial cilia is arranged inside the channel of the respective one of the other artificial cilia. The outer part of each of the other artificial cilia, the inner part of each of the other artificial cilia, and the magnetic unit of each of the other artificial cilia are configured to be placed within the magnetic field so that the magnetic field exerts a magnetic force on the magnetic units of each of the other artificial cilia. The magnetic unit of each of the other artificial cilia is configured to move relative to the outer part of the respective one of the other artificial cilia to allow for a change of a magnetization profile of the outer part of the respective one of the other artificial cilia and for a change of a response of the outer part of the respective one of the other artificial cilia to the magnetic field, wherein the magnetic unit of the artificial cilium and the magnetic units of the other artificial cilia are movable independently from each other within the common magnetic field.

The artificial cilia may be configured to be actuated by the magnetic field and the magnetic field may, for example, be a time varying magnetic field. By individually moving the magnetic units of the individual cilia, the response of the individual cilia to the magnetic field may be changed.

According to an embodiment, the soft robot is configured as a soft gripper,
wherein the body forms a flexible finger of the soft gripper.

According to an embodiment, the outer part is configured as a flat sheet. The flat sheet may extend in a length direction and in a width direction, wherein the flat sheet has a thickness in a height direction that is smaller than a length of the flat sheet in the length direction and a width of the flat sheet in the width direction. The length direction may be parallel to the channel or parallel to the longitudinal direction and the height direction may be parallel to the outer direction. The outer part may be configured to bend in the height direction subject to the force exerted by the magnetic field on the magnetic unit.

According to an embodiment, the outer part comprises at least two walls arranged at an angle with respect to each other and adjoining each other at an edge of the outer part. The at least two walls each may be configured as a flat sheet. Each flat sheet may be configured as mentioned before.

According to an embodiment, the channel is located at the edge. For example, the channel may run along the edge.

According to an embodiment, the outer part is configured as a box having sidewalls adjoining each other at edges of the box, wherein each of the two walls forms one of the sidewalls.

According to an embodiment, the channel forms an edge channel located at one of the edges of the box, wherein the outer part comprises further edge channels located at each of the remaining ones of the edges of the box and wherein each of the edge channels has a respective magnetic unit movably arranged therein.

According to an embodiment, the outer part comprises a first sidewall and a second sidewall, the second sidewall being located opposite the first sidewall, wherein magnetic moments of magnetic units movably arranged within the edge channels of the first sidewall have a first common direction and magnetic moments of magnetic units movably arranged within the edge channels of the second sidewall have a second common direction, wherein the first common direction is opposite to the second common direction.

According to an embodiment, the outer part forms the target member and the inner part forms the magnetic member and the magnetic unit is arranged inside the channel. The channel forms a first channel and the magnetic unit forms a first magnetic unit. Furthermore, the outer part comprises a second channel and a second magnetic unit is movably arranged within the second channel. The second magnetic unit is configured to be placed within the magnetic field so that the magnetic field exerts a magnetic force on the second magnetic unit and the second magnetic unit is configured to move relative to the outer part to allow for a change of a magnetization profile of the outer part and for a change of a response of the outer part to the magnetic field. The second magnetic unit may be movable independently from the first magnetic unit.

The second magnetic unit may be formed by the further magnetic unit described above. Therefore, features and technical effects described for the further magnetic unit may also be implemented with the second magnetic unit and vice versa.

According to an embodiment, the channel and the second channel run parallel to each other.

According to an embodiment, the channel and the second channel are adjacent to each other.

According to an embodiment, a magnetic moment of the magnetic unit and a magnetic moment of the second magnetic unit are opposite with respect to each other.

According to an embodiment, the outer part is configured as the flat sheet and the channel and the second channel are located at the same edge of the flat sheet in the width direction.

According to an embodiment, the outer part is configured as the flat sheet and the channel and the second channel are located at opposite edges of the flat sheet in the width direction. This allows to deform the opposite edges of the flat sheet independently from each other and, for example, twist the flat sheet.

According to an embodiment, the outer part comprises a third channel and a third magnetic unit movably arranged within the third channel, wherein the third magnetic unit is configured to be placed within the magnetic field so that the magnetic field exerts a magnetic force on the third magnetic unit. The third magnetic unit is configured to move relative to the outer part to allow for a change of a magnetization profile of the outer part and for a change of a response of the outer part to the magnetic field. The third channel and the third magnetic unit may be configured as it is disclosed for the first magnetic unit and the first channel and vice versa. For example, the third magnetic unit may be formed by the further magnetic unit described above.

According to an embodiment, the third channel is located in between the channel and the second channel in the width direction of the flat sheet.

Thereby, the third channel may be located at a first distance from the channel in the width direction of the flat sheet and at a second distance from the second channel in the width direction of the flat sheet, wherein a ratio of the first distance to the second distance is at least 0.5 and at most 1.5.

According to an embodiment, the channel and the third channel are located adjacent to each other at the same edge of the flat sheet in the width direction. The second channel then may be located at an opposite edge of the flat sheet.

According to an embodiment, the outer part comprises a fourth channel and a fourth magnetic unit movably arranged within the fourth channel, wherein the fourth magnetic unit is configured to be placed within the magnetic field so that the magnetic field exerts a magnetic force on the fourth magnetic unit and wherein the fourth magnetic unit is configured to move relative to the outer part to allow for a change of a magnetization profile of the outer part and for a change of a response of the outer part to the magnetic field. The fourth channel and the fourth magnetic unit may be configured as it is disclosed for the first magnetic unit and the first channel and vice versa. For example, the fourth magnetic unit may be formed by the further magnetic unit described above.

According to an embodiment, the second channel and the fourth channel are located adjacent to each other at the same edge of the flat sheet in the width direction. The edge may form an edge of the flat sheet opposite to the edge at which the first channel and the third channel are located.

According to an embodiment, a direction of a magnetic moment of the magnetic unit and a direction of a magnetic moment of the third magnetic unit are opposite with respect to each other and a direction of a magnetic moment of the second magnetic unit and a direction of a magnetic moment of the fourth magnetic unit are opposite with respect to each other. Furthermore, the direction of the magnetic moment of the magnetic unit and the direction of the magnetic moment of the second magnetic unit are parallel to each other.

According to an embodiment, a magnitude of a magnetic moment of the magnetic unit and a magnitude of a magnetic moment of the third magnetic unit are equal and a magnitude of a magnetic moment of the second magnetic unit and a magnitude of a magnetic moment of the fourth magnetic unit are equal.

According to an embodiment, the magnitude of the magnetic moment of the magnetic unit and the magnitude of the magnetic moment of the second magnetic unit are equal.

According to an embodiment, the soft robot comprises an additional magnetic unit forming an additional first magnetic unit, an additional second magnetic unit, an additional third magnetic unit, and an additional fourth magnetic unit. The magnetic unit and the additional magnetic unit are serially connected to each other and spaced apart from each other, the second magnetic unit and the additional second magnetic unit are serially connected to each other and spaced apart from each other, the third magnetic unit and the additional third magnetic unit are serially connected to each other and are spaced apart from each other, and the fourth magnetic unit and the additional fourth magnetic unit are serially connected to each other and are spaced apart from each other.

According to an embodiment, a direction of a magnetic moment of the magnetic unit and a direction of a magnetic moment of the additional magnetic unit are opposite to each other.

According to an embodiment, a direction of a magnetic moment of the second magnetic unit and a direction of a magnetic moment of the additional second magnetic unit are opposite to each other.

According to an embodiment, a direction of a magnetic moment of the third magnetic unit and a direction of a magnetic moment of the additional third magnetic unit are opposite to each other.

According to an embodiment, a direction of a magnetic moment of the fourth magnetic unit and a direction of a magnetic moment of the additional fourth magnetic unit are opposite to each other.

According to an embodiment, a magnitude of a magnetic moment of the magnetic unit and a direction of a magnetic moment of the additional magnetic unit are equal.

According to an embodiment, a magnitude of a magnetic moment of the second magnetic unit and a direction of a magnetic moment of the additional second magnetic unit are equal.

According to an embodiment, wherein a magnitude of a magnetic moment of the third magnetic unit and a direction of a magnetic moment of the additional third magnetic unit are equal.

According to an embodiment, a magnitude of a magnetic moment of the fourth magnetic unit and a direction of a magnetic moment of the additional fourth magnetic unit are equal.

According to an embodiment, the body comprises a set of extra channels located in between the channel and the second channel, wherein, for each of the extra channels, a respective extra magnetic unit from a set of extra magnetic units is placed within the respective extra channel.

According to an embodiment, the extra channels are pairwise located adjacent to each other to form pairs of adjacent extra channels, wherein the pairs of adjacent extra channels are spaced apart from each other, from the channel, and from the second channel.

According to an embodiment, directions of magnetic moments of the respective extra magnetic units of each of the pairs of adjacent extra channels are opposite to each other.

According to an embodiment, magnitudes of magnetic moments of the respective extra magnetic units of each of the pairs of adjacent extra channels are equal.

The present disclosure is also directed at a system comprising the soft robot of one of the preceding embodiments and a magnetic field generator, wherein the magnetic field generator is configured to generate the magnetic field within an interaction zone, and wherein the outer part, the inner part, and the magnetic unit are configured to be placed within the magnetic field within the interaction zone. The interaction zone thereby forms a zone in which the magnetic unit interacts with the magnetic field during operation of the soft robot.

The magnetic field generator may comprise a field source or an assembly of several field source. Each field source may be configured to generate a magnetic field and the magnetic field of the individual field sources may superpose, for example in the magnetic interaction zone, to generate the magnetic field actuating the body of the soft robot. The field sources may, for example, be configured as permanent magnets or as magnetic field coils. For example, the magnetic field generator may be formed by the field source or the assembly of the field sources.

According to an embodiment, the magnetic field is a time-varying magnetic field. For example, a direction and/or magnitude of the magnetic field may be variable in time. For example, the direction of the time-varying magnetic field may rotate. With some of these embodiments, the magnitude of the magnetic field may remain constant.

According to an embodiment, the magnetic field generator comprises a field source and a source actuator, wherein the source actuator is configured to vary a distance of the field source from the interaction zone to modify the magnitude of the magnetic field within the interaction zone.

According to an embodiment, the magnetic field generator comprises a multitude of field sources and a source actuator. The field sources are provided in a source arrangement with respect to the interaction zone and the source actuator is configured to vary respective distances of the field sources to the interaction zone.

For example, the source arrangement may be a circular arrangement of the field sources around the interaction zone and the source actuator may be configured to vary a radius or a diameter of the circular arrangement.

According to an embodiment, the magnetic field is essentially homogeneous, such as homogeneous, within the interaction zone.

With the magnetic field being essentially homogeneous in the interaction zone, a variation of a magnitude of the magnetic field within the interaction zone may be at most 50%, at most 25%, at most 20%, at most 10%, at most 5%, at most 2.5% or at most 1%. Additionally or alternatively, a variation of the direction of the magnetic field within the interaction zone may be at most 50%, at most 25%, at most 20%, at most 10%, at most 5%, at most 2.5% or at most 1%.

According to an embodiment, the actuator and the magnetic field generator are configured to move the magnetic unit within the interaction zone with a magnitude of the magnetic field set to a reduced value, such as zero, and to subsequently increase the magnitude of the electric field while the magnetic unit is present in the interaction zone, such as after moving the magnetic unit within the interaction zone. This eliminates transient deformations of the target member due to movement of the magnetic unit within the interaction zone. As a consequence, prediction of the movement of the target member may be facilitated.

The present disclosure is also directed at an arrangement of a first soft robot according to the present disclosure and a second soft robot according to the present disclosure.

According to an embodiment, the first soft robot and the second soft robot are independently actuatable within the magnetic field to cooperate with each other.

The present disclosure is also directed at a method for operating the soft robot according to the present disclosure, the method comprising the steps of:
- generating the external magnetic field in an interaction zone by a magnetic field generator;
- placing the outer part, the inner part, and the magnetic unit within the magnetic field in the interaction zone so that the magnetic field exerts a magnetic force on the magnetic unit; and
- moving the magnetic member relative to the target member, for example by an actuator, to change the magnetization profile of the target member and to change the response of the target member to the magnetic field.

According to an embodiment, the method further comprises the steps of:
- setting a magnitude of the magnetic field to a reduced value, such as zero, during the moving of the magnetic member relative to the target member; and
- increasing the magnitude of the magnetic field while the magnetic unit is present in the magnetic field zone, for example after moving of the magnetic member relative to the target member.

According to an embodiment, the method further comprises the steps of:
- placing an outer part, an inner part, and a magnetic unit of a further soft robot according to the present disclosure within the magnetic field in the interaction zone so that the magnetic field exerts a magnetic force on the magnetic unit of the further robot; and
- actuating the soft robot and the further soft robot independently from each other to cooperate with each other within the magnetic field.

According to an embodiment, the soft robot comprises a further magnetic unit movably arranged with respect to target member and the further magnetic unit is movable independently from the magnetic unit with respect to the target member. The method then comprises the further steps of:
- placing the magnetic unit at a first location and placing the further magnetic unit at a second location, the first location being at a distance from the second location so that the force on the target member at the first location is given by the magnetic moment of the magnetic unit;
- placing the further magnetic unit next to the magnetic unit at the first location so that the force on the target member in the vicinity of the magnetic unit is given by a superposition of the magnetic moment of the magnetic unit and a magnetic moment of the further magnetic unit.

According to an embodiment, the method further comprises the step of rotating a manipulator of the magnetic member while moving it along the channel.

The present disclosure is also directed at a first further soft robot comprising a body. The body has an outer part, an inner part, and a magnetic unit, wherein the outer part is configured as an elongated cylindrical tube comprising a flexible channel and the inner part has a magnetic member comprising the magnetic unit and a flexible part attached to the magnetic unit. The inner part with the magnetic unit and the flexible part are movably arranged within the channel, wherein the flexible part forms a manipulator for moving the magnetic unit. The outer part, the inner part, and the magnetic unit are configured to be placed within a magnetic field so that the magnetic field exerts a magnetic force on the magnetic unit and the inner part and the outer part are configured to move relative to each other to allow for a change of a magnetization profile of the outer part and for a change of a response of the outer part to the magnetic field. Furthermore, the inner part with the magnetic unit is configured to remain longitudinally stationary with respect to a surrounding while the outer part translates longitudinally in a longitudinal direction of the channel with respect to the inner part and the magnetic unit.

The first further soft robot may be configured as it is described for the soft robot and vice versa. For example, the body of the first further soft robot may be configured as it is disclosed for the body of the soft robot and vice versa. With the first further soft robot, the outer part forms the target member.

The first further soft robot may be configured as a medical instrument, such as an endoscope for medical use. For example, the first further soft robot may be configured as a catheter.

According to an embodiment, movement of the magnetic unit is limited to an interior of the channel.

According to an embodiment, the inner part forming the magnetic member comprises a further magnetic unit, wherein the magnetic unit and the further magnetic unit are fixed to each other and the magnetic unit and the further magnetic unit are placed at a distance from each other.

According to an embodiment, a magnetic moment of the magnetic unit differs from a magnetic moment of the further magnetic unit.

According to an embodiment, the inner part forming the magnetic member comprises a third magnetic unit, wherein the third magnetic unit is longitudinally fixed with respect to the magnetic unit and the further magnetic unit at respective distances from the magnetic unit and the further magnetic unit.

The present disclosure is also directed at a second further soft robot configured as an artificial cilia array, the soft robot comprising a support and a multitude of bodies forming artificial cilia, wherein each artificial cilium comprises an outer part, an inner part, and a magnetic unit. For each artificial cilium, the outer part is configured as an elongated flexible tube comprising a flexible tubular channel, and, for each artificial cilium, the outer part extends in a longitudinal direction between a first end and a second end and the channel runs from the second end to the first end. For each artificial cilium, the second end is fixed to the support and the first end is configured as a free end, wherein, for each artificial cilium, the inner part is movably arranged within the channel. For each artificial cilium, the inner part forms a magnetic member comprising the magnetic unit and the outer part forms a flexible target member and the magnetic unit is arranged inside the channel. For each artificial cilium, the outer part, the inner part, and the magnetic unit are configured to be placed within a magnetic field so that the magnetic field exerts a magnetic force on the magnetic unit. Furthermore, for each artificial cilium, the magnetic unit is configured to move relative to the outer part to allow for a change of a magnetization profile of the outer part and for a change of a response of the outer part to the magnetic field. The magnetic units of the individual cilia thereby are configured to independently move with respect to each other.

The second further soft robot may be configured as it is described for the soft robot and vice versa. For example, the body of the second further soft robot may be configured as it is disclosed for the body of the soft robot and vice versa. With the second further soft robot, the outer part forms the target member.

According to an embodiment, for each artificial cilium, the magnetic unit is configured to be moved between a first position located near the first end and a second position located away from the first end, for example located at the second end.

According to an embodiment, for each artificial cilium, moving the magnetic unit from the first position to the second position changes an amplitude of the response of the outer part of the respective artificial cilium to the magnetic field.

According to an embodiment, for each artificial cilium, moving the magnetic unit to one of the first position and the second position reduces the amplitude of the response of the outer part of the respective artificial cilium to the magnetic field to essentially zero, such as zero.

For example, for each artificial cilium, moving the magnetic unit to one of the first position and the second position deactivates the response of the outer part of the respective artificial cilium to the magnetic field.

According to an embodiment, for each artificial cilium, moving the magnetic unit from the first position to the second position changes a direction of the response of the outer part of the respective artificial cilium to the magnetic field, for example, reverses the direction of the response of the outer part of the respective artificial cilium to the magnetic field.

According to an embodiment, for each artificial cilium, moving the magnetic unit from the first position to the second position changes a phase of the response of the outer part of the respective artificial cilium to a periodic variation of the magnetic field, for example by 180°.

According to an embodiment, each artificial cilium comprises a further magnetic unit, wherein, for each artificial cilium, the further magnetic unit is fixed to the flexible part, for example at the first end.

According to an embodiment, for each artificial cilium, the magnetic unit is configured to be placed next to the further magnetic unit.

According to an embodiment, for each artificial cilium, a direction of a magnetic moment of the further magnetic unit differs from a direction of a magnetic moment of the magnetic unit. For example, the direction of the magnetic moment of the further magnetic unit is opposite to the direction of the magnetic moment of the magnetic unit.

According to an embodiment, a magnitude of the magnetic moment of the further magnetic unit equals a magnitude of the magnetic moment of the magnetic unit.

According to an embodiment, a magnitude of the magnetic moment of the further magnetic unit is essentially twice or half a magnitude of the magnetic moment of the magnetic unit.

According to an embodiment, the artificial cilia are arranged in a regular pattern, such as a row or a two-dimensional matrix.

According to an embodiment, the artificial cilia are configured as an actuator for moving a target, such as a fluid or a solid object.

Exemplarily, the soft robot according to the present disclosure may also be configured as a soft gripper.

Therefore, the present disclosure is also directed at a third further soft robot configured as a soft gripper, comprising a body and a support, wherein the body comprises an outer part, an inner part, a magnetic unit and a counterpart. The outer part is flexible and forms a flexible finger of the soft gripper. Thereby, the outer part extends in a longitudinal direction between a first end and a second end and a flexible channel runs from the second end to the first end within the outer part. The second end is fixed to the support and the first end is configured as a free end. The inner part comprises the magnetic unit and is movably arranged within the flexible channel. Furthermore, the outer part, the inner part, and the magnetic unit are configured to be placed within a magnetic field so that the magnetic field exerts a magnetic force on the magnetic unit and the magnetic unit and the outer part are configured to move relative to each other to allow for a change of a magnetization profile of the outer part and for a change of a response of the outer part to the magnetic field. The magnetic unit is configured to be moved between a first position with respect to the outer part and a second position with respect to the outer part, wherein the first end of the outer part is placed from the counterpart at a first distance in a bending direction when the magnetic unit is at the first position and the first end of the outer part is placed from the counterpart at a second distance in the bending direction when the magnetic unit is at the second position. The second distance thereby is smaller than the first distance so that a gripper part comprising the flexible finger and the counterpart is in an open state when the magnetic unit is at the first position and in a closed state when the magnetic unit is at the second position.

The third further soft robot may be configured as it is described for the soft robot and vice versa. For example, the body of the third further soft robot may be configured as it is disclosed for the body of the soft robot and vice versa. With the third further soft robot, the outer part forms the target member.

For example, the magnetic unit and the outer part are configured to move relative to each other to allow for a change of a magnetization profile of the outer part and for a change of a response of the outer part to the common magnetic field.

With the third further soft robot, the outer part assumes a first shape within the magnetic field if the magnetic unit is at the first position and the outer part assumes a second shape within the magnetic field if the magnetic unit is at the second position, the first shape being different from the second shape. Thereby, the first end of the outer part is bent in a bending direction towards the counterpart when being in the first shape to close a gripper part comprising the flexible finger and the counterpart and the outer part is bent against the bending direction away from the counterpart when being in the second shape to open the gripper part comprising the flexible finger and the counterpart.

According to an embodiment of the third further soft robot, the outer part is configured as a flat sheet extending in the longitudinal direction and in a width direction, wherein the flat sheet has a thickness in the transverse direction that is smaller than a length of the flat sheet in the longitudinal direction and a width of the flat sheet in the width direction.

According to an embodiment of the third further soft robot, the outer part comprises a further channel, wherein a further magnetic unit is movably arranged within the further channel. The further magnetic unit is configured to be placed within the magnetic field so that the magnetic field exerts a magnetic force on the further magnetic unit and the further magnetic unit is configured to move relative to the outer part to allow for a change of a magnetization profile of the outer part and for a change of a response of the outer part to the magnetic field.

According to an embodiment of the third further soft robot, the channel and the further channel run parallel to each other.

According to an embodiment of the third further soft robot, a direction of a magnetic moment of the magnetic unit and a direction of a magnetic moment of the further magnetic unit are opposite with respect to each other.

According to an embodiment of the third further soft robot, a magnitude of the magnetic moment of the further magnetic unit equals a magnitude of the magnetic moment of the magnetic unit.

According to an embodiment of the third further soft robot, a magnitude of the magnetic moment of the further magnetic unit is essentially twice or half a magnitude of the magnetic moment of the magnetic unit, such as twice or half the magnitude of the magnetic moment of the magnetic unit.

According to an embodiment of the third further soft robot, the soft robot comprises an additional body having an additional outer part, an additional inner part, and an additional magnetic unit, wherein the additional outer part is flexible and forms a flexible additional finger of the soft gripper and wherein the additional outer part extends in the longitudinal direction between a first end and a second end of the additional outer part and a flexible additional channel runs from the second end to the first end within the additional outer part. The second end of the additional outer part is fixed to the support and the first end of the additional outer part is configured as a free end. The additional inner part comprises the additional magnetic unit and is movably arranged within the flexible additional channel. The additional outer part, the additional inner part, and the additional magnetic unit are configured to be placed within the magnetic field so that the magnetic field exerts a magnetic force on the additional magnetic unit and the additional magnetic unit and the additional outer part are configured to move relative to each other to allow for a change of a magnetization profile of the additional outer part and for a change of a response of the additional outer part to the magnetic field. The additional magnetic unit is configured to be moved between a first position with respect to the additional outer part and a second position with respect to the additional outer part,
wherein the additional outer part assumes a first shape within the magnetic field with the additional magnetic unit at the first position and the additional outer part assumes a second shape within the magnetic field with the additional magnetic unit at the second position, the first shape being different from the second shape. Furthermore, the outer part and the additional outer part are deformable independently from each other.

According to an embodiment of the third further soft robot, the magnetic unit and the additional magnetic unit are movable independently from each other.

According to an embodiment of the third further soft robot, the soft robot comprises an additional counterpart, wherein the first end of the additional outer part is placed from the additional counterpart at a first distance in a bending direction when the additional magnetic unit is at the first position and the first end of the additional outer part is placed from the additional counterpart at a second distance in the bending direction when the additional magnetic unit is at the second position. The second distance thereby is smaller than the first distance so that an additional gripper part comprising the flexible additional finger and the additional counterpart is in an open state when the additional magnetic unit is at the first position and in a closed state when the additional magnetic unit is at the second position.

According to an embodiment of the third further soft robot, the additional outer part forms the counterpart and the outer part forms the additional counterpart.

According to an embodiment of the third further soft robot, the outer part and the additional outer part are placed next to each other in a width direction.

### DRAWINGS

Exemplary embodiments and functions of the present disclosure are described herein in conjunction with the following drawings, showing schematically:
- Fig. 1: a sunflower with auxin transporters;
- Fig. 2: a system comprising a magnetic field generator and soft robot according to the present disclosure;
- Fig. 3: a first configuration of a body of the soft robot;
- Fig. 4: exemplary numerical values for the parameters of the first configuration shown in Fig. 3;
- Fig. 5: deformations of the first configuration of the body;
- Fig. 6: further deformations of the first configuration of the body;
- Fig. 7: a second configuration of the body of the soft robot according to the present disclosure;
- Fig. 8: exemplary numerical values for parameters of the second configuration of the body;
- Fig. 9: Fig. 9A: an outer part and an inner part of the second configuration of the body; Fig. 9B: the body of the second configuration; Fig. 9C: magnetic neutralization of a magnetic moment of the body of the second configuration; and Fig. 9D: magnetic reversal of a magnetic moment of the body of the second configuration;
- Fig. 10: a variant of the second configuration of the body;
- Fig. 11: Fig. 11A: deformations of the body of the second configuration; and Fig. 11B: changes of a shape of an outer part of the second configuration;
- Fig. 12: Fig. 12A: deformations of the body 10 of the second configuration with the magnetic unit 52 at the same position; Fig 12B: changes of a shape of an outer part 12 of the second configuration;
- Fig. 13: a third configuration of the body of the soft robot according to the present disclosure;
- Fig. 14: exemplary numerical values for parameters of the third configuration;
- Fig. 15: the third configuration with varying distances between a magnetic unit and a further magnetic unit and varying distances of the magnetic units from a free end of the body of the magnetic robot;
- Fig. 16: deformations of the outer part of the third configuration of the body;
- Fig. 17: a fourth configuration of the body of the soft robot according to the present disclosure;
- Fig. 18: exemplary numerical values for parameters of the fourth configuration;
- Fig. 19: an outer part of a body according to the fourth configuration in a magnetic field with varying positions of a magnetic unit and two further magnetic units;
- Fig. 20: a fifth configuration of the body of the soft robot according to the present disclosure;
- Fig. 21: exemplary numerical values for parameters of the fifth configuration;
- Fig. 22: shapes of an outer part of the fifth configuration of the body for parameters listed in Fig. 21;
- Fig. 23: a sixth configuration of the body of the soft robot according to the present disclosure;
- Fig. 24: exemplary numerical values for parameters of the sixth configuration;
- Fig. 25: five states of the sixth configuration of the body;
- Fig. 26: a seventh configuration of the body of the soft robot according to the present disclosure;
- Fig. 27: a method for fabricating magnetic elements of the seventh configuration of the body;
- Fig. 28: exemplary design parameters for an outer part of the seventh configuration of the body, an inner part of the seventh configuration of the body and a jig used for magnetization;
- Fig. 29: Fig. 29A: deformations of the seventh configuration of the body for varying distances between a magnetic element and a further magnetic element in; Fig. 29B: the seventh configuration of the body with the magnetic element and the further magnetic element placed adjacent to each other at varying magnetic fields shown; and Fig. 29C: a comparison of the seventh configuration of the body with the magnetic element and the further magnetic element placed adjacent to each other and a body comprising a non-magnetic inner part;
- Fig. 30: the soft robot according to the present disclosure with an outer part of the body configured as a flat sheet;
- Fig. 31: a schematic of a first configuration of the body having the outer part configured as a flat sheet;
- Fig. 32: a further schematic of the first configuration of the body having the outer part configured as a flat sheet;
- Fig. 33: exemplary values for parameters of the first configuration of the body having the outer part configured as a flat sheet;
- Fig. 34: a second configuration of the body having the outer part configured as a flat sheet;
- Fig. 35: exemplary values for parameters of the second configuration of the body having the outer part configured as a flat sheet;
- Fig. 36: a third configuration of the body having the outer part configured as a flat sheet;
- Fig. 37: exemplary values for parameters of the third configuration of the body having the outer part configured as a flat sheet;
- Fig. 38: a fourth configuration of the body having the outer part configured as a flat sheet;
- Fig. 39: exemplary values for parameters of the fourth configuration of the body having the outer part configured as a flat sheet;
- Fig. 40: a variant of the flat sheet of the third configuration;
- Fig. 41: a soft robot according to the present disclosure with an outer part configured as a three-dimensional body;
- Fig. 42: exemplary dimensions of the three-dimensional body shown in Fig. 41;
- Fig. 43: a three-dimensional view of a first configuration of magnetic units of the three-dimensional body shown in Fig. 41;
- Fig. 44: a bottom view of the first configuration of magnetic units of the three-dimensional body shown in Fig. 43;
- Fig. 45: a three-dimensional view of a second configuration of magnetic units of the three-dimensional body shown in Fig. 41;
- Fig. 46: a bottom view of the second configuration of magnetic units of the three-dimensional body shown in Fig. 45;
- Fig. 47: parameter values for the first and second configuration of the three-dimensional bodies shown in Fig. 43 to Fig. 46;
- Fig. 48: various deformations of the three-dimensional body shown in Fig. 41;
- Fig. 49: deformations of bodies of soft robots according to the present disclosure;
- Fig. 50: shape change characteristics of the first configuration of the body having the flat sheet shown in Fig. 31 and Fig. 32;
- Fig. 51: shape change characteristics of the second configuration of the body having the flat sheet shown in Fig. 34;
- Fig. 52: shape change characteristics of the third configuration of the body having the flat sheet shown in Fig. 36;
- Fig. 53: shape change characteristics of the fourth configuration of the body having the flat sheet shown in Fig. 38;
- Fig. 54: an actuator for actuating the soft robots according to the present disclosure;
- Fig. 55: Fig. 54A: a medical instrument traversing through avoidance tissues; Fig. 54B: deformation of a soft robot having a reference configuration of a control setup and of soft robots having bodies with shape retention capabilities according to the present disclosure;
- Fig. 56: Fig. 56A and Fig. 56B: embodiments of inner parts and outer parts of the body of the soft robot having shape retention capabilities; Fig. 56C: the reference configuration of the control setup;
- Fig. 57: Fig. 57A: a schematic segmentation of the soft robot having shape retention capabilities; Fig. 57B: images of an outer part of the soft robot having shape retention capabilities bypassing an avoidance object;
- Fig. 58: state changes in four segments of the body of the soft robot having shape retention capabilities upon bypassing the avoidance object;
- Fig. 59: a setup for demonstrating movement of the soft robot having shape retention capabilities;
- Fig. 60: dimensional parameters of the setup of Fig. 59;
- Fig. 61: a further setup for demonstrating movement of the soft robot having shape retention capabilities;
- Fig. 62: a magnetic field during the movement of the body in the further setup;
- Fig. 63: a vascular system;
- Fig. 64: a time dependence of a magnetic field for moving an outer part of the soft robot having shape retention capabilities into and out of a branch of the vascular system;
- Fig. 65: different positions of a target member formed by the outer part of the soft robot having shape retention capabilities upon moving into a first further branch of the vascular system;
- Fig. 66: different positions of a target member formed by the outer part of the soft robot having shape retention capabilities upon moving into a second further branch of the vascular system;
- Fig. 67: images of introduction of the soft robot according to the present disclosure into a vessel of a porcine heart;
- Fig. 68: Fig. 68A: two positions of an ultrasonic probe with respect to the vessel of the porcine heart; Fig. 68B: pictures of the soft robot within the blood vessel of the porcine heart taken by the ultrasonic probe;
- Fig. 69: an environment for traversing a single obstacle with the soft robot according to the present disclosure;
- Fig. 70: an environment for traversing two obstacles with the soft robot according to the present disclosure;
- Fig. 71: a design of a body of a soft robot according to the present disclosure adapted for 3D navigation;
- Fig. 72: a variation of a magnitude of a magnetic field during movement of the body shown in Fig. 71 in the environment for traversing two obstacles of Fig. 70;
- Fig. 73: a target member of the body shown in Fig. 71 in an end position within the environment having two obstacles shown in Fig. 70;
- Fig. 74: a soft robot according to the present disclosure configured as an artificial cilia array;
- Fig. 75: another version of a soft robot according to the present disclosure configured as an artificial cilia array;
- Fig. 76: an outer part and two possible versions of an inner part of a body forming an artificial cilium;
- Fig. 77: cilia activation in a single cilium;
- Fig. 78: photographs and schematic diagrams of an artificial cilia array according to the present disclosure;
- Fig. 79: examples of targeted activation of cilia;
- Fig. 80: a method for using targeted cilia activation to achieve path-directed propulsion;
- Fig. 81: an outer part and an inner part of a cilium together with exemplary dimensions of the outer part and the inner part;
- Fig. 82: various motion trajectories of a single cilium actuated by a rotating magnetic field;
- Fig. 83: images of a one-dimensional artificial cilia array with cilia exhibiting bending amplitude modulation in a common uniform magnetic field;
- Fig. 84: images of a three-dimensional artificial cilia array with cilia exhibiting bending amplitude modulation in a common uniform magnetic field;
- Fig. 85: Fig. 85A: a body of a cilium configured for phase modulation; Fig. 85B: two cilia configured to have variable motional phases in a common rotating magnetic field;
- Fig. 86: images of a 1x6 cilia array exhibiting phase modulations;
- Fig. 87: images of a 2×4 cilia array submerged in a fluid driving the fluid in one direction;
- Fig. 88: images of the 2×4 cilia array submerged in the fluid driving the fluid in two directions;
- Fig. 89: images of the 2×4 cilia array submerged in the fluid driving the fluid in a first direction in a first step and in a second direction in a second step;
- Fig. 90: a paddle for attachment to a tip of a cilium;
- Fig. 91: images and schematic diagrams of a cilia array combining targeted cilia activation and ciliary phase modulation;
- Fig. 92: a minimally invasive surgery scenario;
- Fig. 93: a first soft robot, a second soft robot and third soft robot configured as two types of integrated soft tubes;
- Fig. 94: exemplary parameter values of the first soft robot, the second soft robot and the third soft robot;
- Fig. 95: operation of the first soft robot, a second soft robot and third soft robot within a common uniform magnetic field;
- Fig. 96: a diagram illustrating steps of a method for operating the first soft robot, the second soft robot and the third soft robot;
- Fig. 97: a first environment for operation of the first soft robot, the second soft robot and the third soft robot;
- Fig. 98: a second environment for operation of the first soft robot, the second soft robot and the third soft robot;
- Fig. 99: a third environment for operation of the first soft robot, the second soft robot and the third soft robot;
- Fig. 100: images of operation of the first soft robot, the second soft robot and the third soft robot in the third environment;
- Fig. 101: a detailed top view of a soft robot according to the present disclosure configured as a soft gripper;
- Fig. 102: a schematic layout of the soft robot configured as the soft gripper;
- Fig. 103: a side view of the soft robot configured as the soft gripper;
- Fig. 104: images of various bending modes of the soft gripper;
- Fig. 105: Fig. 105A: images of grasping a T-shaped first object with the soft gripper; Fig. 105B: images of grasping an F-shaped second object with the soft gripper; Fig. 105C: images of grasping an n-shaped third object with the soft gripper;
- Fig. 106: a method for fabricating inner and outer parts of the soft robots according to the present disclosure as hollow elements, such as tubes;
- Fig. 107: a method for fabricating inner and outer parts of the soft robots according to the present disclosure as solid elements, such as rods;
- Fig. 108: a method for fabricating outer parts configured as two-dimensional flat sheets and three-dimensional boxes;
- Fig. 109: experimental platforms and a magnetic field generator according to the present disclosure;
- Fig. 110: schematic diagrams of quantification of soft tube deformation;
- Fig. 111: a model of a body of a soft robot according to the present disclosure,
- Fig. 112: a body of a soft robot according to the present disclosure with an actuator comprising a first part and a second part;
- Fig. 113: the body of a soft robot according to the present disclosure with a magnetic interaction zone, a feeding zone and an actuation zone;
- Fig. 114: the body of the soft robot according to the present disclosure with an overlapping region of an outer part and an inner part of the body of the soft robot;
- Fig. 115: Fig. 115A: a diagram of a resistive force for different radii of curvature of the body of the soft robot; Fig. 115B: a diagram of the resistive force for different number of bends of the body of the soft robot; Fig. 115C: a diagram of a resistive force for different inner part diameters of the body of the soft robot;
- Fig. 116: Fig. 116A: a diagram illustrating an effect of different materials on relative motion resistance between the outer part and the inner part of the body of the soft robot; Fig. 116B: a diagram illustrating an effect of different motion modes on relative motion resistance between the outer part and the inner part of the body of the soft robot. Fig. 116C: a diagram illustrating an effect of different lubricants according to the present disclosure on relative motion resistance between the outer part and the inner part of the body of the soft robot;
- Fig. 117: a force analysis of a tube forming a part of the body of the soft robot according to the present disclosure in the feeding zone;
- Fig. 118: an elastic modulus of different materials used for part of the body of the soft robot according to the present disclosure;
- Fig. 119: Fig. 119A: bending deformation of a tube formed by the outer part and the inner part in the actuation zone; Fig. 119B: a body having an outer part and an inner part comprising a first inner part section and a second inner part section;
- Fig. 120: a variant of the first configuration of the body shown in Fig. 3;
- Fig. 121: a variant of the body shown in Fig. 120;
- Fig. 122: different modes of long-range operation of the body of the soft robot according to the present disclosure;
- Fig. 123: an embodiment of the soft robot according to the present disclosure having a body with an outer part, an inner part, and a further inner part;
- Fig. 124: an inner part of the soft robot of Fig. 123 with a first magnetic element having a first magnetization profile and a second magnetic element having a second magnetization profile with exemplary parameter values;
- Fig. 125: Fig. 125A: a magnetic field generator according to the present disclosure configured as a a large-scale magnetic field generator; Fig. 125B: an arrangement of multiple magnetic field generators according to the present disclosure;
- Fig. 126: a schematic of long-range operation of a soft robot according to the present disclosure with a large magnetic interaction length;
- Fig. 127: stiffness variations in a body having an outer part and an inner part of a body according to the present disclosure;
- Fig. 128: a body of a soft robot according to the present disclosure configured to operate ends of individual parts in distinct working zones;
- Fig. 129: the body of the soft robot of Fig. 128 with the working zones comprising individual magnetic fields;
- Fig. 130: a comparison of theoretical and experimental values for deformation of soft tubes in 1D magnetization reprogramming;
- Fig. 131: a comparison of experimental and simulation results of the second configuration for 2D magnetization reprogramming;
- Fig. 132: an experimental setup for analysis of deformation of bodies of the soft robot;
- Fig. 133: an impact of magnetic field strength on the deformation of soft tubes forming the body of the soft robot;
- Fig. 134: an impact of magnetic unit distance on the deformation of the soft tubes forming the body of the soft robot;
- Fig. 135: an impact of elastic modulus on the deformation of the soft tubes forming the body of the soft robot;
- Fig. 136: an elastic modulus of different materials;
- Fig. 137: a diagram for error analysis of magnetic unit position control;
- Fig. 138: an analysis diagram of a body of a soft robot formed as a catheter navigating through blood vessels;
- Fig. 139: an experimental setup assessing an impact of a radius of curvature on operation of an inner part of the bod of the soft robot;
- Fig. 140: an effect of various parameters on the relative motion resistance involved in manipulating parts of the body of the soft robot;
- Fig. 141: mixing ratios and corresponding magnetization magnitudes of magnetic elements of the soft robot according to the present disclosure;
- Fig. 142: a force-torque space generated by different devices and methods including the devices and methods according to the present disclosure;
- Fig. 143: a template-based magnetization profile design method;
- Fig. 144: a magnetization and an analysis of elongated parts of the soft robot having a helical magnetisation profile;
- Fig. 145: an influence of a diameter of a magnet distribution circle of a magnet arrangement on magnetic field intensity in different regions of an environment of the soft robot;
- Fig. 146: an impact of a magnetic field angle on a magnetic field in different regions of the environment of the soft robot;
- Fig. 147: an impact of a spacing of two magnetic field generators on a magnetic field in different regions of the environment of the soft robot;
- Fig. 148: an impact of a magnetic field plane angle on a magnetic field in different regions of the environment of the soft robot.

### DETAILED DESCRIPTION

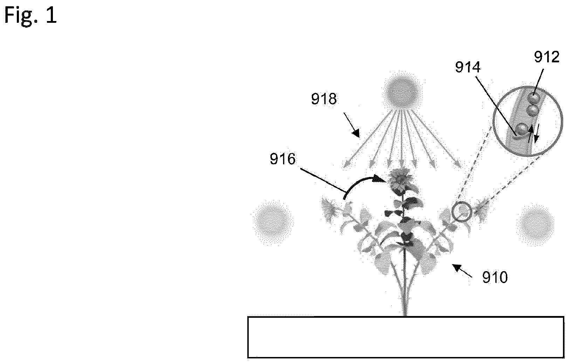

In connection with the present disclosure, it has been observed that sunflowers 910 in nature achieve adaptive deformation 916 by redistributing internal auxin 912 in response to changes in solar position and sunlight 816, see Fig.1. Auxin transporters 914 actively reposition auxin 912, altering its spatial distribution, thus inducing variations in local growth rates and resulting in differential deformation. Inspired by this biological phenomenon, the present disclosure describes a real-time in-situ magnetization reprogramming approach that achieves deformation by redistributing internal magnetic units through the movement of magnetic unit carriers, which are also called magnetic members in the present disclosure. Specifically, changes in the positions of magnetic unit carriers relocate magnetic units, altering their spatial distribution (manifesting as distinct magnetization profiles), thus inducing variations in forces and torques experienced under magnetic fields and resulting in differential deformation. Both the sunflower 910 and the method according to the present disclosure achieve differential deformation through redistribution of their respective internal elements-auxin 912 in the sunflower 910 and magnetic units in the method according to the present disclosure. The difference is that auxin transporters 914 in sunflowers 910 are driven internally through active biological mechanisms, whereas magnetic unit carriers in the method according to the present disclosure are externally controlled by various actuators or stimuli capable of inducing different modes of motion. This approach enables real-time, in-situ reprogramming of the overall magnetization profile, capable of not just one-dimensional (1D) reprogramming for elongated objects, such as tubular objects, but also extending to two- and three-dimensional (2D, 3D) reprogramming on planar and spatial objects.

Additionally, it has been discovered that the method and devices according to the present disclosure exhibit two unique characteristics (i.e., magnetic neutralization and magnetic reversal) that facilitate specific operations. Applications according to the present disclosure include contact-free object navigation, reprogrammable cilia arrays, coordinated multi-instrument operation, and shape-adaptable soft grippers, which involve operations that existing methods cannot achieve. The devices and methods according to the present disclosure achieve unprecedented modes and varieties of deformations, thereby broadening the application scope of magnetic actuation technologies. Besides, by allowing magnetic soft robots to adjust their own magnetization profiles in situ and in real-time, the method according to the present disclosure frees them from total reliance on changing external magnetic fields for deformation - especially for complex deformations - thus reducing the need for complex magnetic field generation systems. This approach opens new pathways for the advancement of magnetic actuation technologies.

In the following, several embodiments of a soft robot 1 according to the present disclosure are described in connection with various Figures. Unless indicated otherwise, each scale bar 900 is in the Figures has a length of 5 mm.

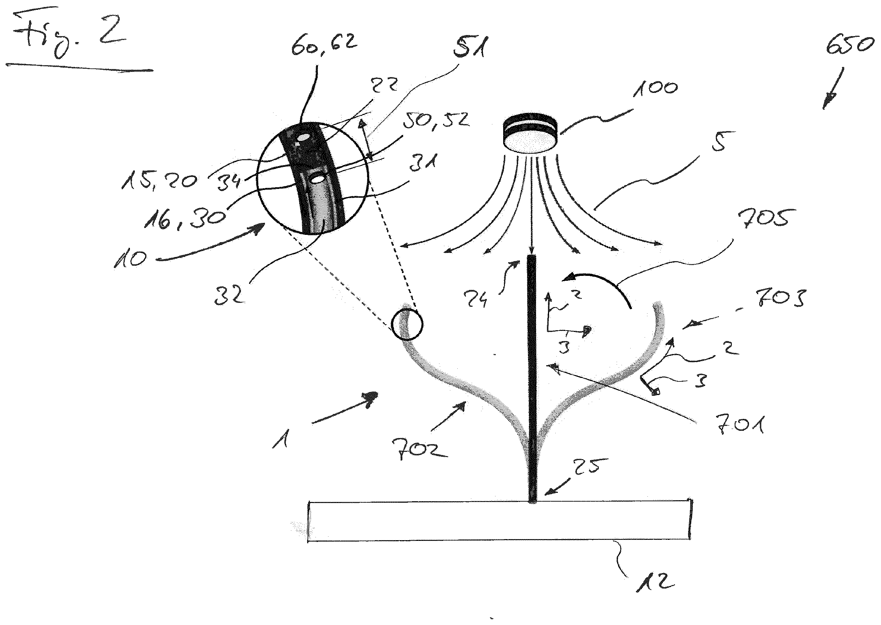

Fig. 2 depicts a system 650 comprising a magnetic field generator 100 and soft robot 1 according to the present disclosure. The soft robot 1 comprises a body 10 having an outer part 20, an inner part 30 and a support 12. The outer part 20 extends in a longitudinal direction 2 from a first end 24 to a second end 25, whereby the first end 24 is configured as a free end that is free to move in a bending direction 3 perpendicular to the longitudinal direction 2 and the second end 25 is fixed to the support 12. The longitudinal direction 2 and the bending direction 3 are thereby defined relative to the outer part 20 so that the longitudinal direction 2 follows the shape of the outer part 20 and the bending direction 3 is perpendicular to the longitudinal direction 2 at every location along the outer part 20.

With other embodiments, also the second end 25 may be configured as a free end.

The outer part 20 is configured as a flexible member that is configured to bend perpendicular to the longitudinal direction 2 in the bending direction 3. With the present embodiment, the outer part 20 is configured as an elongated cylindrical member, such as a tubular member. With other embodiments, the outer part 20 may also have other shapes. For example, the outer part 20 may be configured as a flat sheet or as a three-dimensional box.

The outer part 20 comprises an internal channel 22, which is exemplarily configured as a tubular channel. With other embodiments, the channel 22 may also be configured as an open channel. The inner part 30 is located within the channel 22. With the present embodiment, the inner part 30 is movably arranged within the channel 22. The inner part 30 comprises a magnetic unit 52, which itself is movably arranged within the channel 22. The magnetic unit 52 is configured to be actuated through the channel 22 so that it moves back and forth along the longitudinal direction 2 within the channel 22. With the present embodiment, the inner part 30 comprises a flexible part 31 that is fixedly connected to the magnetic unit 52. The inner part 30 is configured to actuate movement of the magnetic unit 52 within the channel 22. Exemplarily, the flexible part 31 is connected to an actuator (not shown) that is configured to move the magnetic unit 52 within the channel 22, for example in the longitudinal direction 2.

With the embodiment shown in Fig. 2, the magnetic unit 52 is located at an end of the outer part 30, namely at a first end 34 pointing towards the free first end 24 of the outer part 20. The first end 34 is configured as a free end of the inner part 30. With other embodiments, the magnetic unit 52 may also be located away from the first end 34 of the inner part 30.

The inner part 30 comprises a channel 32 that runs along the longitudinal direction 2. With the embodiment shown, the channel 32 extends through the flexible part 31. Although not explicitly depicted in Fig. 2, the channel 32 may also extend through the magnetic unit 52. In particular, the magnetic unit 52 may be configured as a hollow member, such as a hollow cylindrical member.

The body 10 of the soft robot 1 further comprises a further magnetic unit 62. The further magnetic unit 62 is part of a further magnetic element 60. The further magnetic unit 62 is configured for being placed at a distance 51 from the magnetic unit 52. With the present embodiment, the further magnetic unit 62 is configured for being placed at the distance 51 by moving the magnetic unit 52 away from the further magnetic unit 62. Furthermore, the magnetic unit 52 and the further magnetic unit 62 are configured for being placed adjacent to each other, exemplarily by moving the magnetic unit 52 next to the further magnetic unit 62.

With the present embodiment, the further magnetic unit 62 is arranged within the channel 22, namely radially within the channel 22. With other embodiments, the further magnetic unit 62 may also be arranged radially outside the channel 22. For example, the further magnetic unit 62 may circumferentially surround the channel 22. Furthermore, the further magnetic unit 62 may be arranged adjacent to the channel 22 in a width direction perpendicular to the channel 22, for example, if the outer part 20 is configured as a flat sheet. The further magnetic unit 62 may be arranged adjacent to the channel 22 in the width direction and be placed in a further channel that is located next to the channel 22. The further channel may, for example, run parallel to the channel 22.

The further magnetic unit 62 is fixed to the outer part 20. Exemplarily, it is located at the first end 24 of the outer part 20.

The magnetic field generator 100 is configured to generate a magnetic field 5 and the outer part 20, the inner part 30 and the magnetic unit 52 are configured to be placed within the magnetic field 5. With the exemplary embodiment shown in Fig. 2, also the further magnetic unit 62 is configured to be placed within the magnetic field 5.

With the soft robot 1, the outer part 20 forms a target member 15 and the inner part 30 forms a magnetic member 16. The magnetic member 16 comprises the magnetic unit 52 and is movably arranged with respect to the target member 15. By moving the magnetic member 16 with respect to the target member 15, a shape of the target member 15 changes within the magnetic field 5. Movement of the magnetic member 16 induces a deformation 705 of the target member 15.

Exemplarily, the target member 15 is configured to have a first shape 701 when the magnetic member 16 with the magnetic unit 52 is located at a first position with respect to the target member 15 and to have a second shape 702 when the magnetic member 16 with the magnetic unit 52 is located at a second position with respect to the target member 15.

With the present embodiment, the target member 15 is essentially straight in the first shape 701 and the target member 15 is bent perpendicular to the channel 22 and perpendicular to the longitudinal direction 2 in the second shape 702.

Furthermore, the target member 15 is configured to have a third shape 703. In the third shape 703, the target member 15 is also bent perpendicular to the channel 22 and perpendicular to the longitudinal direction 2. Thereby, the target member 15 is bent in a first direction in the second shape 702 and in a second direction in the third shape 703, whereby the first direction is perpendicular to the second direction. With other embodiments of the present disclosure, the first shape 701, the second shape 702 and the third shape 703 may be configured as various other shapes.

In the following, various configurations of the inner part 20 and the outer part 30 are described.

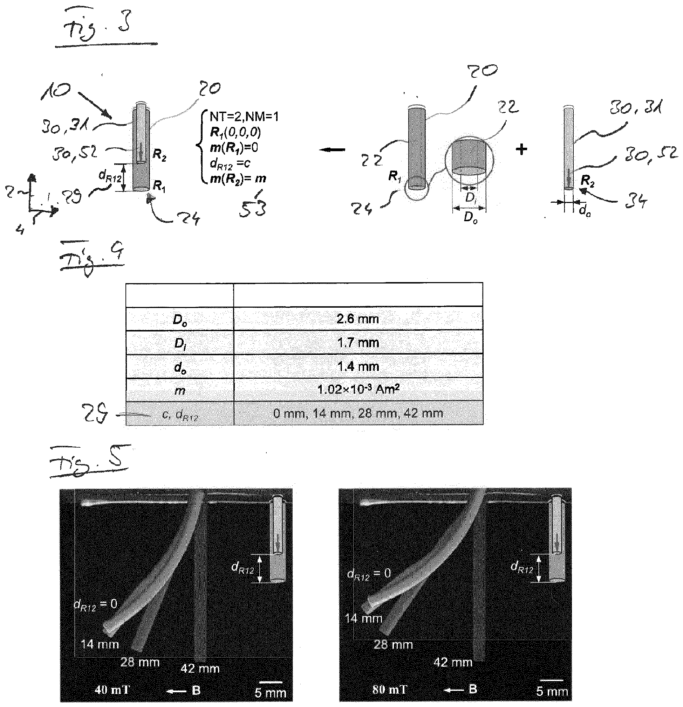

Fig. 3 depicts a first configuration of the body 10 of the soft robot 1 with the inner part 20 and the outer part 30. The assembled first configuration thereby is depicted to the left in Fig. 3 and the individual components of the first configuration are shown to the right in Fig. 3. As far as no differences are disclosed in the description and the Figures, the first configuration is configured as it is disclosed in connection with the configuration of Fig. 2 and vice versa.

With the first configuration, the inner part 30 comprises the flexible part 31 and the magnetic unit 52, whereby the magnetic unit 52 is located at the first end 34 of the flexible part 31. Exemplarily, the inner part 30 is configured as a solid cylinder, such as a rod. With other embodiments, the inner part 30 may also be configured as a hollow member, such as a tubular member, as it is disclosed in connection with Fig. 2. The inner part 30 has an outer diameter dₒ. Furthermore, the magnetic unit 52 has a magnetic moment 53, also denoted as **m.** With the present embodiment, a direction of the magnetic moment 53 is orientated parallel to the longitudinal direction 2. The direction of the magnetic moment 53 thereby points to the first end 34 of the inner part 30. With other embodiments, the direction of the magnetic moment 53 may also be orientated in an opposite direction. Alternatively, it may also be tilted with respect to the longitudinal direction 2.

The outer part 20 lacks a magnetic unit attached thereto, such as the further magnetic unit 65 shown in Fig. 2.

The outer part 20 is configured as a tubular member having an inner diameter Dᵢ and an outer diameter Dₒ, whereby the inner diameter Dᵢ defines the diameter of the channel 22.

The inner part 30 is movably arranged within the outer part 20, namely within the channel 22 of the outer part 20. Thereby, the magnetic unit 52 is configured to be moved along the longitudinal direction 2 between a first position located at the first end 24 of the outer part 20 and a second position located at a distance 29 away from the first end 24 of the outer part 20. The distance 29 equals a distance d_{R12} between the respective free first ends 24, 34 of the outer part 20 and the inner part 30.

With the first configuration, the target member 15 formed by the outer part 20 has a first magnetization profile when the magnetic unit 52 is located at the first position and a second magnetization profile when the magnetic unit 52 is located at the second position. Moving the magnetic member 16 formed by the inner part 30 and comprising the magnetic unit 52 with respect to the target member 15 results in a change of the magnetization profile of the target member 15.

Fig. 4 lists exemplary numerical values for the parameters of the first configuration shown in Fig. 3.

Fig. 5 shows deformations of the first configuration of the body 10 with the magnetic unit 52 placed at various distances 29 of d_{R12} = 0.14 mm, 28 mm, 42 mm under uniform magnetic fields 5 of 40 mT and 80 mT. Deformations under the same magnetic field 5 are consolidated into a single frame.

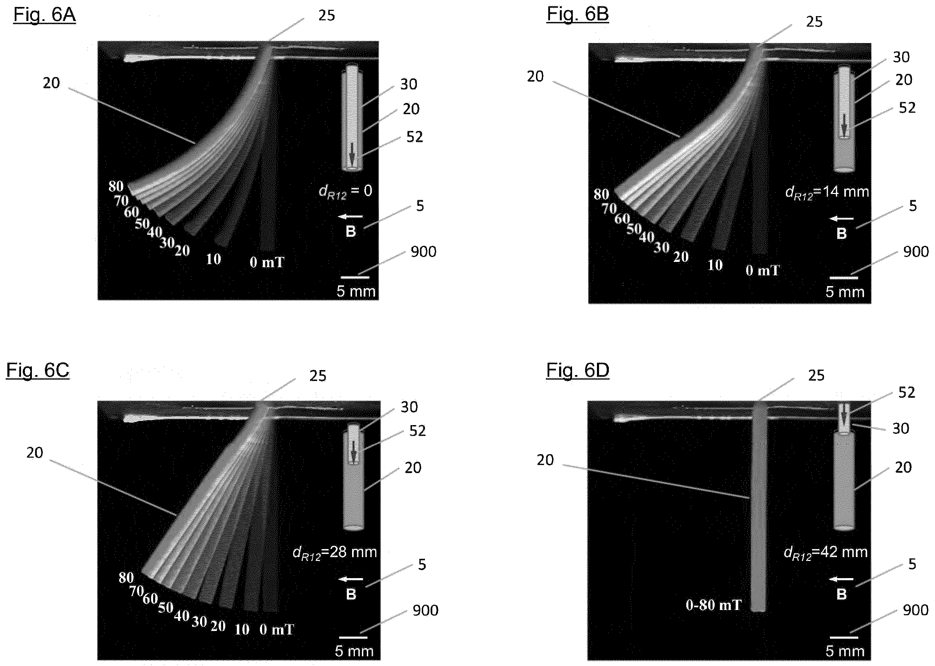

Figs. 6A to 6D show deformations of the first configuration of the body 10 with same positions of the magnetic unit 52 consolidated into a single Figure, with each Figure depicting the magnetic field 5 incrementally increasing from 0 mT to 80 mT in 10 mT increments. These Figures illustrate the various deformations 705 with different positions of the magnetic unit 62 under various magnetic field strengths. In Fig. 6A, the distance 29 is *d_{R12}* = 0, in Fig. 6B *d_{R12}* = 14 mm, in Fig. 6C *d_{R12}* = 28 mm and in Fig. 6D *d_{R12} =* 42 mm. In Fig. 6D, the body 10 and the outer part 20 do not undergo deformation in response to changes in the magnetic field 5, as the magnetic unit 52 is located outside the outer part 20. This characteristic allows for selective control over whether the outer part 20 is influenced by the magnetic field 5 or not.

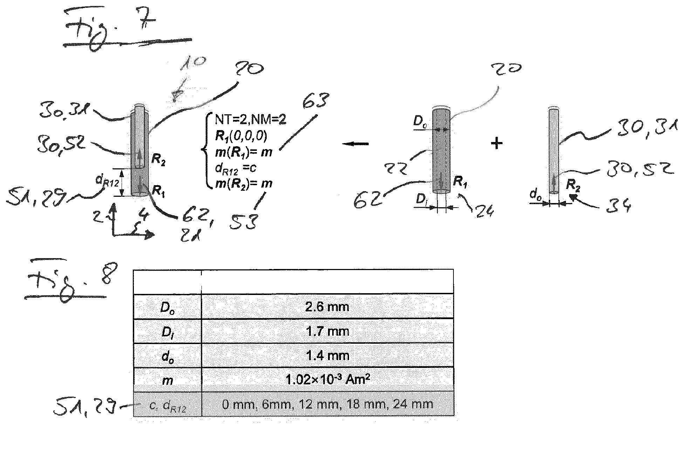

Fig. 7 depicts a second configuration of the body 10 of the soft robot 1 with the inner part 20 and the outer part 30. The assembled second configuration is depicted to the left in Fig. 7 and the individual parts of the second configuration are shown to the right in Fig. 7. As far as no differences are disclosed in the description and the Figures, the second configuration is configured as it is disclosed in connection with the first configuration and/or the configuration of Fig. 2 and vice versa. In particular, the second configuration corresponds to the configuration of the inner part 20 and the outer part 30 shown in Fig. 2.

Compared to the first configuration, the second configuration has the further magnetic unit 62 attached to the first end 24 of the outer part 20. The distance 51 between the magnetic unit 52 and the further magnetic unit 62 then corresponds to the distance 29 between the first end 24 of the outer part 20 and the first end 34 of the inner part 30.

A magnetic moment 63 of the further magnetic unit 62 has a direction that is opposite to the direction of the magnetic moment 53 of the magnetic unit 52. Furthermore, a magnitude of the magnetic moment 63 of the further magnetic unit 62 essentially equals, such as equals, a magnitude of the magnetic moment 53 of the magnetic unit 52.

Fig. 8 lists exemplary numerical values for the parameters of the second configuration shown in Fig. 7.

The second configuration is an example of a configuration, in which the magnetic member 16 and the target member 15 are configured to switch a response of the target member 15 to the magnetic field 5 on or off depending on the relative position between the magnetic member 16 and the target member 15. Movement of the magnetic member 16 and the target member 15 relative to each other thereby leads to a change of the magnetization profile of the target member 15 between a first magnetization profile, in which the magnetic moment of the target member 15 is essentially zero, such as zero, and a second magnetization profile, in which the target member 15 has a non-zero magnetic moment.

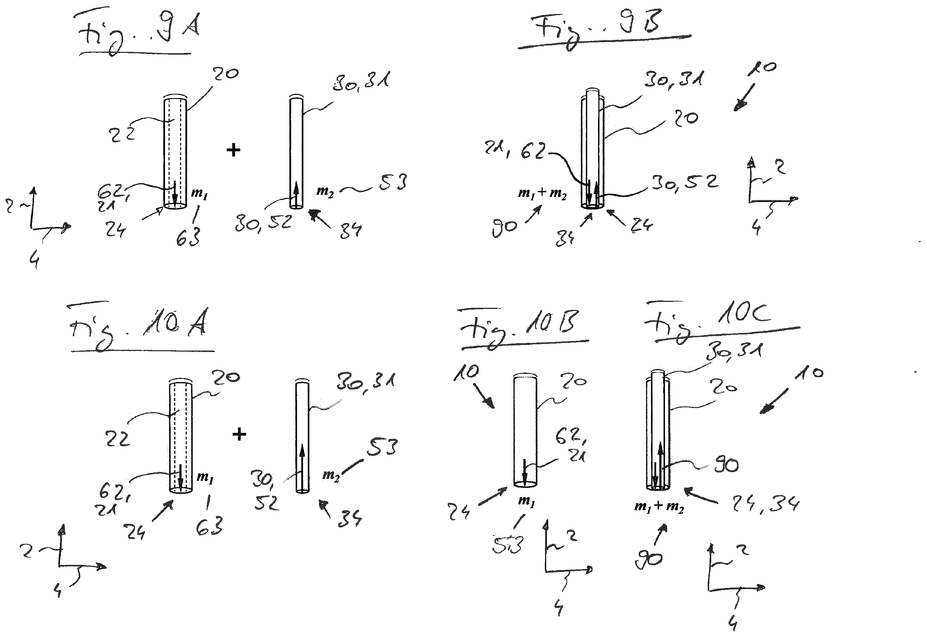

With the second configuration, a response of the target member 15 formed by the outer part 20 to the magnetic field 5 may be switched on and off depending on the position of the magnetic unit 52 and the magnetic member 16 comprising the magnetic unit 52 with respect to the outer part 20. Fig. 9A depicts the outer part 20 and the inner part 30, whereby the outer part 20 has the further magnetic unit 62 with the magnetic moment 63 located at its first end 24 and the inner part 30 has the magnetic unit 52 with the magnetic moment 53 located at its first end 34. The magnetic moment 53 of the magnetic unit 52 equals the magnetic moment 63 of the further magnetic unit 62.

Upon placing the magnetic unit 52 at a first position adjacent to the further magnetic unit 62, the response of the target member 15 and of the further magnetic unit 62 to the magnetic field 5 is switched off, as shown in Fig. 9B. For the resulting first magnetization profile, a resulting magnetic moment of the target member 15 is given by a superposition of the magnetic moment 53 of the magnetic unit 52 and the magnetic moment 63 of the further magnetic unit 62. The magnetic moment 53 of the magnetic unit 52 thereby compensates for the magnetic moment 63 of the further magnetic unit 62. This may be termed magnetic neutralization.

By placing the magnetic unit 52 at a second position away from the further magnetic unit 62, the response of the target member 15 and of the further magnetic unit 62 to the magnetic field 5 is switched on.

Fig. 10A depicts a variant of the second configuration, in which the change of the magnetization profile of the target member 15 reverses a direction of a magnetic moment of the target member 15 depending on the relative position between the magnetic member 16 and the target member 15.

Movement of the magnetic member 16 and the target member 15 relative to each other thereby leads to a change of the magnetization profile of the target member 15 between a first magnetization profile, in which the magnetic moment of the target member 15 has a first direction, and a second magnetization profile, in which the magnetic moment of the target member 15 has a second direction opposite to the first direction. Thereby, a magnitude of the magnetic moment of the target member 15 is essentially the same, such as the same, with both the first and second magnetization profiles.

As shown in Fig. 10A, the magnetic unit 52 has a magnetic moment 53 that is opposite to a magnetic moment 63 of the further magnetic unit 62 and a magnitude of the magnetic moment 53 of the magnetic unit 52 is twice a magnitude of the magnetic moment 63 of the further magnetic unit 62.

The magnetic unit 52 is configured to be moved between a first position, in which it is located adjacent to the further magnetic unit 62 and a second position, in which it is located away from the further magnetic unit 62. Fig. 10B shows the body 10 with the magnetic member 16 and the target member 15 in a relative position, in which the magnetic unit 52 is located at the second position away from the further magnetic unit 62. The second magnetization profile of the target member 15 is given by the magnetic moment 63 of the further magnetic unit 62 at the location of the further magnetic unit 62. Fig. 10C shows the body 10 with the magnetic member 16 of the target member 15 in a relative position, in which the magnetic unit 52 is located at the first position adjacent to the further magnetic unit 62. The first magnetization profile of the target member 15 is given by a magnetic moment at the location of the further magnetic unit 62, whereby the magnetic moment has a direction that is opposite to the direction of the magnetic moment 63 of the further magnetic unit 62 and has essentially the same magnitude, such as the same magnitude, as the magnetic moment 63 of the further magnetic unit 62. This may be termed magnetic reversal.

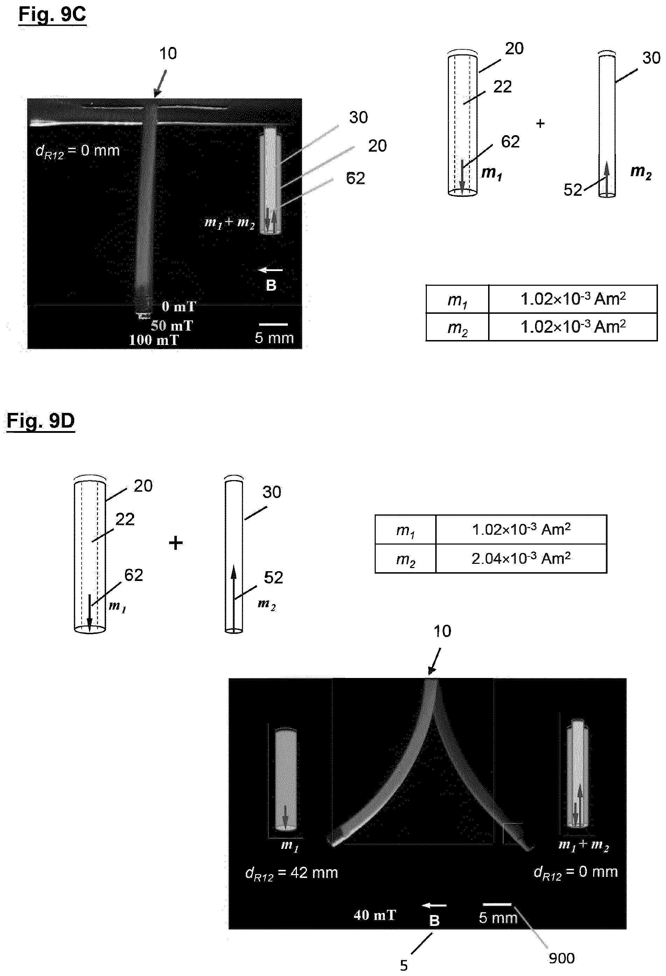

Fig. 9C again depicts magnetic neutralization. The body 10 formed as an integrated tube comprises the outer part 20 and the inner part 30 and has two magnetic units 52, 62 with equal magnetization magnitudes of the magnetic moments 53, 63 but opposite directions of the magnetic moments 53, 63. The outer part 20 maintains nearly the same position and shape under varying magnetic fields 5, which incrementally increase from 0 mT to 100 mT in 50 mT steps. This behavior demonstrates magnetic neutralization.

Fig. 9D again depicts magnetic reversal. The body 10 formed as an integrated tube comprises the outer part 20 and the inner part 30 and has two magnetic units 52, 62 with the further unit 62 having a magnitude of the magnetic moment 63 that is twice the magnitude of the magnetic moment 53 of the magnetic unit 52, both magnetic moments 53, 63 oriented in opposite directions. This results in a total reversal in deformation by adjusting the positions of the magnetic units when subjected to a uniform magnetic field 5 of 40 mT.

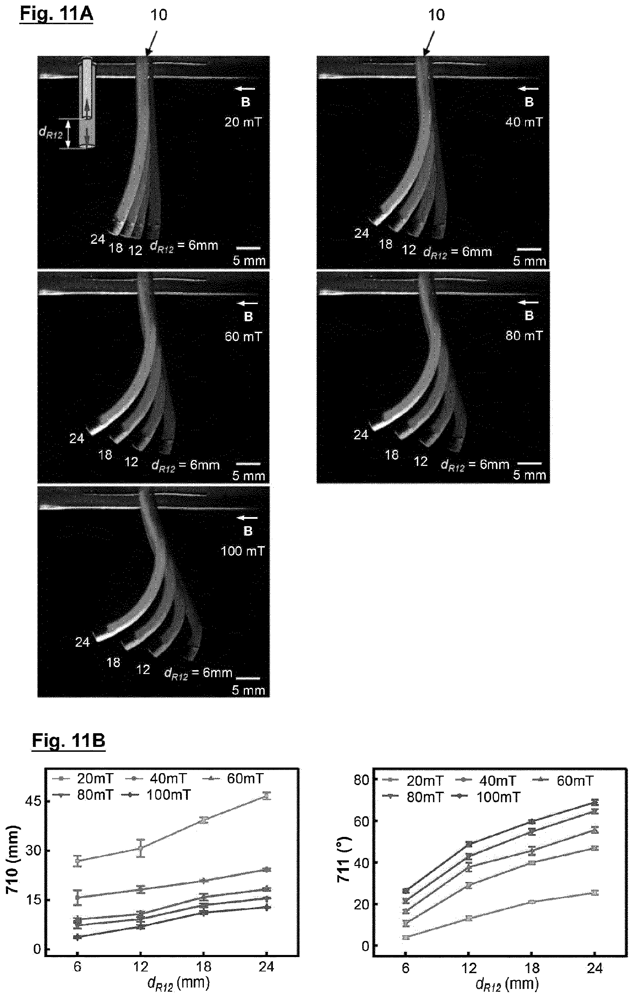

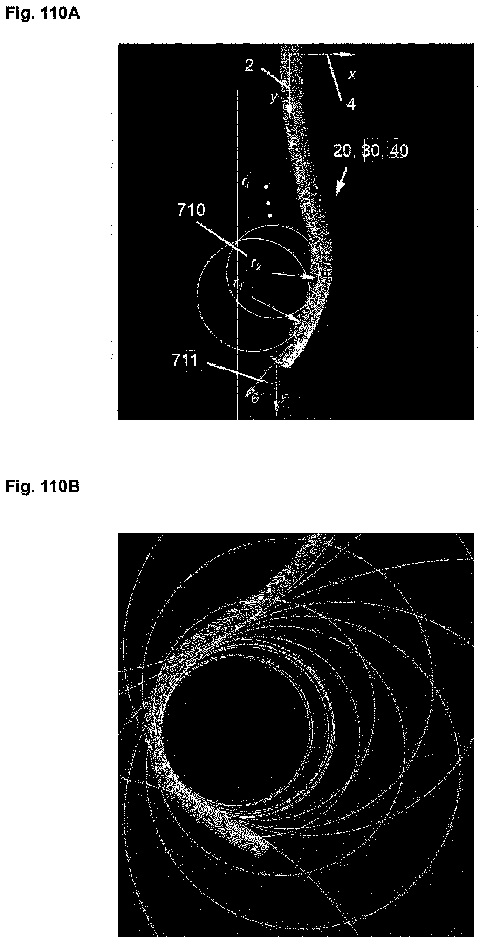

Figs. 11A shows deformations of the body 10 of the variant of the second configuration shown in Figures 7, 9A, and 9B with the magnetic unit 52 at various distances 29, 51 of *d_{R12} =* 6 mm, 12 mm, 18 mm, 24 mm, consolidated into single frames. Each frame depicts deformations under the same uniform magnetic field 5, and the magnetic field strength in these five figures incrementally increases from 20 mT to 100 mT in 20 mT increments. Fig. 11B depicts changes of the shape of the body 10, characterized by two representative descriptors (radius of curvature 710 and terminal bending angle 711, see Fig. 110).

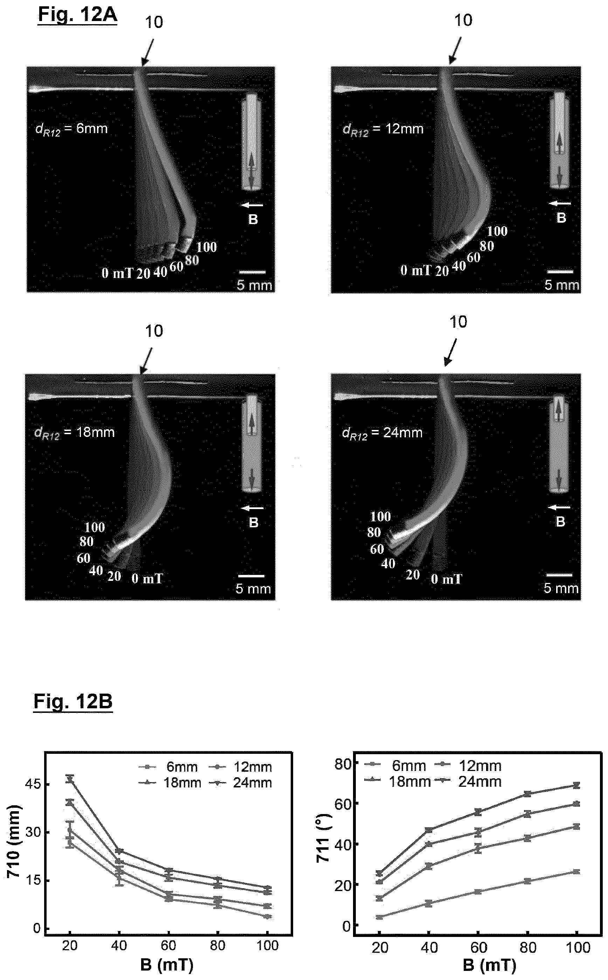

In Fig. 12A, deformations of the body 10 with the magnetic unit 52 at the same position are consolidated into a single frame. Each frame depicts the deformation with the magnetic field 5 incrementally increasing from 0 mT to 100 mT in 20 mT increments. The positions of the magnetic unit 52 have distances 29, 51 of 6 mm, 12 mm, 18 mm, and 24 mm, respectively. In Fig. 12B, changes of the shape of the outer part 12 are characterized by two representative descriptors (radius of curvature 710 and terminal bending angle 711, see Fig. 110), and the magnitude of the magnetic field 5 serves as the independent variable on the *x*-axis.

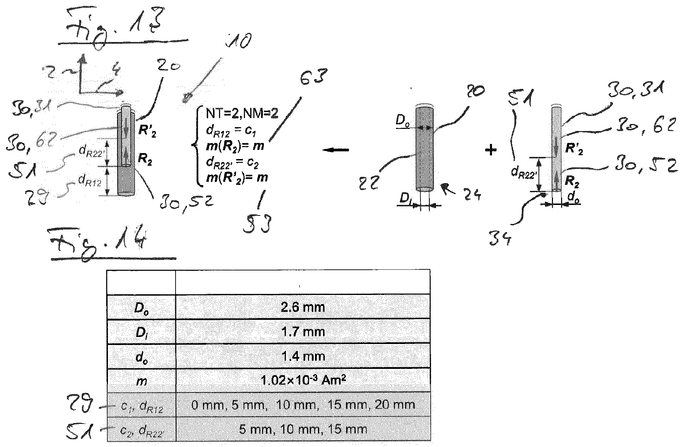

Fig. 13 depicts a third configuration of the body 10 of the soft robot 1 with the inner part 20 and the outer part 30. The assembled third configuration is depicted to the left in Fig. 13 and the individual components of the third configuration are shown to the right in Fig. 13. As far as no differences are disclosed in the description and the Figures, the third configuration is configured as it is disclosed in connection with the first configuration and/or the second configuration and/or the configuration of Fig. 2 and vice versa.

With the third configuration, the inner part 30 forming the magnetic member 16 comprises the further magnetic unit 62. The further magnetic unit 62 is configured to be placed at the distance 51 from the magnetic unit 52 by being fixed with respect to the magnetic unit 52 at the distance 51 away from the magnetic unit 52. With the third configuration, the further magnetic unit 62 is exemplarily fixed to the flexible part 31 of the inner part 30 and placed at the distance 51 away from the magnetic unit 52. The magnetic unit 52 is located at the first end 34 of the inner part 30 and the further magnetic unit 62 is spaced apart from the first end 34 by the distance 51.

Both the magnetization 53 of the magnetic unit 52 and the magnetization 63 of the further magnetic unit 62 have a direction that is parallel to the longitudinal direction 2, whereby the direction of the magnetization 53 of the magnetic unit 52 is opposite to the direction of the magnetization 63 of the further magnetic unit 62. The inner part 30 may be placed in the magnetic field 5 with a direction of the magnetic field 5 being perpendicular to the longitudinal direction 2. Sections of the inner part 30, at which the magnetic unit 52 and the further magnetic unit 62 are located, then become bended in the bending direction 3 due to the magnetic unit 52 and the further magnetic unit 62 deflecting to align with the direction of the magnetic field 5.

The magnetic unit 52 and the further magnetic unit 62 are configured to bend in opposite directions within the common magnetic field 5. Therefore, a portion of the flexible part 31 in between the magnetic unit 52 and the further magnetic unit 62 is deformed into a smooth arc.

Fig. 14 lists exemplary numerical values for the parameters of the third configuration shown in Fig. 13.

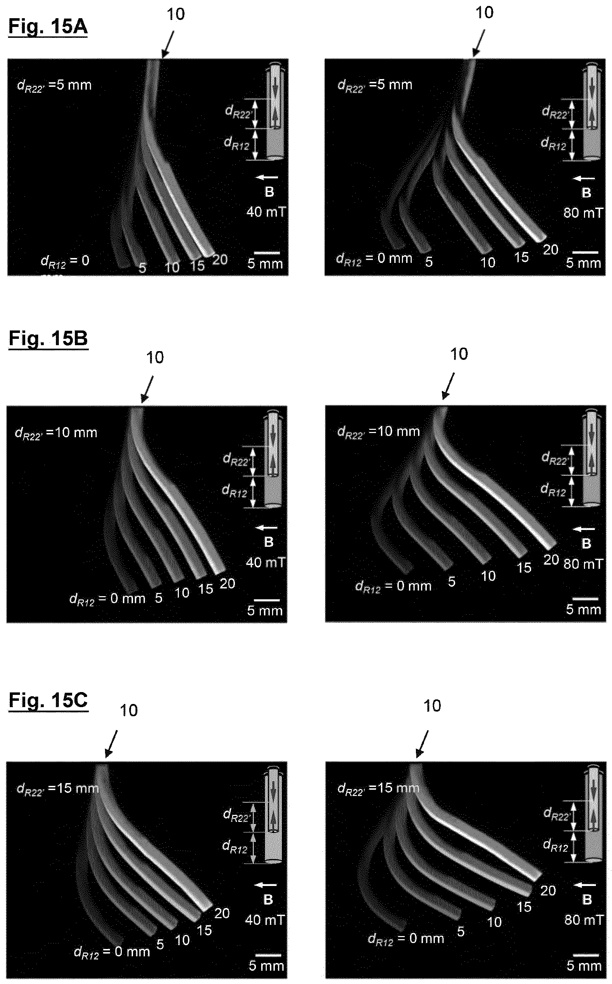

As shown in Figs. 15A to 15C, the distance 51 between the magnetic unit 52 and the further magnetic unit 62 of the third configuration, the distance 51 being denoted as *d_{R22'},* may be set to three distinct values, and, for each value of the distance 51, *d_{R22'},* the value of the distance 29 between the first end 34 of the inner part 30 and the first end 24 of the outer part 20, the distance 29 being denoted as *d_{R12},* may be incrementally increased from 0 to 20 mm in increments of 5 mm to observe the deformation of the outer part 20. Additionally, magnitudes of the magnetic field 5, denoted as B, of 40 mT and 80 mT may be established as varying conditions. For the same value of the distance 51, *d_{R22'},* and an constant magnitude of the magnetic field 5, deformations corresponding to five different values of the distance 29, *d_{R12},* are integrated into a single frame for ease of observation. In Fig 15A, when the distance 51 is set to *d_{R22'} =* 5 mm and the distance assumes various values *d_{R12},* the resultant deformations under magnitudes of the magnetic field 5 of 40 mT and 80 mT are observed. In Fig 15B, when the distance 51 is assigned a value of *d_{R22'}* = 10 mm and the distance 29 (*d_{R12}*) varies, the corresponding deformations 705 at magnitudes of the magnetic field 5 of 40 mT and 80 mT are observed. In Fig 15C, when the distance 51 is set to *d_{R22'}* = 15 mm and the distance 29 assumes various values *d_{R12},* the resultant deformations under magnitudes of the magnetic field 5 of 40 mT and 80 mT are obtained.

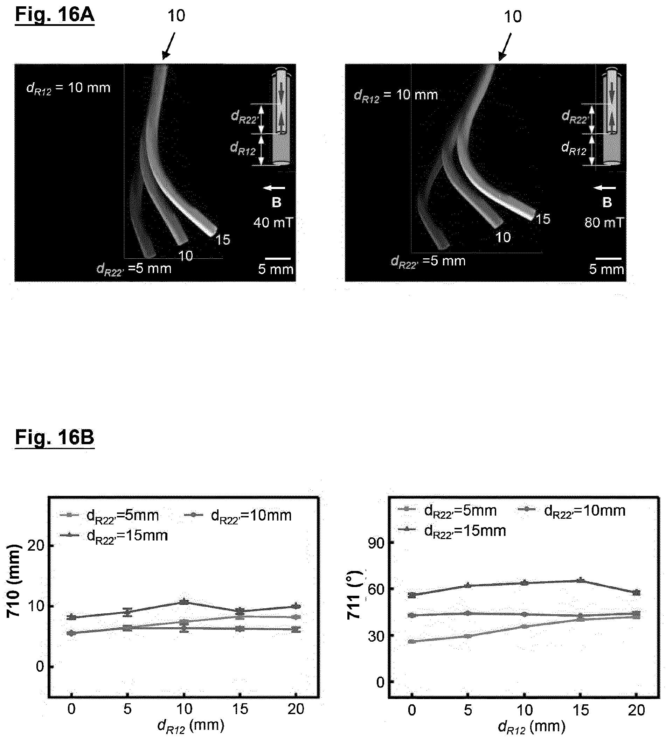

Fig. 16A shows deformations of the body 10 of the third configuration with the magnitude of the magnetic field 5 being set to B = 40mT and B = 80mT, respectively, with distance 29 between the first ends 24, 34 of the inner part 20 and the outer part 30, respectively, being set to *d_{R12}* = 10mm and the distance 51 between the magnetic unit 52 and the further magnetic unit 62 being set to *d_{R22'}* = 5 mm, 10 mm, 15 mm, 20 mm. Deformations under the same magnetic field 5 are merged into a single table for easier observation. Figs. 16B shows the radius of curvature 710 and the terminal bending angle 711 of the outer tube 20 of the third configuration with different parameters exposed to a magnetic field 5 having a magnitude of 80 mT. The trend lines in these two frames indicate that the radius of curvature 710 and the terminal bending angle 711 change with variations in *d_{R22'}* while showing no significant correlation with changes in *d_{R12}.*

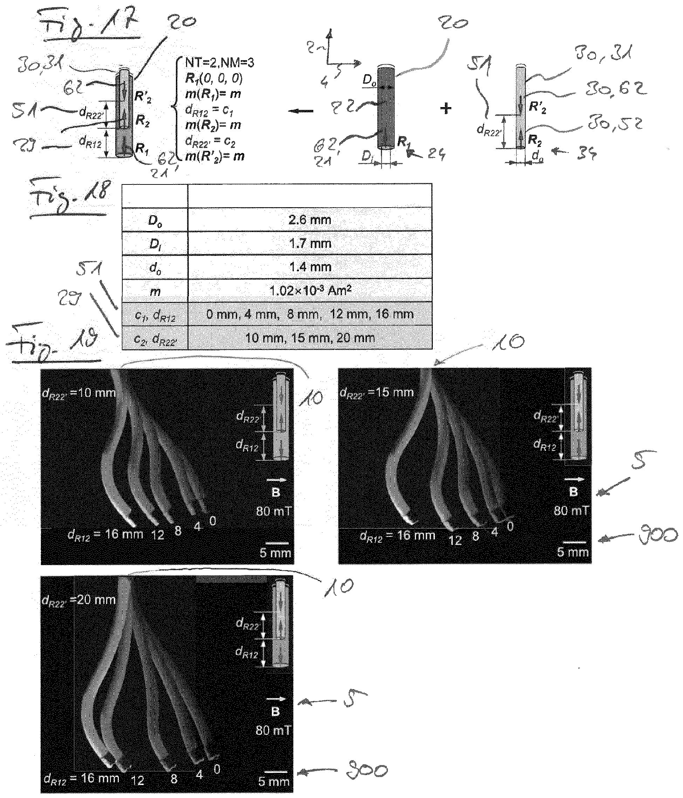

Fig. 17 depicts a fourth configuration of the body 10 of the soft robot 1 with the inner part 20 and the outer part 30. The assembled fourth configuration is depicted to the left in Fig. 17 and the individual components of the fourth configuration are shown to the right in Fig. 17. As far as no differences are disclosed in the description and the Figures, the fourth configuration is configured as it is disclosed in connection with the first configuration and/or the second configuration and/or the third configuration and/or the configuration of Fig. 2 and vice versa.

The inner part 30 of the fourth configuration is the same as with the third configuration, comprising the magnetic unit 52 and the further magnetic unit 62. Different from the third configuration, the fourth configuration has an outer part 20 that also has a further magnetic unit 62 fixed thereto, whereby the further magnetic unit 62 of the outer part 20 is located at the first end 24 of the outer part 20. A direction of a magnetic moment of the further magnetic unit 62 of the outer part 20 is opposite the direction of the magnetic unit 52 located at the first and 34 of the inner part 30. Therefore, the magnetic unit 52 and the further magnetic unit 62 of the outer part 20 are configured to cancel each other when the magnetic unit 52 is placed next to the further magnetic unit 62 of the outer part 20.

Fig. 18 lists exemplary numerical values for the parameters of the fourth configuration shown in Fig. 17.

Fig. 19 depicts the outer part 20 of the body 10 according to the fourth configuration with the distance 51 set to three distinct values (*d_{R22'}* = 10 mm, 15 mm, and 20 mm), and, for each value of the distance 51, the distance 29 is incrementally increased from *d_{R12}* = 0 to *d_{R12}* = 16 mm in increments of 4 mm to observe the deformation of the integrated tube forming the body 10. The magnitude of the magnetic field 5 is B = 80 mT. For the same value of *d_{R22'},* deformations corresponding to five different values of *d_{R12}* are integrated into a single figure for ease of observation.

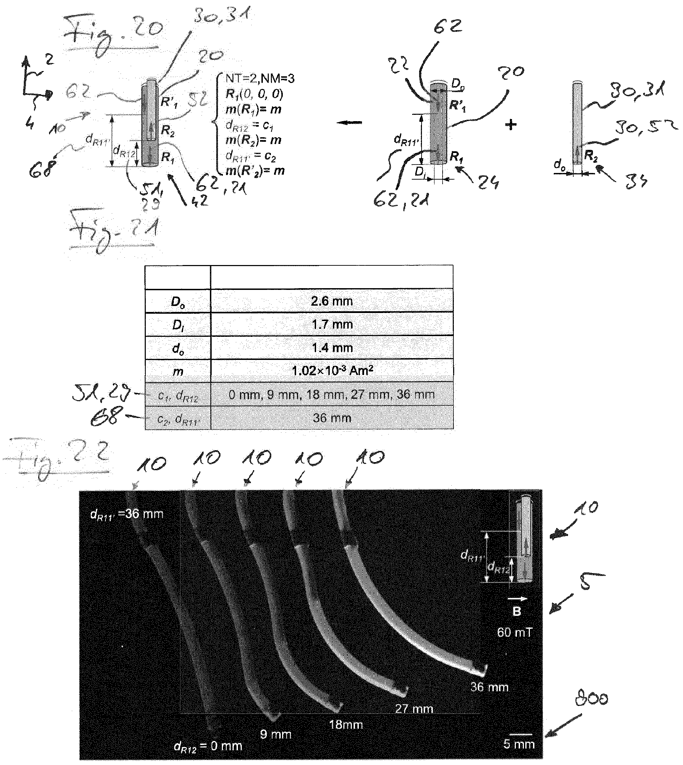

Fig. 20 depicts a fifth configuration of the body 10 of the soft robot 1 with the inner part 20 and the outer part 30. The assembled fifth configuration is depicted to the left in Fig. 20 and the individual components of the fifth configuration are shown to the right in Fig. 20. As far as no differences are disclosed in the description and the Figures, the fifth configuration is configured as it is disclosed in connection with the first configuration and/or the second configuration and/or the third configuration and/or the fourth configuration and/or the configuration of Fig. 2 and vice versa.

With the fifth configuration, the outer part 20 comprises two further magnetic units 62, which are placed at a distance 68 from each other. Thereby, a first further magnetic unit 62 is exemplarily located at the first end 24 of the outer part 20 and a second further magnetic unit 62 is placed at the distance 68 from the first end 24.

Both further magnetic units 62 have the same magnetic moment 63 so that directions of the magnetic moments 63 and magnitudes of the magnetic moments 63 are the same for both further magnetic units 62. The magnetic moment 53 of the magnetic unit 52, which is part of the inner part 30, has a direction opposite to the direction of the magnetic moments 63 of the further magnetic unit 62. The magnetic unit 52 is configured to be placed adjacent to the first further magnetic unit 62 and to be placed adjacent to the second further magnetic unit 62. This allows to selectively modify the magnetic moment 63 of one of the further magnetic units 62. With the exemplary embodiment, the magnitude of the magnetic moment 53 of the magnetic unit 52 equals the magnitude of the magnetic moments 63 of the further magnetic units 62. Therefore, an influence of the magnetic field 5 on either one of the further magnetic units 62 may be selectively switched off by placing the magnetic unit 53 adjacent to the respective further magnetic unit 62.

Fig. 21 lists exemplary numerical values for the parameters of the fifth configuration shown in Fig. 20.

Fig. 22 depicts shapes of the outer part 20 of the body 10 of the fifth configuration for the parameters listed in Fig. 21. Thereby, the distance 68, denoted as *d_{R11'},* is set to 36 mm, and the distance 51 between the magnetic unit 52 and the further magnetic unit 62, the distance 51 being denoted as *d_{R12},* is incrementally increased from 0 to 36 mm in increments of 9 mm to observe the deformation of the integrated tube that is formed by the outer part 20 and the inner part 30. The magnitude of the magnetic field is B = 60 mT.

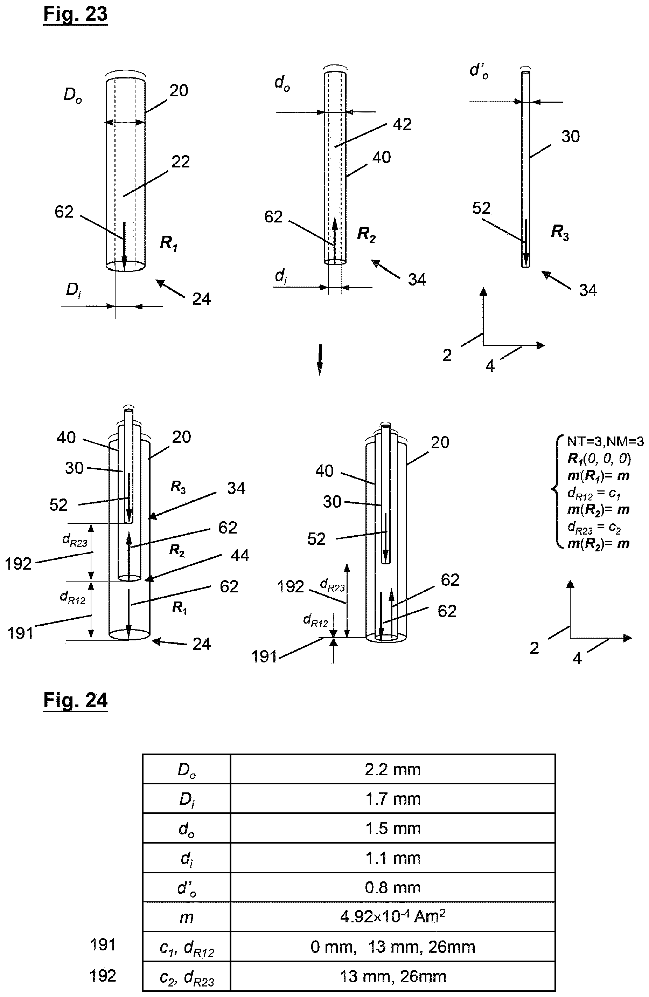

Fig. 23 depicts a sixth configuration of the body 10 of the soft robot 1 with the inner part 20 and the outer part 30. The assembled sixth configuration is depicted at the bottom of Fig. 23 and the individual components of the sixth configuration are shown at the top of Fig. 23. As far as no differences are disclosed in the description and the Figures, the sixth configuration is configured as it is disclosed in connection with the first configuration and/or the second configuration and/or the third configuration and/or the fourth configuration and/or the fifths configuration and/or the configuration of Fig. 2 and vice versa.

The sixth configuration comprises the inner part 30 with the magnetic unit 52 and the outer part 20 having the further magnetic unit 62. A further inner part 40 is stacked in the outer direction 4 in between the outer part 20 and the inner part 30 and the further inner part 40 is movable with respect to the inner part 30 and the outer part 20. Like the outer part 20, the further inner part 40 is configured as a tubular part, having an inner diameter *dᵢ* and an outer diameter *dₒ*. The inner diameter *dᵢ* is more than or equals the outer diameter *d'ₒ* of the inner part 30. The outer diameter *dₒ* is less than or equals the inner diameter *Dₒ* of the outer part 20. The further inner part 40 has a further channel 42 and the inner part 30 is located within the further channel 42. Furthermore, the further inner part 40 is located within the channel 22 of the outer part 20.

The inner part 30 comprises the magnetic unit 52, which is exemplarily located at the first end 34 of the inner part 30. Furthermore, the outer part 20 comprises the further magnetic unit 62, which is exemplarily located at the first end 24 of the outer part 20. With the configuration shown, the magnetic unit 52 and the further magnetic unit 62 have magnetic moments with the same magnitude and the same direction.

The further magnetic unit 62 of the outer part 20 forms a first further magnetic unit 62. The further inner part 40 comprises a second further magnetic unit 62, which is located exemplarily at a free first end 44 of the further inner part 40. The second further magnetic unit 62 has a magnetic moment with a magnitude that equals the magnitude of the magnetic moments of the magnetic unit 52 and the first further magnetic unit 62. Furthermore, the magnetic moment of the second further magnetic unit 62 has a direction that is opposite to the direction of the magnetic moments of the magnetic unit 52 and the first further magnetic unit 62.

With the sixth configuration, the inner part 30, the further inner part 40 and the outer part 20 are independently movable with respect to each other. Thereby, both a distance 191 between the first end 24 of the outer part 20 and the first end 44 of the further inner part 40 and a distance 192 between the first end 44 of the further inner part 40 and the first end 34 of the inner part 30 may be adjusted independently from each other.

For example, the second further magnetic unit 62 of the further inner part 40 is configured to be selectively placed adjacent to the magnetic unit 52 or adjacent to the first further magnetic unit 62. Due to the magnitude of the magnetic moment of the second further magnetic unit 62 being equal to the magnitude of the magnetic moments of the magnetic unit 52 and the first further magnetic unit 62 and due to the direction of the magnetic moment of the second further magnetic unit 62 being opposite to the direction of the magnetic moments of the magnetic unit 52 and the further magnetic unit 62, the body 10 is configured to selectively switch off the action of the first further magnetic unit 62 and the action of the magnetic unit 52 by placing the second further magnetic unit 62 adjacent to the first further magnetic unit 62 or adjacent to the magnetic unit 52.

Fig. 23 depicts the seventh configuration of the body 10 in two different states. With the first state, the first distance 191 and the second distance 192 are both different from zero so that the magnetic unit 52, the first further magnetic unit 62 and the second further magnetic unit 62 are all located at respective distances from each other. With the second state, the first distance 191 is zero and the second distance 192 is different from zero. Therefore, the first further magnetic unit 62 and the second further magnetic unit 62 are located adjacent to each other in the longitudinal direction 2 and exemplarily cancel each other. The magnetic unit 52 is located at the distance 192 from the first further magnetic unit 62 and the second further magnetic unit 62.

The seventh configuration of the body 10, like any other configuration of the body 10 shown in the foregoing, may be expanded by stacking additional further inner parts 40 in between the outer part 20 and the inner part 30, whereby all further inner parts 40 are sequentially stacked within each other in the outer direction.

Fig. 24 lists exemplary numerical values for the parameters of the sixth configuration shown in Fig. 23.

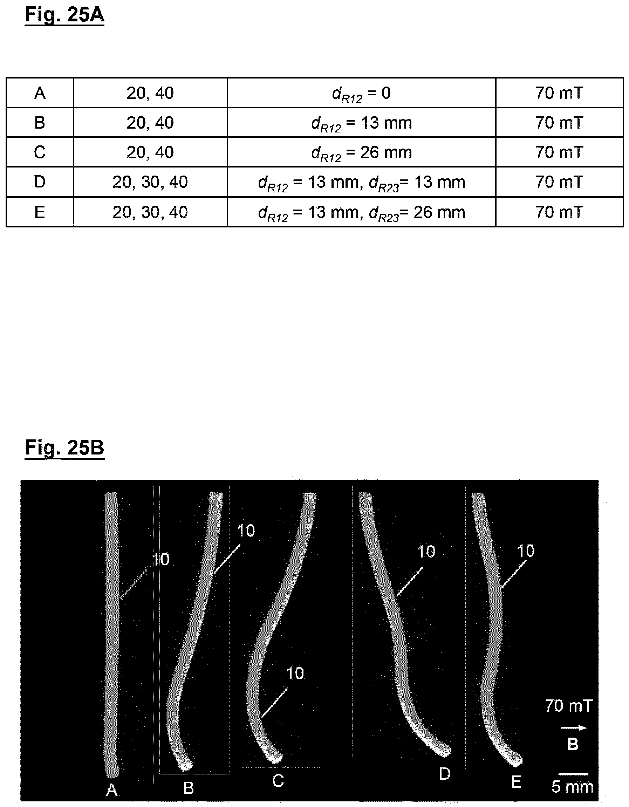

Fig. 25A lists five states of the body 10 of the sixth configuration of the soft robot 1. The first column lists states, the second column lists parts of the body 10 interacting with the magnetic field 5, the third column lists the distances 191, 192, and the fourth column lists the magnitude 106 of the magnetic field 5.

With states A, B and C, the magnetic unit 52 of the inner part 30 is retracted from a bending portion of the outer part 20 that is configured to deform on the influence of the magnetic field 5. For example, the magnetic unit 52 of the inner part 30 may be located at the second end 25 and the second end 25 may be fixed, for example to a rigid structure, such as a support. Both the first further magnetic unit 62 of the outer part 20 and the second further magnetic unit 62 of the further inner part 40 are located within the bending portion of the outer part 20. The distance 191 between the first further magnetic unit 62 of the outer part 20 and the second further magnetic unit 62 of the further inner part 40, the distance 191 being denoted as *d_{R12},* is varied to be *d_{R12}* = 0 mm, 13 mm, 26mm. With states D and C, also the magnetic unit 52 is located within the bending portion of the outer part 20.

Fig. 25B depicts respective shapes of the body 10 for the states listed in Fig. 25A. The deformations corresponding to these five combinations result from a magnetic field of 70 mT.

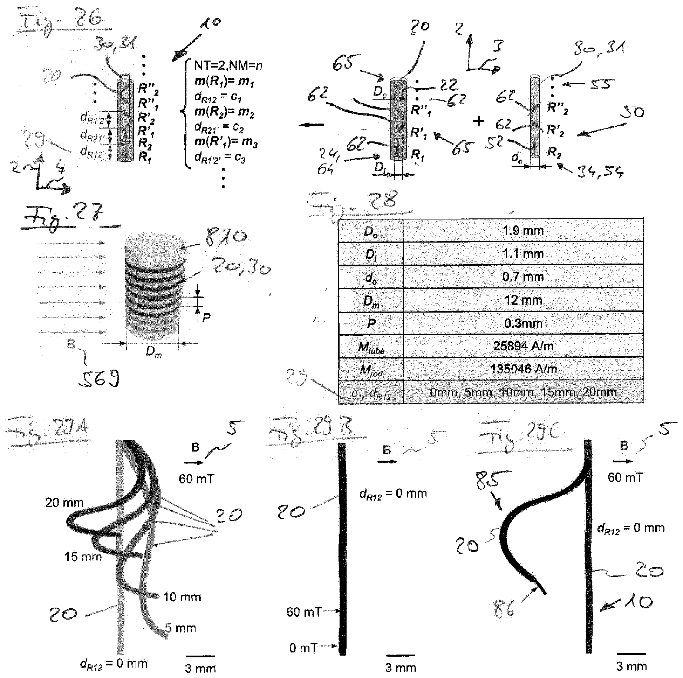

Fig. 26 depicts a seventh configuration of the body 10 of the soft robot 1 with the inner part 20 and the outer part 30. The assembled seventh configuration is depicted to the left in Fig. 26 and the individual components of the sixth configuration are shown to the right in Fig. 26. As far as no differences are disclosed in the description and the Figures, the seventh configuration is configured as it is disclosed in connection with the first configuration and/or the second configuration and/or the third configuration and/or the fourth configuration and/or the fifth configuration and/or the sixth configuration and/or the configuration of Fig. 2 and vice versa.

Like with the second configuration of the body 10, the seventh configuration comprises the magnetic unit 52 located at the first end 34 of the inner part 30 and the further magnetic unit 62 located at the first end 24 of the outer part 20. The magnetic unit 52 and the further magnetic unit 62 thereby have the same magnetic moment as the magnetic unit 52 and the further magnetic unit 62 of the second configuration of the body 10.

With the seventh configuration, the magnetic unit 52 forms part of a magnetic element 50 that extends from a first end 54 to a second end 55. The first end 54 of the magnetic element 50 is exemplarily located at the first end 34 of the inner part 30 and the second end 55 of the magnetic element 50 is located at a distance from the first end 34 of the inner part 30. With other embodiments, the first end 54 may also be located at a distance from the first end 34 of the inner part 30. A magnetic moment or magnetization of the magnetic element 50 changes continuously from the first end 54 to the second end 55. A multitude of magnetic units comprising the magnetic unit 52 and multiple further magnetic units 62 are integrated into the magnetic element 50.

Furthermore, the further magnetic unit 62 of the outer part 20 forms part of a further magnetic element 60 that extends from a first end 64 to a second end 65. The first end 64 is exemplarily located at the first end 24 of the outer part 20. With other embodiments, the first end 64 may also be located at a distance from the first end 24 of the outer part 20. A magnetic moment of the further magnetic element 60 changes continuously from the first end 64 to the second end 65. A multitude of magnetic units comprising the further magnetic unit 62 and multiple additional further magnetic units 62 are integrated into the further magnetic element 60.

With the seventh configuration, a length of the magnetic element 50 between the first and 54 and the second and 55 equals a length of the further magnetic element 60 between the first end 64 and the second end 65.

A direction of a magnetic moment of the magnetic element 50 is opposite to a direction of a magnetic moment of the further magnetic element 60. Thereby, the direction of the magnetic moment of the magnetic element 50 at every location along the length of the magnetic element 50 between the first end 54 and the second end 55 is opposite to the direction of the magnetic moment of the further magnetic element 60 at its respective location between the first end 64 and the second end 65.

The magnetic element 50 comprises magnetic particles that are homogeneously embedded in a flexible substrate material as a powder, each magnetic particle forming a magnetic unit and the magnetic particles comprising the magnetic unit 52. Likewise, the further magnetic element 60 comprises magnetic particles that are homogeneously embedded in a flexible substrate material as a powder, each magnetic particle forming a magnetic unit and the magnetic particles comprising the further magnetic unit 62. A number of magnetic units 52, 62 of the respective magnetic element 50, 60 in this configuration equals the number of magnetic particles embedded in the substrate material.

The magnetic element 50 comprises a helical magnetization profile of its magnetic moment so that the magnetic element 52 forms into a helical shape under the influence of the homogeneous magnetic field 5.

A projection of the direction of the magnetic moment of the magnetic element 50 onto a plane perpendicular to the channel 22 or the longitudinal direction 2 continuously rotates from a first direction of a first magnetic moment at the first end 54 to a second direction of a second magnetic moment at the second end 55. Furthermore, a projection of the direction of the magnetic moment of the further magnetic element 60 onto a plane perpendicular to the channel 22 or the longitudinal direction 2 continuously rotates from a first direction of a first magnetic moment at the first end 64 to a second direction of a second magnetic moment at the second end 65.

As shown in Fig. 27, a method for fabricating the magnetic elements 50, 60 having the magnetic moment that changes continuously from the first end 54, 64 to the second end 55, 65 may comprise providing the respective magnetic element 50, 60 with a continuous deformation, for example by placing the magnetic element 50, 60 into a jig 810. Furthermore, the method may comprise applying a magnetization field 569, such as a homogeneous magnetization field, to the respective magnetic element 50, 60 having the deformation, such as to the magnetic element 50, 60 within the jig 810, for magnetizing all sections of the deformed magnetic element 50, 60 along the magnetization field 569.

To obtain the helical magnetization profile of the magnetic element 50 and the further magnetic element 60, the tube forming the outer part 20 and the rod forming the inner part 30 may be coiled around a jig 810 for magnetization and may be placed into the magnetization field 569 so that they receive completely opposite magnetization profiles. Thereby, the magnetic element 50 and the further magnetic element 60 may be magnetized with opposite directions of the magnetization field 569.

Fig. 28 lists exemplary design parameters for the outer part 20, the inner part 30 and the jig 810 used for magnetization. The jig 810 has a helical channel provided on an outside surface of a cylindrical base, the cylindrical base having an outer diameter *Dₘ* and the helical channel having a pitch P.

Fig. 29A depicts deformation of the seventh configuration of the body 10 formed as an integrated tube comprising the inner part 30 and the outer part 20 for various values of the distance 29, *d_{R12},* between the first end 24 of the outer part 20 and the first end 34 of the inner part 20 demonstrating the capability of the integrated tube to switch between linear and helical deformations under the same magnetic field (60 mT). For *d_{R12}* = 0, due to the inner part 30 and the outer part 20 being located next to each other and having opposite directions of their magnetic moments, magnetic neutralization is achieved, resulting in negligible deformation of the integrated tube in the magnetic field 5.

To further validate the magnetic neutralization characteristic, a comparative experiment was designed. By applying magnetic fields of B = 0 mT and B = 60 mT to the body 10 of the seventh configuration, it was observed that the integrated tube comprising the outer part 20 maintained a nearly linear state for both magnitudes of the magnetic field 5, with only extremely minor differences between the states, see Fig. 29B. To confirm that the negligible deformation at a magnitude of the magnetic field 5 of B = 60 mT shown in Fig. 29B was not due to changes in stiffness caused by the inclusion of the internal rod forming the inner part 30, a control experiment was conducted. As shown in Fig. 29C, an integrated tube containing the body 10 with the outer part 20 and the inner part 30 of the seventh configuration remains linear under the magnetic field 5, whereas a control body 85 with the outer part 20 of the seventh configuration and a control inner part 86 formed by a non-magnetic rod assumes a helical shape. It was found that the increase in stiffness induced by the inner part 30 does not cause the body 10 comprising the integrated tube to remain linear under magnetic fields.

With all configurations shown in the foregoing Figures, the value NT in the Figures denotes the number of parts, such as outer parts 20 and inner parts 30, 40, nested together within the body 10 and the value NM denotes the numbers of magnetic units, such as magnetic units 52 and further magnetic units 62 integrated into the body 10.

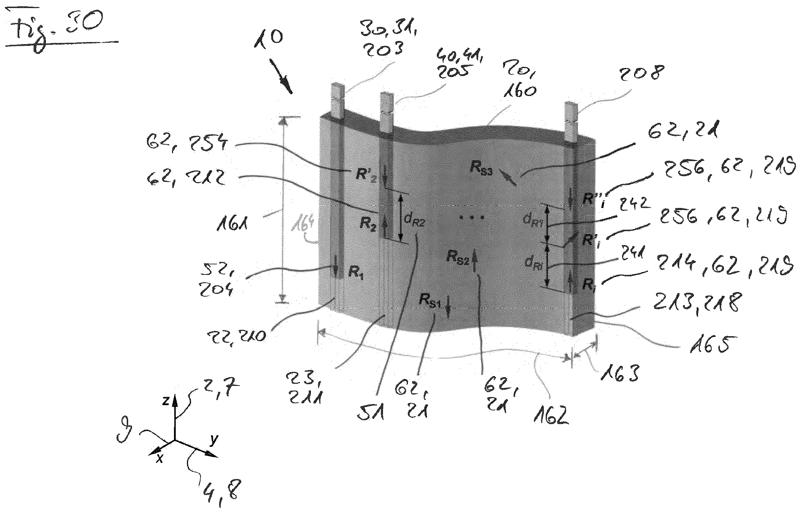

The outer part 20 of the body 10 of the soft robot 1 may also be configured as a flat sheet 160, as shown in Fig. 30. The flat sheet 160 has a length 161 in a length direction 7, a width 162 in a width direction 8 and a height 163 in a height direction 9. The length direction 7 is parallel to the longitudinal direction 2, the width direction 8 is parallel to the outer direction 4 and the height direction 9 is perpendicular to both the length direction 7 and the width direction 8. The flat sheet 160 extends in the width direction 8 between a first edge 164 and a second edge 165. The first edge 164 and the second edge 165 run parallel to the length direction 7.

In the sheet 160, multiple channels having a rectangular, such as a square, or a round cross-section can be designed for rods forming inner parts to pass through. Magnetic units may be mounted to the inner parts. By adjusting the position of these rods relative to the sheet 160, changes in the magnetization profile of the sheet 160 can be achieved. In Fig. 30, ***Rᵢ*** denotes positions of magnetic units in the rods forming the inner parts, ***Rₛᵢ*** denotes positions of magnetic units positioned in the sheet 160, and *d_{Ri}* indicates the distance between two corresponding magnetic units.

With the body 10 shown in Fig. 30, the outer part 20 has a channel 22 that is located at the first edge 164 of the outer part 20 and runs parallel to the first edge 164. Furthermore, the outer part 20 has a further channel 23 that is located at a distance from the first edge 164 and runs parallel to the first edge 164 and the channel 22. The channel 22 forms a first channel 210 and the further channel 23 forms a second channel 211.

The body 10 comprises an inner part 30 that forms a first inner part 203. As long as no differences are disclosed in the description and the Figures, the first inner part 203 is configured as it is disclosed for the inner part 30 of the foregoing embodiments. In particular, the first inner part 203 has the flexible part 31 and the magnetic unit 52 attached to the flexible part 31 at the first end 34 of the inner part 30, whereby the magnetic unit 52 forms a first magnetic unit 204. The first inner part 203 is movably arranged within the first channel 210 of the outer part 20. With the embodiment shown, the first inner part 203 exemplarily has a rectangular cross-section. Likewise, the first channel 210 of the outer part 20 exemplarily has a rectangular cross-section.

The body 10 comprises a further inner part 40 that forms a second inner part 205. As long as no differences are disclosed in the description and the Figures, the second inner part 205 is configured as it is disclosed for the further inner part 40 of the foregoing embodiments. In particular, the second inner part 205 has the further flexible part 41 and the further magnetic unit 62 attached to the further flexible part 41 at the first end 44 of the second inner part 205, whereby the further magnetic unit 62 forms a second magnetic unit 212. Furthermore, an additional second magnetic unit 524 of the second inner part 205 is located at a distance 51 from the second magnetic unit 212.

The second inner part 205 is movably arranged within the second channel 211 of the outer part 20. With the embodiment shown, the second inner part 205 exemplarily has a rectangular cross-section, namely a square cross-section. Likewise, the second channel 211 of the outer part 20 exemplarily has a rectangular cross-section.

The first magnetic unit 204 and the second magnetic unit 212 have magnetic moments with opposite directions. Furthermore, a magnitude of the electric dipole moment of the first magnetic unit 203 equals a magnitude of the magnetic moment of the second magnetic unit 212. The magnetic moment of the second magnetic unit 212 and the magnetic moment of the additional second magnetic unit 524 have equal magnitudes and opposite directions.

The body 10 may comprise several extra channels 218 and individual extra inner parts 208 may be movably arranged within each of the extra channels 218. Each extra inner part 208 may comprise at least one extra magnetic unit 219. The extra inner parts 208 may be configured as it is disclosed for the first inner part 203 and the second inner part 205 and the extra magnetic units 219 may be configured as it is disclosed for the first magnetic unit 204 and the second magnetic unit 212.

Exemplarily, the body 10 depicted in Fig. 30 comprises a third channel 213 forming an extra channel 218 with a third inner part 206 forming an extra inner part 208 movably arranged therein. The third inner part 206 comprises three further magnetic units 62, which form a third magnetic unit 214 and two additional third magnetic units 526, respectively. The third magnetic unit 214 is located at a first end of the third inner part 206, one of the additional third magnetic units 526 is located at a distance 241 from the third magnetic unit 214 and the other one of the additional third magnetic units 526 is located at a distance 242 from the one of the additional third magnetic units 526. The further magnetic units of the third inner part 206 form extra magnetic units 218.

As shown in Fig. 30, the outer part 20 may also comprise a further magnetic unit 62 that forms a fixed magnetic unit 21 and that is fixed to the outer part 20, for example embedded within the outer part 20. With the exemplary embodiment, the outer part 20 comprises a multitude of fixed magnetic units 21, each fixed magnetic unit 21 forming a further magnetic unit 62.

In the following, several possible alternative configurations of the body 10 having the outer part 20 that is configured as a flat sheet 160 are described. As long as no differences are disclosed in the description or the Figures, the alternative configurations are configured as it is disclosed for the configuration in Fig. 30 and vice versa.

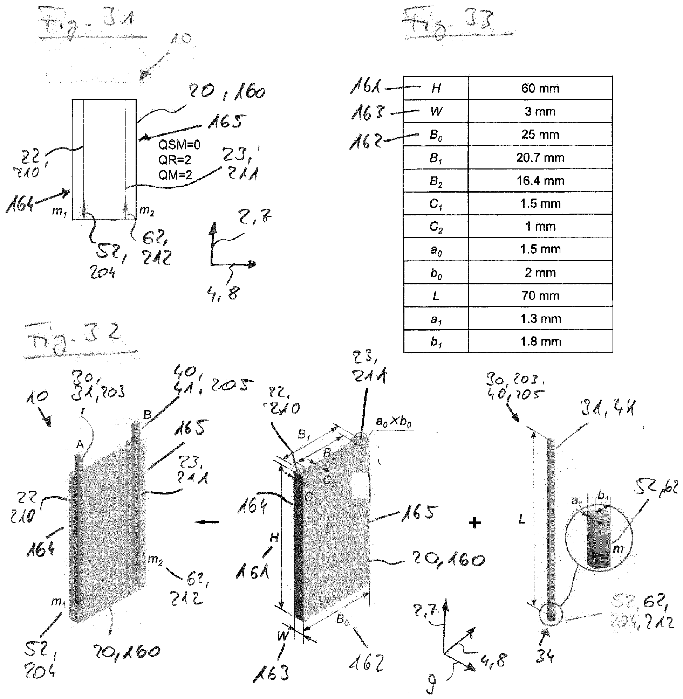

Figs. 31 and 32 depict a first configuration of the body 10 having the outer part 20 configured as a flat sheet 160. The channel 22 is located at the first edge 164 of the outer part 20 and runs parallel to the first edge 164. Furthermore, the further channel 23 is located at the second edge 165 of the outer part 20 and runs parallel to the second edge 165. The channel 22 forms the first channel 210 and the further channel 23 forms the second channel 211.

The body 10 further comprises the inner part 30 already described in connection with Fig. 30. The body 10 comprises a further inner part 40 that forms a second inner part 205. As long as no differences are disclosed in the description and the Figures, the second inner part 205 of the first configuration is configured as it is disclosed for the further inner part 40 shown in Fig. 30 and vice versa. With the first configuration, the second inner part only has the second magnetic unit 214 located at the first end of the second inner part 205. The first magnetic unit 204 and the second magnetic unit 212 have magnetic moments with opposite directions. Furthermore, a magnitude of the electric dipole moment of the first magnetic unit 204 equals a magnitude of the magnetic moment of the second magnetic unit 212.

Fig. 33 lists exemplary values for parameters of the flat sheet 160 shown in Fig. 32.

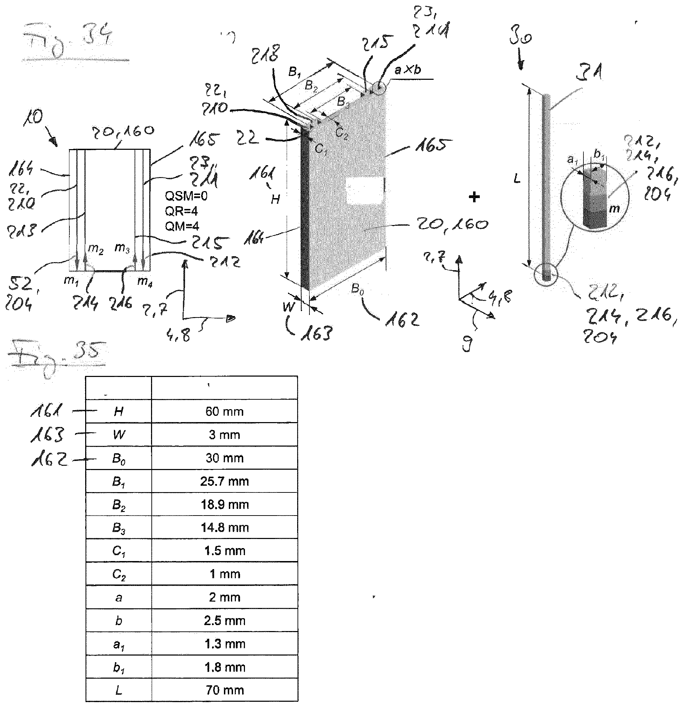

Fig. 34 depicts a second configuration of the body 10 having the outer part 20 configured as a flat sheet 160. As far as no differences are disclosed in the description and the Figures, the body 10 of the second configuration is configured as it is disclosed for the body 10 of the first configuration and vice versa.

With the second configuration of the body 10, the outer part 20 has a third channel 213 located next to and orientated parallel to the first channel 210 and a fourth channel 215 located next to and orientated parallel to the second channel 211. The first channel 210 and the third channel 230 form a first pair of channels located at the first edge 164 of the flat sheet 160 and the second channel 211 and the fourth channel 215 form a second pair of channels located at the second edge 165 of the flat sheet 160. In each of the channels 210, 211, 230, 215, an inner part 30 is arranged, whereby each inner part 30 has a flexible part 31 and a magnetic unit located at a free end of the flexible part 31.

In addition to the first magnetic unit 204 movably arranged within the first channel 210 and the second magnetic unit 212 movably arranged within the second channel 211, a third magnetic unit 214 is movably arranged within the third channel 213 and a fourth magnetic unit 216 is movably arranged within the fourth channel 215.

Magnetic units 204, 212, 214, 216 located in pairs of adjacent channels 210, 211, 213, 215 may have magnetic moments with opposite directions. Furthermore, magnetic units located in pairs of adjacent channels may have magnetic moments with equal magnitudes. With the second configuration, the first magnetic unit 204, the second magnetic unit 212, the third magnetic unit 214 and the fourth magnetic unit 216 have magnetic moments with equal magnitudes. Furthermore, a direction of the magnetic moment of the first magnetic unit 204 is opposite to a direction of the magnetic moment of the third magnetic unit 214 and a direction of the magnetic moment of the second magnetic unit 212 is opposite a direction of the magnetic moment of the fourth magnetic unit 216. With the second configuration, the direction of the magnetic moment of the first magnetic unit 204 is exemplarily parallel to the direction of the magnetic moment of the second magnetic unit 212.

Fig. 35 lists exemplary values for parameters of the flat sheet 160 shown in Fig. 34.

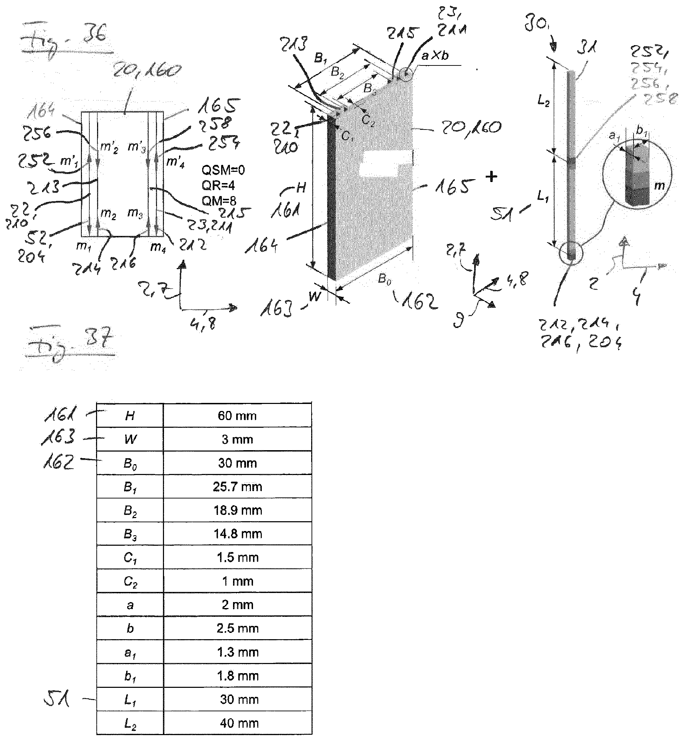

Fig. 36 depicts a third configuration of the body 10 having the outer part 20 configured as a flat sheet 160. As far as no differences are disclosed in the description and the Figures, the body 10 of the third configuration is configured as it is disclosed for the body 10 of the second configuration and vice versa.

With the third configuration, each channel 210, 211, 214, 215 comprises an additional magnetic unit. Exemplarily, an additional first magnetic unit 252 is movably arranged within the first channel 210, an additional second magnetic unit 254 is movably arranged within the second channel 211, an additional third magnetic unit 256 is movably arranged within the third channel 213 and an additional fourth magnetic unit 258 is movably arranged within the fourth channel 215.

For each channel 210, 211, 213, 215, directions of the magnetic moments of the two magnetic units 204, 212, 214, 216, 252, 254, 256, 258 arranged within the respective channel 210, 211, 213, 215 are opposite to each other and magnitudes of the magnetic moments of the two magnetic units 204, 212, 214, 216, 252, 254, 256, 258 arranged within the respective channel 210, 211, 213, 215 are equal. A direction of the magnetic moment of the additional first magnetic unit 252 is opposite to the direction of the magnetic moment of the first magnetic unit 204 and a magnitude of the magnetic moment of the additional first magnetic unit 252 is equal to the magnitude of the magnetic moment of the first magnetic unit 204. A direction of the magnetic moment of the additional second magnetic unit 254 is opposite to the direction of the magnetic moment of the second magnetic unit 212 and a magnitude of the magnetic moment of the additional second magnetic unit 254 is equal to the magnitude of the magnetic moment of the second magnetic unit 212. A direction of the magnetic moment of the additional third magnetic unit 256 is opposite to the direction of the magnetic moment of the third magnetic unit 214 and a magnitude of the magnetic moment of the additional third magnetic unit 256 is equal to the magnitude of the magnetic moment of the third magnetic unit 214. A direction of the magnetic moment of the additional fourth magnetic unit 258 is opposite to the direction of the magnetic moment of the fourth magnetic unit 216 and a magnitude of the magnetic moment of the additional fourth magnetic unit 258 is equal to the magnitude of the magnetic moment of the fourth magnetic unit 216.

For each channel 210, 211, 213, 215, the magnetic unit 204, 212, 214, 216 is located at a distance 51 from the additional magnetic unit 252, 254, 256, 258 of the respective channel 204, 211, 213, 215. Exemplarily, a respective flexible part 31 is located within each channel 210, 211, 213, 215, whereby the respective flexible part 31 comprises the magnetic unit 204, 212, 214, 216 and the additional magnetic unit 252, 254, 256, 258 located within the respective channel 210, 211, 213, 215.

Fig. 37 lists exemplary values for parameters of the flat sheet 160 shown in Fig. 36.

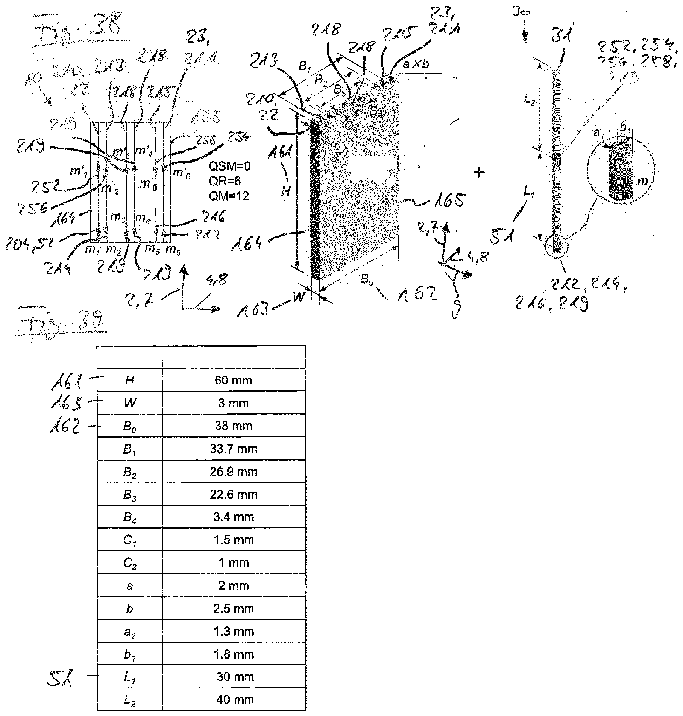

Fig. 38 depicts a fourth configuration of the body 10 having the outer part 20 configured as a flat sheet 160. As far as no differences are disclosed in the description and the Figures, the body 10 of the fourth configuration is configured as it is disclosed for the body 10 of the third configuration and vice versa.

With the fourth configuration, a set of extra channels 218 is located in between the first channel 210 and the second channel 211. Exemplarily, two extract channels 218 are located in between the first channel 210 and the second channel 211. The extra channels 218 are exemplarily placed adjacent to each other. The two extra channels 218 form a pair of adjacent extra channels 218. With other embodiments, the set of extra channels 218 may only comprise a single extra channel 218. Alternatively, the set of extract channels 218 may comprise more than two extra channels 218. The extra channels 218 may be located pairwise adjacent to each other.

Within each extra channel 218, two extra magnetic units 219 are located that have magnetic moments with a same direction and a same magnitude. Furthermore, the extra magnetic units 219 of one of the extra channels 218 and the extra magnetic units 219 of the other one of the extra channels 218 have magnetic moments with the same magnitude and opposite directions. Like with the magnetic units 204, 212, 214, 216, 252, 254, 256, 258 of the remaining channels 210, 211, 213, 215, the extra magnetic units 219 of each extra channel 218 are placed at the distance 51 from each other. Furthermore, the extra magnetic units 219 of each extra channel 218 form part of an inner part 30 located within the respective extra channel 218. Each inner part 30 comprises a flexible part 31 and the extra magnetic units 219 are fixed to the flexible part 31 at the distance 51.

With the fourth configuration, the outer part 20 comprises three pairs of channels 210, 211, 213, 215, 218, whereby the channels 210, 211, 213, 215, 218 of each pair are located adjacent to each other.

Fig. 39 lists exemplary values for parameters of the flat sheet 160 shown in Fig. 38.

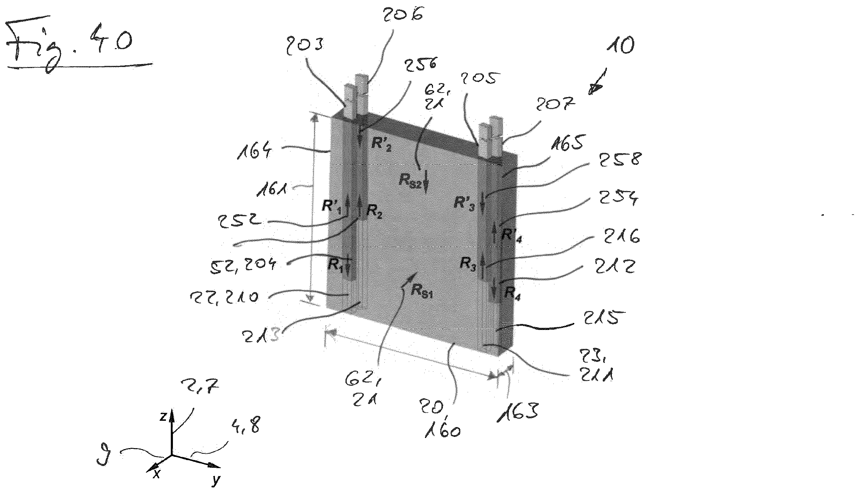

Fig. 40 depicts a variant of the flat sheet 160 of the third configuration shown in Fig. 36. With the variant shown in Fig. 40, the first channel 210 and the third channel 213 are arranged next to each other in the height direction 9 and the second channel 211 and the third channel 215 are arranged next to each other in the height direction 9. The first channel 210 and the third channel 213 are thereby located at the first edge 164 of the flat sheet 160 and the second channel 211 and the fourth channel 214 are located at the second edge 165 of the flat sheet 160.

Furthermore, the variant of Fig. 40 comprises two fixed magnetic units 21 embedded within the flat sheet 160, wherein each fixed magnetic unit 21 forms a further magnetic unit 62.

As can be seen from Fig. 40, the inner part 31 located within the first channel 210 forms a first inner part 203, the inner part 31 located within the second channel 211 forms a second inner part 205, the inner part 31 located within the third channel 213 forms a third inner part hundred 6 and the inner part 31 located within the fourth channel 215 forms a fourth in the part 207. The same designations also apply to the inner parts 31 located within the respective channels 210, 211, 213, 215 of the variant shown in Fig. 36.

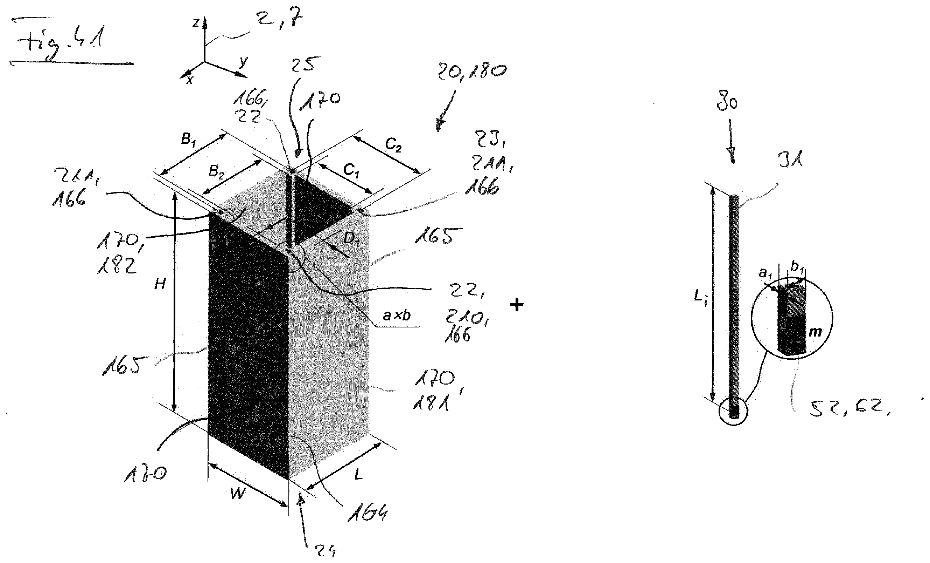

Fig. 41 depicts a soft robot 1 according to the present disclosure that has an outer part 20 that is configured as a three-dimensional body. Exemplarily, the outer part 20 is configured as a box 180. The box 180 is elongated in a length direction 7 and has a height H along the length direction 7. It has a length L in a first transverse direction x and a width W along a second transverse direction y, wherein the length direction 7, the first transverse direction x and the second transverse direction y are perpendicular to each other.

The box 180 has walls 170 that are configured as flat sheets 160. The walls 170 are parallel to the length direction 7 and arranged at right angles with respect to each other. The walls 170 form a first sidewall 181 and a second sidewall 182 that are orientated parallel to each other and parallel to the first transverse direction x. The first sidewall 181 and the second sidewall 182 have the length L in the first transverse direction x and the height H in the second transverse direction y. Furthermore, they have a thickness D₁ in the second transverse direction y that is smaller than the length L and the height H. As far as no differences are disclosed in the description and the Figures, the first sidewall 181 and the second sidewall 182 are configured as it is disclosed for the flat sheet 160 shown in Figs. 31 and 32. The first edges 164 and the second edges 165 of both the first sidewall 181 and the second sidewall 182 each form an edge of the box 180 running along the length direction 7.

The walls 170 form two additional sidewalls that connect the first sidewall 181 with the second sidewall 182 along the second transverse direction y. The additional sidewalls 170 have the width W along the second transverse direction y and the height H along the length direction 7. Furthermore, they have a thickness D₁ along the first transverse direction x that is smaller than the height H and the width W.

At each of the edges 164, 165 of the box 180, an edge channel 166 is located that extends in the longitudinal direction 2 that is parallel to the length direction 7. The edge channels 166 located at the first edges 164 of the first sidewall 181 and the second sidewall 182 form respective channels 22 or first channels 210 and the edge channels 166 located at the second edges 165 of the first sidewall 181 and the second sidewall 182 form respective further channels 23 or second channels 211. The first and second channels 210, 211 are configured as it is disclosed for the first and second channels 22, 211 of the flat sheet 160 shown in Fig. 31.

Each channel 22, 23 is exemplarily configured with a rectangular cross section and has a first width a in the first transverse direction x and a second width b in the second transverse direction y.

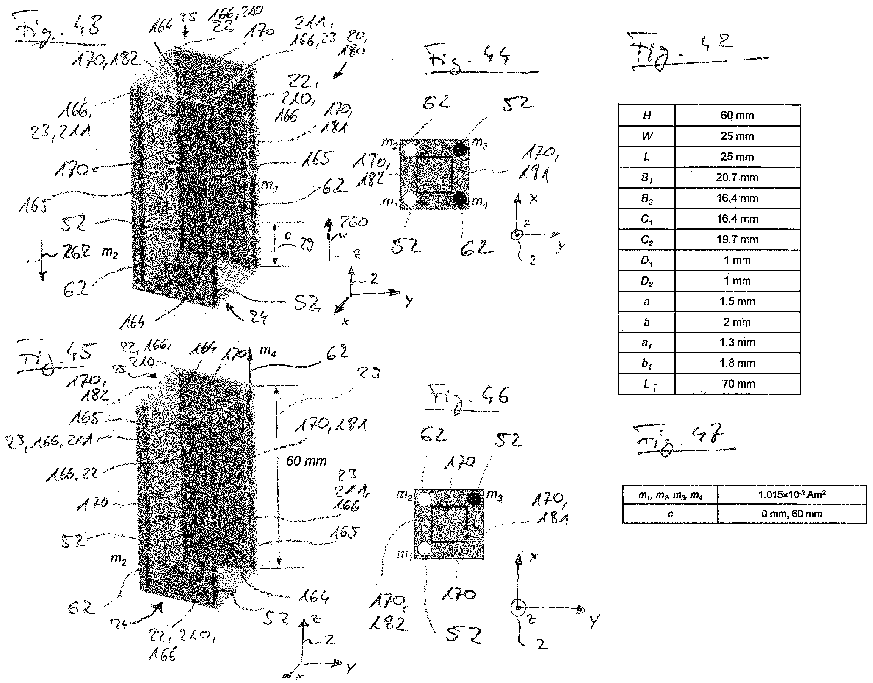

Within each of the edge channels 166, an inner part 30 is located, whereby the inner part 30 comprises a flexible part 31 and a magnetic unit 52, 62 located at a free 34 end of the flexible part 31. The inner part 30 is separately shown to the right in Fig. 46 and has a first width a₁ in the first transverse direction x and a second width a₂ in the second transverse direction y. Furthermore, it has a length Lᵢ along the length direction 2. Within the edge channels formed by the channels 22 at the first edges 164, respective magnetic units 52 are located and within the edge channels formed by the further channels 23 at the second edges 165, respective further magnetic unit 62 are located.

Fig. 42 lists exemplary dimensions of the body 10 shown in Fig. 41.

Figs. 43 to 46 exemplarily show a possible configuration of the magnetic units 52, 62 of the body 10 shown in Fig. 41. Thereby, Figs. 43 and 44 show the individual magnetic units 52, 62 located at first positions along the channels 22, 23 with Fig. 43 depicting a schematic three-dimensional view of the body 10 and Fig. 44 showing a schematic bottom view of the body 10 shown in Fig. 43. Furthermore, Figs. 45 and 46 show the individual magnetic units 52, 62 located at second positions along the channels 22, 23 with Fig. 45 depicting a schematic three-dimensional view of the body 10 and Fig. 46 showing a schematic bottom view of the body 10 shown in Fig. 46.

Thereby, the magnetic moments of the magnetic units 52, 62 arranged within the channels 22, 23 at the first edge 164 and the second edge 165 of the first sidewall 181 have a first common direction 260 and the magnetic moments of the magnetic units 52, 62 arranged within the channels 22, 23 at the first edge 164 and the second edge 165 of the second sidewall 182 have a second common direction 262. The second common direction 262 is opposite to the first common direction 260. This configuration has the technical effect that the first sidewall 181 and the second sidewall 182 bend in opposite directions within a homogeneous magnetic field 5 that is orientated parallel to the second transverse direction y.

Fig. 47 lists possible values for the parameters of Figs. 43 to 46.

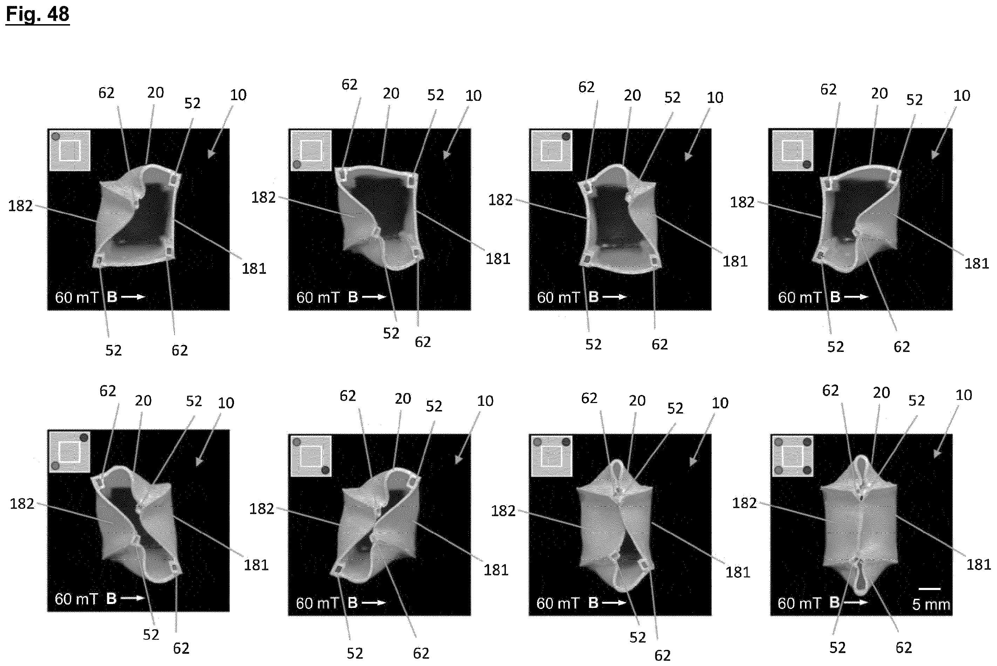

Fig. 48 depicts various deformations of the body 10 for different positions of the magnetic units 52, 62 in a bottom view onto the free first end 24 of the outer part 20. The second end 25 of the outer part 20 is fixed to a support (not shown). The sketches in the upper left corner of the individual panels indicate first magnetic units 52, 62 that are located at the free first end 24 of the outer body 20 with a filled circle.

The devices and methods according to the present disclosure are further detailed in the following sections.

### Results

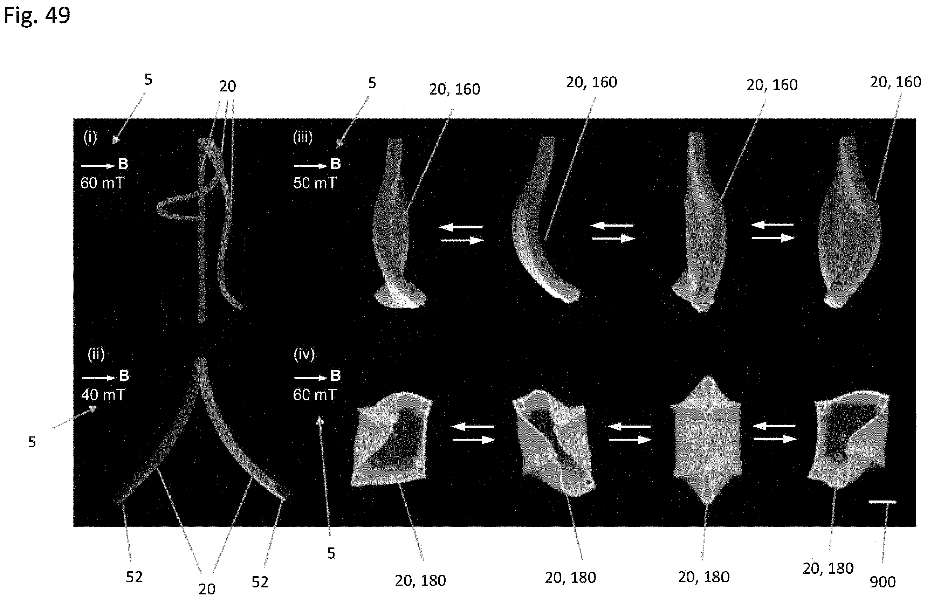

The present disclosure introduces real-time, in-situ magnetization reprogramming, which modifies the relative positions of magnetic units 52, 62 within a soft robot 1 by dynamically and locally manipulating a magnetic unit carrier formed by the magnetic member 16, see Fig. 2. The variation of the position of the magnetic unit 52, 62 enables the real-time and in-situ reprogramming of the magnetization profile of soft robots 1. "Magnetic unit" may be defined as a collection of magnetic material within a continuous region exhibiting a magnetization or magnetic moment with a uniform direction. This unit is independent of its volume size and is defined solely by the direction of the magnetization. For example, in Fig. 49 at (ii), which is further detailed below, the black region at the end of the tube forming the outer part 20, which black region has a magnetization in the downward direction, constitutes one magnetic unit 52, 62 (dimensions Φ1.9 × 2 mm). In contrast, in Fig. 49 at (i), also further detailed below, the tube formed by the body 10 displays a helical magnetization profile where magnetic particles along its longitudinal axis 2 have varying magnetization directions, with each particle representing a separate magnetic unit 52, 62 (dimension Φ0.05 mm). "Magnetic unit carriers" refer to components in soft robots 1 that are capable of transporting or moving magnetic units 52, 62. They can take the form of nested tubes (see, e.g., Fig. 2 and Fig. 23) or built-in rods (see, e.g., Fig. 30, Fig. 41), and they undergo movement under the influence of external forces generated by actuators or other stimuli. This approach embodies a universal framework that can be adapted to various configurations of soft robots 1 and their components. It accommodates diverse forms of soft robotic systems or soft robots 1, such as tubular robots 1 (see, e.g., Figs. 2 to 29) or sheet-shaped robots 1 (see, e.g., Figs. 30 to 40), among others. The magnetic units 52, 62 integrated within this framework may vary widely, from magnetic particles (see, e.g., Fig. 49 at (i) and Fig. 26) to permanent magnets (see, e.g., Fig. 49 at (iii)), each differing in size and magnetization capacities. This approach allows for the customization or modification of the positions of magnetic units 52. 62 according to various requirements; thereby generating different magnetization profiles to achieve diverse deformations. For instance, as illustrated in Fig. 49, various shapes, such as helical and linear shapes under the same uniform magnetic field 5, along with their real-time interconversion, are depicted. These deformation modes are unachievable through existing methodologies. The method of reconfiguring positions of magnetic units 52, 62 can be applied to configure a single magnetic unit 52, 62 (Fig. 49 at (ii)) or multiple magnetic units 52, 62 (Fig. 49 at (iii)-(iv)), and even a virtually infinite number of magnetic units 52, 62, formed by magnetic particles embedded in a flexible matrix material (Fig. 49 at (i)); it can be utilized in magnetization reprogramming in 1D robot morphologies (Fig. 49 at (i)-(ii)), 2D robot morphologies (Fig. 49 at (iii), and 3D robot morphologies (Fig. 49 at (iv)). Additionally, when combined with varying magnetic fields 5, it can broaden the range and diversity of shape deformations, as detailed below.

In Fig. 49, under a uniform magnetic field 5, several deformations are achieved through the proposed real-time in-situ magnetization reprogramming, ranging from bodies 10 configured as one-dimensional (1D) tubes (at (i) and (ii)) to bodies 10 configured as two-dimensional (2D) sheets (at (ii)), and progressing to bodies 10 configured as three-dimensional (3D) box-shaped structures (at (iii)), with transformations occurring at different moments. The tops of the objects forming the outer part 20 are suspended at a carrier (not shown) with their respective second ends 25, with the bodies 10 at (i-iii) shown in the front view and the bodies 10 at (iv) depicted in the bottom view. In (i), the tube forming the outer part 20 transitions from a straight line into a helix under the same uniform magnetic field 5; in (ii), the tube forming the outer part 20 achieves deformations to the left and right under the same uniform magnetic field 5. In (i), the tube's color was digitally adjusted from black to red for enhanced visualization, whereas the objects in (ii-iv), originally red, were left unmodified. The scale bar 900 is 5mm.

For the 1D magnetization reprogramming, a tubular robot 1 may be selected as the platform, as shown in Fig. 23. A nested approach may be employed with multiple tubes forming the outer part 20 and the inner parts 30, 40, each containing preprogrammed magnetic units 52, 62. The relative positions between tubes (*d_{Rii+1}*) are tunable according to requirements, see Fig. 23. Altering *d_{Rii+1}* induces changes in the positions of the magnetic units 52, thereby achieving various magnetization profiles.

Moreover, the number of magnetic units 52 in each tube 20, 30, 40 can be expanded as needed. Possible configurations and shapes of the tubular robot 1 are shown in Figs. 3 to 29, providing the possibility of generating multiple deformations in real time and in situ by using the method according to the present disclosure without the need for altering the magnetic field 5. Each configuration exhibits distinct characteristics and capabilities. For instance, the first configuration of Figs. 3 to 5 enables selective activation of magnetism in the robot 1, with specific applications detailed below. The third configuration of Figs. 13 to 16 possesses shape retention capabilities, with specific applications detailed below. The seventh configuration of Figs. 26 to 29 enables rapid switching between various complex deformations, such as linear and helical shapes, with specific applications detailed below.

Additionally, with the same configuration, different shapes may be achieved by altering the relative positions between tubes forming the outer part 20 and the inner parts 30, 40. For instance, in the second configuration of Figs. 7 to 12, within a uniform magnetic field 5 of 80 mT, as the distance 51between the first magnetic unit 52 and the further magnetic unit 62 varies from *d_{R12} =* 0 mm in increments of 6 mm up to *d_{R12} =* 24 mm, the radius of curvature 710 of the tube forming the outer part 20 changes from +∞ to 7.3 mm and then to 15.6 mm, while the terminal bending angle 711 shifts from 0° to 21.5° and then to 64.7°, see Figs. 11 and 12. The radius of curvature 710 and terminal bending angle 711 are specifically defined to describe the shape changes of the tube, as detailed in connection with Fig. 110 below.

Moreover, within various magnetic fields 5, more shape changes may be obtained under the same configuration. For example, in the second configuration of Figs. 7 and 12A with the distance 51 set at *d_{R12} =* 6 mm, the increase in magnitude of the magnetic field 5 from 20 mT to 100 mT leads to a decrease in the radius of curvature 710 from 26.9 mm to 3.8 mm and an increase in the terminal bending angle 711 from 3.9° to 26.3°. These experiments demonstrate the ability of 1D magnetization reprogramming according to the present disclosure to achieve various deformations.

For the 2D magnetization reprogramming, a sheet-shaped robot 1 may be selected as the platform. This robot 1 is designed with strategically placed holes or channels 22, 23 to facilitate the movement of rods forming the inner parts 30, which are equipped with preprogrammed magnetic units 52, 62 within their structure, see, for example, Fig. 30. Four exemplary distinct configurations have been described in connection with Figs. 31 to 38, which are configured to undergo shape deformation as shown in Fig. 49 at (iii),

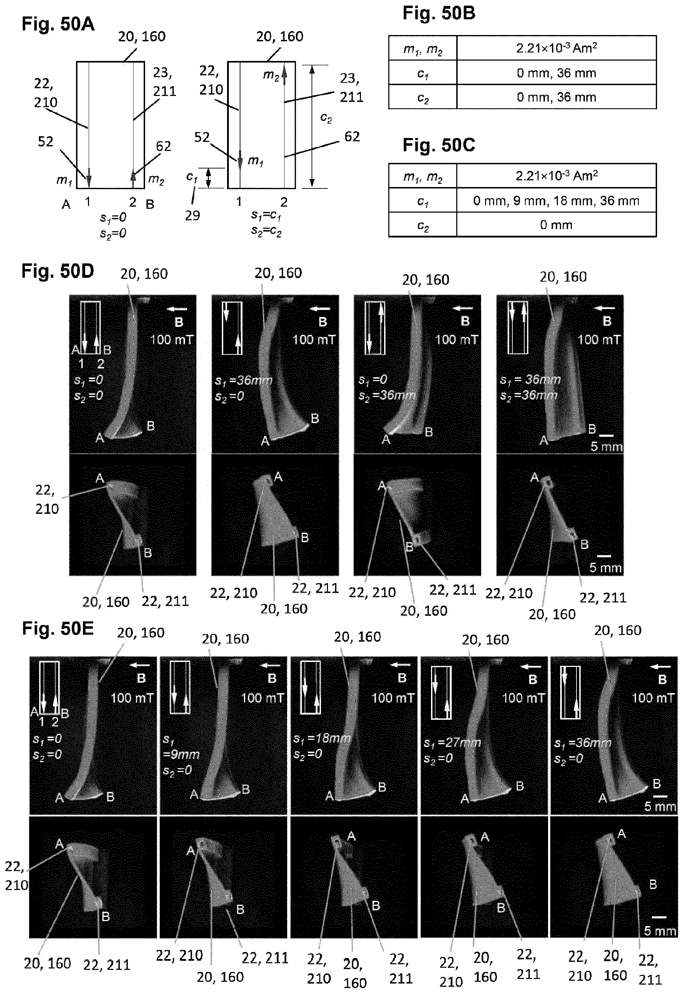

Figs. 50A to 50E depict shape change characteristics of the first configuration of the body 10 having the outer part 20 configured as a flat sheet 160 shown in Fig. 31. Fig. 50A is a schematic illustration depicting the positions of the magnetic units 52, 62. To describe the correspondence between the front and bottom views in the deformation of the sheet 160, the two lower corners of the sheet 160 are denoted as points A and B. Fig. 50B lists possible parameter settings for observing deformation at extreme positions, whereby two extreme positions (0 mm and 36 mm) are listed for each magnetic unit 52, 62. Fig. 50C lists possible parameter settings for observing deformation, aimed at controlling the variables: the further magnetic unit 62 is fixed (0 mm), while the magnetic unit 52 is positioned variably. Fig. 50D depicts deformations at the extreme positions listed in Fig. 50B with a front view in the first row and a bottom view in the second row. Fig. 50E depicts deformations with the magnetic units 52, 63 at various positions listed in Fig. 50C with the front view in the first row and the bottom view in the second row.

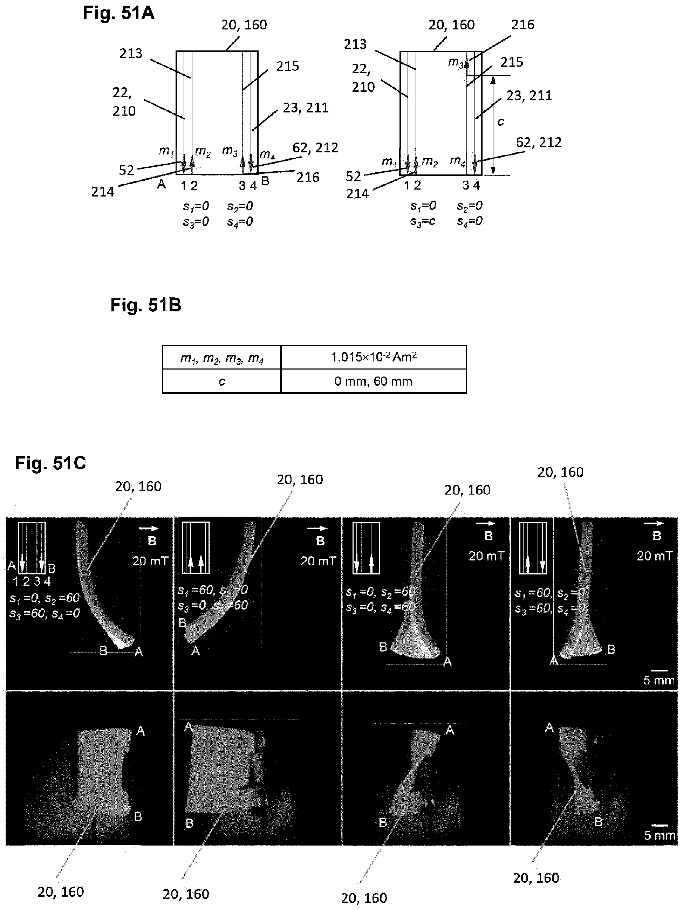

Figs. 51A to 51C depict shape change characteristics of the second configuration of the body 10 having the outer part 20 configured as a flat sheet 160 shown in Fig. 34. Fig. 51A is a schematic illustration depicting the positions of the magnetic units 52, 62, 212, 214, 216. To describe the correspondence between the front and bottom views in the deformation of the sheet, the two lower corners of the sheet are denoted as points A and B. Fig. 51B lists parameter settings for observing deformation with the magnetic units 62, 212, 214, 216 at various positions. Note that when c is 60 mm, the magnetic unit, 52, 62, 212, 214, 216 is located outside of the sheet 160 at its fixed end. Fig. 51C shows deformations with the magnetic units 52, 62, 212, 214, 216 at the various positions listed in Fig. 51B with the front view in the first row and the bottom view in the second row.

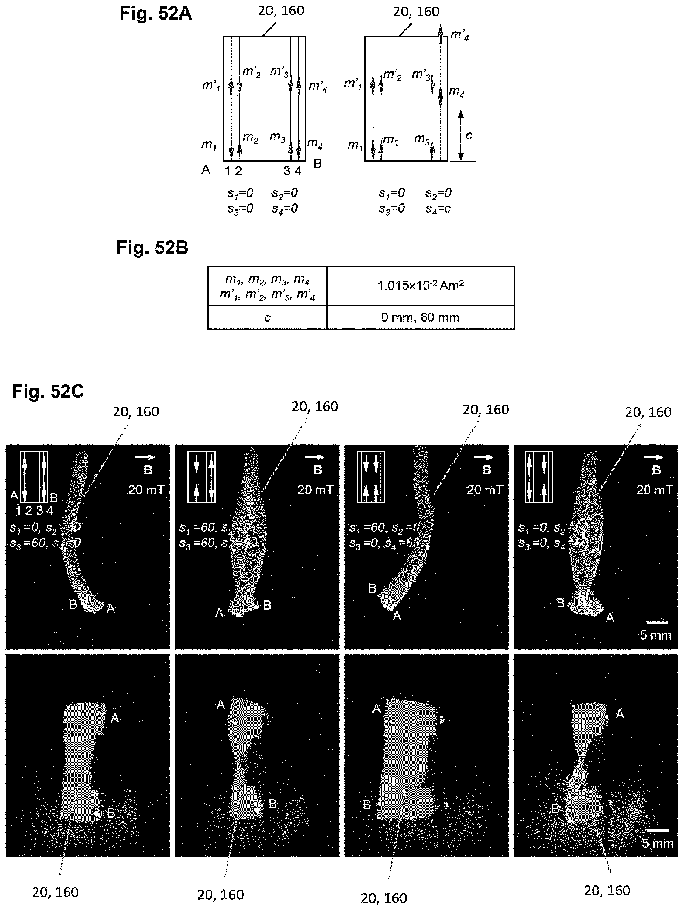

Figs. 52A to 52C depict shape change characteristics of the third configuration of the body 10 having the outer part 20 configured as a flat sheet 160 shown in Fig. 36. Fig. 52A is a schematic illustration depicting the positions of the magnetic units 52, 62, 212, 214, 216, 252, 254, 256, 258. Points A and B are used to designate the two lower corners of the sheet 160, in order to illustrate the relationship between the front and bottom views during the deformation of the sheet 160 in Fig. 52C. Fig. 52B lists parameter settings for observing deformation at various positions. Note that when c is 60 mm, the magnetic unit is located on the outside of the sheet 160 at its fixed end. Fig. 52C shows deformations with the magnetic units 52, 62, 212, 214, 216, 252, 254, 256, 258 at the various positions listed in Fig. 52B with the front view in the first row and the bottom view in the second row.

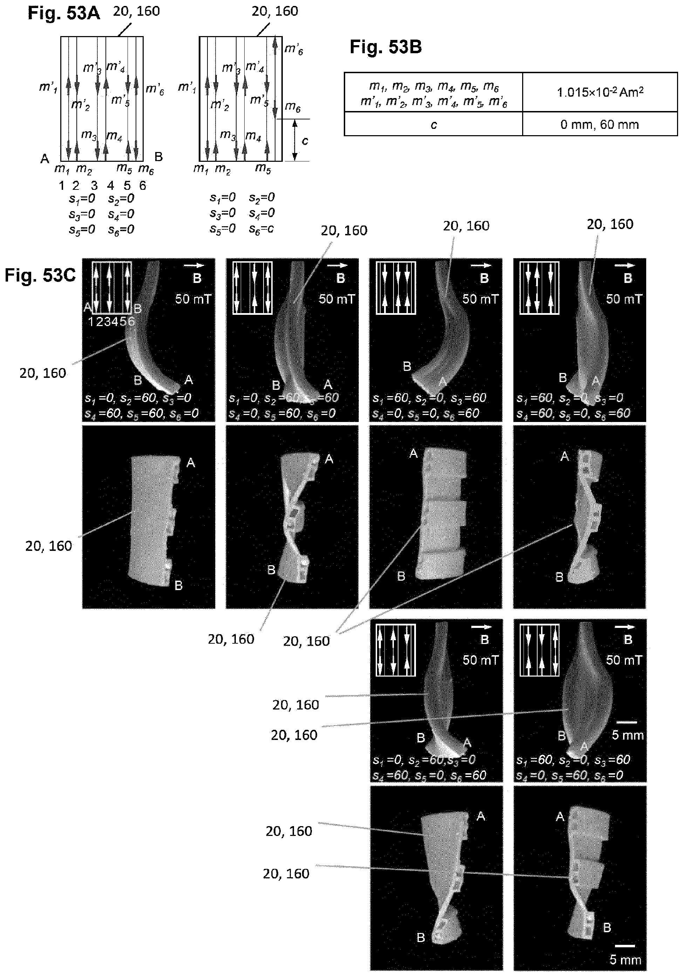

Figs. 53A to 53C depict shape change characteristics of the fourth configuration of the body 10 having the outer part 20 configured as a flat sheet 160 shown in Fig. 38. Fig. 53A is a schematic illustration depicting the positions of the magnetic units 52, 62, 212, 214, 216, 219, 252, 254, 256, 258. Points A and B are used to designate the two lower corners of the sheet 160, in order to illustrate the relationship between the front and bottom views during the deformation of the sheet 160. Fig. 53B lists parameter settings for observing deformation at various positions. Note that when c is 60 mm, the magnetic unit 52, 62, 212, 214, 216, 219, 252, 254, 256, 258 is located on the outside of the sheet 160 at its fixed end. Fig. 53C shows deformations with the magnetic units 52, 62, 212, 214, 216, 219, 252, 254, 256, 258 at the various positions listed in Fig. 53B with the front view in the first and third rows and the bottom view in the second and fourth rows.

Similarly, the method according to the present disclosure comprises 3D magnetization reprogramming. The soft robot 1 may be configured as a soft 3D box-shaped robot, and, by adjusting the layout of magnetic units 52, 62, different deformations are achieved in real time under the same magnetic field 5, see Fig. 49 at (iv) and Figs. 41 to 48. 2D and 3D magnetization reprogramming are spatial expansions of 1D magnetization reprogramming. The present disclosure tested the feasibility of these expansions by setting up several potential configurations. Considering the wide array of deformations and characteristics already demonstrated in 1D magnetization reprogramming, the 2D and 3D reprogramming methods enable a spatial expansion of these deformation characteristics, thus facilitating the emergence of diverse properties.

In connection with the present disclosure, two distinct characteristics have been discovered: magnetic neutralization and magnetic reversal.
(1) Magnetic neutralization: when two magnetic units 52, 62 with magnetic moments having identical magnitude but opposite directions (i.e., having magnetic moments: **m** and **-m)** are placed adjacent to each other, for example within the tube forming the outer part 20, their magnetic moments cancel each other out overall. In this configuration, the outer part 20, such as the tube forming the outer part, appears non-magnetized externally and the magnetic forces are effectively neutralized, thereby preventing any deformation. For example, as shown in Fig. 9C, the tube remains upright with almost no change in shape as the magnetic field 5 increases, any minor changes being attributable to manufacturing errors in the magnetic units 52, 62. Utilizing this characteristic, the tube 20 transforms substantially from a straight to a helical shape without altering the magnetic field 5, see Fig. 49 at (i) and Fig. 29A.
   Furthermore, to eliminate the influence of changes in stiffness due to the movement of internal rods, the present disclosure presents comparative experiments that verified that these changes in stiffness did not impact the deformations, see Figs. 29B and 29C. Magnetic neutralization allows for the magnetization of soft robots 1 to be selectively activated or deactivated, thereby eliminating or initiating the influence of the external magnetic field 5 on magnetic robots 1. Such control makes it possible to selectively manipulate a specific magnetic robot 1 within a magnetic field 5 that includes multiple magnetic robots 1. Potential applications include coordinated multi-instrument operation within the same magnetic field 5, see below. Additionally, when the two aforementioned magnetic units 52, 62 are moved from an overlapping position to distinct locations, the magnetization profiles of the tube forming the outer part 20 will be regenerated. These different magnetization profiles, caused by distinct locations of the magnetic units 52, 62, subsequently result in various shapes.
(2) Magnetic reversal: If the further magnetic unit 62 has a magnetic moment with twice the magnitude but opposite direction as the magnetic unit 52 forming a target magnetic unit (i.e., the magnetic moments being: -2m and m) and if the further magnetic unit 62 moves into a position adjacent to the magnetic unit 52, such as into the same axial position as the target magnetic unit 52 within a tube forming the outer part 20, the magnitude of the magnetic moment of the outer part 20 remains unchanged, yet the direction of its magnetic moment is reversed (i.e., changing from **m** to **-m**). As illustrated in Fig. 49 at (ii) and Fig. 9D, without altering the magnetic field 5, the outer part 20 changes from bending left to bending right. In the deformations shown in Fig. 49 at (ii), the degrees of bending to the left and right are not identical, which is due to changes in stiffness caused by the introduction of an inner tube forming the inner part 30. The magnetic moment of the inner tube 30, that is, the magnetic moment of the magnetic unit 52 of the inner part 30, may be set to - 2**m**+Δ**m**, where Δ**m** constitutes a margin magnetic moment that compensates for the stiffness changes resulting from the introduction of the inner tube forming the inner part 30. This ability to completely reverse the deformation direction of the tube 20 without altering the magnetic field 5 offers new possibilities for complex operations within the same magnetic field 5, see below.

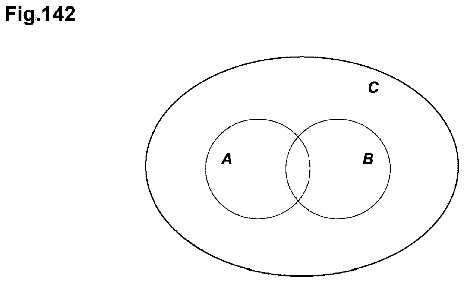

The foregoing showcased a range of deformations across 1D, 2D, and 3D magnetization programming. It thereby demonstrated that the real-time, in-situ magnetization reprogramming according to the present disclosure allows for various shape changes instantaneously and locally under the same uniform magnetic field 5. These deformations are unattainable with existing methodologies, thereby broadening the potential applications of magnetic actuation technology. As detailed below, it may be theoretically demonstrated that the force-torque space achievable by existing methods is strictly a subset of that enabled by the method according to the present disclosure when combined with a tunable magnetic field 5, highlighting the broader actuation capability of the approach according to the present disclosure (see Fig. 142 and section VIII). While the foregoing presented several configurations, additional configurations are possible tailored to specific applications. In practical applications, these configurations can be used alone or in combination, and they can also be utilized alongside varying magnetic fields 5, such as time-varying magnetic fields 5, to achieve more complex and diverse deformation modes.

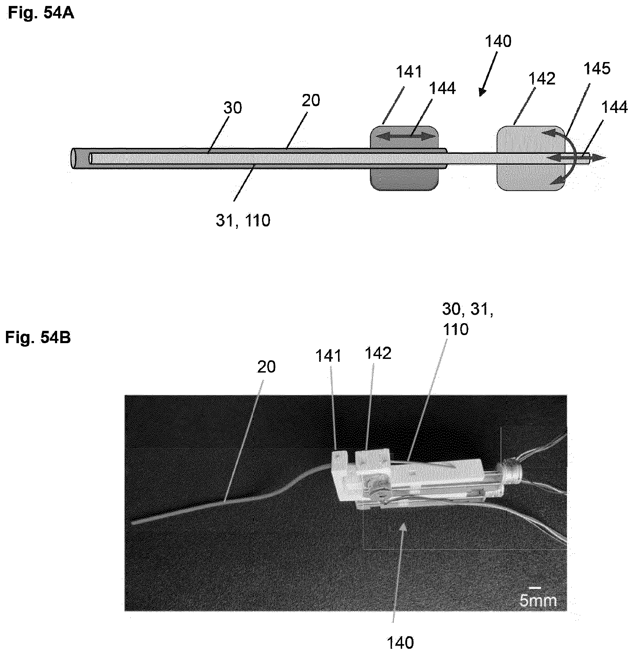

The devices and methods according to the present disclosure do not impose specific constraints on the external forces used to actuate relative motion between magnetic unit carriers or magnetic members 16. These forces can originate from a wide range of actuators 140 or from any form of stimulus capable of generating movement. One embodiment of an actuator 140 is depicted in Fig. 54. Thereby, Fig. 54A depicts a schematic diagram that illustrates the operating mechanism of the body 10. The operating mechanism comprises the actuator 140 that has the first part 141 and the second part 142, wherein the first part 141 is connected to the outer part 20 of the body 10 and the second part 142 is connected to the inner part 30 of the body 10. The first part 141 of the actuator 140 is configured to perform translational motion 144, whereas the second part 142 of the actuator 140 is configured to perform both translational motion 144 and rotational motion 146. For example, the inner part 30 may be configured to be rotated within the outer part 20. As a result, a manipulator 110 of the magnetic unit 25, which manipulator 110 may exemplarily be formed by the flexible part 31 of the inner part 30, may be rotated within the outer part 20. Naturally, changes in the tube configuration may lead to variations in the number of tubes and their motion modes, thereby necessitating corresponding modifications to the parts 141, 142 of the actuator 140. Fig. 54B depicts a photograph of an embodiment of the actuator 140.

In general, the flexible parts 31 of the inner parts 30 of all embodiments of the soft robot 1 according to the present disclosure may form manipulators 110 that are configured to transfer an actuation force from the actuator 140 to the magnetic unit 52,62.

The following subsections detail four applications to demonstrate the potential of the devices and methods according to the present disclosure, which certainly extends beyond these examples.

### Application I: Contact-free object navigation

Minimally invasive surgery (MIS) is now widely utilized due to its benefits in reducing tissue trauma, decreasing postoperative recovery time, and lowering the risk of complications (*37-39*). Common forms of MIS include vascular interventional surgery (*40-45*), laparoscopic surgery (*46-48*), endoscopic surgery (*49*, *50*)*,* and ophthalmic procedures (*51-53*). In MIS, minimally invasive instruments are externally manipulated through incisions or natural orifices to reach the target site for diagnosis or treatment (*39*).

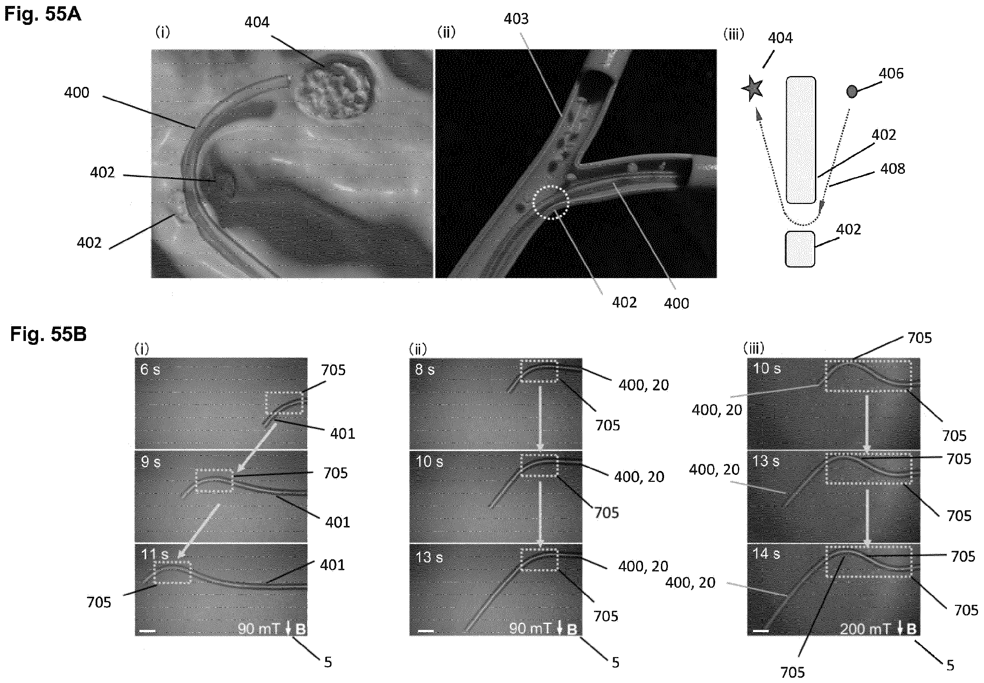

One prevalent operating technique, as depicted in Fig. 55A at (i), involves navigating a medical instrument 400, such as an endoscope or catheter, around avoidance objects 402, also called obstacles, demonstrating the flexibility of MIS. Fig. 55A thereby depicts at (i) the medical instrument 400 traversing through avoidance tissues forming obstacles 402 to reach the target area with the target 404 for treatment. Fig. 55A further depicts at (ii) a medical instrument 400 that is formed as a catheter and that navigates through blood vessels 403, whereby convex portions of the walls of the blood vessel 403 form obstacles 402 or avoidance objects.

Operation of the medical instrument 400 may be facilitated by control over the form of the instrument 400, commonly achieved through pneumatic systems (*54*), hydraulic systems (*55*), magnetic systems(*56, 26*), and tendon/cable mechanisms (*46, 57*). However, during the movement of common surgical instruments, unavoidable contact with avoidance objects or obstacles 402 may occur, leading to varying degrees of tissue damage. This is detrimental to patient recovery and may even cause complications, particularly when the avoidance tissue is already damaged (*39, 44, 58*)*.*

Specifically, for vascular intervention surgeries, see Fig. 55A at (ii), the seriousness of vascular complications makes such contact particularly noteworthy. Even in successful surgeries, contact between instruments 400 and blood vessels 403 is inevitable. Such damage is particularly concerning for older patients whose vascular physical properties are compromised (e.g., reduced elasticity of vascular walls), not only increasing their surgical risks but also limiting their options for diagnosis or treatment through vascular intervention.

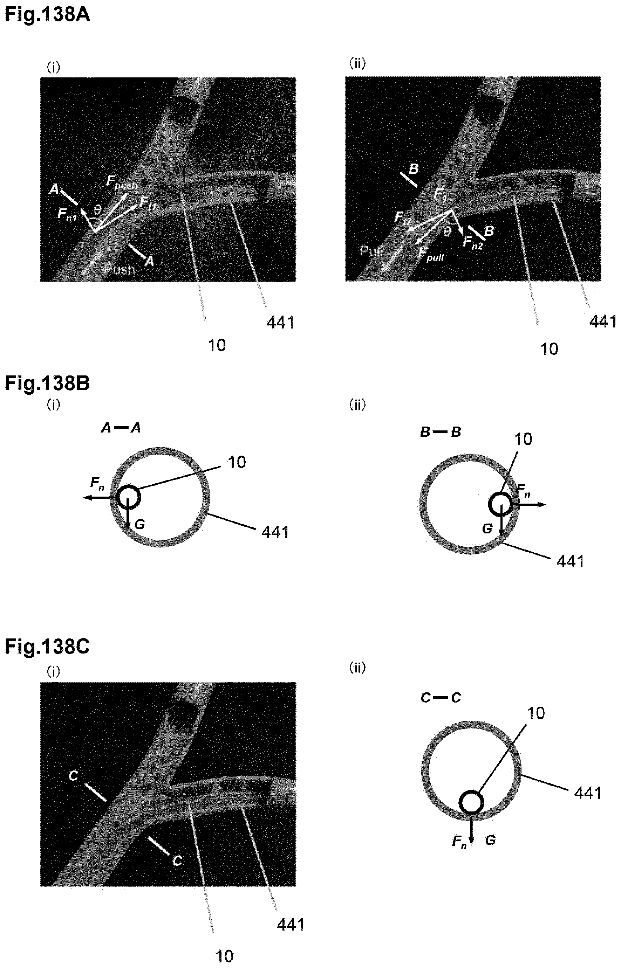

In vascular interventional surgeries, the parts of the catheter 400 that collide or contact with the vascular wall are divided into the head and body. Damage to the vascular wall by the catheter head primarily results from direct impact. The primary source of damage to the vascular wall from the catheter body is friction. This friction is less impactful in blood vessels 403 with smaller curvatures but cannot be ignored in blood vessels 403 with larger curvatures or at bifurcations. The present disclosure provides a detailed analysis and discussion on this issue below, see section V. and Fig. 138. Even by the most conservative estimates, the friction caused by this contact is 62.5 times greater than that caused by the weight of the catheter 400. Efforts must be made to eliminate this friction and ensure the catheter 400 is in an optimal contact condition, which optimal contact condition is shown in Fig. 138C.

Regarding the contact of the catheter head with the vascular wall, numerous studies have been conducted, mainly focusing on measuring the operating force at the proximal or distal end of the catheter 400 and combining it with safe operation mechanisms and algorithms (*44, 59*) to reduce vessel damage. The most common approach to mitigate the catheter body's contact with the vessel 403 involves surface treatments, such as hydrogel skin (*26*) or commercial resin coatings (*60*), to reduce friction between the catheter 400 and the vessel. However, these methods do not fundamentally eliminate such contacts.

A study in literature (*61*) introduces an advanced method of manipulating magnetization combinations to alter its shape near obstacles, enabling the catheter to traverse them, a significant improvement over previous research. However, due to the limitation that its magnetization profile cannot freely adjust to positional changes, the bent part of the catheter is influenced by its forward or backward movements, and thus lacks independent motion. As depicted in Fig. 55B at (i), the bent section or deformation 705 moves along with the overall motion under the unidirectional magnetic field 5, which still fails to address the issue fundamentally. In addition, a technique that combines magnets with low-melting-point alloys has been developed (*62*, *63*)*.* This approach uses magnetic forces to deform the tube and leverages the alloy's solidification and melting processes to maintain or reverse the tube's shape. The technique shows the potential to reduce vascular damage. However, heating and cooling within a sealed chamber is time-consuming, which precludes real-time operation, and also raises concerns about excessive heat accumulation.

To address the limitations of existing research, a method for operating a soft robot according to the present disclosure is exemplarily based on the principles of the third configuration of the soft robot 1 having a cylindrical outer part 20 for 1D magnetization reprogramming, as shown in shown in Figs. 13 to 16. This method leverages the shape retention capabilities of the third configuration to fundamentally eliminate the damage to tissue at bends 705 by preventing contact of the instrument 400 with avoidance objects 402. The outer tube forming the outer part 20 of the instrument 400 may comprise or consist of Polydimethylsiloxane (PDMS) and, for ease of manufacture, rods may replace the inner tubes forming the inner parts 30. With other embodiments, the inner parts 30 may also be fabricated as hollow elongated members, such as additional tubes.

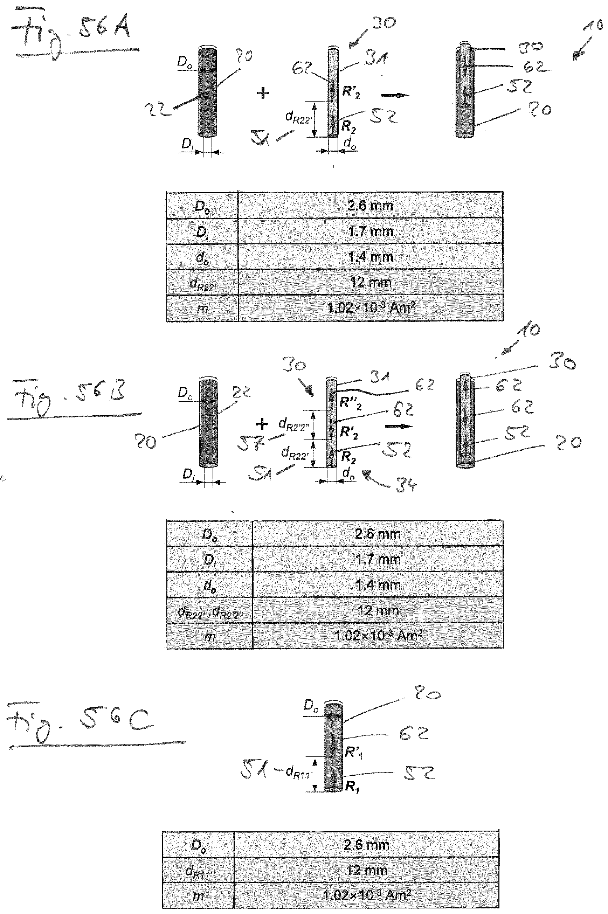

Figs. 56A and 56B depict embodiments of inner parts 30 and outer parts 20 that form components of a body 10 of a soft robot 1 having shape retention capabilities. The embodiment shown in Fig. 56A is configured as it is disclosed for the third configuration shown in Fig. 13. The table included in Fig. 56A lists exemplary parameter values of this configuration. Fig. 56B depicts an alternative configuration, in which the inner part 30 comprises the magnetic unit 52 and two further magnetic units 62 that are arranged, one after the other, along the flexible part 31. The magnetic unit 52 is placed at the first end 34 of the inner part 30 and a first one of the further magnetic units 62 have the distance 29 and the first one of the further magnetic units 62 and the second one of the further magnetic units 62 have a distance 57, which exemplarily equals the distance 51. The magnetic unit 52 and the further magnetic units 62 have magnetic moments with alternating directions.

Fig. 56C depicts a reference configuration of a control setup that lacks the inner part 30 having the magnetic units 52, 62 and only comprises an outer part 22 to which the magnetic unit 52 and the further magnetic unit 62 are attached spaced at the distance 51.

By nesting the inner rods forming the inner parts 30 within the outer tubes 20, the bodies 10 are assembled, wherein each body 10 forms an elongated tube configured as a medical instrument 400, namely an endoscope or catheter. To achieve different deformations, two types of tube configurations were employed: the first configuration of Fig. 56A features an inner part 30, such as a tube or a rod, with two magnetic units 52, 62, while the second configuration of Fig. 56B includes an inner part 30, such as a tube or a rod, with three magnetic units 52, 62.

In Fig. 55B at (i), under a uniform magnetic field 5 of 90 mT directed downward, the soft tube 20 of the control setup of Fig. 56C, which soft tube forms a prior art instrument 401, was pushed forward or backward, and its bend or deformation 705 moved with it, preventing it from bypassing avoidance objects 402 without contacts. In contrast, with the method according to the present disclosure and with the first configuration shown in Fig. 56A, the bend or deformation 705 in the soft tube forming the outer part 20 remains stationary with respect to a surrounding of the soft robot 1 regardless of whether the soft tube forming the outer part 20 is moved forward or backward with respect to the inner part 30, see Fig. 55B at (ii). This feature of the method according to the present disclosure facilitates contactless navigation around avoidance objects or obstacles 402 because the avoidance objects 402 remain static, and the deformation 705 of the outer part 20 is configured to also stay stationary during movement of the outer part 20. Additionally, Fig. 55B shows at (iii) a soft tube having the body 10 according to the second configuration shown in Fig. 56B with three magnetic units 52, 62 that form two bent sections or deformations 705 within the magnetic field 5, enabling it to bypass more intricate avoidance objects or obstacles 402 without contact.

With the movements of the body 10 shown in Fig. 55B at (ii) and (iii), the outer part 20 forming the target member 15 is moved relative to the inner part 30 forming the magnetic member 16, whereby the inner part 30 is stationary with respect to the surrounding of the body 10. This results in the deformation 705 of the outer part 20 remaining at the same position with respect to the environment.

Fig. 55A schematically shows at (iii) a possible application of the method according to the present disclosure having further steps to bypass between two obstacles 402, as shown in Fig. 55A at (i). The soft tube forming the body 10 is controlled to navigate a designated path 408 from a starting point 406 around the avoidance object 402 to a target 404 without contact with the avoidance object 402. During this process, the magnetization profile of the body 10 is modified in real-time, exemplarily by moving the magnetic member 16 with respect to the target member 15.

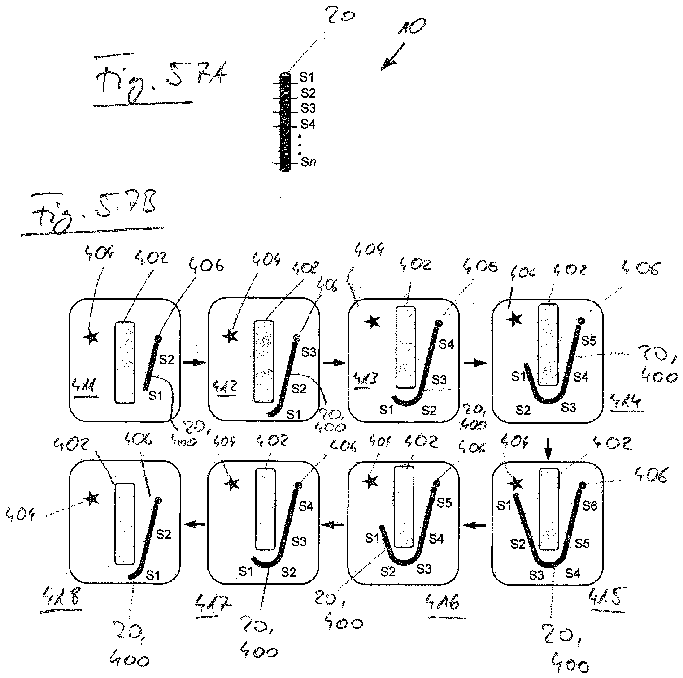

Thereby, the soft tube forming the body 10 and therefore also the outer part 20 has multiple small segments, labeled as S1, S2, ... Sn, as shown in Fig. 57A. Each segment may have a first state 421 or a second state 422, wherein the first state 421 is a non-magnetic state, resulting in no bending in the magnetic field 5, and the second state 422 is a magnetic state, causing bending in the magnetic field 5. Fig. 57B illustrates the outer part 20 bypassing the avoidance object 402 depicted in Fig. 55A at (iii) in a sequence divided into eight steps 411, 412, 413, 414, 415, 416, 417, 418. The outer part 20 thereby is exemplarily divided into four segments S1, S2, S3, S4. Correspondingly, different states 421, 422 are set in the segments S1, S2, S3, S4 of the soft tube forming the outer part 20 at each step 411, 412, 413, 414, 415, 416, 417, 418, whereby the states 421, 422 are set by moving the inner part 30 with respect to the outer part 20.

The medical instrument 400 advances to reach the position of the target 404 in a first step 411, a second step 412, a third step 413, a fourth 414, and a fifth 415 and then retracts again in a sixth step 416, a seventh step 417, and a third step 418. During steps 411, 412, and 413, the inner part 30 and the outer part 20 are both moved together with respect to the surrounding so that the inner part 30 and the outer part 20 do not move relative to each other. In steps 414, 415, and 416, the inner part 30 remains stationary with respect to the surrounding and the outer part 20 is moved. Thereby, the inner part 30 moves relative to the outer part 20. In steps 414, 415, and 416, the outer part 20 is exemplarily moved towards the target 404. During the remaining steps 417 and 418, the inner part 30 and the outer part 20 are moved together with respect to the surrounding so that there is no relative movement between the inner part 30 and the outer part 20.

As can be seen from Fig. 57B, the method according to the present disclosure may comprise a step of moving the target member 15 and the magnetic member 16 together with respect to a surrounding of the body 10 and a further step of keeping the magnetic member 16 stationary with respect to the surrounding and moving the target member 15 with respect to the surrounding. Thereby, only the magnetic member 16 may comprise magnetic units and the target member 15 may be free from magnetic units. Additionally or alternatively, the magnetic member 16 may be formed by the inner part 30 and the target member 15 may be formed by the outer part 20.

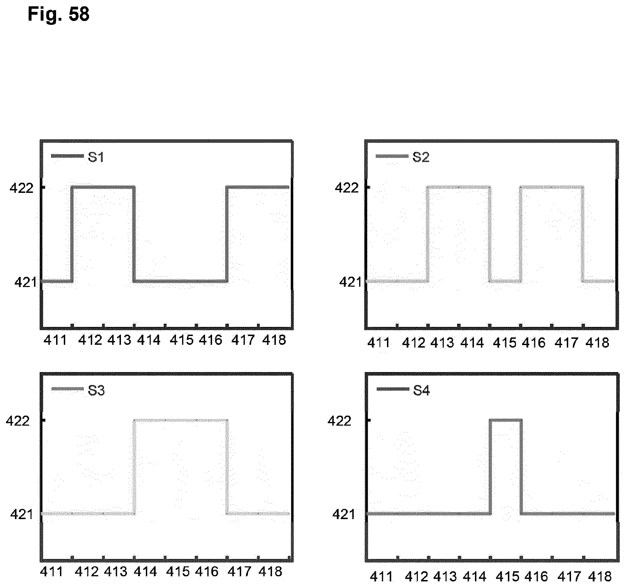

Fig. 58 illustrates the state changes in the four segments of the soft tube 20 employed for moving the outer part 20 along the designated path 408, whereby Fig. 58 is a diagram of the magnetization states 421, 422 of each segment S1, S2, S3, S4 of the medical instrument 400 during the process of passing the avoidance object 402 as depicted in Fig. 55 at (iii) and Fig. 57B. The segments S1, S2, S3, and S4 correspond to the four sections of the medical instrument 400 as shown in Fig. 57B.

If a segment S1, S2, S3, and S4 is in the first state 421, the magnetic units 52, 62 of the inner part 30 are placed away from the corresponding segment S1, S2, S3, and S4 and, if a segment S1, S2, S3, S4 is in the second state 422, at least one of the magnetic units 52, 62 is placed within the corresponding segment S1, S2, 3, S4. The corresponding segment S1, S2, S3, S4 has a shape that is straight in the first state 421 and a shape that is bent in the second state 422.

Using the existing method employing the control setup, it is challenging to achieve real-time, in-situ modifications of the magnetization profile, thereby hindering the completion of the aforementioned task. Although theoretically possible through the superposition of multiple magnetic fields 5, this approach is overly complex and primarily of theoretical interest, with limited practical applicability and no documented cases in existing literature.

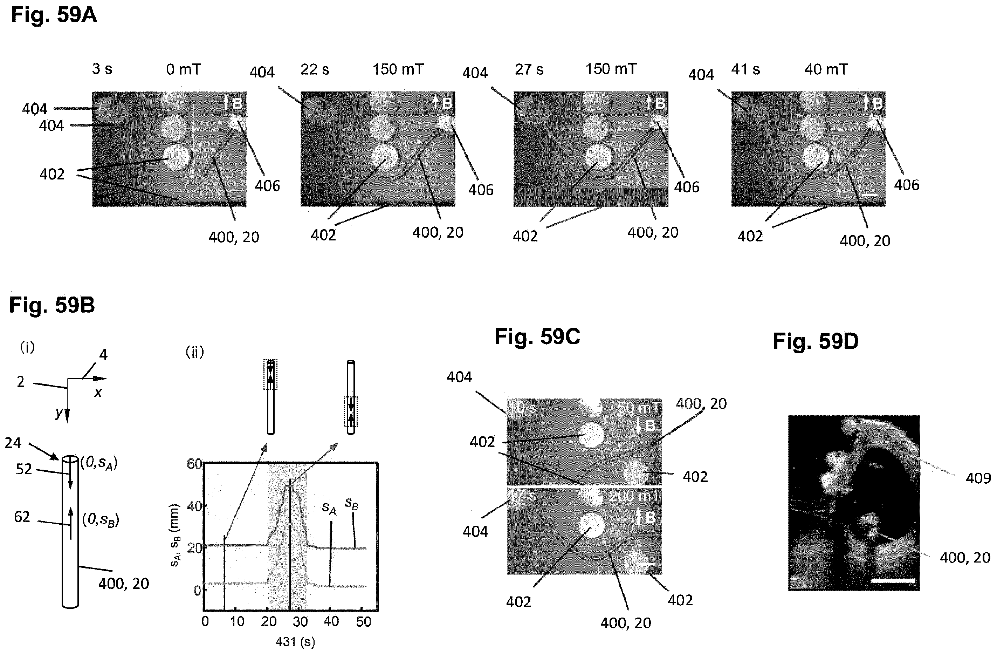

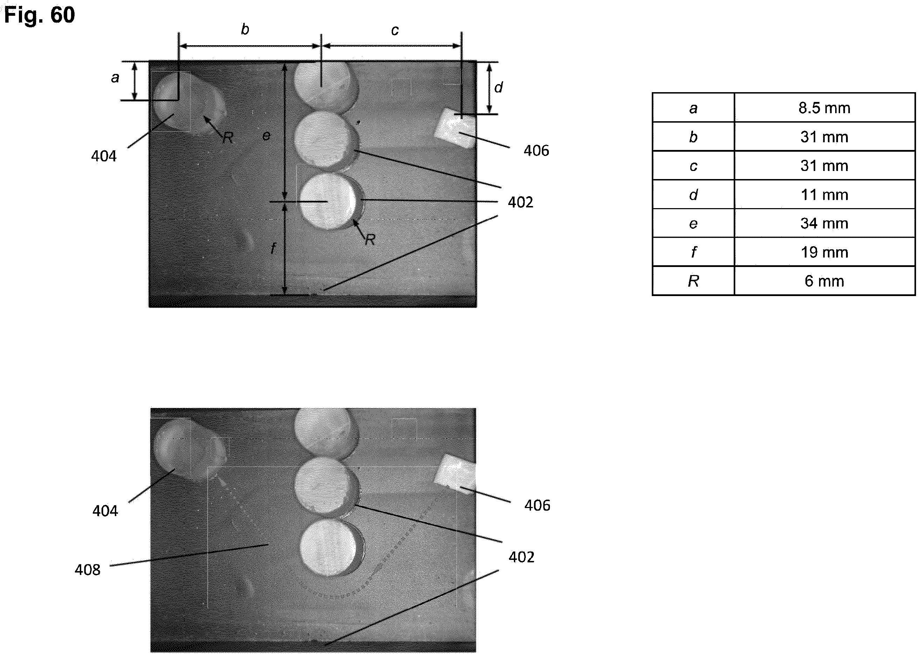

Fig. 59A depicts a setup based on Fig. 55A at (iii), with specific dimensional parameters provided in Fig. 60. Using existing methods employing the configuration of the control setup of Fig. 56C, contacts with the avoidance objects 402 are unavoidable. However, by employing the method according to the present disclosure with the first configuration of Fig. 56A, the body 10 is configured for contact-free navigation around the avoidance object 402 to reach the location of the target 404, see Fig. 59A. Fig. 59B defines at (i) distances s_{A} and s_{B} of the two magnetic units 52, 62 in the soft tube forming the outer part 20 to the free first end 24 of the outer part 20 to describe the changes in the magnetization profile upon performing the method. As can be seen from Fig. 59B at (ii), the body 10 is configured to change the position of magnetic units 52, 62 and the magnetization profile in real-time with the movement of the outer part 20 with respect to the inner part 30, as highlighted in the shaded area in Fig. 59B at (ii), whereby the diagram indicates time 431 on the horizontal axis and the distances s_{A} and s_{B} on the vertical axis. Thereby, a relative position (*s_{A} - s_{B}*) of the two magnetic units 52, 62 remains unchanged, enabling the possibility of navigating around the avoidance object 402. The shaded area thereby comprises steps 414, 415, 416 shown in Fig. 57B.

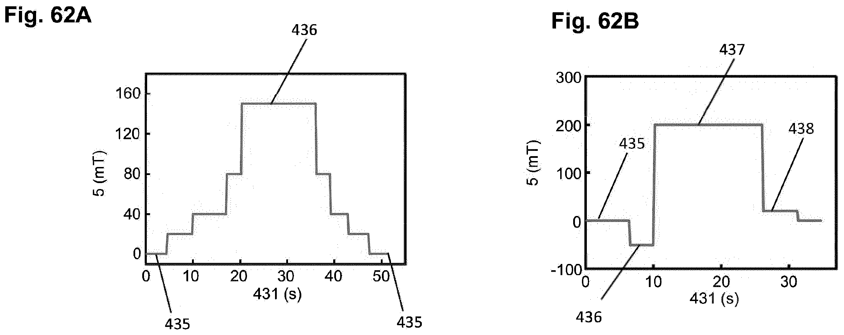

In addition to moving the magnetic member 16 and the target member 15 with respect to each other for actuation of the soft robot 1, the soft robot 1 may also be configured to alter the magnetic field 5 for inducing movement of the body 10. This is exemplarily depicted in Fig. 62A, which shows the magnitude of the magnetic field 5 over time 431 as the target member 15 formed by the outer part 20 and the magnetic member 16 formed by the inner part 30 move relative to each other, as depicted in Fig. 59 at (ii). The magnetic field 5 is switched from a first magnetic field 435, which exemplarily is zero, to a second magnetic field 436, which exemplarily is different from zero. The body 10 is first moved within the first magnetic field 435 and then within the second magnetic field 436. Exemplarily, the body 10 is first moved within the first magnetic field 435 during the first step 411 shown in Fig. 57B. The body 10 is then moved, while ramping the magnetic field 5 gradually from the first magnetic field 435 to the second magnetic field 436, exemplarily during the second steps 412 and the third step 413. With the exemplary embodiment, the target member 15 exemplarily formed by the outer part 20 and the magnetic member 16 exemplarily formed by the inner part 30 are moved relative to each other within the second magnetic field 436 during the fourth step 414, the fifth step 415, and the sixth step 416. During the seventh step 417, and the eight step 418, the target member 15 and the magnetic member 16 are moved together without relative motion while gradually decreasing the magnetic field 5 from the second magnetic field 436 to the first magnetic field 435.

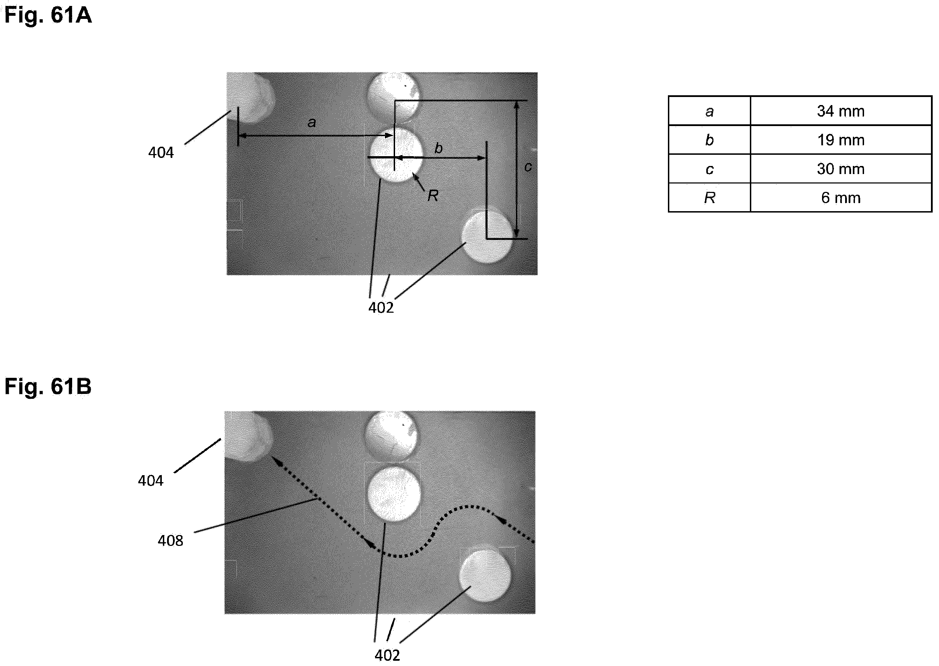

Fig. 61A and Fig. 61B depict a more complex environment, in which the soft robot 1 according to the present disclosure is configured to navigate. Fig. 61A thereby depicts the layout of the environment with the target object 404 and the obstacles 402, as well as a table with dimensions of the environment. Fig. 61B depicts the path 408 along which the outer part 20 of the soft robot 1 navigates according to the method according to the present disclosure. The body 10 thereby has the second configuration shown in Fig. 56B.

As shown in Fig. 59C, the outer part 20 is configured to navigate around the obstacles 402 by advancing the outer part 20 with the inner part 30 remaining stationary with respect to the outer part 20 in a first magnetic field 435, the first magnetic field 435 exemplarily being zero. The outer part 20 and the inner part 30 are then positioned at a first obstacle 402 and the magnetic field 5 is switched from the first magnetic field 435 to a second magnetic field 436, which exemplarily has a magnitude of 50 mT in a first direction, causing a deformation and bending of the outer part 20. The outer part 20 and the inner part 30 are then moved until they are located at a second obstacle 402 and the magnetic field 5 is switched from the second magnetic field 436 to a third magnetic field 437. The third magnetic field 437 has a second direction, which is different from the first direction, namely opposite to the first direction, and a magnitude different from the magnitude of the second magnetic field 436, for example larger than the magnitude of the second magnetic field 436, exemplarily of 200 mT. Subsequently, the method comprises moving the outer part 20 while keeping the inner part 30 stationary with respect to the surrounding in the third magnetic field 437. The outer part 20 is thereby moved to the target 404 while keeping the inner part 30 stationary within the third magnetic field 437. The outer part 20 then may be retracted while still keeping the inner part 30 stationary within the third magnetic field 437. In a subsequent step, the magnetic field 5 may be switched to a fourth magnetic field 438 while keeping the outer part 20 and the inner part 30 stationary with respect to the surrounding. Subsequently, the inner part 30 and the outer part 20 may be retracted together without relative movement within the fourth magnetic field 438. Fig. 62B depicts the magnetic field 5 during the movement of the body 10 according to the method depicted in Fig. 59C.

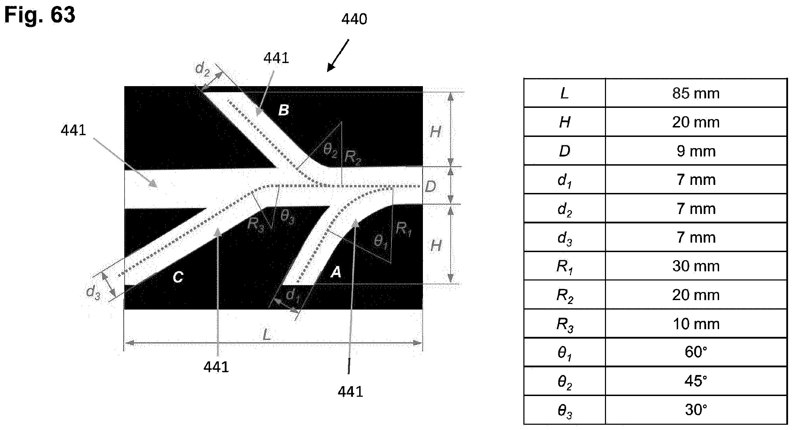

Fig. 63 depicts a vascular system having blood vessel 441 with three branches A, B, C with different radii of curvature R₁, R₂, R₃ and bending angles θ₁, θ₂, θ₃. By applying a magnetic field 5 with the same direction and gradually changing magnitude B, the soft tube forming the outer part 20 enters and exits the three branches A, B, C without any contacts, achieving the ideal contact scenario discussed in section V. below, where there is no contact with the sides and only contact with the bottom of the vessel 441.

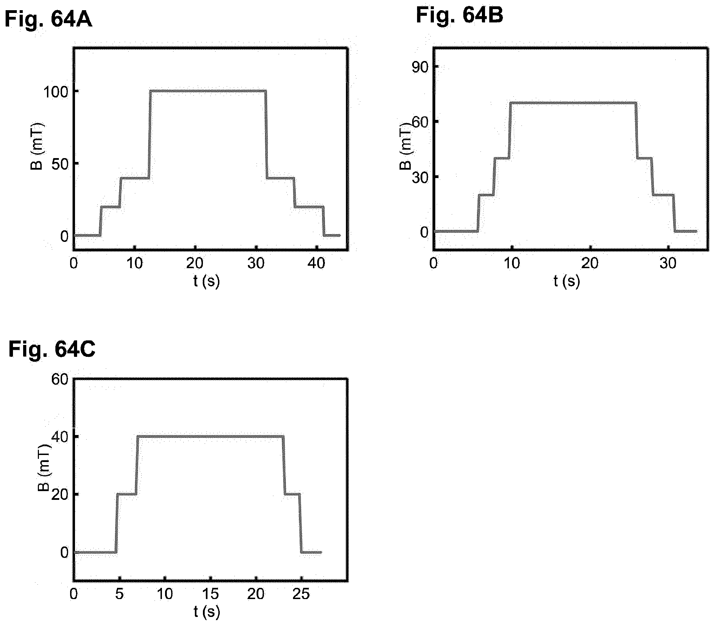

Fig. 64A depicts the magnetic field 5, denoted as B, over time t for moving the outer part 20 into and out of branch A, Fig. 64B depicts the magnetic field 5 over time t for moving the outer part 20 into and out of branch B, and Fig. 64C depicts the magnetic field 5 over time t for moving the outer part 20 into and out of branch C.

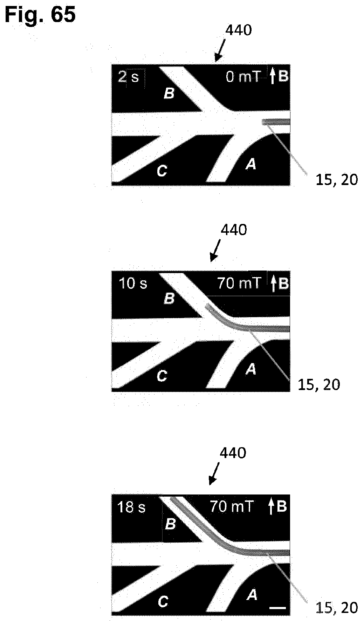

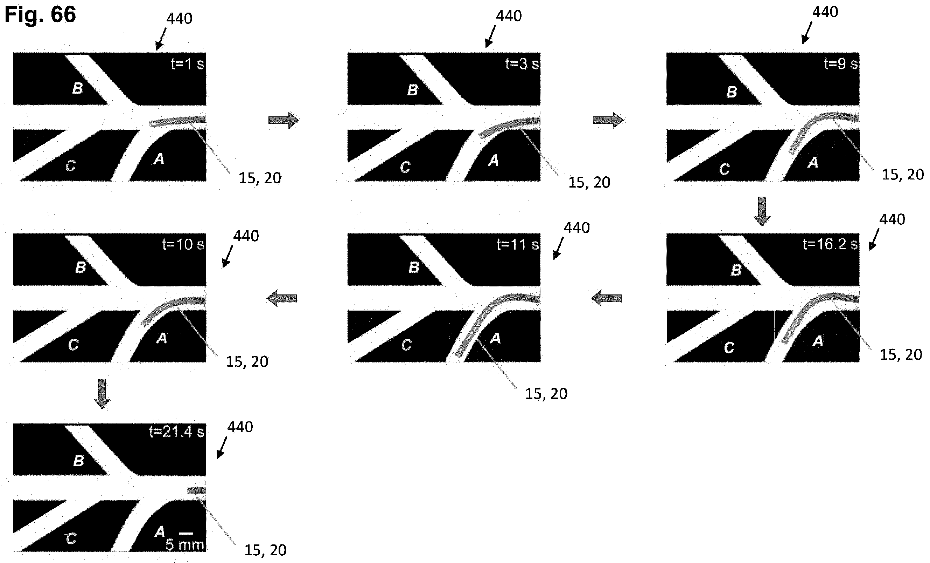

Furthermore, Fig. 65 depicts different positions of the target member 15 formed by the outer part 20 upon moving into branch B. Likewise, Fig. 66 depicts different positions of the target member 15 upon moving into and out of branch A.

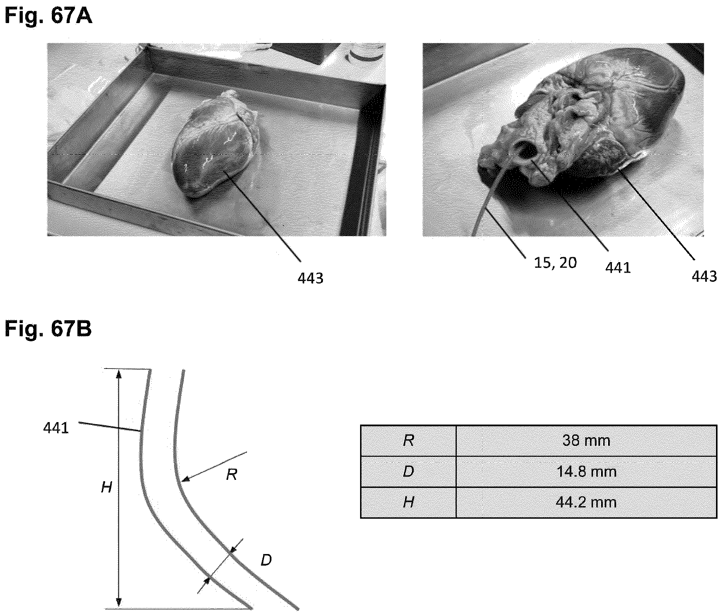

Fig. 67A depicts introduction of the soft robot 1 according to the present disclosure into a vessel 441 of a porcine heart 443 in an *ex vivo* experiment. Fig. 67B depicts the dimension of the vessel 441. The soft robot 1 having the outer part 20 thereby is configured to navigate through the vessel 441 without any contact to the walls of the vessel 441 by using the method according to the present disclosure.

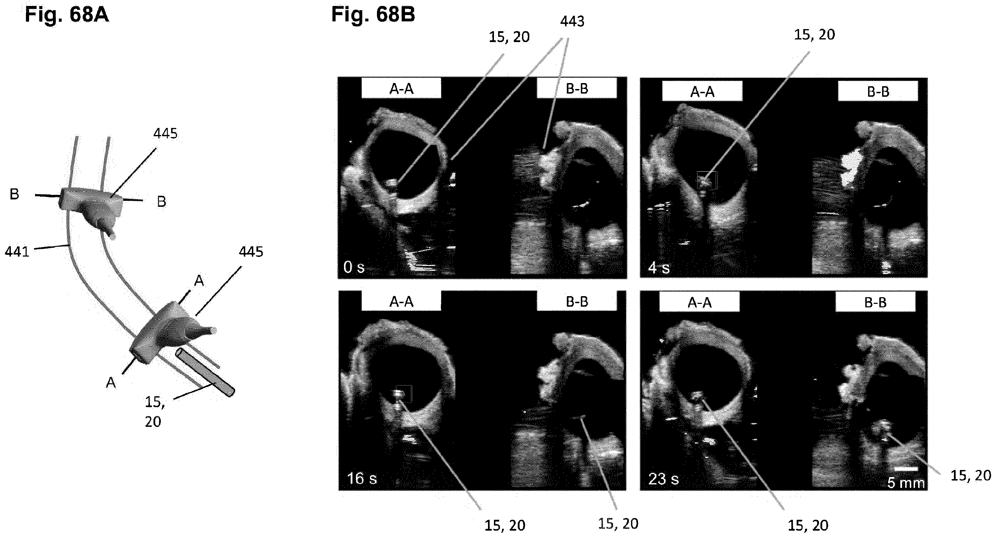

The movement of the outer part 20 through the vessel 441 may be monitored by an ultrasonic probe 445. Fig. 68A schematically depicts two positions of the ultrasonic probe 445 within scan planes A-A and B-B perpendicular to the axis of the vessel 441, whereby a bent of the blood vessel 441 is located in between scan planes A-A and B-B. The target member 15 formed by the outer part 20 is moved within the blood vessel 441 and is thereby passing first scan plane A-A and then scan plane B-B. Fig. 68B depicts pictures taken by the ultrasonic probe 445 within planes A-A and B-B. Initially, the outer part 20 appears only in scan plane A-A. Later, the outer part 20 is observed in both scan planes A-A and B-B, indicating the movement of the outer part 20 from scan planes A-A to B-B as shown in Fig. 68A. The pictures demonstrate that the outer part 20 does not collide with the walls of the vessel 441 on either side. The gravity of the outer part 20 may be counteracted by the reaction force of the vessel 441, resulting in the outer part 20 contacting the lower side of the vessel 441. This corresponds to the ideal state described in section V. below, thus preventing damage to the vessel 441.

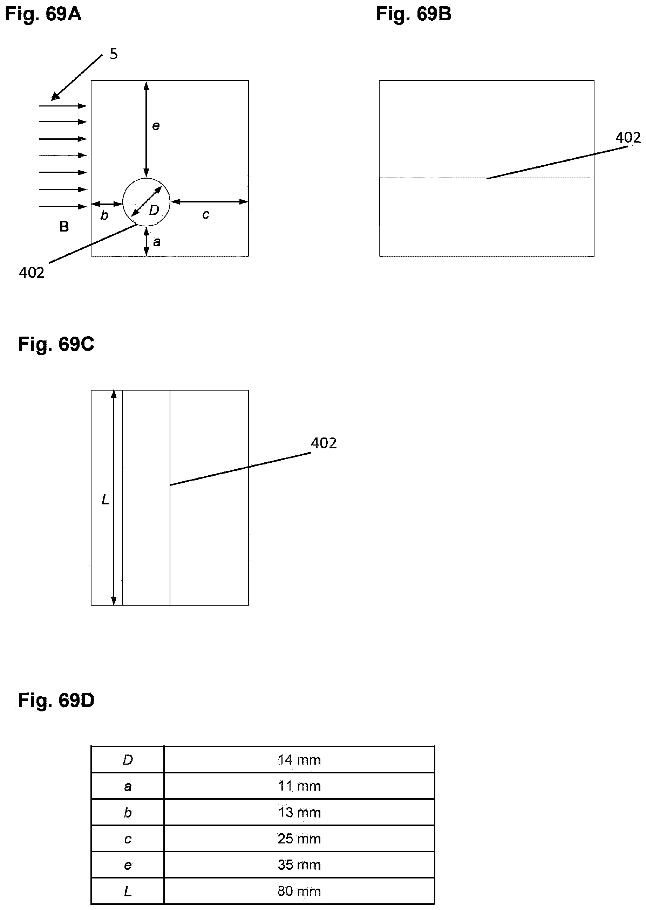

The soft robot 1 according to the present disclosure may also be configured for 3D contact-free object navigation. Fig. 69 depicts an environment for traversing a single obstacle 402 with a three-dimensional movement of the target member 15. Within the environment, the magnetic field 5 is unidirectional and orientated from left to right in the front view shown in Fig. 69A. Fig. 69B depicts a side view of the environment and Fig. 69C depicts a top view of the environment. Furthermore, Fig. 69D lists exemplary parameters of the environment and the obstacle 402.

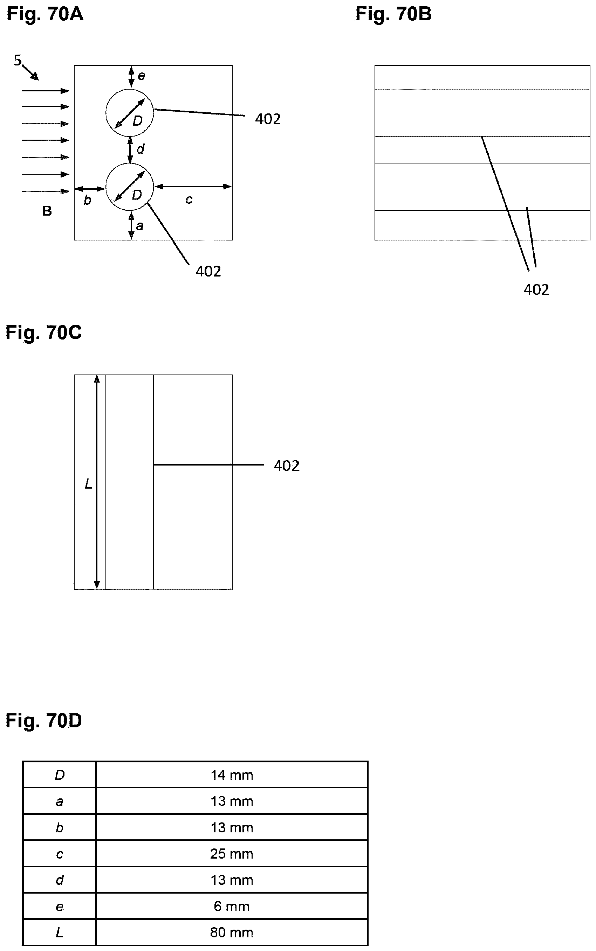

Fig. 70 depicts an environment for traversing two obstacles 402 with a three-dimensional movement of the target member 15. Within the environment, the magnetic field 5 is unidirectional and orientated from the left to right in the front view shown in Fig. 70A. Fig. 70B depicts a side view of the environment and Fig. 70C depicts a top view of the environment. Furthermore, Fig. 70D lists exemplary parameters of the environment and the obstacles 402.

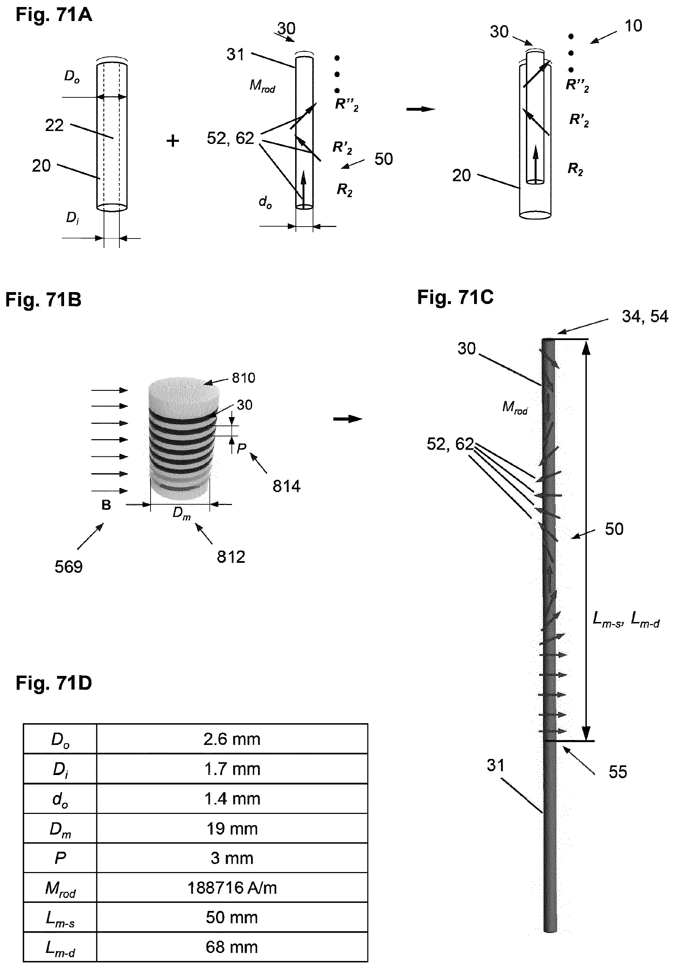

Fig. 71 depicts an exemplary design of the body 10 for 3D navigation. The body 10 has the outer part 20 formed as a tubular member with a channel 22 and the inner part 30, which may be formed as a solid rod or a further tubular member. As far as no differences are disclosed in the description and the Figures, the design of the body 10 is the same as the design of the seventh configuration of the body 10 shown in Figures 26 to 28. With the design of Fig. 71, the outer part 20 does not comprise any magnetic unit and the inner part 30 comprises the magnetic element 50 already described in connection with Figures 26 to 28. Fig. 71A depicts the outer part 20, the inner part 30 and the assembled body 10. Fig. 71B depicts the jig 810 for imparting a helical magnetic moment onto the magnetic element 50, the jig 810 having a pith 840 and a diameter 812. Fig. 71C depicts the inner part 30 after removal from the jig 810, wherein the magnetic moment of the magnetic element 50 continuously changes from the first end 54 to the second end 55. The magnetic element 50 thereby has a length *Lₘ₋ₛ* in a version for traversing the single obstacle 402 shown in Fig. 69 and a length *L_{m-d}* in a version for traversing the two obstacles 402 shown in Fig. 70. Fig. 71D lists exemplary parameters of the jig 810 and the body 10. The variation in the lengths *Lₘ₋ₛ, L_{m-d}* results in distinct magnetization profiles, which in turn lead to different deformations.

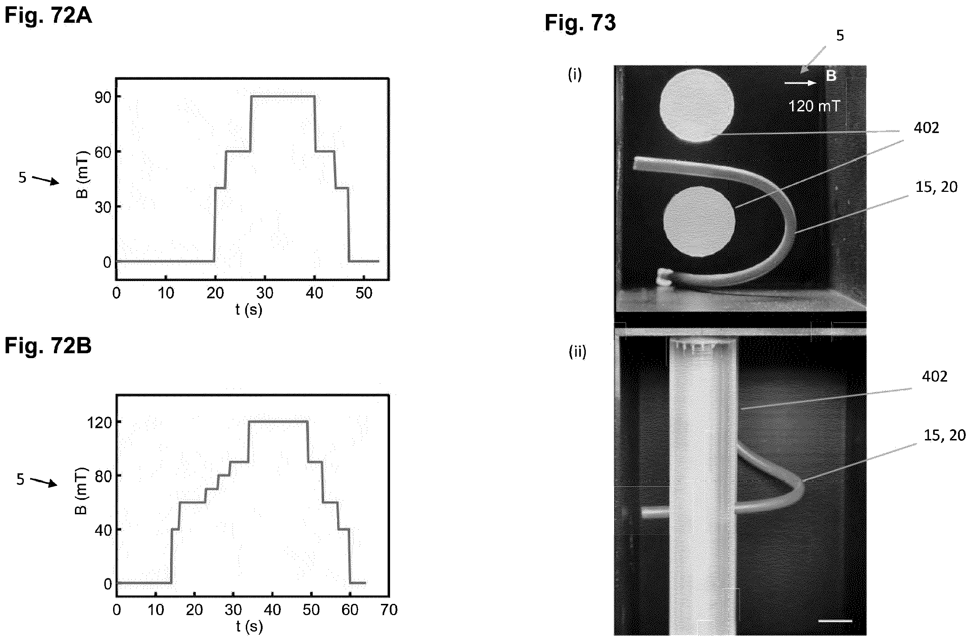

The variant of the body 10 shown in Fig. 71 having the length *Lₘ₋ₛ* is configured to move around the obstacle 402 of the environment having the single obstacle 402, as shown in Fig. 69 without contact to the obstacle 402 in a single magnetic field 5 having a constant direction. During movement of the outer part 20 of the body 10, the magnitude of the magnetic field 5 is varied, as shown in Fig. 72A. Furthermore, the variant of the body 10 shown in Fig. 71 having the length *L_{m-d}* is configured to move around the obstacles 402 of the environment having two obstacles 402, as shown in Fig. 70 without contact to the obstacles 402 in a single magnetic field 5 having a constant direction. During movement of the outer part 20 of the body 10, the magnitude of the magnetic field 5 may be varied, as shown in Fig. 72B. With both variants shown in Fig. 72A, 72B, the direction of the magnetic field 5 remains constant, while its magnitude was gradually varied to facilitate smooth deformation of the outer part 20 forming the target member 15.

Fig. 73 depicts the target member 15 in an end position within the environment having two obstacles 402 shown in Fig. 70. Upon moving into the end position, the target member 15 had been moved while undergoing a deformation in three dimensions within the unidirectional magnetic field 5 shown in Fig. 72B. The target member 15 has spiraled around one of the obstacles 402 and moved through a slot in between the two obstacles 402. Fig. 73 depicts the front view corresponding to Fig. 70A at (i) and the top view corresponding to Fig. 70B at (ii).

With other applications, the soft robot 1 may be configured to have a larger operational length, a larger number of bends, differing radii of curvature, such as smaller radii of curvature, and/or a higher number of layers or nested inner parts 30, 40. The soft robot 1 then may have features designed according to four new methods - namely, a segmented design method, a multi-magnetic field control method, a multi-bend deformation design method, and a zone-based tube nesting design method - targeting long-range operation, multi-bend deformation, high-curvature deformation, and multi-nested tube operation, respectively.

For example, regarding the long-range operation, the body 10 may have a tubular shape with a length of, for example, more than 100 mm, such as more than 200 mm, such as more than 300 mm, for example 305 mm, and may be configured to be manipulated while achieving multiple bending deformations. This length can be further increased, for example to at least 400 mm, at least 500 mm, at least 750 mm, or at least 1000 mm, such as 1240 mm. Thereby, a stiffness of the outer part 20 and the inner parts 30, 40, which may be tubular, may be enhanced along the length. For example, commercial catheters may be integrated into the body 10 for manipulation, which may result in an operational length of the body 10 of at least 500 mm, at least 750 mm, or at least 1000 mm, such as 1240 mm. With other embodiments, the length of the body 10 may be at least 1500 mm, at least 2000 mm or at least 3000 mm. The aforementioned operational distances essentially satisfy most application requirements. The detailed designs, tuning strategies, and related discussions of these methods are presented below.

By real-time, in-situ adjustment of the magnetization profile of the body 10 of the soft robot 1, the body 10 may maintain a constant magnetization profile at the location of the avoidance object 402 as it moves forward, ensuring the same curved shape as the avoidance object 402. This allows the soft tube forming the body 10 to avoid contact with the avoidance object 402. Traditional methods struggle to achieve this manipulation; however, the devices and methods according to the present disclosure achieve contact-free navigation in a straightforward and effective manner. In applications, for example, in interventional surgery, the method according to the present disclosure can eliminate damage to blood vessels 403 or tissue caused by the catheter or endoscope at bent sections, significantly enhancing the safety of the procedure. Additionally, devices and methods according to the present disclosure have the potential to reduce the health requirements for patients undergoing vascular interventions, particularly for elderly patients with fragile vessel walls. Furthermore, the devices and methods according to the present disclosure, which avoid contact with the surrounding environment, can also be applied to other medical diagnostic and treatment methods as well as to industrial applications.

### Application II: Reprogrammable cilia array

The ubiquitous cilia in nature play a paramount role in various creatures, including human beings, due to their essential functionalities such as cerebrospinal fluid transportation (*64*), mucus clearance (*65*), egg cell delivery (*66*), self-propulsion (*67*), food capture (*68*), as well as self-cleaning (*69*). Inspirations were lever-aged to create artificial cilia for versatile on-chip integrated microfluidic pumps and mixers, controlled particle and droplet manipulators, self-cleaning and antifouling surfaces, environmental information sensors, and soft robots 1(*70-72*)*.* Although dramatic progress has been made in the field of artificial cilia (*73-77*)*,* there is still a long way to go for the real application of artificial cilia in areas including microfluidics, chemical engineering, biological engineering, biomedical science, and robotics. The primary reason is the lack of in-situ reprogrammability of existing artificial cilia targeting desired reconfigurable fluid and particle/cell manipulation capabilities as well as reprogrammable locomotion capacities that are tunable on the fly using a simple and efficient strategy. For example, programmable magnetic artificial cilia could only generate specific flow patterns using predefined cilia coordination or phase shift (*77-79*). Moreover, electrically actuated artificial cilia are either cost-intensive or require a cumbersome sophisticated actuation platform inhibiting their actual application despite their ability to induce reconfigurable flow patterns (*73, 74*).

To address the aforementioned issues, the devices and methods according to the present disclosure may provide for reprogramming cilia, such as cilia based on the first configuration of the body 10 shown in Figures 3 to 6 or cilia based on the second configuration of the body 10 shown in Figures 7 to 12. These configurations provide three possibilities for reprogramming cilia: targeted cilia activation, ciliary bending amplitude modulation, and ciliary phase modulation. These possibilities can be employed individually as well as in combination to achieve more complex functionalities.

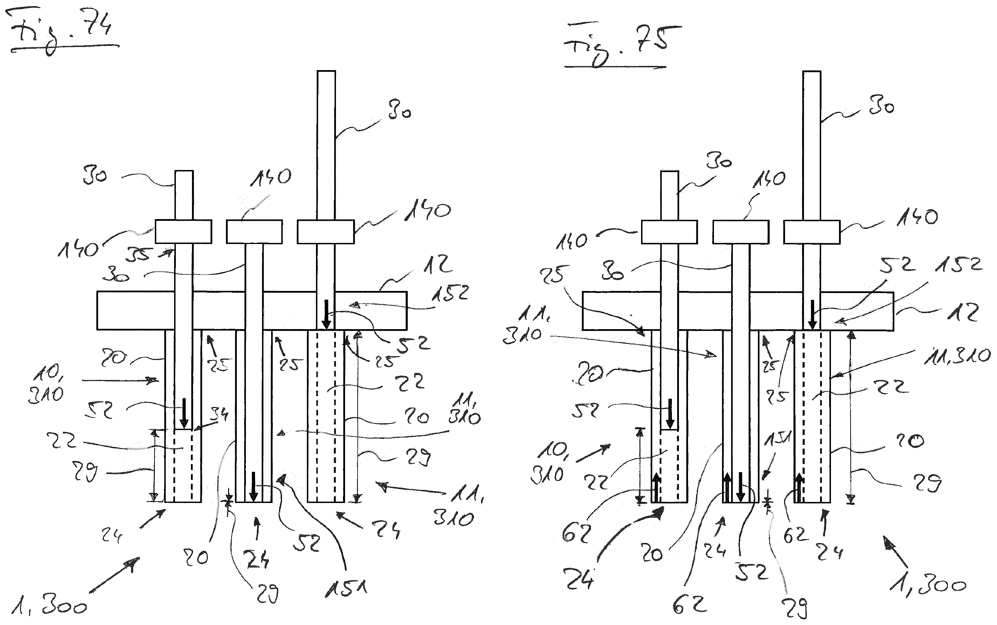

Fig. 74 schematically depicts a soft robot 1 that is configured as an artificial cilia array 300. The soft robot 1 comprises a support 12 to which a body 10 forming an artificial cilium 310 is fixedly attached. The body 10 is exemplarily configured as it is disclosed for the first configuration of the body 10 comprising a tubular outer part 20 shown in Figures 3 to 6. The outer part 20 has the tubular channel 22, in which the inner part 30 is movably arranged. With its second end 25, the outer part 20 is fixed to the support 12 and the first end 24 of the outer part 20 forms a free end. The inner part 30 has the magnetic unit 52 arranged at its free first end 34. At its second end 35, the inner part 30 is connected to an actuator 140 that is configured to at least linearly move the inner part 30 within the channel 22 of the outer part 20. Thereby, the distance 29 between the free end 24 of the outer part 20 and the free end 34 of the inner part 30 is varied. With the embodiment shown in Fig. 74, the outer part 20 is free from any magnetic units.

The artificial cilia array 300 comprises a multitude of other bodies 11, wherein each other body 11 is configured as it is disclosed for the body 10 of the artificial cilia array 300. The other bodies 11 form other artificial cilia 310 of the artificial cilia array 300.

With the artificial cilia array 300 shown in Fig. 74, the distance 29 is zero for the artificial cilium 310 arranged in the middle, so that the magnetic unit 52 is located at a first position 151 at the free end 24 of the outer part 20 of the artificial cilium 310. With the artificial cilium 310 placed to the right in Fig. 74, the distance 29 is maximum so that the magnetic unit 52 is completely retracted from the channel 22 of the outer part 24 and positioned at a second position 152. As a consequence, the artificial cilium 310 in the middle has a magnetization profile comprising the magnetic unit 52 located at the free end 24 and the artificial cilium 310 to the right as a magnetization profile that does not comprise any magnetic unit.

The artificial cilium 310 to the left in Fig. 74 has the inner part 30 placed at an intermediate distance 29 in between zero and the maximum distance. This results in a magnetization profile in which the magnetic unit 52 is located at the intermediate position in between the free end 24 and the support 12.

Fig. 75 schematically depicts another version of a soft robot 1 that is configured as an artificial cilia array 300. As far as no differences are disclosed in the description and the Figures, the soft robot 1 of Fig. 75 is configured as it is disclosed for the soft robot 1 shown in Fig. 74.

The soft robot 1 of Fig. 75 comprises artificial cilia 310 that are formed by the second configuration of the body 10 shown in Figures 7 to 12. With the second configuration, the outer part 20 comprises the further magnetic unit 62 and the direction of the magnetic moment of the magnetic unit 52 is opposite to the direction of the magnetic moment of the further magnetic unit 62. Furthermore, the magnitude of the magnetic moment of the magnetic unit 52 equals the magnitude of the magnetic moment of the further magnetic unit 62.

Upon placing the magnetic unit 52 next to the further magnetic unit 62 by reducing the distance 29 to zero, the magnetic moments of the magnetic unit 52 and the further magnetic unit 62 cancel and the body 10 has a magnetization profile that has zero magnetic moment. The other body 11 placed in the middle of the artificial cilium array 300 shown in Fig. 75 has this configuration. Furthermore, upon placing the magnetic unit 52 at the maximum distance 29 and thereby retracting the magnetic unit 52 from the channel 22 of the outer part 20, the body 10 has a magnetization profile that comprises the magnetic moment of the further magnetic unit 62 located at the first and 24 of the outer part 20. The other body 11 placed to the right of the artificial cilia array 300 shown in Fig. 75 has this configuration.

For targeted cilia activation, the bodies 10, 11 may have the first configuration for 1D magnetization reprogramming shown in Figures 3 to 6 and Fig. 74. By setting the distance 29 to either its minimum value, exemplarily to *d*_{*R*12}=0 mm, or its maximum value, exemplarily *d*_{*R*12}=42 mm, each cilium 310 may be selectively magnetized, see Fig. 6A for the distance 29 reduced to zero and Fig. 6D for the distance 29 said to its maximum value.

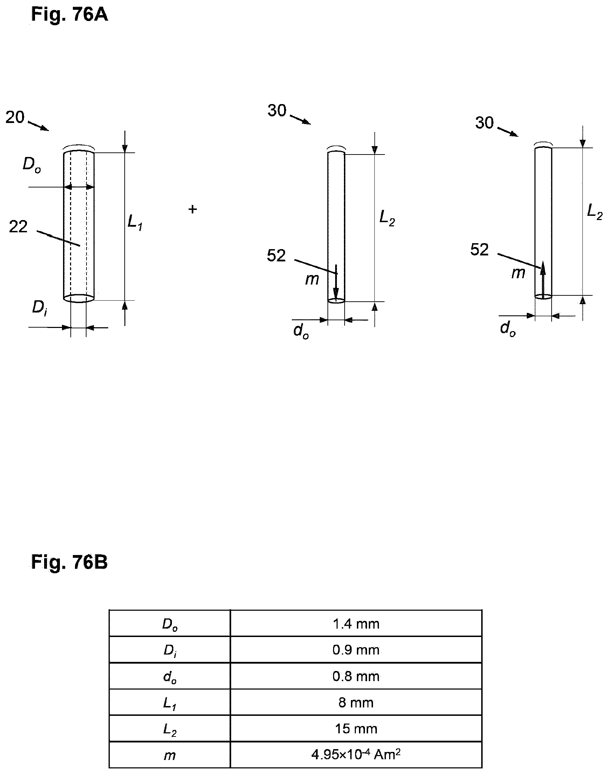

To facilitate manipulation and observation, the cilia 310 may have a length in the millimeter-scale. Fig. 76A depicts the outer part 20 and two possible versions of the inner part 30 that may be employed as part of the bodies 10, 11 forming the artificial cilia 310, 11. Thereby, the two versions of the inner part 30 have opposing directions of the magnetic moment of the magnetic unit 52. Fig. 76B lists exemplary parameters of the configuration shown in Fig. 76A.

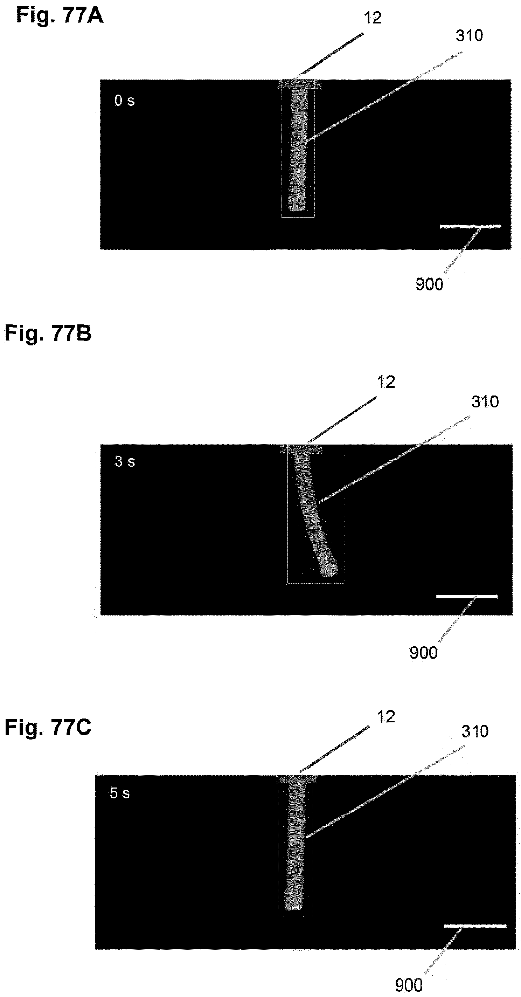

Fig. 77 demonstrates the ciliary activation in a single cilium 310. The cilium 310 may be switched between active and inactive by controlling the presence of the magnetic unit 52 at the first end 24 via the distance 29 (*d*_{*R*12}), enabling transitions between stationary and moving states under a same time-varying magnetic field 5 (exemplarily generated by a Halbach array, 20mT). The time-variety magnetic field 5 may be a rotating magnetic field.

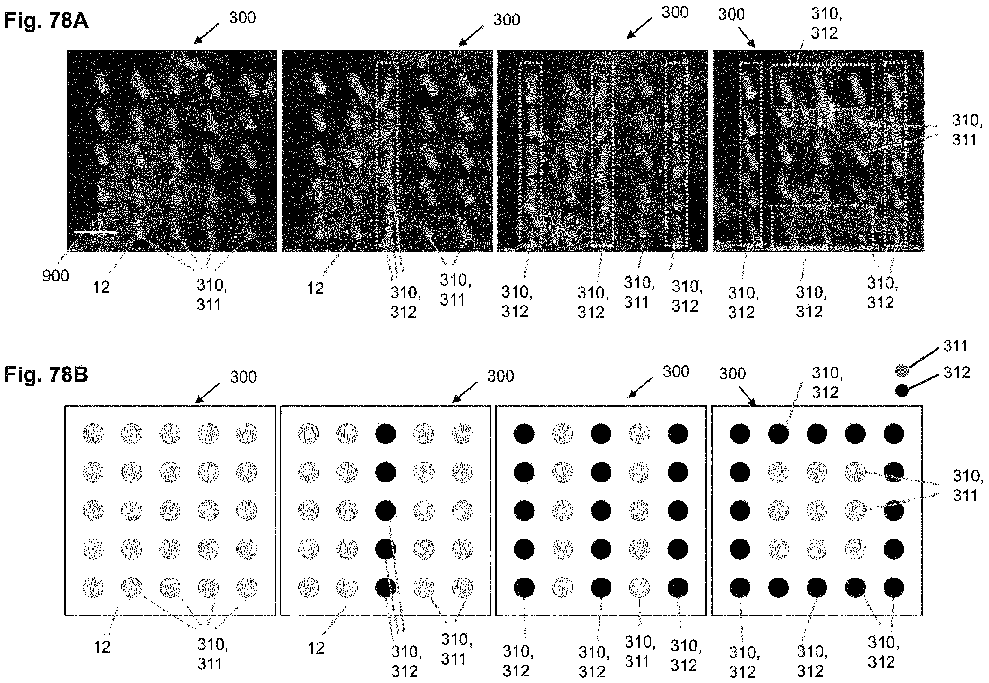

Control over individual cilia 310 may be extended to cilia arrays 310, allowing for selective activation of specific cilia 310 to be active or inactive, as shown in Fig. 78. Thereby, Fig. 78A depicts images of the artificial cilia array 300 and Fig. 78B depict corresponding diagrams indicating whether individual cilia 310 are active or inactive.

In general, the artificial cilia array 300 may have at least a first cilium 311, such as a multitude of first cilia 311, that exhibit a first response to the magnetic field 5 and at least a second cilium 312, such as a multitude of second cilia 312, that exhibit a second response to the magnetic field 5. The first cilia 311 may form a first pattern and the second cilia 312 may form a second pattern. Exemplarily, the first pattern and/or the second pattern may comprise a single row or multiple rows within the artificial cilia array 300. Each pattern may comprise a first row and a second row that meet at an angle, such as a right angle.

With the embodiment shown in Fig. 78, the first response of the first cilia 311 is remaining stationary upon applying the magnetic field 5 and the second response of the second cilia 312 is a deflection within the magnetic field 5.

To facilitate observation, the schematic diagrams of Fig. 78B mark active second cilia 312 in a dark shade and inactive first cilia 311 in a light shade. These diagrams illustrate the variety of patterns that can be formed.

By utilizing the targeted cilia activation, various states and driving modes of cilia arrays 300 may be implemented, offering new possibilities for different functionalities. One potential application of this technique is to transport matter along a desired pathway.

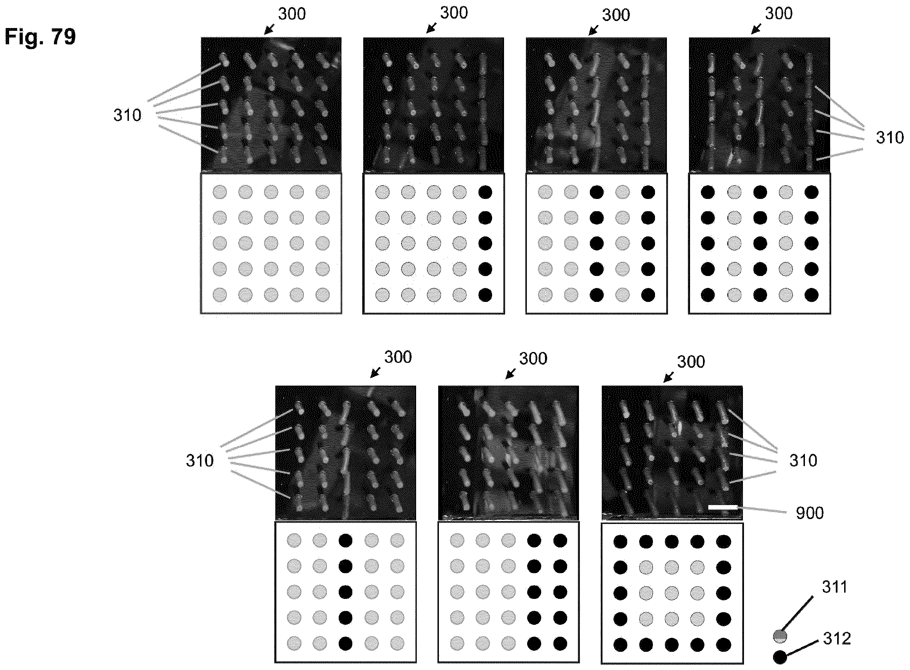

Fig. 79 depicts further examples of targeted activation of cilia 310. The cilia array 300 is shown in various states in images, with corresponding schematic diagrams directly below. These diagrams illustrate the activation states of the ciliary array 300, including the inactive state of the first cilia 311 and the active state of the second cilia 312. This Figure demonstrates the control of targeted activation of cilia 310 in numerous configurations, achievable through the real-time in-situ magnetization reprogramming method according to the present disclosure. The cilia 310 may be placed in a rotating magnetic field 5 having a magnitude of, for example, 45mT. The scale bar 900 is 5mm.

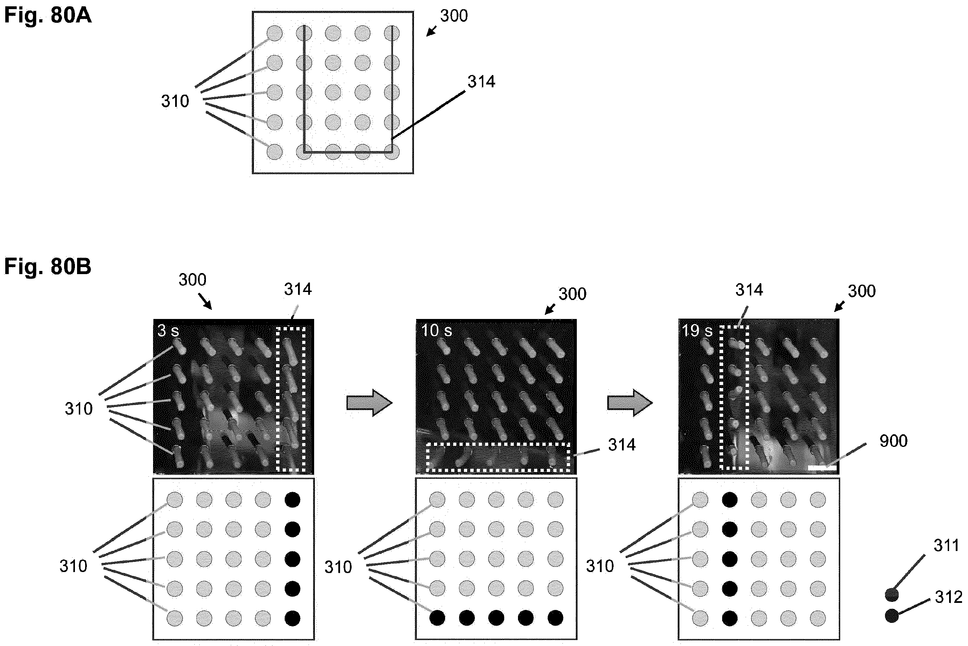

Fig. 80 depicts a method for using targeted cilia activation to achieve path-directed propulsion. In Fig. 80A, a predefined path 314 for propulsion is shown as a U-shaped path. Fig. 80B illustrates steps for activating patterns of second cilia 312 that are arranged in individual rows 315, whereby the individual rows 315 form a sequence of adjoining rows 315 of active second cilia 312 and the rows 315 of the sequence are activated one after the other. To achieve propulsion along the U-shaped path 314, the cilia 310 in the far-right row 314 are initially activated, followed by the activation of the cilia 310 in the bottom row 314. Finally, the cilia 310 in the second to left row 314 are activated. Fig. 80B depicts images of the cilia array 300 in various states with corresponding schematic diagrams showing first cilia 311 and second cilia 312 directly below. These diagrams illustrate the activation states of the ciliary array 300, including the inactive states of first cilia 311 and active states of second cilia 312. The cilia 310 may be placed in a rotating magnetic field 5 having a magnitude of, for example, 45mT. The scale bar 900 is 5mm.

With the artificial cilia array 300, fluids or solids may be transported along predetermined trajectories by sequentially activating cilia 310 in specific rows 314, such as the U-shaped path 314 shown in Fig. 80. This U-shaped path 314 is an example of the ability of the method according to the present disclosure to transport matter along arbitrary paths 314. The method of targeted activation of cilia 310 thus lays the groundwork for directional driving in various contexts.

Ciliary bending amplitude modulation may be realized with artificial cilia 310 having the second configuration of the body 10 forming an elongated cylindrical member for 1D magnetization reprogramming, as shown in Figs. 7 to 12 and Fig. 81A. By varying the distance 29, *d*_{*R*12}, the outer part 20 exhibits various bending amplitudes with varying radii of curvature 710 and terminal bending angles 711 as defined in connection with Fig. 110. A single cilium 310 thereby is configured to perform diverse motion patterns in the same rotating magnetic field 5.

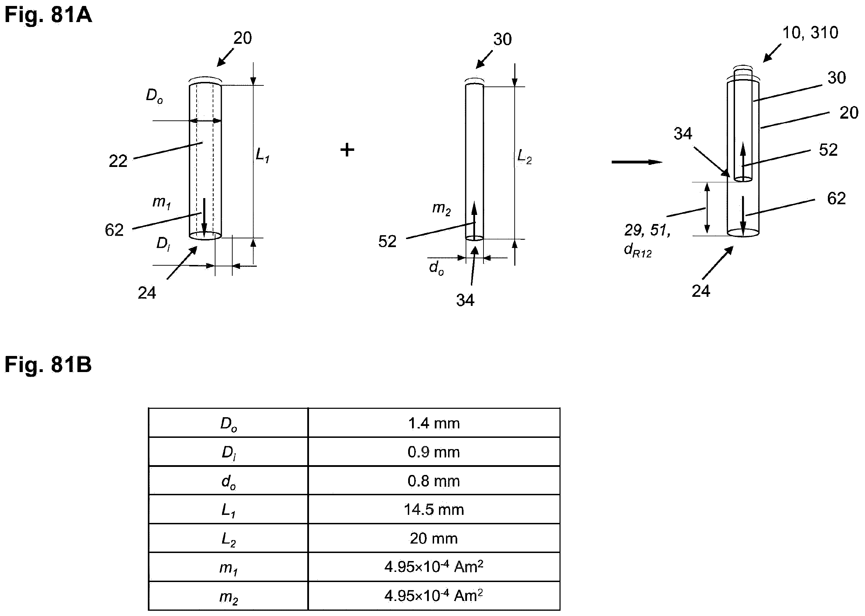

The individual cilia 310 may be configured as shown Fig. 81A and may have the parameters listed in Fig. 81B. In particular, the magnetic unit 52 and the further magnetic unit 62 may have magnetic moments with the same magnitude and differing, namely opposite, directions. Fig. 75 depicts an artificial cilia array 300 comprising these cilia 310.

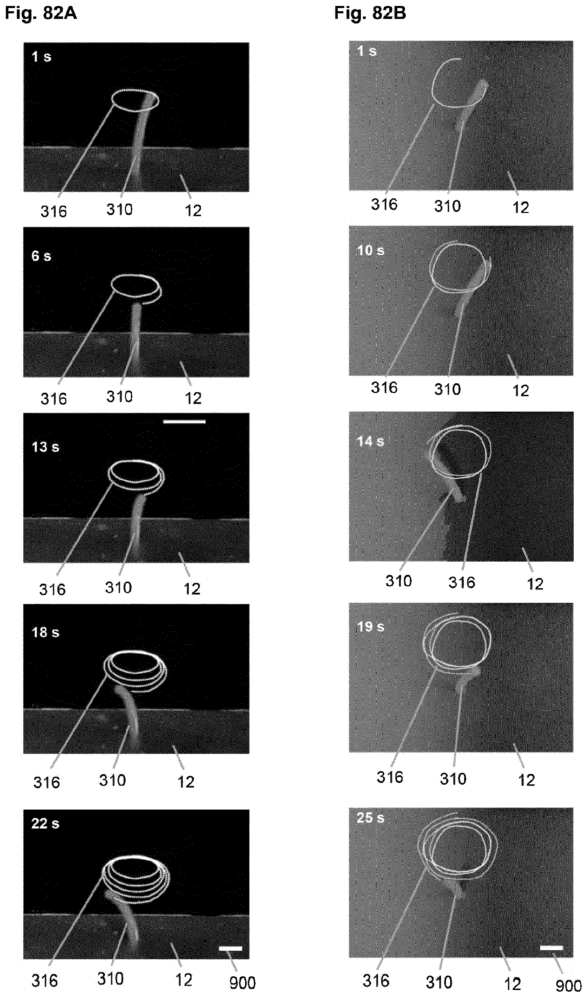

Fig. 82 shows the various motion trajectories 316 of a single cilium 310 actuated by a rotating magnetic field 5 having constant magnitude, exemplarily 45 mT. By altering the distance 29 (*d*_{*R*12}), the bending amplitude of the cilium 310 is adjusted. For example, the distance 29 may be set to five distinct values, whereby a radius of the motion of the cilium 310 is different for each value. This leads to five distinct trajectories 316 that enable different ciliary motion patterns. Fig. 82A depict side views of the cilium 310 with the trajectory 316 and Fig. 82B depicts top views. The dashed lines in the images represent the trajectory 316 of the midpoint at the end of the cilium 310. With each panel in Fig. 82, the distance 29 is enlarged from top to bottom increasing the radius of the trajectory 316.

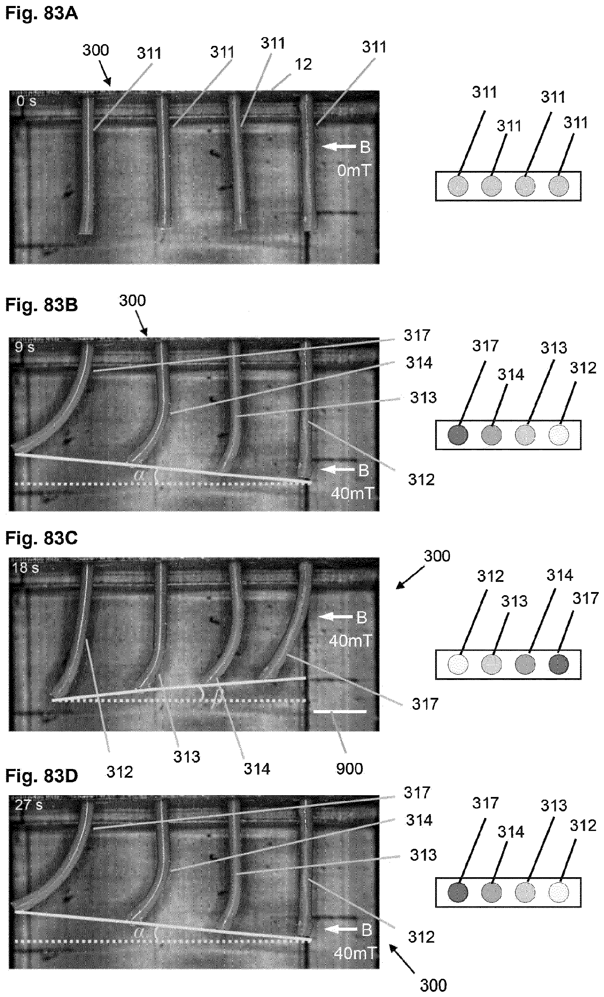

Fig. 83 depicts images of a one-dimensional artificial cilia array 300 with cilia exhibiting bending amplitude modulation in a common uniform magnetic field 5 (B), exemplarily having a magnitude of 40 mT. Each individual cilium may be configured as a first cilium 311 having a first bending amplitude, as a second cilium 312 having a second bending amplitude, as a third cilium 313 having a third bending amplitude, a fourth cilium 314 having a fourth bending amplitude or as a fifth cilium 317 having a fifth bending amplitude. The first bending amplitude is zero and the second to fifth bending amplitudes increase. The individual bending amplitudes are generated by configuring the first cilium 311 to the fifth cilium 317 with increasing distances 29 between the magnetic unit 52 and the further magnetic unit 62.

In Fig. 83A, each cilium is configured as a first cilium 311. With the configurations in Figs. 83B, 83C, 83D, the cilia in the artificial cilium array 300 are configured as the second cilium 312, the third cilium 313, the fourth cilium 314 and the fifth cilium 317, one after the other. The variation in bending amplitude across the different cilia 312, 313, 314, 370 results in the tilting angles α and β at the tips of the cilia. To the right of the images in Fig. 83, corresponding schematics are displayed that highlight the different bending amplitudes with circles of different shade. The variation of bending amplitude from left to right is gradual and can also be adjusted as needed to achieve the desired bending amplitudes. The scale bar 900 is 5mm.

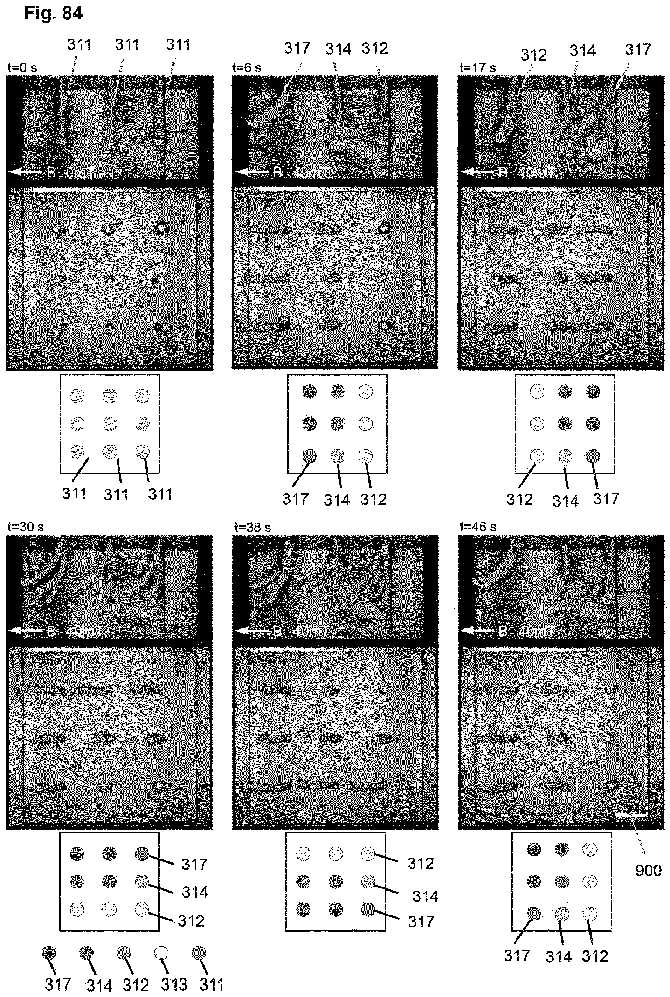

Fig. 84 depicts images of ciliary bending amplitude modulation in a 3x3 cilia array 300 under a common uniform magnetic field 5, denoted as B. Corresponding schematics are displayed at the bottom of the images. The individual images show a side view of the artificial cilia array 300 at the top and a top view of the artificial cilia array 300 at the bottom of each respective image. The images show some exemplary patterns, although any pattern that meets specific requirements can be obtained. The scale bar 900 is 5mm.

With the embodiment shown in Figs. 81 to 84, the first response of the first cilia 311 is remaining stationary or deflecting with a first amplitude that is zero upon applying the magnetic field 5 and the second response of the second cilia 312 is a deflection within the magnetic field 5 with a second amplitude. A third response of the third cilia 313 is a deflection with a third amplitude, a fourth response of the fourth cilia 340 is a deflection with a fourth amplitude and a fifth response of the fifth cilia 317 is a deflection with a fifth amplitude. Thereby, the first amplitude, the second amplitude, the third amplitude, the fourth amplitude and the fifth amplitude are different from each other. Exemplarily, the amplitudes continuously increase from the first amplitude to the fifth amplitude.

As detailed in the foregoing, it is possible to generate and switch between different motion patterns for a single cilium 310 and cilia array 300 without altering the external magnetic field 5. The method according to the present disclosure allows for the creation of various distinct motion patterns, which hold the potential to realize functionalities that existing cilia configurations cannot achieve.

Ciliary phase modulation may be realized by implementing the magnetic reversal characteristic of the second configuration of the body 10 for 1D magnetization reprogramming, as shown in Figures 9D and 10, which is configured for alteration of the direction of the magnetic moment of the body 10, thus facilitating the modulation of the cilia phase.

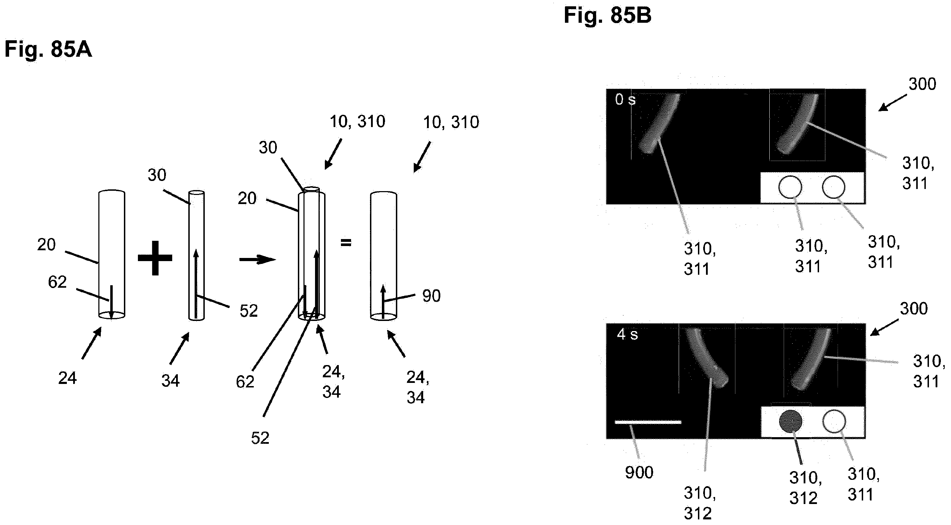

As shown in Fig. 85A, the magnetic unit 52 of the inner part 30 has a magnetic moment **-**2***m*** with twice the magnitude and opposite direction as the magnetic moment **m** of the further magnetic unit 62 of the outer part 20. A superposition 90 of the magnetic moments *-2**m***, ***m*** of the magnetic unit 52 and the further magnetic unit 62 reverses the direction of the magnetic moment of the body 10 forming the artificial cilium 310.

Each artificial cilium 310 may have a length L₁ of 10 mm, a length L₂ of 14 mm, a magnetic moment m₂ of the magnetic unit 52 of 9.9×10⁻⁴ Am², and a magnetic moment m₁ of the further magnetic unit 62 of 4.95×10⁻⁴ Am², with the remaining dimensions being equal to the values listed in Fig. 81B.

Fig. 85B shows that two cilia 310 may be configured to have the same motional phase in a common rotating magnetic field 5 (45mT) or may have opposite phases, depending on the position of the magnetic unit 52 within the outer part 20. With the magnetic unit 52 positioned at the first end 24 of the outer part 20, each cilium 310 is configured as a first cilium 311 and, with the magnetic unit 52 positioned at the second end 25 of the outer part 20, each cilium 310 is configured as a second cilium 312. Thereby, the first cilium 311 and the second cilium 312 have magnetic moments with the same magnitude and opposite direction. This results in different phases of the motion of the first cilium 311 and the second cilium 300 1210 a common time-varying magnetic field 5. The phase differences are visualized by circles of different shade to denote the first cilia 311 at a first phase, such as a phase of 0, and the second cilia 312 at a second phase, such as at a phase of π, as displayed in the lower right corner of each sub-figure in Fig. 85B.

With the embodiment shown in Fig. 85, the first response of the first cilia 311 is motion with the first phase upon applying the magnetic field 5 and the second response of the second cilia 312 is motion with a second phase within the magnetic field 5.

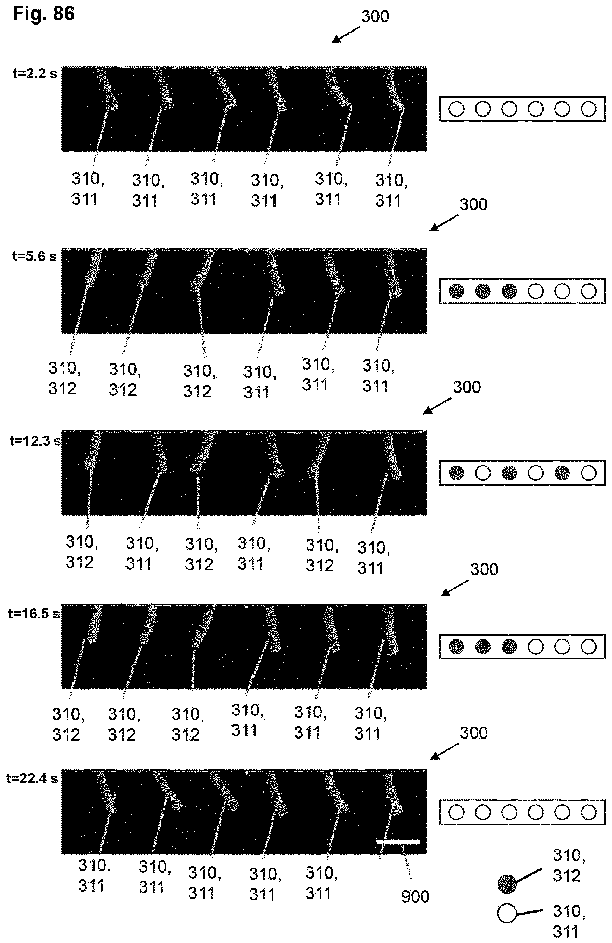

Furthermore, the phase modulation changes may be applied in a 1×6 cilia array 300, as shown in Fig. 86, which depicts images of ciliary phase modulation in the 1x6 cilia array 300 under a common rotating magnetic field 5 of exemplarily B = 45 mT. To clearly depict the changes in phase, corresponding schematics are displayed on the right side of the images. Thereby, white circles represent first cilia 311 at a phase of zero and dark circles denote second cilia 312 at a phase of π. The scale bar 900 is 5mm.

The foregoing demonstrates the feasibility of using the method according to the present disclosure for phase modulation of cilia arrays 300, providing a new approach for implementing complex ciliary motion patterns and potentially expanding the applications of cilia 310.

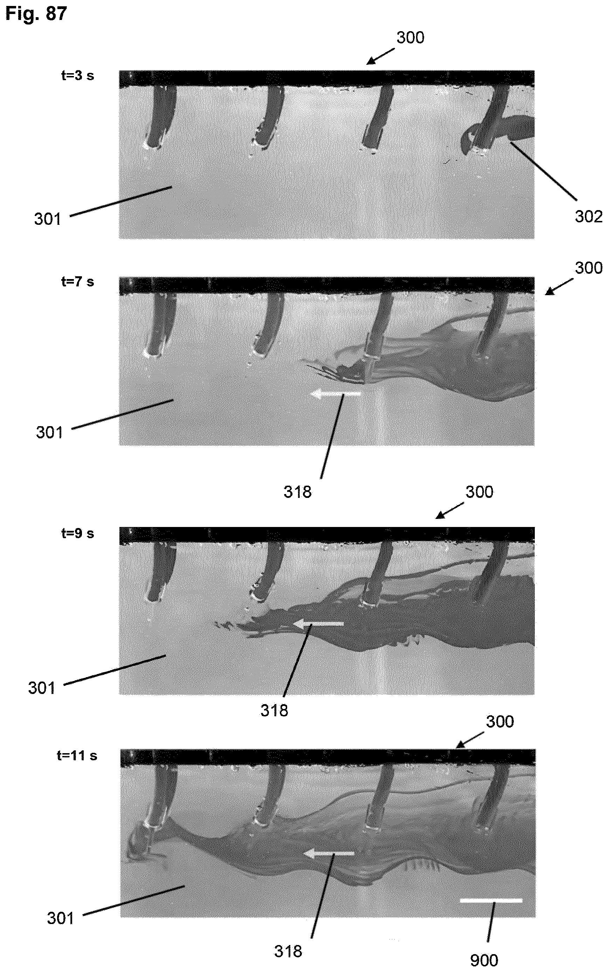

One potential application is the real-time modulation of fluid driving directions. Continuing with the cilia 310 used in the embodiments described in the foregoing, a cilia array 300, such as a 2×4 cilia array 300, may be submerged in a fluid 301, such as 85% glycerin, and may be driven by a rotating magnetic field 5 of B = 45 mT. If all cilia 310 are configured as first or second cilia 311, 312 moving with the same phase, they drive the fluid 301 in a first flow direction 318, which exemplarily is leftward in Fig. 87. The first flow direction 318 thereby is highlighted by an ink 302 added to the fluid 301.

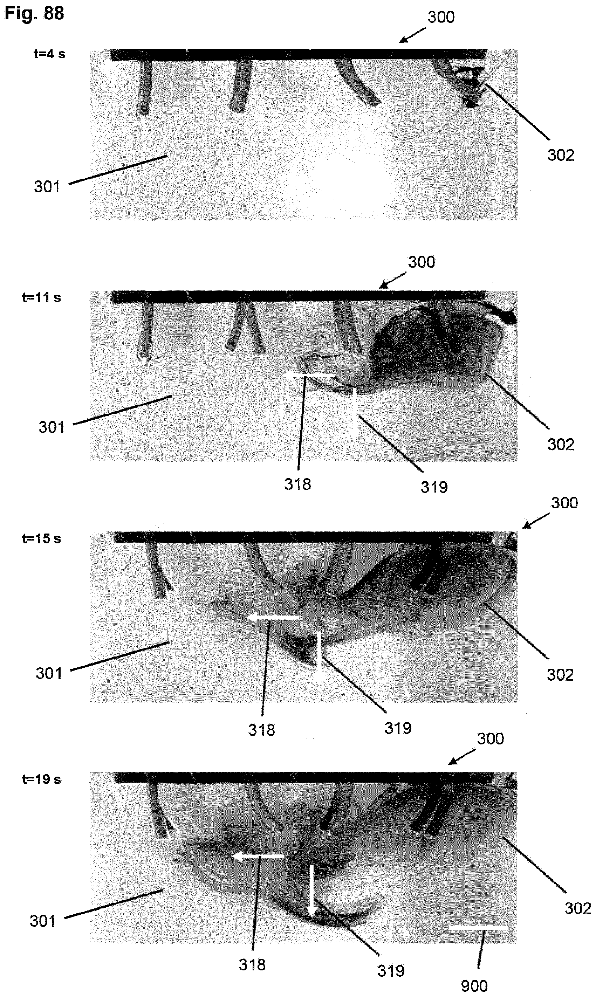

With the configuration shown in Fig. 88, one group of cilia 310 may be configured as the first cilia 311 and another group of cilia 310 may be configured as the second cilia 312, whereby the first cilia 311 and the second cilia 312 exhibit differing phases within the rotating magnetic field 5. Exemplarily, the first cilia 311 and the second cilia 312 are out of phase by π. The fluid 301 is then driven both in the first direction 318 and in a second direction 319, whereby the second direction exemplarily is downward. With the embodiment shown in Fig. 88, the first group is formed by the cilia 310 in the two rows to the left and the second group is formed by the cilia 310 in the two rows to the right. The scale bar 900 is 5mm in Figs. 87 and 88.

The cilia array 300 is configured to switch between the configuration of Fig. 87 and the configuration of Fig. 88 by moving the magnetic unit 52 within the individual cilia 310.

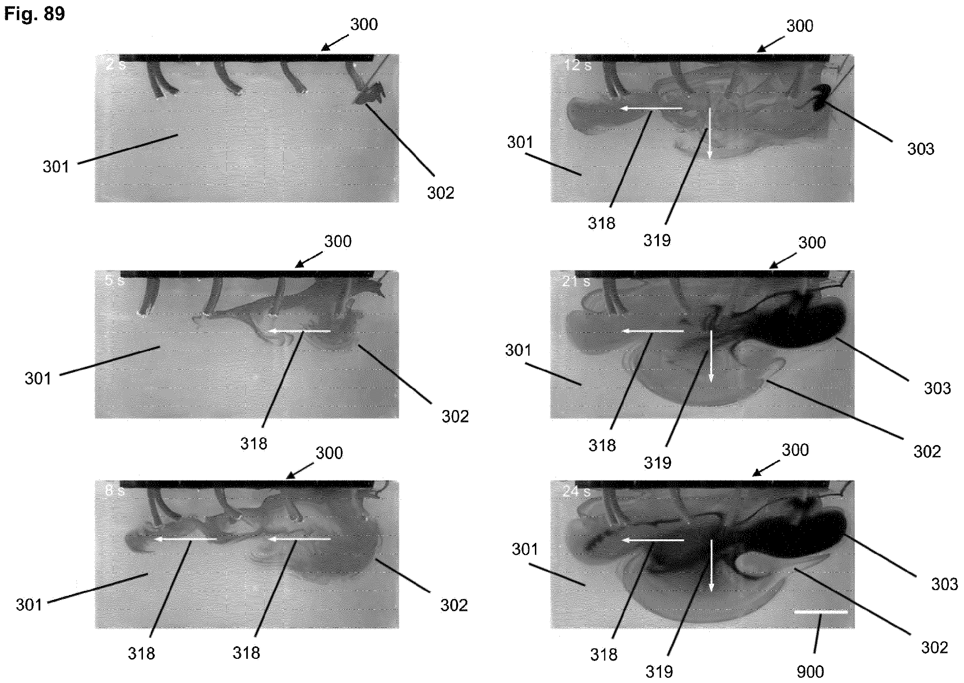

To further illustrate the specific process of phase modulation, Fig. 89 depicts a step of initially driving the fluid 301 in the first direction 318, namely to the left, (see images taken at 2 s, 5 s, and 8 s) and a step of driving the fluid 301 in both the first direction 318 and the second direction 319, namely downward and leftward, see images taken at 12 s, 21 s, and 24 s. During the first step, all cilia 310 move with the same phase and in the second step, the phase of the second group of cilia 310 is switched, whereby the second group of cilia 310 is exemplarily out of phase by π with the first group.

With alternative embodiments, the fluid 301 may only be driven in the second direction 319 in the second step.

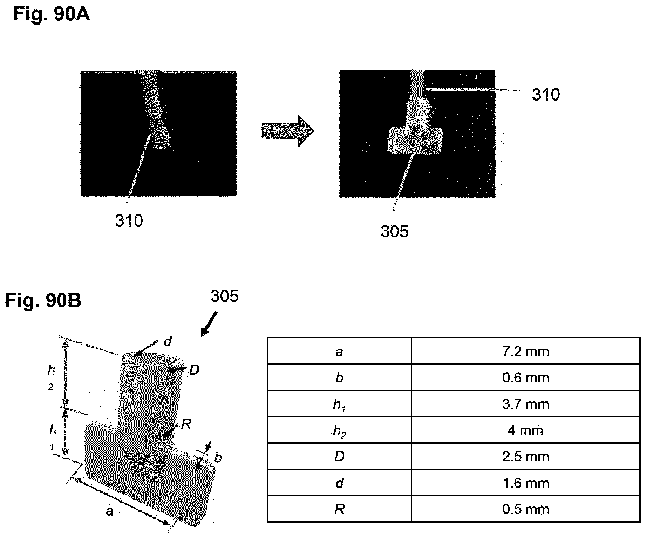

Paddles 305, such as square-shaped paddles, may be attached to the tip of each cilium 310 to enhance the fluid flow, see Fig. 90. This design merely amplifies the driving effect without changing the driving mode. Fig. 90A depicts a cilium 310 with the paddle 305 at its tip and Fig. 90B lists exemplary design parameters of the paddle.

The ability to control the phase of the cilia 310 to change driving directions 318, 319 allows fluid manipulation to be adjusted on demand, enhancing driving efficiency and providing greater operational flexibility. Of course, the embodiments described are merely demonstrations of potential capabilities. For instance, while the phase difference in the aforementioned cilia is π, more complex designs of magnetization directions may be employed to achieve various desired phase differences.

The dimensions of the cilia 310 can be adjusted according to specific operational requirements. The cilia 310 demonstrated above have an outer diameter Dₒ of 1.4 mm. Smaller-scale cilia 310 can also be controlled using the method according to the present disclosure. Below, we describe the mechanical criteria required to achieve relative motion between tubes forming the outer part 20 and the inner parts 30, 40. Therefore, regardless of the scale, as long as these mechanical conditions are satisfied, the method according to the present disclosure can be applied to realize a reprogrammable cilia array 300.

Targeted cilia activation, ciliary bending amplitude modulation, and ciliary phase modulation can be combined to enable operations and functionalities that are not possible if these techniques are applied independently. For example, by combining targeted cilia activation with ciliary phase modulation, it is possible to achieve more complex ciliary driving patterns.

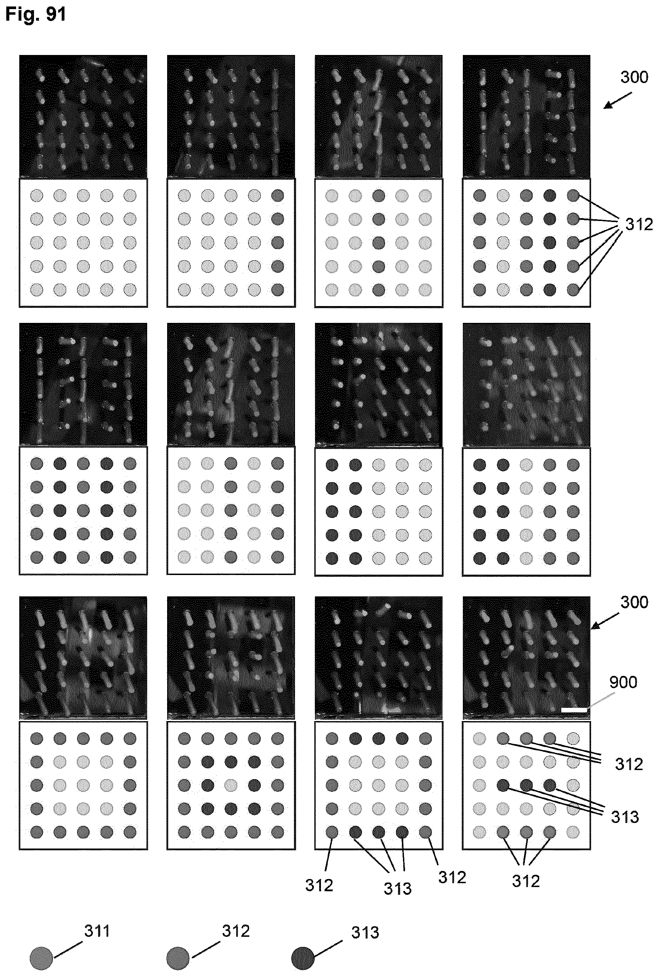

Fig. 91 depicts a driving method of the cilia array 300 that combines targeted cilia activation and ciliary phase modulation. The cilia array 300 is shown in various states in the images, directly below of which corresponding schematic diagrams are shown. These diagrams illustrate the activation states of the cilia 310 in the ciliary array 300, including the first cilia 311 with the first response, which is remaining inactive in the magnetic field 5, representing an inactive state, the second cilia 312 with the second response, which is motion with phase 0 in the magnetic field 5, representing an active state with phase 0, and the third cilia 313 with the third response, which is motion with phase *π* in the magnetic field 5, representing an active state with phase *π*. The diverse patterns in Fig. 91 demonstrate the capability to achieve more complex driving patterns through the combination of targeted cilia activation and ciliary phase modulation. The scale bar 900 is 5 mm.

The cilia 310 of the cilia array 300 shown in Fig. 91 may, for example, be formed by bodies 10 that comprise the outer part 20, the inner part 30 and the further inner part 40, whereby the further inner part 40 is nested in between the inner part 30 and the outer part 20. The inner part 30 may comprise the magnetic unit 52 and the further inner part 40 may comprise the further magnetic unit 62, while the outer part 20 may be free from any magnetic units. The magnetic unit 52 and the further magnetic unit 62 may have magnetic moments with the same magnitude and opposite directions. By selectively moving only the magnetic unit 52 or only the further magnetic unit 62 to the first end 24 of the outer part 20, the magnetization profile of the body 10 may be switched between two profiles having opposing magnetic moments with the same magnitude. By either retracting both the magnetic unit 52 and the further magnetic unit 62 from the channel 22 or by placing both the magnetic unit 52 and the further magnetic unit 62 at the first end 24 of the channel 22, the body 10 is configured with a magnetization profile that has a magnetic moment of zero.

The targeted cilia activation, ciliary bending amplitude modulation, and ciliary phase modulation described before have been based solely on the first configuration and the second configuration of the body 10 for 1D magnetization reprogramming, as depicted in Figures 3 to 12. Other configurations shown in Figures 3 to 29 may also be implemented to enable even more complex motion patterns for individual cilia 310, thus allowing for greater possibilities for expanding ciliary functions and for more complex applications.

### Application III: Coordinated multi-instrument operation

In medical diagnosis and treatment, multiple medical instruments are commonly needed to work together to achieve the desired outcome. For example, catheters and guidewires are used together in vascular interventional surgeries (*42, 45*), and multiple robotic arms or grippers are employed in laparoscopic procedures (*80*). Currently, various actuation mechanisms have been investigated and implemented in medical procedures *(26, 46, 54, 55, 81*)*.* In magnetic actuation, the locomotion or shape control of instruments is achieved through the gradient force or torque exerted by an external magnetic field. Nevertheless, due to the pervasive nature of magnetic fields, which affect magnetic objects within their range non-selectively, independently controlling multiple targets using the same magnetic field poses a considerable challenge (*82*).

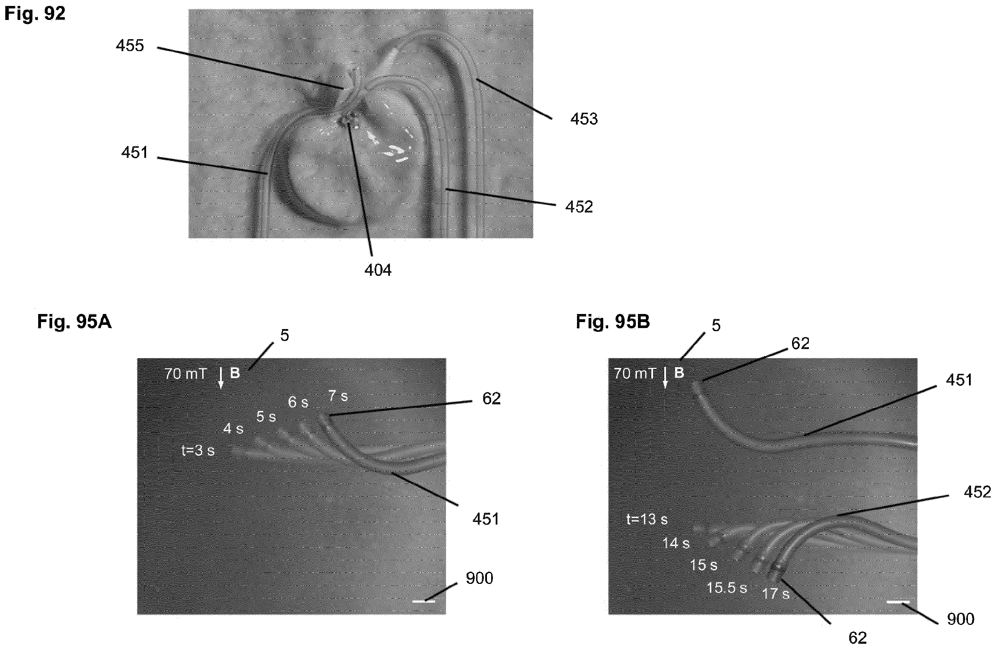

A typical minimally invasive surgery scenario is illustrated in Fig. 92, which is a typical multi-instrument coordinated operational medical scenario. Three instruments collaborate to diagnose or treat the target tissue. The target 404, such as a diagnostic or treatment target, is obscured by an obstruction 455, such as tissue. This necessitates the use of a first soft robot 451 to appropriately reposition the obstruction 455, ensuring sufficient workspace for a second soft robot 452 to either diagnose or treat a target 404, such as a target tissue. Additionally, a third soft robot 453 may be required to enhance visibility (by obtaining operational images) within the body or to assist during the diagnostic and treatment processes. Exemplarily, the soft robots 451, 452, 453 each are configured as a medical instrument 400. With other embodiments, the soft robots 451, 452, 453 may also form other devices, for example for industrial use.

The conventional method of single magnetic field control from the prior art is inadequate for such operations; the use of multiple magnetic fields, though posing significant challenges, also raises questions about the feasibility of completing such tasks. Therefore, magnetic actuation methods are often limited to manipulating a single instrument, restricting their use in scenarios requiring multiple instruments.

The devices and methods according to the present disclosure provide effective means to address the aforementioned challenges.

As an example, the feature of magnetic neutralization, as provided by the second configuration of the body 10 configured for 1D magnetization reprogramming of Fig. 9C, enable the instruments formed by the soft robots 451, 452, 453 to selectively respond to an external magnetic field 5 based on demand. Moreover, by adjusting the distance 51 (*d*_{*R*12}) between the magnetic unit 52 and the further magnetic unit 62, the soft robots 451, 452, 453 can achieve real-time shape transformation, see Figs. 7 to 12, thereby attaining deformations necessary to accomplish various tasks.

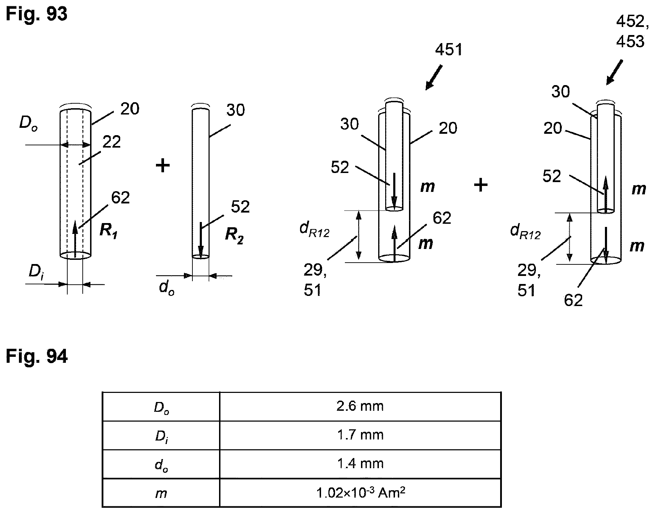

According to the present disclosure, the first soft robot 451 and the second soft robot 452 may be configured as two types of integrated soft tubes, as shown in **Fig. 93****.** As far as no differences are disclosed in the description or the Figures, the first soft robot 451 and the second soft robot 452 each have the second configuration of the body 10 described in Figures 7 to 12. Both have the outer part 20 with the further magnetic unit 62 and the inner part 30 with the magnetic unit 52. For each soft robot 451, 452 the magnetic unit 52 has a magnetic moment with the same magnitude and the opposite direction as the magnetic moment of the further magnetic unit 62.

The magnetic moment of the magnetic unit 52 of the first soft robot 451 and the magnetic moment of the magnetic unit 52 of the second soft robot 452 have opposite directions. Furthermore, the further magnetic unit 62 of the first soft robot 451 and the further magnetic unit 62 of the second soft robot 452 have magnetic moments with opposite directions. As an example, the magnetic moments of the magnetic units 52 of the first and second soft robot 451, 452 may have the same magnitude and the magnetic moments of the further magnetic units 62 of the first and second soft robot 451, 452 may have the same magnitude. With some embodiments, the inner part 30 may be configured as a soft rod to facilitate manufacturing and manipulation. With other embodiments, the inner part 30 may be configured as a hollow tube.

The third soft robot 453 is configured identical to the second soft robot 452.

Fig. 94 lists exemplary parameter values of the first, second, and third soft robot 451, 452, 453 shown in Fig. 93.

Fig. 95 depicts operation of the first soft robot 451 and the second soft robot 452 within a common uniform magnetic field 5 directed downward with a strength of 70 mT. Thereby, images of a movie have been superimposed with the time t of the individual images indicated in the Figures. The first soft robot 451 and the second soft robot 452 are independently controlled in the same uniform magnetic field 5. Within the uniform magnetic field 5 of B = 70 mT, directed downward, the first soft robot 351 undergoes deformation into various shapes, see Fig. 95A. In the same uniform magnetic field 5, the first soft robot 351 maintains a specific shape or deformation while the second soft robot 352 undergoes deformation into different shapes as required.

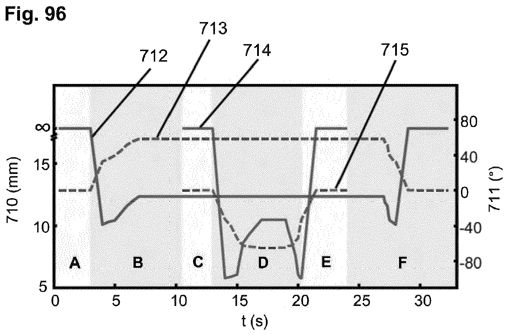

To visually demonstrate the independent and controllable changes in the states of both soft robots 451, 452 within the same magnetic field 5, Fig. 96 depicts the variations in the radius of curvature 710 and the terminal bending angles 711, as defined in Fig. 110, throughout the operation. Note that, in the straight state of the outer parts 20, the radius of curvature 710 is infinite, and the terminal bending angle 711 is 0°. Fig. 96 thereby depicts a first radius of curvature 712 and a first terminal bending angle 713 of the first soft robot 451 and a second radius of curvature 714 and a second terminal bending angle 715 of the second soft robot 452. A deformation of the first soft robot 451 and a deformation of the second soft robot 452 are independently controlled within the common magnetic field 5. For example, the first soft robot 451 and the second soft robot 452 are configured to adjust the first radius of curvature 712 and the second radius of curvature 714 independently from each other. Furthermore, the first soft robot 451 and the second soft robot 452 are configured to adjust the first terminal bending angle 713 and the second terminal bending angle 715 independently from each other.

Referring to Fig. 96, the entire movement of the first soft robot 451 and the second soft robot 452 is divided into six stages, outlined as follows: In a first step, the first soft robot 451 is moved forward with a straight state under the magnetic field 5. At this stage, the first soft robot 451 is non-magnetic because the distance 51 is *d*_{*R*12} = 0, corresponding to 0-3 s in Fig. 95A and Zone A in Fig. 96. In a first step, the relative positions of the two magnetic units 52, 62 within the first soft robot 451 is altered by changing the distance 51 (*d*_{*R*12}), which allows the first soft robot 451 to assume various states. This operation corresponds to 3 s to 10.5 s in Fig. 95A and Zone B in Fig. 96. In a third step, while maintaining the state of the first soft robot 451, the second soft robot 452 is advanced or retracted. At this time, the first soft robot 451 maintains a specific state under the magnetic field 5 with a fixed distance 51 (*d*_{*R*12}), while the second soft robot 452 is in a straight state as a result of the distance 51 being set to *d*_{*R*12} = 0, corresponding to 10.5 s to 13 s in Fig. 95B and Zone C in Fig. 96. In a fourth step, with the state of the first soft robot 451 unchanged, the state of the second soft robot 452 is adjusted by changing the distance 51 (*d*_{*R*12}), corresponding to 13 s to 20.3 s in Fig. 95B and Zone D in Fig. 96. In a fifth step, the second soft robot 452 is returned to a straight state (with the distance 51 set to *d*_{*R*12} = 0) and retracted, corresponding to Zone E in Fig. 96. In a sixth step, the first soft robot 451 is returned to a straight state (by setting the distance 51 to *d*_{*R*12} = 0) and retracted, corresponding to Zone F in Fig. 96.

This operation demonstrated the feasibility of manipulating multiple soft robots 451, 452 forming multiple instruments under the same magnetic field 5. The settings of the magnetization profiles of the soft robots 451, 452 are relatively simple, resulting in straightforward deformations. More complex deformations or functionalities can be achieved by employing more complex magnetization designs, such as the helical transformations shown in the seventh configuration of the body 10 for 1D magnetization reprogramming, as disclosed in Figs. 26 to 51.

The devices and methods according to the present disclosure may also be used in three further scenarios or environments based on the typical application setting depicted in Fig. 92. Experimental phantoms for these scenarios were fabricated using 3D printing (Form 3B+, Formlabs, USA) with Elastic 50A as the printing material. The instruments used in these operations are the same as those in Fig. 95.

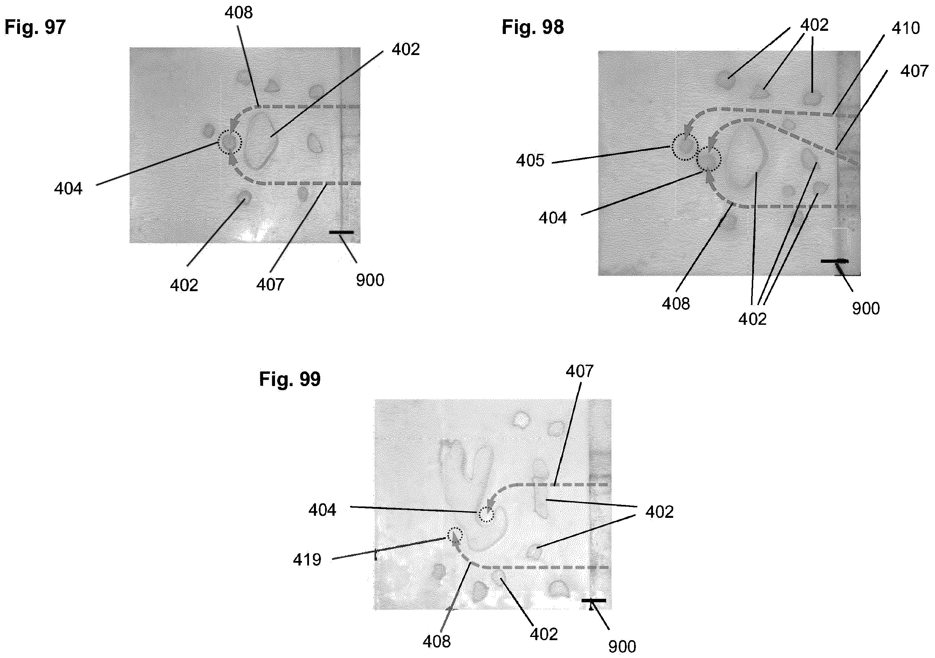

The first experimental scenario or environment, as illustrated in Fig. 97, involves numerous tissues between the target 404 and the entry site, which act as obstacles 402 to diagnostic or therapeutic procedures. The scale bar 900 in Fig. 97 is 5 mm.

The target 404 requires the coordinated action of two instruments formed by soft robots 1: one instrument follows a path 408 to the target 404 to complete preliminary treatment tasks; subsequently, a second instrument follows a further path 407 to the target 404 to administer adjunctive therapy. Thereby, the first soft robot 451, which is configured as a soft tube, travels along the path 408, it initially maintains a straight state under a uniform magnetic field 5 (90 mT, directed downwards) due to the obstacles 402 within the path 408. Upon reaching the location of the target 404, it transforms into a curved state under the same uniform magnetic field 5 by enlarging the distance 51, enabling its tip to reach the position of the target 404. To maintain the shape of the first soft robot 451, the magnetic field 5 is kept constant. Then, the second soft robot 452, configured as a soft tube, operating under the same uniform magnetic field 5, traverses the obstacles 402 in a straight state. Near the location of the target 404, the second soft robot 452 transitions to a curved state to position its tip formed by the free end 24 at the target 404. After completing all diagnostic and therapeutic procedures, the two soft robots 451, 452 revert to a straight state to retract through the obstacles 402.

The second experimental scenario or environment has the target 404 and a further target 405, as shown in Fig. 98. These targets 404, 405 require sequential operations/treatments, necessitating three instruments formed by the first soft robot 451, the second soft robot 452, and the third soft robot 453, which follow paths 408, further path 407, and additional further path 410 to reach targets 404, 405, respectively. After completing the tasks at the targets 404, 405, the instruments are retracted. The soft robots 451, 452, 453 used as instruments are the same as those depicted in Fig. 95, with the third soft robot 453 being configured like the second soft robot 452. The scale bar 900 in Fig. 98 is 5 mm.

Similar to the first experimental scenario, the entire operation takes place under a 90 mT downward uniform magnetic field 5. The three soft robots 451, 452, 453 forming the instruments navigate through the obstacles 402 in a straight state and then adjust their shapes to position their tips formed by the free ends 24 at the targets 404, 405. Finally, they retract back through the obstacles 402 in a straight state after completing the procedures.

The third experimental scenario or environment shown in Fig. 99 comprises a target 404 that requires one instrument formed by the first soft robot 451 to perform auxiliary operations near position 419 along the path 408, and another instrument formed by the second soft robot 452 to proceed along the further path 407 to carry out diagnostic and therapeutic operations at the location of the target 404. However, the further path 407 is obstructed by barriers, necessitating an additional instrument formed by the third soft robot 453 to clear the pathway. The scale bar 900 is 5 mm.

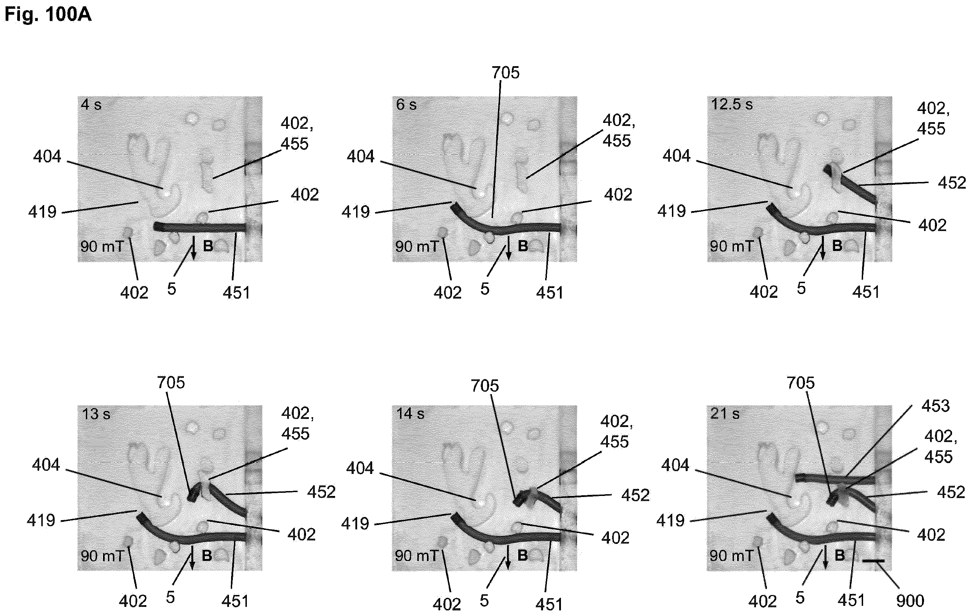

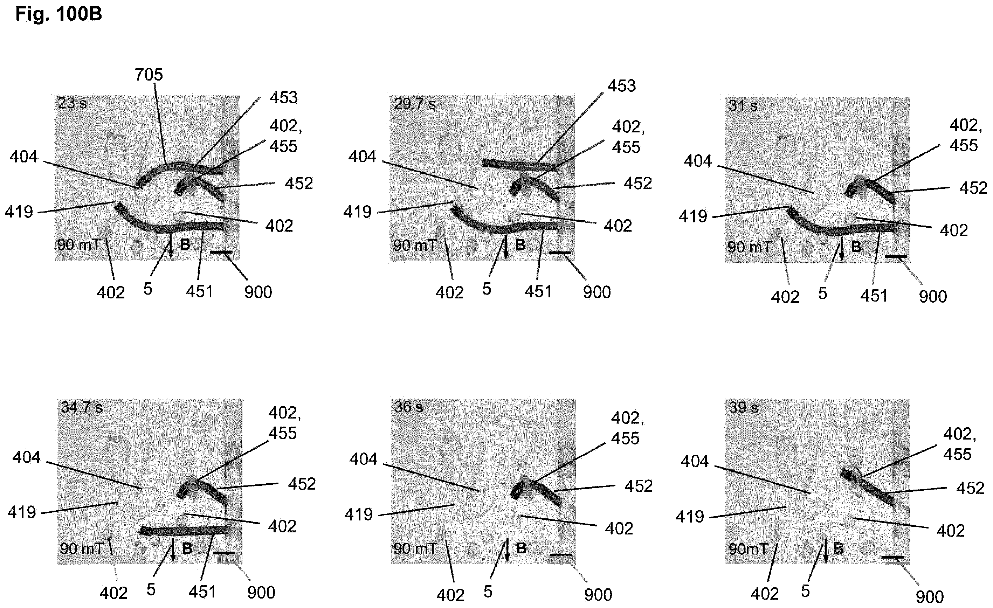

Fig. 100 depicts images of operation of the first soft robot 451, the second soft robot 452 and the third soft robot 451 in the third environment of Fig. 99. The soft robots 451, 452, 453 used as instruments in this operation are the same as those in the second experimental scenario, and the entire procedure is conducted under a 90 mT downward uniform magnetic field 5. Fig. 100 illustrates the entire procedure with Fig. 100A showing the first part of the procedure up to 21 seconds and Fig. 100B showing the second part of the procedure. Before 12.5 s, the first soft robot 451 forming a first soft tube passes through obstacles 402 in a straight state along the path 408, then adjusts to a deformation 705 configured as a curved state to reach position 419 and maintains that shape. Between 12.5 s and 14 s, the second soft robot 452 forming a secondary soft tube enters the obstacles 402 in a straight state, then undergoes a deformation 705 to a curved state to hook onto one of the obstacles 404, which forms an obstruction 455. Subsequently, the secondary soft tube retracts, pulling the obstruction 455 with it, and maintains this state to clear the further path 407. From 21s to 23s, the third soft robot 453 forming a main soft tube travels in a straight state along the further path 407 near to the location of the target 404, then undergoes a deformation 705 to a changed or bent shape to reach the target 404. After completing the diagnostic and therapeutic operations (not shown), all soft robots 451, 452, 453 revert to a straight state and retract through the entry site.

The above design utilizes the magnetic neutralization feature and the second configuration of the body 10 for 1D magnetization reprogramming shown in Figs. 7 to 12. Since the direction of the magnetic field 5 remains constant, the resulting operation is confined to a single plane. The soft robots 1 formed by the tubes may also operate beyond this plane (i.e., 3D operation). One approach is to rotate the external magnetic field 5. However, this method is only effective for specific tasks, as changes in the magnetic field direction may interfere with the functioning of other soft robots or tubes (medical instruments). A more effective solution is to adopt the seventh configuration of the body 10 for 1D magnetization reprogramming shown in Figs. 26 to 29. The seventh configuration enables 3D deformation under a uniaxial constant magnetic field 5. By combining the seventh configuration with the magnetic neutralization feature, it becomes possible to achieve both diverse 3D deformation modes and non-deformation states under a constant magnetic field 5, see Figs. 26 to 29. This capability makes it particularly well-suited for realizing 3D coordinated multi-instrument operations.

The foregoing experiments validate the feasibility of performing coordinated multi-instrument operations under the same magnetic field 5. The execution of complex maneuvers has been demonstrated but it had not been detailed how to carry out specific diagnostics or treatments. Diagnostics and treatments could involve actions like drug delivery or sampling, which can be achieved by equipping the soft robots 1, 451, 452, 453 forming the instruments with internal drug reservoirs or sampling needles. These operations can be tailored to specific surgical requirements; however, these modifications do not fundamentally affect the feasibility of coordinated multi-instrument operations and are therefore not further discussed in this disclosure.

Additionally, the shape of the soft robots 1, 451, 452, 453 forming the instruments/soft tubes is influenced by external magnetic fields 5, the magnetic moments of the magnetic units 52, 62, the distance 51 between the magnetic units 52, 62, and the elastic modulus of the soft tube forming the outer and inner parts 20, 30, 40. These effects are analyzed and discussed theoretically, through simulation, and experimentally below. By strategically designing these factors, the necessary shapes and maneuvers for applications can be achieved. The coordinated multi-instrument operation method proposed in this disclosure allows instruments formed by soft robots 1, 451, 452, 453 to be selectively influenced by, or shielded from, external magnetic fields 5 and enables the generation of required deformations based on specific needs without altering external magnetic fields 5. This characteristic makes it possible to control multiple instruments formed by soft robots 1, 451, 452, 453 under the same magnetic field 5, thereby enabling tasks that cannot be accomplished with existing methods. The devices and methods according to the present disclosure provide potential applications in medical settings and can be extended to other scenarios, such as micro-assembly and collaborative operation of multiple robotic arms, for example in industrial manufacturing and processing environments.

### Application IV: Shape adaptable soft grippers

Compared to traditional grippers, soft grippers exhibit unique advantages in interacting with objects due to their excellent compliance and adaptability. Their common actuation mechanisms include cable-driven actuation (83-85), pneumatic-driven actuation (86-88), dielectric elastomer actuators (89-91), and shape memory alloys (92-94). In contrast to these actuation methods, magnetically-driven actuation offers notable advantages in reducing size and simplifying components. The manipulation can be easily accomplished when utilizing magnetic fields to control soft grippers; however, it is generally limited to basic gripping actions. The actuation of soft grippers depends on their magnetization profiles and external magnetic fields, posing challenges for scenarios requiring adaptive gripping conforming to the shape of the object. Achieving specific shapes in soft grippers through external magnetic fields results in a limited number of shapes, satisfying only highly specialized needs.

The approach according to the present disclosure involves modifying the magnetization profile of the soft gripper itself to achieve the necessary deformations for effective gripping - this method may be termed "real-time in-situ magnetization reprogramming."

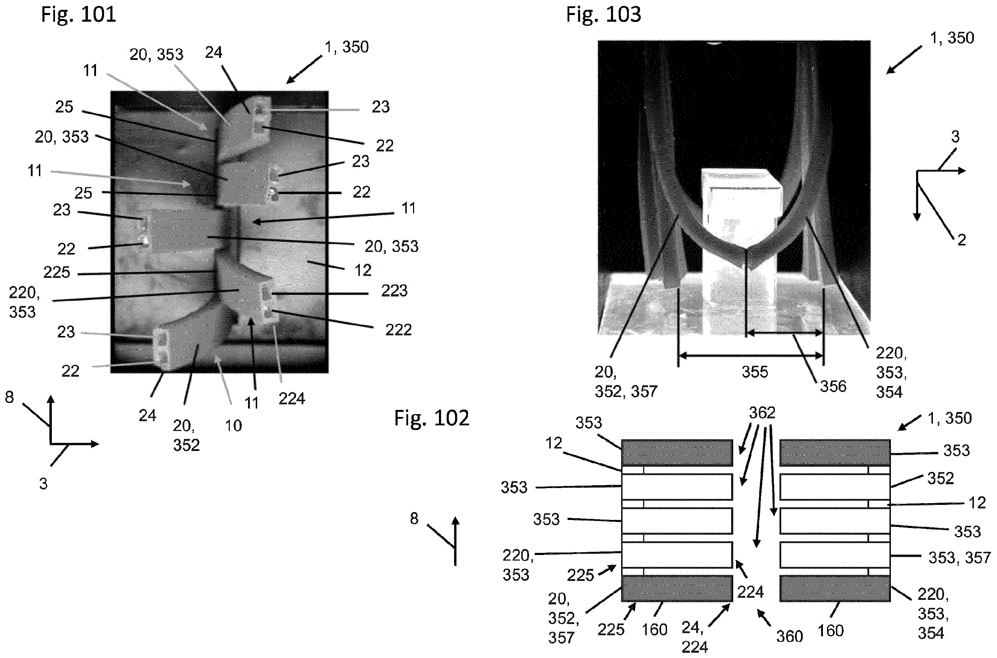

Fig. 101 depicts a soft robot that is configured as a soft gripper 350. The soft gripper 350 comprises a body 10 that has an outer part 20 that is configured as a flexible finger 352 of the soft gripper 350. As far as no differences are disclosed in the description and the Figures, the body 10 is configured as it is disclosed for the first configuration of the body 10 having the flat sheet 160 as the outer part 20, as shown in Figs. 31 and 32. With the outer part 20 forming the flexible finger 352, the channel 22 and the further channel 23 are located directly adjacent to each other. The magnetic unit 52 located within the channel 22 has a magnetic moment with a direction that is opposite to the direction of the magnetic moment of the further magnetic unit 62 located within the further channel 23. Furthermore, a magnitude of the magnetic moment of the magnetic unit 52 equals a magnitude of the magnetic moment of the further magnetic unit 62.

The outer part 20 is fixed to a support 12 at the second end 25 and the first end 24 of the outer part 20 forms a free end.

The magnetic unit 52 is configured to be moved between a first position with respect to the outer part 20 forming the flexible finger 352 and a second position with respect to the outer part 20. Exemplarily, the magnetic unit 52 is located at the free end 24 of the outer part 20 in the second position and retracted from the free end 24 of the outer part 20 in the first position. For example, the magnetic unit 52 may be completely retracted into the support 12 at the first position.

The further magnetic unit 62 is configured to be moved between a first position with respect to the outer part 20 forming the flexible finger 352 and a second position with respect to the outer part 20. Exemplarily, the further magnetic unit 62 is located at the free end 24 of the outer part 20 in the second position and retracted from the free end 24 of the outer part 20 in the first position. For example, the further magnetic unit 62 may be completely retracted into the support 12 at the first position.

As shown in Fig. 101, the soft gripper 350 comprises an additional body 11, that is configured as it is disclosed for the body 10 of the soft gripper 350. The outer part 20 of the additional body 11 forms an additional outer part 220 having an additional channel 222 and the inner part 30 of the additional body 11 forms an additional inner part. Furthermore, the magnetic unit arranged within the additional channel 222 forms an additional magnetic unit. The further channel of the additional outer part 220 forms an additional further channel 223 and the further magnetic unit arranged within the additional further channel 223 forms an additional further magnetic unit.

The additional outer part 220 forms a flexible additional finger 353 of the soft gripper 350. It has a free first end 224 and a second end 225 that is fixed to the support 12.

The outer part 20 and the additional outer part 220 are located next to each other in a width direction 8. Thereby, the width direction 8 is orientated parallel to a width direction of the flat sheets 160 forming the outer part 20 and the additional outer part 222.

Furthermore, the soft gripper 350 comprises three further additional bodies 11, which are configured like the additional body 11 and which are arranged one after the other along the width direction 8. With other embodiments, the soft gripper 350 may comprise a smaller or larger number of further additional bodies 11.

The outer parts 20, 220 are configured to bend along a bending direction 3 within the magnetic field 5, whereby the bending direction 3 is perpendicular to the width direction 8 and a length direction 2 that runs parallel to the channels 22, 23, 222, 223. The outer parts 20, 220 thereby may bend in the bending direction 3 or against the bending direction 3.

While Fig. 101 depicts a detailed top view of the soft gripper 350, Fig. 102 depicts a schematic layout of the soft gripper 350 and Fig. 103 depicts a side view of the soft gripper 350 in the width direction 8.

The soft gripper 350 comprises a further row of further outer parts that are located opposite to the row of outer parts shown in Fig. 101 in the bending direction 3. The soft gripper 350 thus comprises pairs of flexible outer parts 20, 220 that are located opposite to each other in the bending direction 3. With other embodiments, the soft gripper 350 may comprise at least one pair of flexible outer parts 20, 220 that are located opposite to each other in the bending direction 3, such as a pair formed by the outer part 20 located at the bottom left in Fig. 102 and a flexible additional outer part 220 forming a flexible additional finger 353 located opposite to the outer part 20 in the bending direction 3.

For each outer part 20, 220, a corresponding opposing outer part 20, 220 located opposite to the respective outer part 20, 220 in the bending direction 3 forms a counterpart. For example, with the outer part 20 at the bottom left in Fig. 102, the additional outer part 220 forms the counterpart 354 of the flexible finger 252. Likewise, the outer part 20 forms an additional counterpart 357 of the additional outer part 220 at the bottom right in Fig. 102.

Each pair of opposing outer parts 20, 220 forms a gripper part of the soft gripper 350. Therefore, the flexible gripper 350 has at least a gripper part 360. With the exemplary embodiment, the soft gripper 350 has additional gripper parts 362 that are located one after the other next to the gripper part 360 in the width direction 8. The soft gripper 350 has four additional gripper parts 362.

As can be seen from Fig. 103, a distance between the outer part 20 and the counterpart 354 may be adjusted by adjusting the position of the magnetic unit 52 and the further magnetic unit 62 within the outer part 20. With the magnetic unit 52 at the first position, the outer part 20 has a first distance 355 from the counterpart 354 and, with the magnetic unit 52 at the second position, the outer part 20 has a second distance 356 from the counterpart 354, whereby the second distance 356 is smaller than the first distance 355. Thereby, the outer part 20 bends in a first direction. During bending in the first direction, the further magnetic unit 53 is located at the first position away from the free end 24.

By keeping the magnetic unit 52 at the first position away from the free end 24 and by moving the further magnetic unit 62 to the free end 24, the outer part 20 is configured to bend in a second direction opposite to the first direction. Therefore, the outer part 20 is straight if both the magnetic unit 52 and the further magnetic unit 62 are located in their respective first positions and the outer part 20 bends in the second direction if the further magnetic unit 62 is moved to the second position at the free end 24.

As can be seen from Fig. 103, the gripper part 360 formed by the outer part 20 and the additional outer part 220 may completely close by bending the outer part 20 in the first direction and by bending the additional outer part 122 forming the counterpart 354 in the second direction.

In summary, the soft gripper 350 shown in Figs. 101, 102 and 103 is hand-like, featuring five pairs of strip-shaped fingers 352, 353, each of which can bend forwards in the first direction or backwards in the second direction independently or in combination under the same magnetic field 5.

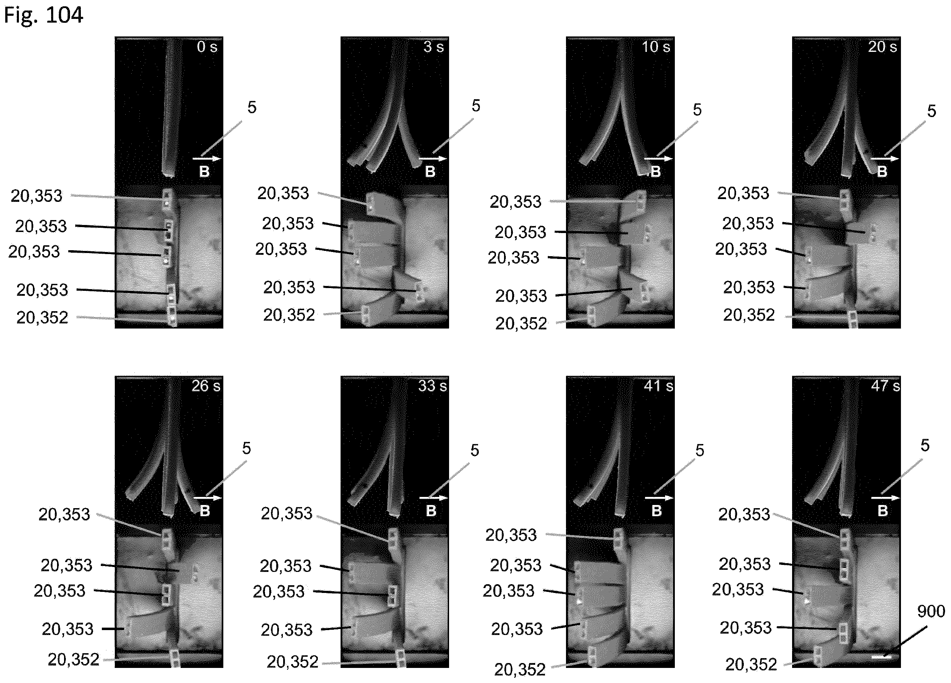

Fig. 104 shows images of various bending modes of the soft gripper 350 under the same uniform magnetic field 5, such as a magnetic field of B = 35 mT directed to the right, wherein the upper parts of the images depict the side view in the width direction 8 and the lower parts of the images depict the corresponding top view onto the outer parts 20, 220.

The gripper 350 has the ability to flexibly bend like a human hand under the same uniform magnetic field 5, such as a magnetic field of B = 35 mT. The gripper 350 may realize various bends by altering the positions of the magnetic units 52, 62 within each outer part 20, 220, forming the fingers 352, 353, which changes the magnetization profiles of the individual outer parts 20, 220 in the same magnetic field 5.

The gripper 350 is further configured to handle objects of various shapes. For example, it may have the functionality of a human hand by positioning gripper parts 360, 362 side by side in a row, allowing them to collaborate in performing diverse gripping tasks.

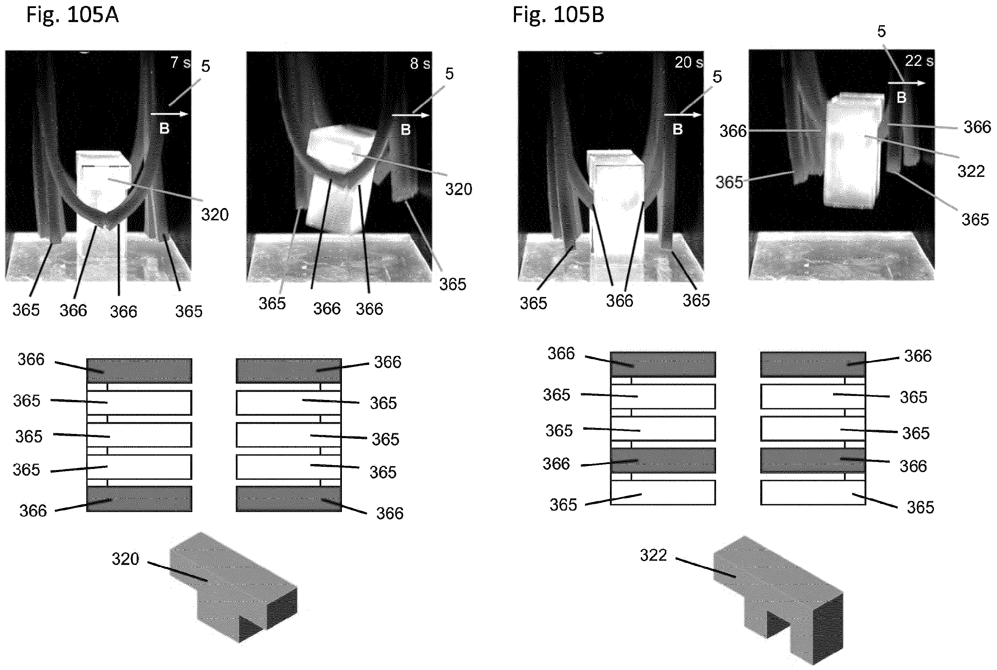

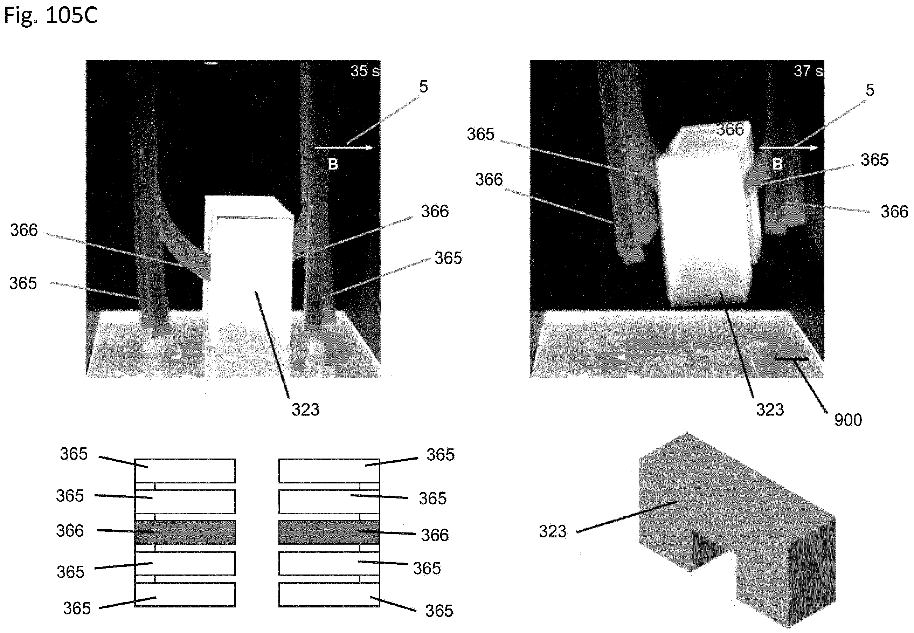

Fig. 105 depicts the gripper 350 having the two rows of outer parts 20, 220 forming the flexible fingers 352, 353 placed side by side, which are configured to grasp different objects 320, 322, 323. All operations are conducted under the same uniform magnetic field 5, with the direction of the magnetic field 5 pointing right and the magnetic field having a magnitude of B = 60 mT. Each Figure 105A, 105B, 105C includes two images of the gripping action, a schematic diagram describing the state of the gripper 350, and another schematic diagram illustrating the shape of the object 320, 322, 323 being grasped. In the schematic diagram illustrating the state of the gripper 350, the two rows on the left and right correspond to the two hand-like rows of flexible fingers 352, 353. Each row comprises five rectangles, representing the five fingers 352, 353 of the hand-like gripper section. The light portions indicate inactive outer parts 365 in a relaxed straight position, while the dark (red) portions denote active outer parts 366 in a bent position (grasping state).

Fig. 105A depicts grasping a T-shaped first object 320. Fig. 105A depicts grasping an F-shaped second object 322. Fig. 105C depicts grasping an n-shaped third object 323. The scale bars in Fig. 105 have a length of 5mm.

The objects 320, 322, 323 may be grasped under the same uniform magnetic field 5, exemplarily having a magnitude of B = 60 mT, and having a constant direction along the bending direction 3, the direction exemplarily being to the right in Fig. 105. To grasp the T-shaped first object 320, the first and fifth outer parts 20, 220 of each row are simultaneously configured as active outer parts 366 and the remaining outer parts 20, 220 are configured as inactive outer parts 365, thereby matching the shape of the T-shaped first object 320 and thus successfully completing the grip. Similarly, when gripping the F-shaped and n-shaped second and third objects 322, 323, outer parts 20, 220 matching the shape of the respective one of the second and third object 322, 323 are configured as active outer parts 366 that bend in the bending direction 3 and the remaining outer parts 20, 220 are configured as inactive outer parts 365 that keep a straight shape. The steps of operating the soft grouper 350 shown in Fig. 105 demonstrate that the shape-adaptable soft gripper 350 can effectively grasp objects 320, 322, 323 of different shapes under the same magnetic field 5.

In general, the soft robot 1 configured as the soft gripper 350 has a multitude of outer parts 20, 220, whereby each outer part 20, 220 may be selectively configured as an active outer part 366 that has a first shape or as an inactive outer part 365 that has a second shape. The first shape may be a bent shape. The second shape may be a straight shape or a shape bent in direction different from, such as opposite to, a direction in which the first shape is bent.

The active outer part 366 thereby has the magnetic unit 52 in the second position and the inactive outer part 365 has the magnetic unit 52 in the first position. Furthermore, the soft gripper 350 is configured to activate a first group of active outer parts 366 to manipulate the first object 320 and to activate a second group of active outer parts 366 to manipulate the second object 322. Thereby the first group is different from the second group.

The foregoing examples demonstrate that the devices and methods according to the present disclosure allow for successfully grasping objects 320, 322, 323 of three different shapes. These shapes 320, 322, 323 have the potential to be further developed into more complex forms. In practical applications, there may be a need to handle objects with various complex shapes, and it is possible to develop more intricate designs of the soft gripper 350 based on the devices and methods of the present disclosure to meet such demands. Additionally, for ease of manufacturing and demonstration, the shape-adaptable soft gripper 350 described in the foregoing has centimeter-scale dimensions. However, this does not limit the applicability of the principles of the present disclosure to smaller scales.

### Discussion

The present disclosure is directed to a magnetization reprogramming method that can achieve real-time in-situ reprogramming not possible with existing methods from the prior art. The devices and methods for magnetization reprogramming in 1D, 2D, and 3D soft robot morphologies allow for various configurations and corresponding deformations. In addition, the devices and methods according to the present disclosure exhibit two distinctive features, magnetic neutralization and magnetic reversal, which provide significant advantages over current techniques. The potential of the method according to the present disclosure is validated by four distinct applications: it can enable contact-free navigation through avoidance objects 402; modify motion patterns of cilia 310 through targeted activation, bending amplitude modulation, and phase modulation for complex functions; allow for independent and coordinated operations of multiple instruments 400, 401 under a single magnetic field 5; and achieve grasping of objects 320, 322, 323 with varying shapes. These applications demonstrate functionalities beyond existing research capabilities, pushing the boundaries in their respective fields. The devices and methods according to the present disclosure free magnetic soft robots 1 from complete dependence on external magnetic fields 5 for morphological transformations, lessening the requirement for intricate magnetic field generation systems. Moreover, they enable novel modes and diversity in deformations, significantly expanding the range of uses for magnetic actuation technology.

The following considerations are instrumental in advancing the application of the devices and methods according to the present disclosure. First, where needed, the impact of stiffness variations caused by positional changes between the components of the soft robot 1 on its deformation may be considered. For instance, when utilizing the magnetic reversal feature of the second configuration of the cylindrical body 10 for 1D magnetization reprogramming, the degrees of bending to the left and right may be not identical, see Fig. 9D. This may be addressed by modeling methods and solutions detailed below. These are optional and may be implented when precise deformation control is necessary.

Second, the form and size of the magnetic units 52, 62 may be considered in relation to the demands of specific application scenarios, striking an appropriate trade-off. The form and size of the magnetic units 52, 62 directly impact the precision of the magnetization profile generation. Smaller dimensions lead to higher precision in creating these profiles; however, the complexity of fabrication and control increases accordingly. For instance, with the seventh configuration of the cylindrical body 10 for 1D magnetization reprogramming, see Fig. 26, the magnetic elements 50, 60 may comprise magnetic particles approximately 50 micrometers in size. In Application II detailed above, the magnetic units 52, 62 may be permanent magnets sized Φ0.75mm × 1mm. Although the precision of magnetization profiles generated using permanent magnets is lower than that achieved with 50-micrometer magnetic particles, both configurations satisfy the application requirements adequately.

Finally, possible solutions may be considered when applying the devices and methods according to the present disclosure on more minor scales. For miniaturization, the devices and methods according to the present disclosure easily apply at millimeter and sub-millimeter scales. However, further size reduction introduces two main issues: friction between components and positional accuracy of magnetic units 52, 62. Friction can be addressed by improving manufacturing precision and lubrication. Positional accuracy can be enhanced with precise positional control systems, and their complexity increases as size decreases. For even smaller scales, alternative actuation mechanisms such as thermal, optical, or chemical actuation may be implemented to overcome the limitations of positional control systems.

### Materials and Methods

### Fabrication

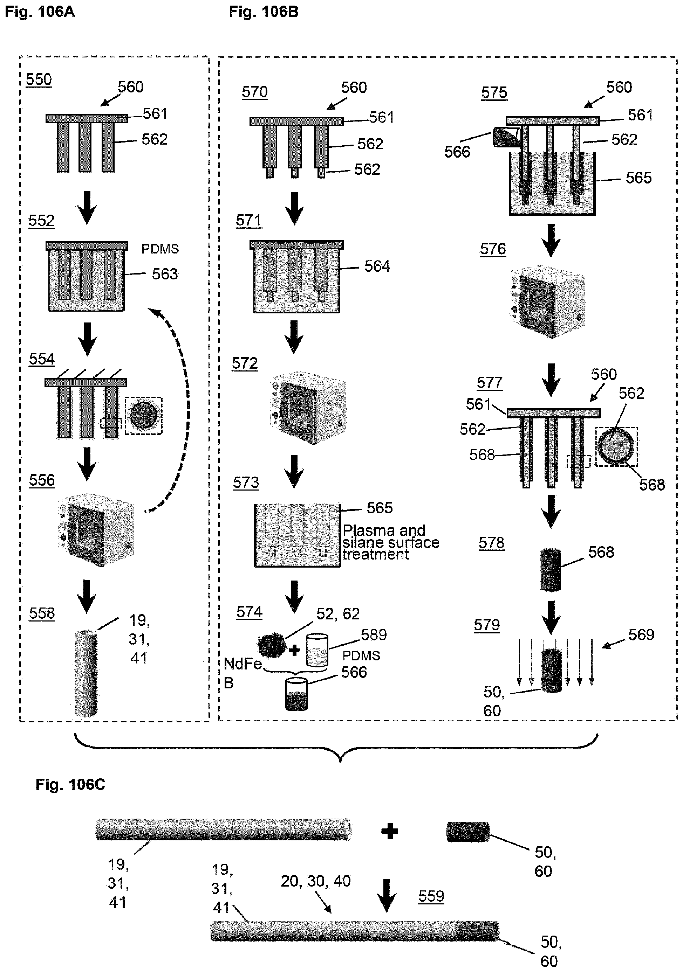

All the outer parts 20, and inner parts 30, 40 involved may be fabricated as soft tubes using a dip-coating molding method. As shown in Fig. 106A, initially, a positive mold is provided (550) by fixing a commercial pin 562 (steel test pin, ORION, DE) onto a flat plate 561 to form the positive mold 560. Subsequently, this mold 560 is placed into a container with a part material 563 of the flexible parts 20, 30, 40 and the mold 560 is immersed (552) in the part material 563, such as in a polymer, such as mixed Polydimethylsiloxane (PDMS) (Sylgard 184, Dow Corning, US) with a base-to-curing agent mass ratio of 20:1. The coated mold 560 is then positioned or suspended vertically (554) and cured (556) in an oven at 70°C. Finally, by repeating the steps of coating 552, 554 and curing 556, a soft tube of the desired thickness is obtained, the soft tube forming a flexible part 19 of the outer part 20, the flexible part 31 of the inner part 30, or the flexible part 41 of the further inner part 40. In this process, the mass ratio of base to curing agent in the part material 563, such as PDMS, can be adjusted according to requirements. Steps 552, 5554, 556 can be repeated to obtain soft tubes with varying wall thicknesses.

Fig. 106B depicts a fabrication method for magnetic soft tubes, which may form the magnetic elements 50, 60. Thereby, Neodymium Iron Boron (NdFeB) may be used as magnetic material. Fig. 106C depicts steps for assembling the soft tubes of Fig. 106A and the magnetic soft tubes of Fig. 106B by connecting 559 them together, for example by using PDMS. After connecting 559, the assembly may be cured in an oven to solidify the PDMS.

The entire outer part 20, inner part 34, and further inner part 40 may be fabricated as soft tubes containing magnetic units 52, 62. Such soft tubes containing magnetic units 52, 62 may be fabricated by first creating a soft tube, as detailed in Fig. 106A, and then connecting it with a soft tubular magnetic element 52, 62 fabricated with the method of Fig. 106B.

As shown in Fig. 106B, the magnetic element 50, 60 may be manufactured using a molding method. Initially, a positive mold 560 is produced (570) using 3D printing technology. For example, a Form 3B printer by Formlabs, US may be used with Clear V4 as the specified printing material. The positive mold 560 may subsequently receive plasma treatment to enhance its hydrophilicity. Subsequently, the positive mold 560 may receive a silanization treatment, for example with trichloro(1H,1H,2H,2H-perfluorooctyl) silane (97%, Merck KgaA, DE), to facilitate demolding. The positive mold 560 is then immersed (571) into a mold material 564, for example a material comprising a base and curing agent of Dragon Skin 10 (Smooth-On Inc., US) mixed in a 1:1 mass ratio. Afterwards degassing and curing 572, such as after 10 minutes of degassing and curing 572 at 90°C for 1 hour, the positive mold 560 is removed (573) to obtain a negative mold 565. The negative mold 565 may then be subjected to plasma treatment and silanization.

Next, the method comprises providing 574 a mixture 566 comprising magnetic units 52, 62, such as magnetic particles, for example Neodymium Iron Boron (NdFeB) microparticles (MQP-15-7, Magnequench GmbH, DE), within a fluid substrate material 589, such as a fluid polymer, for example PDMS (base and curing agent mixed in a 10:1 mass ratio). Thereby, the magnetic units 52, 62 and the substrate material 589 are blended, for example in a certain proportion, which vary according to different requirements. The mixture 566 is then filled (575) into the negative mold 565, in which a complementary positive mold 560 defining the channels 22, 23, 32, 42 is placed, for example a 3D-printed positive mold. Subsequently, the mixture 566 within the mold comprising the negative mold 565 and the positive mold 560 is degassed in a vacuum chamber and subsequently cured (567) in an oven, for example at 90°C for 1 hour. The molds 565, 566 are then removed (578) to obtain a magnetic tube 568. Finally, the magnetic tube 568 is magnetized (579) in a magnetic field 569, for example in a vibrating sample magnetometer (EZ7, MicroSense, USA) at 1.8 T, to form the magnetic elements 50, 60.

As shown in Fig. 106C, the element 50, 60 formed by the magnetic soft tube is then connected (559) to the soft tube forming the flexible element 19, 31, 41 using the part material 563, such as PDMS, and the assembly is then cured in an oven to obtain a soft tube containing magnetic units 52, 62.

To facilitate processing and manufacturing, magnetic soft rods, which form solid cylindrical members, may be used to replace the inner magnetic soft tubes in the composition of bodies 10 with multilayer tubes.

A possible fabrication method for a magnetic soft rod is similar to that for the magnetic soft tube. The fabrication steps for the magnetic soft rod are simpler than those for the magnetic soft tube, with the main difference being the type of mold used.

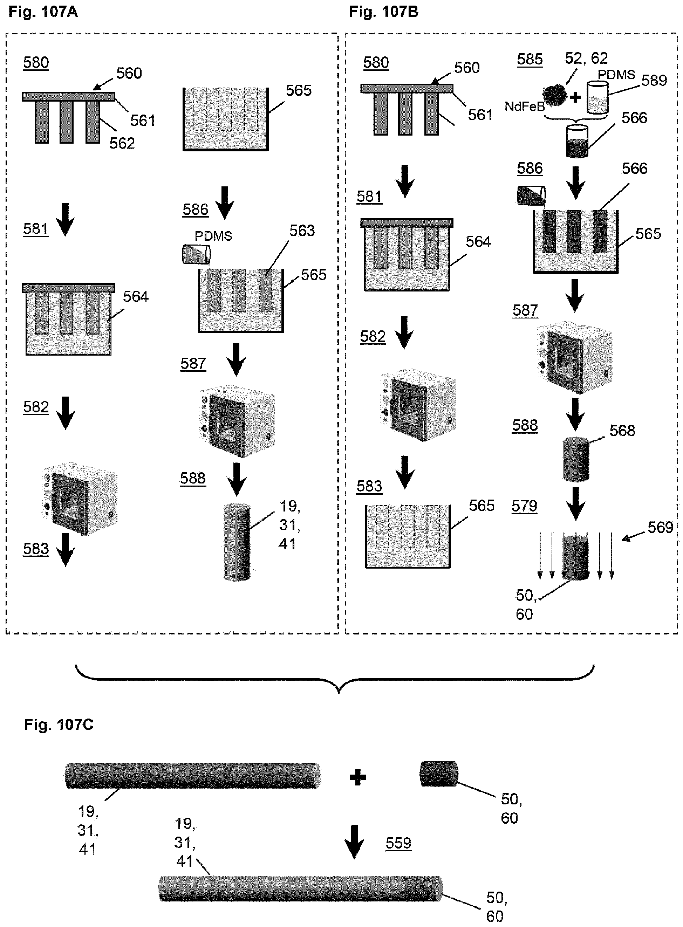

Fig. 107A depicts method steps for fabricating a solid rod that may form the flexible member 19, 31, 41 of the outer and inner part 20, 30, 40. The method comprises providing 580 a positive mold 564 defining the outer diameter of the solid rod. The positive mold 564 comprises a plate 561 and at least one pin 562, such as several pins, that are fixed to the plate 561. The method then comprises placing 581 the positive mold 560 into the mold material 564, which may be configured as described before. The mold material 564 in the positive mold 560 is then degassed in a vacuum chamber. The method then comprises curing 582 the mold material 564 to obtain a negative mold 565 and removing 583 the negative mold 565 from the positive mold 562. The negative mold 565 then receives a surface treatment, for example with plasma and silane. Subsequently, the method comprises filling 586 the negative mold 565 with the part material 563, for example PDMS as detailed above. The part material 563 may then be degassed in a vacuum chamber and cured 587 in an oven. Finally, the method comprises removing 588 the part material 563 forming the soft rod from the negative mold 565 to obtain the flexible part 19, 31, 41 formed by the soft rod.

Fig. 107B depicts method steps for fabricating a solid magnetic rod forming the magnetic elements 52, 62. The method comprises providing 580 a positive mold 564 defining the outer diameter of the magnetic elements 52, 62. The positive mold 564 comprises a plate 561 and several pins 562 that are fixed to the plate 561. The method then comprises placing 581 the positive mold 560 into the mold material 564. The mold material 564 in the positive mold 560 is then degassed in a vacuum chamber. The method then comprises curing 582 the mold material 564 to obtain a negative mold 565 and removing 583 the negative mold 565 from the positive mold 562. The negative mold 565 then receives a surface treatment, for example with plasma and silane. Subsequently, the method comprises providing 585 the mixture 566 containing the magnetic units 52, 62 and the fluid 589, which may be configured as detailed above and which may comprise the part material 563 as the fluid 589. The method then comprises filling 586 the negative mold 565 with the mixture 566. The mixture 566 may then be degassed in a vacuum chamber and cured 587 in an oven. Finally, the method comprises removing 588 the soft magnetic rod from the negative mold 565. Finally, the soft magnetic rod is magnetized (579) in a magnetic field 569, for example in a vibrating sample magnetometer (EZ7, MicroSense, USA) at 1.8 T, to form the magnetic element 50, 60 as the magnetic soft rod.

As detailed in Fig. 107C, the soft rod forming the flexible element 19, 31, 41 and the magnetic soft rod forming the magnetic element 50, 60 can be connected (559) together using the part material 563, such as PDMS. After connection, the assembly may be cured in an oven to solidify the part material 563.

A method for fabricating the spirally deformable soft tube of the seventh configuration of the tubular body 10 for 1D magnetization reprogramming shown in Fig. 26 to 29 may comprise the following steps. Initially, a magnetic soft tube may be formed by the steps 550 to 558 of the dip-coating molding method from Fig. 106A. Thereby, the part material 563 used in step 552 may comprise a silicone, such as Ecoflex (ECO-FLEX^{™} 0050, KauPo, DE). Additionally, the part material 563 may comprise a cure retarder, such as Platinum Silicone Cure Retarder (SLO-JO^{™}/1, KauPo, DE). The part material 563 may further comprise the magnetic units 52, 62 as particles, such as NdFeB microparticles (MOP-15-7, Magnequench GmbH, DE). The part material 563 may be prepared with a mass ratio of Retarder to Ecoflex at 3%, and the ratio of Ecoflex to microparticles may be adjusted as per specific requirements. After obtaining the magnetic soft tube, it is placed in the jig 810, which may have a diameter Dₘ of 12 mm, and the magnetic soft tube is fixed in place, see Fig. 27. The soft tube is then magnetized in the magnetization field 569, for example by using a 1.8T magnetization field 569 in a vibrating sample magnetometer, resulting in the desired magnetic moment. The soft tube may form the magnetic elements 50, 60 of the outer and inner parts 20, 30, 40.

A fabrication method of magnetic rods and non-magnetic rods, which may be paired with the spiral-deformable soft tube of the seventh configuration of the tubular body 10, may comprise the same steps as those described for the production of the soft rods and the magnetic soft rods shown in Fig. 107.

With the seventh configuration of the tubular body 10 shown in Fig. 26, the outer part 20 with the further magnetic element 60 may be magnetized in a magnetization field 569 that is opposite to a magnetization field 569 used for magnetizing the magnetic element 50 of the inner part 30.

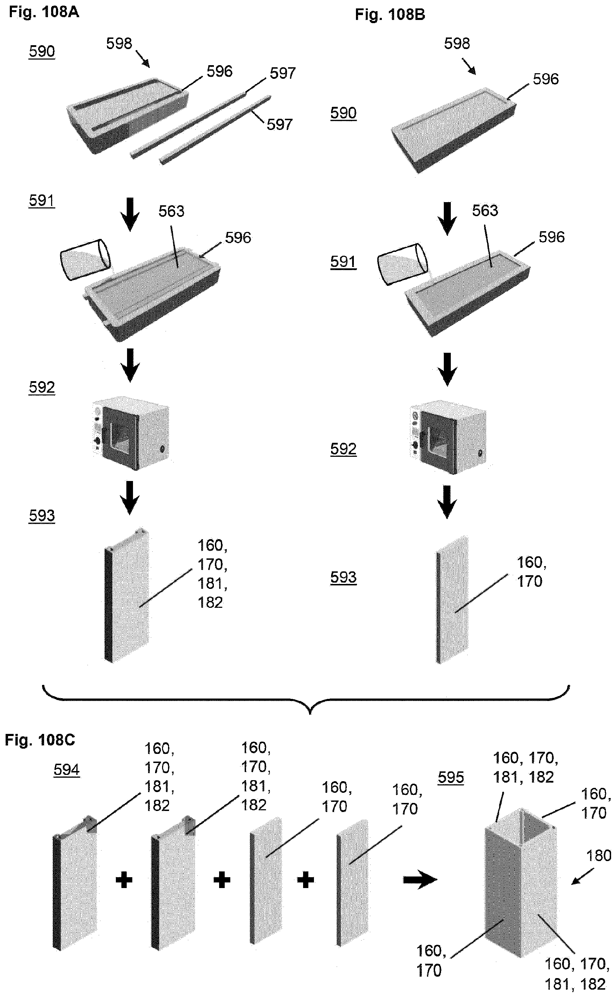

Fig. 108 depicts a fabrication method for the two-dimensional flat sheets 160 and the three-dimensional box-shaped outer part 20 forming the box 180. Fig. 108A thereby depicts a fabrication method for a perforated sheet having the channels 22, 23 and Fig. 108B depicts a fabrication method for an unperforated sheet 160 having no channels. Fig. 108C depicts a fabrication method for the three-dimensional box-shaped soft structure forming the box 180.

The method for producing the perforated sheet used as the body 10 for 2D magnetization reprogramming shown in Fig. 108A comprises providing (590) parts of a three-dimensional mold 598, for example by 3D printing. The mold 598 may comprise a base 596 having slots and defining the outer walls of the channels 22, 23. The base may comprise receptacles for receiving inner mold parts 597 that define the interior of the channels 22, 23. The inner mold parts 597 may be configured as elongated rods. When being placed within the receptacles, the inner mold parts 597 are located within the slots of the base and allow the molding material to flow around the inner mold parts 597. The receptacles may be configured as holes within the outer wall of the base 596 and the inner mold parts 597 may be configured as rods, such as square rods.

The mold 598 may receive a surface treatment, for example with plasma and silane. The method then comprises a step 591 of assembling the mold 598 and filling the mold 598 with a part material 563. The part material 563 may comprise a mixture of Ecoflex (ECOFLEX^{™} 0030, KauPo, DE) and platinum silicone cure retarder (SLO-JO^{™}/1, KauPo, DE) having a mass ratio of the retarder to Ecoflex at 3%. The mold 598 with the part material 563 is then placed in a vacuum chamber to remove air and, for example after 10 minutes, cured 592 in an oven, for example for 60 minutes. Upon completion of curing, the method comprises removing 593 the mold 598 to obtain the perforated sheet 160 having the channels 22, 23.

The rod with magnetic units 52, 62 for pairing with this perforated sheet is fabricated in a manner similar to the magnetic soft rods, as shown in Fig. 107. The rod may be fabricated as a square rod, and the primary difference then is replacing the pins 562 having a cylindrical shape with pins having a square shape.

As shown in Fig. 108C, a method for fabricating a 3D box-shaped soft structure forming the box 180 comprises providing (594) multiple sidewalls 170 formed by flat sheets 160, whereby two of the flat sheets 160 are produced with the method of Fig. 108A are provided as the first sidewall 181 and the second sidewall 182. Two further sidewalls 170 are configured as unperforated flat sheets 160 having no channels, whereby these two flat sheets 160 may be produced with the method depicted in Fig. 108B.

As shown in Fig. 108B, the method for fabricating the unperforated flat sheets 160 may comprise the same steps 590, 591, 592, 593 as the method for fabricating the perforated sheets shown in Fig. 108A. Thereby the mold 598 has a base 596 without slots and lacks the inner mold parts 597.

The providing (594) of the multiple sidewalls 170 comprises providing two sidewalls 170 configured as unperforated sheets 160 and two sidewalls 170 configured as perforated sheets 160. The method further comprises connecting (559) the sidewalls 170, for example by bonding them with Ecoflex and subsequent curing, which results in the formation of the 3D box-shaped soft structure forming the box 180.

To enhance visibility of the body 10, 11, a red pigment (SILC-PIG^{™}, KauPo, DE) may be added to all 1D, 2D, and 3D parts during fabrication, with the exception of those containing magnetic particles. The parts comprising magnetic particles are black due to the inherent color of the magnetic material, which cannot be masked by the pigment.

With other embodiments, elongated parts, such as tubes or rods, forming the outer or inner parts 20, 30, 40 may also be fabricated by providing a precursor part having a larger dimension than the elongated part, such as a larger extension in the outer direction 4, and by reshaping the precursor part to have a smaller dimension, such as a smaller dimension in the outer direction 4. Reshaping may comprise heating the precursor part and/or melting the precursor part and/or stretching the precursor part, for example along the longitudinal direction 2 perpendicular to the outer direction 4. For example, the precursor part may be a commercially available tube (such as 06420-01, Cole-Parmer, US). Reshaping may comprise heating, melting and stretching the tube to fabricate a tube at a smaller scale. The reshaped precursor part may have, for example, an outer diameter of 0.7 mm, or 0.3 mm. For the case of a tube, it may have an inner diameter of 0.4 mm.

### Experimental platforms

Two types of magnetic elements 50, 60 will be described in the following that may be used to magnetically actuate the soft robots 1 according to the present disclosure. A first type of the magnetic elements 50, 60 is configured as a permanent magnet, such as a permanent magnet with a pre-set magnetization profile, for example a commercially purchased tiny permanent magnet with a pre-set magnetization profile. The magnetic elements 50, 60 configured as permanent magnets may be rigid. The second type of magnetic elements 50, 60 comprises soft elastomeric materials, in which magnetic particles are embedded, see steps 574 in Fig. 106B and 584 in Fig. 107B. The soft elastomeric material forms the substrate material 589. A vibrating sample magnetometer (VSM) (EZ7, MicroSense, USA) may be used to magnetize these materials at 1.8 Tesla to achieve the required magnetization profiles. The magnetic characterization of different materials mixed with varying proportions of magnetic particles was also measured using the VSM in the framework of the present disclosure.

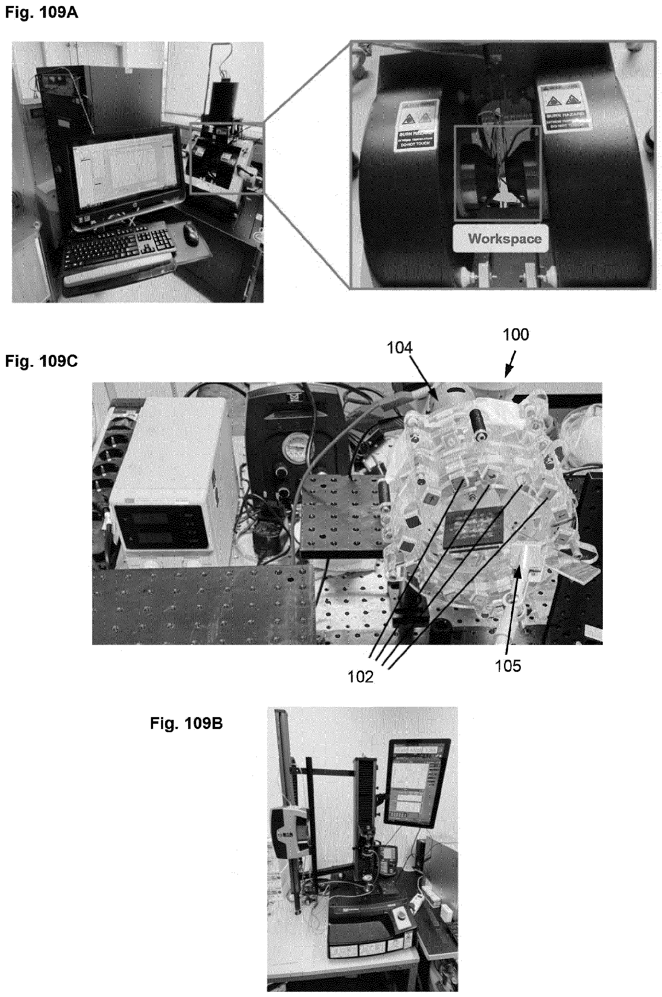

Fig. 109A depicts the vibrating sample magnetometer that may be utilized for magnetizing materials and generating uniform magnetic fields for control of soft robots 1. Fig. 109B depicts a universal testing system that is employed for conducting tensile strength tests and measuring friction forces. Fig. 109C depicts a magnetic field generator 100, configured as a Halbach array, that is designed to generate rotating magnetic fields 5.

At least two types of external magnetic fields 5 may be used for actuation of the soft robots 1 according to the present disclosure: a uniform magnetic field 5 and a rotating magnetic field 5. The uniform magnetic field 5 may be generated by a magnetic field generator 100 formed by the VSM, with its magnitude adjustable as required.

As depicted in Fig. 109C, a magnetic field generator 100 may also comprise a source arrangement 105, such as a Halbach array, comprising field sources, such as permanent magnets 102. The source arrangement 105 may be driven by a source actuator 102, such as a motor. For example, the source actuator 102 may be configured to rotate the source arrangement 105. The source arrangement 105 may be mounted on a shaft of a direct current (DC) motor via connecting components. Rotating the source arrangement 105 results in the creation of a rotating magnetic field 5. By adjusting the rotational speed of the motor, the frequency of the rotating magnetic field 5 can be precisely controlled.

In general, the source arrangement 105 may be configured to produce a uniform magnetic field 5, for example at least in the interaction zone 130.

The elastic modulus of different materials used in the devices according to the present disclosure may be determined by the universal testing system (5942, INSTRON, USA) for tensile testing, as illustrated in Fig. 109B. The test involves continuously applying a tensile load until the sample fractures. This process allows to obtain the displacement and load applied during the stretching of the sample, thereby facilitating the calculation of the material's elastic modulus. Furthermore, this instrument may also be utilized to measure the pulling force of rods in soft tubes with varying curvatures.

### Demonstration platforms

For demonstrating application I, a model with the avoidance objects or obstacles 402 with cylinders was produced using 3D printing (clear V4, Formlabs, US). The cylinder has a diameter of 12 mm and a height of 20 m. It was positioned on a flat surface to form the required model. Specific geometric parameters of the model are shown in Figs. 60 and 61. The vascular model in application I was also fabricated using 3D printing and subsequently underwent surface treatment (SCS PDS 2010 Labcoter^{®}, Specialty Coating Systems Inc., USA) with Parylene C. Specific geometric parameters of the vascular model are provided in Fig. 63. In Application II, the base plate for securing the cilia 310 was crafted from acrylic using laser processing to create an array of small holes on its surface, into which the cilia 310 were then fixed. The ciliary paddles at the tips of the cilia were also produced using 3D printing technology (clear V4, Formlabs, US) and the specific geometric parameters are detailed in Fig. 90. In Application III, the tissue model was fabricated using 3D printing with the soft material Elastic 50A (Formlabs, US) selected to mimic the soft properties of tissue. In Application IV, the object model was also created through 3D printing, utilizing the material clear V4.

### I. Tube Manipulation Schemes

To facilitate the application of the methods and devices according to the present disclosure across various scenarios, the following presents detailed design, analysis, and optimization solutions.

According to the present disclosure, the body 10 may be configured as a 1D soft body, such as a soft tube. The outer part 20 and the inner part 30 of such a 1D soft body may be configured as elongated members, such as tubes or rods, as disclosed, inter alia, in connection with Figures 2 to 29A. Alternatively, the body 10 and the outer part 20 may also be configured as a 2D soft body, such as the bodies 10 having the outer part 20 configured as the flat sheet 160 exemplarily disclosed, inter alia, in connection with Figures 30 to 40, or as a 3D soft body, such as the bodies 10 having the outer part 20 configured as the box 180 exemplarily disclosed, inter alia, in connection with Figures 41 to 49.

Since the deformation of bodies 10 configured as 1D soft bodies, namely 1D soft tubes, forms the basis of the present disclosure - with bodies 10 forming 2D and 3D soft bodies being extensions of the 1D soft body - the following focusses solely on the design of the 1D soft body manipulation scheme, namely on a 1D soft tube manipulation scheme In the design of the 1D soft body, configurations involving either a two-part or a multi-part nested arrangement of the outer and inner parts 20, 30, 40 of the body 10 are considered. The following focusses on the two-part configuration for detailed analysis, while a brief overview of the multi-part design is also presented below.

Unless indicated otherwise in the following, the parts 20, 30, 40 of the body 10 mentioned in the following each may be configured as tubes, such as soft tubes.

With the two-part nested arrangement, the body 10 comprises the outer part 20 and the inner part 30, whereas, with the multi-part nested arrangement, the body 10 comprises additionally the further inner parts 40 nested in between the outer part 20 and the inner part 30.

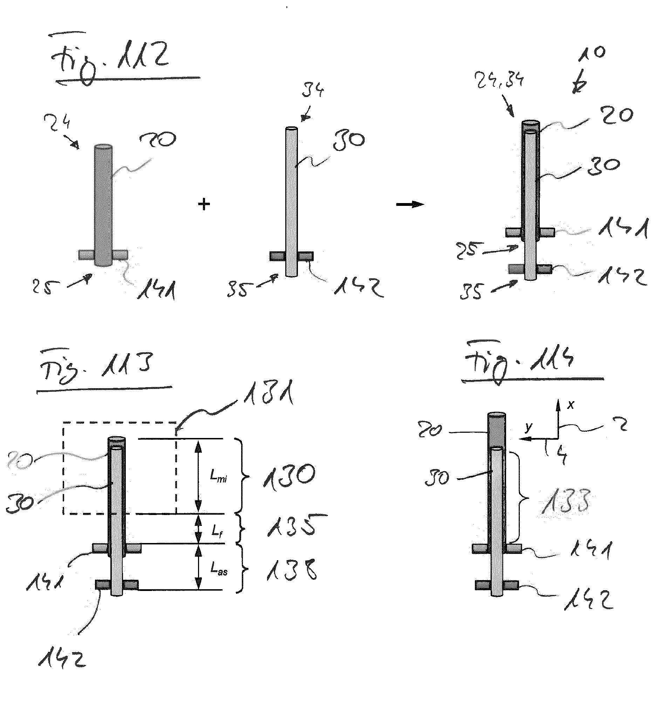

For the manipulation of two nested soft parts, namely the outer part 20 and the inner part 30, relative displacement between the two parts 20, 30 is achieved by manipulating the terminal ends formed by the second ends 25, 35, causing the parts 20, 30 to advance or retract. Although there are multiple methods to manipulate the parts 20, 30, operation may be simplified by using an actuator 140 comprising first and second parts 141, 142 configured as grippers. As shown in Fig. 54, two grippers are employed to grasp the outer part 20 and the inner part 30, respectively. The parts 20, 30 are then assembled as shown in Fig. 112 and placed within the magnetic field 5 to achieve the desired deformations.

To clarify the discussion, relevant concepts may be defined as follows. As depicted in Fig. 113, the effective area covered by the magnetic field 5 is termed the workspace 131. Once the parts are assembled and placed within the workspace 131, the zone where the parts 20, 30, 40 interact with the magnetic field 5 and undergo deformation is defined as the magnetic interaction zone 130, with the corresponding part length denoted as the magnetic interaction length (*Lₘᵢ*). The zone between the interaction zone 130 and the actuator 140 is called the feeding zone 135, and the corresponding part length is defined as the feeding length (*L_{f}*). Finally, the region where the actuator 140 holds the parts 20, 30, 40 and controls their motion is referred to as the actuation zone 138, with the spacing between the parts 141, 142 of the actuator 140 in this region being defined as the actuation spacing (*Lₐₛ*).

The following individually analyzes and discusses the three zones: the magnetic interaction zone 130, the feeding zone 135, and the actuation zone 138. For each zone 130, 135, 138, a mechanical analysis of the soft part is first conducted and appropriate design solutions and optimization strategies based on this analysis are then proposed. Finally, the present disclosure presents an exemplary configuration. After discussing these three zones, the disclosure focusses on several special operations: long-range operation, multi-bend deformation operation, high-curvature deformation operation, and multi-nested-part operation.

### 1.1. Magnetic Interaction Zone

### 1.1.1 Mechanics Analysis

Fig. 114 depicts the body 10 of the soft robot 1 with an overlapping region 133 of the outer part 20 and the inner part 30 of the body 10.

When operating the inner and outer parts 20, 30, the forces involved in the overlapping region 133 (i.e., an interaction area, as non-overlapping sections do not involve relative motion or interaction), see Fig. 114, can be categorized as follows. 1) With the outer part 20 stationary while pushing the inner part 30 along the longitudinal direction 2, namely in the longitudinal direction 2 (in the *+x* direction), the outer part 20 experiences tensile force and the inner part 30 experiences compressive force. 2) With the outer part 20 stationary while pulling the inner part 30 along the longitudinal direction 2, namely against the longitudinal direction 2 (in the - *x* direction), the outer part 20 experiences compressive force and the inner part 30 experiences tensile force. 3) With the inner part 30 stationary while pushing the outer part 20 along the longitudinal direction 2, namely in the longitudinal direction 2 (in the *+x* direction), the inner part 30 experiences tensile force and the outer part 20 experiences compressive force. 4) With the inner part 30 stationary while pulling the outer part 20 along the longitudinal direction 2, namely against the longitudinal direction 2 (in the *-x* direction), the inner part 30 experiences compressive force and the outer part 20 experiences tensile force. Since the inner and outer parts 20, 30 are nested and essentially equivalent, these four cases can be reduced to an analysis of two scenarios: one part 20, 30 remains fixed while the other part 20, 30 moves, being subjected either to tensile or compressive forces.

Tensile and compressive forces are influenced by various factors, including part bending, gravity, and friction. In addition, because the part 20, 30 is located within the magnetic interaction zone, its bending is also affected by magnetic forces/torques. Under the combined influence of these factors, the dynamic process of relative part motion becomes exceedingly complex. Since the static bending profile can be calculated using the formulas provided below, we simplify the dynamic process by modeling it as the inner part 30 moving along a fixed bending profile maintained by the outer part 20. Because this static profile is established under the combined effects of gravity, magnetic forces, and other external loads, it inherently accounts for these influences on the inner part 30. Therefore, the magnetic forces and gravity acting on the bent segment do not require separate calculations.

Assuming that the part 20, 30 undergoes only bending deformation without any torsion, and following a pseudo-rigid model, we assume that the part 20, 30 is composed of n serial link segments. If the curvature of a certain segment is denoted as *κᵢ*, then the bending moment corresponding to that segment is: ${M}_{i} = {E}_{i} {I}_{i} {κ}_{i}$ (S9-1) where *Eᵢ* is the elastic modulus of that section; *Iᵢ* is the area moment of inertia of that segment. Therefore, the bending moment of all the bent segments is: ${M}_{total} = \sum {E}_{i} {I}_{i} {κ}_{i}$ (S9-2)

The force required to move the inner part 30 to form or eliminate a bend of the same shape as the outer part 20 is: ${F}_{c} = \frac{{M}_{total}}{{L}_{e}}$ (S9-3) where *Lₑ* is the equivalent lever arm.

The formation of bending is a result of the action of magnetic torque. In segments where there is no bending, there is an absence of magnetic torque. Consequently, in these non-bent segments, the only external force acting is gravity. The gravity acting on the part 20, 30 primarily serves two functions: it generates resistance in the direction of the part's end movement, and it also creates frictional force in the direction perpendicular to the part 20, 30. The force exerted by gravity in the direction of the part's end movement is: ${F}_{e}^{G} = \sum {m}_{i}^{m} g ⋅ {s}_{e}$ (S9-4) where ${m}_{i}^{m}$ represents the mass per unit length of the part; **g** is the acceleration due to gravity; **s*ₑ*** is the unit directional vector in the direction of the part's end movement.

When the inner and outer parts 20, 30 move relative to each other, frictional force is generated. The total friction force *F^{f},* is given by the sum of the friction forces acting on each individual unit. The frictional force experienced by the *i*^{th} segment is: ${F}_{i}^{f} = {μ}_{i} {N}_{i}$ (S9-5) where *µᵢ* is the coefficient of friction corresponding to the *i*^{th} segment;

*Nᵢ* means the normal force corresponding to the *i*^{th} segment.

In the non-bent segments, the normal force is the component of gravity in the direction perpendicular to the movement; in the bent segments, this normal force arises from the compressive force due to the bending of the inner part 30. Thus, there is ${N}_{nb} = {F}_{N}^{G}$ (S9-6) ${N}_{b} = {F}_{N}^{C}$ (S9-7) where *N_{ub}* and *N_{b}* represent the normal force in the non-bent segments and bent segments, respectively; ${F}_{N}^{G}$ means the component of gravity; ${F}_{N}^{C}$ is normal force arises from the compressive force due to the bending of the inner part 30. The normal force generated by gravity is: ${F}_{N}^{G} = \sum {m}_{i}^{m} g ⋅ {s}_{i}^{N}$ (S9-8)

Here, ${s}_{i}^{N}$ is the unit direction vector of the normal force for the *i*^{th} segment. As elaborated below, the angle of this segment relative to the *+x* axis is *αᵢ*, and ${s}_{i}^{N}$ can be calculated by ${s}_{i}^{N} = \left(-sin {α}_{i},cos {α}_{i}\right)$ (S9-9)

The normal force exerted by the inner part 30 on the outer part 20 originates from the compression of the outer part 20 by the bent profile of the inner part 30, and the formation of this bent profile depends on the bending moment experienced during bending. To simplify calculations, we approximate the normal force generated by the bending of the inner part 30 as the force corresponding to the bending moment, denoted as *F_{c}*, and thus there is ${F}_{N}^{C} = {F}_{c}$ (S9-10)

Therefore, the resultant force required for the inner part 30 to move within the outer part 20 is: ${F}_{m - total} = {F}_{c} + {F}_{e}^{G} + \sum {F}_{i}^{f}$ (S9-11)

The tension rod exhibits stability and can operate as long as the axial tensile stress remains below its yield strength. Therefore, if the relative motion resistance between the parts 20, 30 is less than the part's yield force, pulling the part 20, 30 is feasible, i.e., ${F}_{m - total} < {F}_{y}$ (S9-12) where *F_{y}* represents the part's yield force, which can be calculated using the following formula: ${F}_{y} = {σ}_{y} A$ (S9-13)

Here, *σ_{y}* denotes the part's yield strength, and *A* is its cross-sectional area.

When pushing the part 20, 30, since it is subject to compressive force, the instability of the compression rod must be considered. Specifically, once the applied compressive load exceeds the critical buckling load, the compression rod loses stability and is unable to transmit axial force. Furthermore, since the part 20, 30 is located within the magnetic interaction zone 130 and undergoes bending deformation under magnetic forces, and given that the gap between the inner and outer parts 20, 30 is relatively small in this disclosure, multiple contact points between the parts 20, 30 are likely to form. For this situation, one simplified computational method is to calculate the critical buckling load of the part segment between every two contact points (refer to Section I.2.1 below for the critical buckling load calculation of a straight rod), and then take the smallest of these calculated values as the critical buckling load *F_{cr}*, for that state, i.e., ${F}_{cr} = min \left({F}_{cr}^{i}\right)$ (S9-14) where ${F}_{cr}^{i}$ denotes the critical buckling load for the part segment between the *i*^{th} and (*i*+1)^{th} contact points.

Due to the formation of multiple overlapping arcs of the inner part 30 within the outer part 20, determining the number of contact points is challenging. Furthermore, once the part 20 buckles, additional contact points may emerge, leading to a subsequent equilibrium state. This situation is exceedingly complex, and the aforementioned calculation method can only provide a rough estimate. More precise calculations may be carried out using simulation software or determined experimentally. In simulation, a simplified approach similar to the calculation of the relative motion resistance *Fₘ₋ₜₒₜₐₗ* for part movement can be adopted, whereby the outer part 20 maintains a constant bending profile while the inner part 30 moves along that profile.

For experimental methods, different bending curvatures and numbers of bends can be designed based on the specific operation, taking into account other factors, and measurements can then be taken. For instance, as illustrated in Fig. 114, an experimental approach may be employed to assess the influence of various parameters-including radius of curvature, number of bends, and gap-on the pushing and pulling operations. This method not only provides design reference data but also enables the feasibility validation of the completed design.

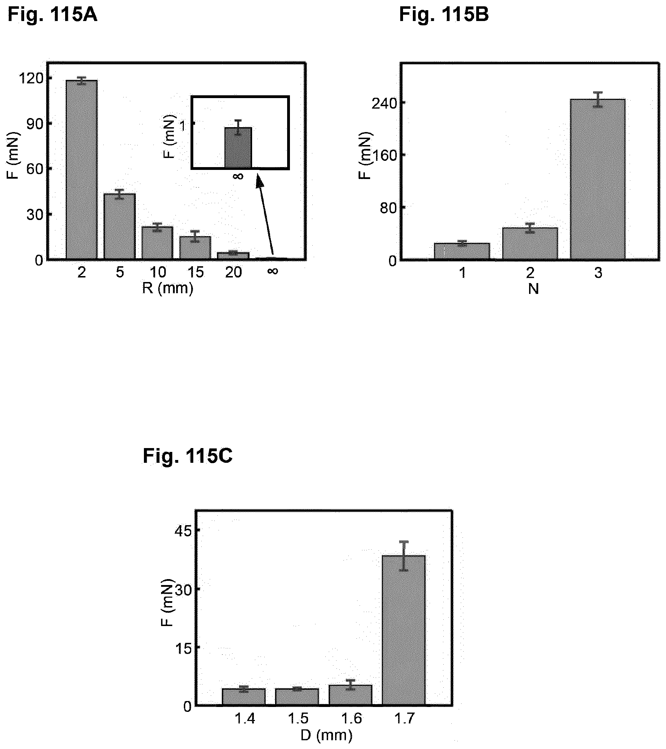

Fig. 115 depicts the effect of various parameters on the relative motion resistance F involved in manipulating tubes, wherein Fig. 115A depicts force F for different radii of curvature R, Fig. 115B depict force F for different numbers of bends N and Fig. 115C depicts force F for different diameters D of the inner part 30.

Whether determined via experiments or simulations, the critical buckling load *F_{cr}*, can be obtained. In order to push the part 30 and achieve relative motion between the parts 20, 30, the following condition must be satisfied: ${F}_{m - total} < {F}_{cr}$ (S9-15)

Considering the condition for achieving relative motion through pulling, the mechanical criterion for realizing relative part motion within the magnetic interaction zone 130 - and thereby enabling real-time magnetic reprogramming - is given by: $\left\{\begin{matrix}{F}_{cr} > {F}_{m - total} \\ {F}_{y} > {F}_{m - total}\end{matrix}\right.$ (S9-16)

### I.1.2 Design and Optimization Strategies

For operations within the magnetic interaction zone 130, the part's deformation will vary depending on the magnetic field strength. As the magnetic field 5 increases, the bending of the part 20, 30 intensifies, and the relative motion resistance between the parts 20, 30 correspondingly increases. However, regardless of variations in the magnetic field 5 or part deformation, as long as the operational condition expressed in Equation (S9-16) is satisfied, relative motion between the parts 20, 30 can be achieved.

If, based on operational requirements, the magnetic interaction length needs to be further increased such that the relative motion resistance between the parts *Fₘ₋ₜₒₐₜₐₗ,* exceeds the original critical buckling load *F_{cr}* and yield force *F_{y}*, then the pushing and pulling operations will no longer be effective. In response, we propose the following solutions.

### I.1.2.1 Reducing the Relative Motion Resistance Fₘ₋ₜₒₜₐₗ

Based on the analysis in Section I.1.1, under these conditions, the part curvature cannot be altered (as the required curvature is necessary to fulfill specific operational demands). Therefore, reducing friction is the most effective strategy. The following discussion details methods for reducing friction between the parts 20, 30, 40 along with the corresponding design and optimization strategies.

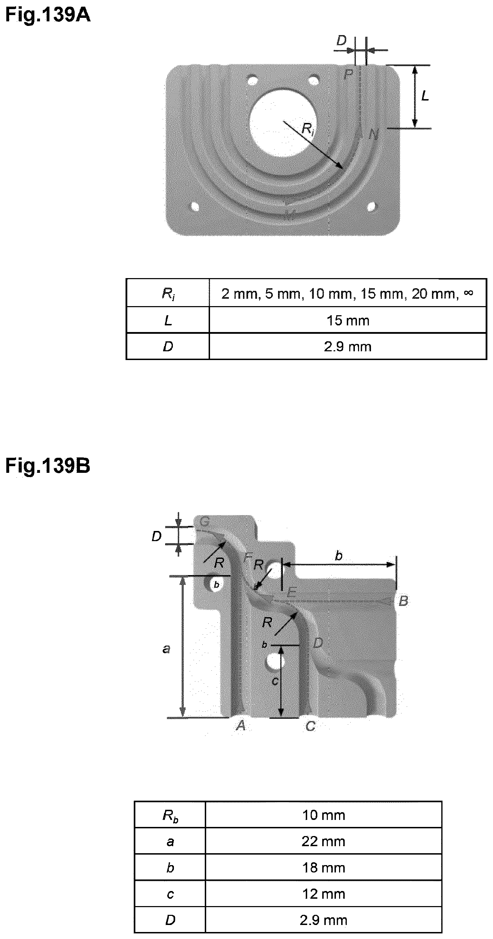

Fig. 166 depicts the influence of various parameters on the relative motion resistance involved in manipulating tubes forming the parts 20, 30, 40. Fig. 166A depicts the effect of different materials on relative motion resistance. Fig. 166B depicts the effect of different motion modes on relative motion resistance. Fig. 166C depicts the effect of different lubrication methods or lubricants on relative motion resistance. All experiments were conducted using the setup shown in Fig. 139A, with a radius of curvature of the bend of Rᵢ = 10 mm.

### a) Utilization of low-friction materials

For soft materials, a variety of options are available for reducing friction. Under the constraint of meeting other performance requirements, materials with a low friction coefficient may be selected. For example, parts fabricated from Ecoflex exhibit a slightly lower friction coefficient compared to those made from Polydimethylsiloxane (PDMS). However, while Ecoflex reduces friction, its lower elastic modulus relative to PDMS can affect other properties such as deformation behavior and the critical buckling load. Therefore, a comprehensive evaluation may be necessary. With the present disclosure, most parts are exemplarily fabricated from PDMS, for example the cylindrical parts 20, 30, 40 used for contact free object navigation, the artificial cilia array 300 and multi-instrument operation, as shown, inter alia, in Figs. 55 to 100 and 2 to 25), whereas some cylindrical parts are made from Ecoflex, such as the seventh configuration shown in Fig. 26 to 29, and both the 2D and 3D structures forming the flat sheets 160 and the box 180 shown, inter alia, in Figs. 30 to 48 are entirely fabricated using Ecoflex. In general, all flexible parts of the bodies 10 according to the present disclosure may comprise a silicone, such as silicone rubber.

### b) Surface treatment of the part

During or after fabrication, the surface of the parts 20, 30, 40 can be chemically treated to reduce its friction coefficient. For instance, when fabricating parts 20, 30, 40 using silicone rubber, a surface tension diffuser (Slide STD-1, Kaupo, DE) can be added. This additive diffuses to the material's surface during the curing process, forming a thin, low-surface-energy film that effectively reduces friction. According to the present disclosure, the flat sheets 160 may incorporate this surface tension diffuser to lower surface viscosity, like, for example, the sheets 160 shown in Figs. 31 to 40. Also, all other flexible parts 20, 30, 40 may comprise surface tension diffuser.

### c) Enhancing lubrication conditions

Various lubricants can be introduced between the parts 20, 30, 40 to reduce friction. For example, incorporating talcum powder between the parts 20, 30, 40 can markedly lower friction; in addition, other lubrication methods or lubricants, such as oil-based lubricants, may also be employed. With the present disclosure, talcum powder may be added to all parts 20, 30, 40 to reduce the friction between them, for example to the parts 20, 30, 40 shown in Figs. 2 to 25.

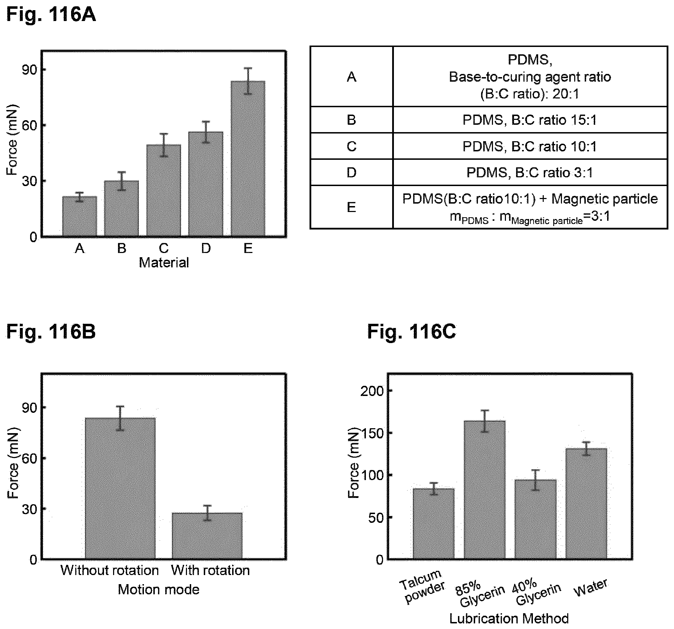

The relative motion resistance for different lubricants, as experimentally measured and, is illustrated in Fig. 116C, wherein the average relative motion resistance was 163.8 mN with 85% glycerol, 131 mN with water, and decreased to 83.6 mN when talcum powder was used. Additional analysis is provided in section VI. below. Any of the foregoing lubricants may be used as lubricant according to the present disclosure.

### d) Altering the part's motion mode

When pushing or pulling the parts 20, 30, 40, introducing a rotational component-rotating while simultaneously pushing or pulling-can modify the frictional interaction between the parts 20, 30, 40. This change transforms pure sliding friction into a combination of sliding and rolling friction, with the latter typically having a much lower friction coefficient. Additionally, rotation weakens the adhesive effects between the parts 20, 30, 40 by continuously reordering and refreshing the local contact surfaces, thereby reducing adhesive friction. The direct consequence of this operational modification is a significant reduction in the frictional force between the parts 20,30, 40. For instance, for scenarios involving relatively long parts 20, 30, 40 or parts 20, 30, 40 with pronounced curvature, a method combining rotation with pulling/pushing may be adopted, for example with the embodiments shown in Fig. 55B at (iii), 59A, and 59C. To assess the impact of motion modes, relative motion resistance under different operational conditions have been measured, as illustrated in Fig. 116B. If the part's motion mode is changed from simple pulling to pulling combined with rotation, its relative motion resistance significantly decreased from 83.6 mN to 27.4 mN. Additional analysis is provided in section VI. below.

### I.1.2.2 Increasing the Critical Buckling Load F_{cr}

Although the classical formula for the critical buckling load of a straight rod cannot yield precise values, more complex calculations involving multiple contact points and simulations are based on similar principles. Thus, the parameters involved in the calculation of the critical buckling load for a straight rod at least reflect the positive or negative correlations of each parameter with the steady-state behavior. According to the analysis of the critical load in section I.2.1 above (Equation (S9-28)), the critical buckling load can be increased by raising the elastic modulus of the parts 20, 30, 40, by increasing its area moment of inertia, or by reducing its length. The following analysis examines these three aspects in detail.

### a) Increasing the elastic modulus

The critical buckling load *F_{cr}* is directly proportional to the elastic modulus of the part 20, 30, 40. Increasing the elastic modulus can effectively raise the critical buckling load. However, the elastic modulus also directly influences the deformation characteristics of the part 20, 30, 40. Assuming that the part 20, 30, 40 bends under the influence of a magnetic field, and neglecting other factors, the energy density for a segment of arc length s is given by ${u}_{ela} \left(s\right) = \frac{1}{2} EIκ {\left(s\right)}^{2}$ (S9-17) where *κ*(*s*) is the curvature corresponding to the segment of arc length s. Let the angle between the tangent of this segment and a reference direction be *θ*(*s*), and then the curvature can be expressed as $κ \left(s\right) = \frac{d θ}{d s}$ (S9-18)

Therefore, the total bending energy of the part 20, 30, 40 is given by ${U}_{ela} = \frac{1}{2} EI \int {\left(\frac{d θ}{d s}\right)}^{2} d s$ (S9-19)

The magnetic potential energy associated with the part 20, 30, 40 is ${U}_{mag} = - mB \int cosθ \left(s\right) d s$ (S9-20)

Neglecting other influencing factors for the time being, the total energy of the system is ${U}_{sys} = \frac{1}{2} EI \int {\left(\frac{d θ}{d s}\right)}^{2} d s - mB \int cos θ \left(s\right) d s$ (S9-21)

In equilibrium, the system attains an extreme value, and the above expression can be reformulated as follows: $\frac{EI}{mB} = - \frac{{d}^{2} θ}{sin θ \left(s\right) d {s}^{2}}$ (S9-22)

When increasing the elastic modulus to enhance the critical buckling load, the part's deformation becomes smaller than the originally prescribed deformation. To maintain the target deformation, and given that the right-hand side of Equation (S9-22) remains constant according to the previous analysis, it is necessary to increase either the magnetic moment *m* or the magnetic field strength *B* to preserve the part's deformation. In other words, an increased elastic modulus *E* enhances the part's resistance to bending, requiring the magnetic field to supply additional energy to induce the desired bending. Therefore, if the approach is to increase the magnetic moment, one must design the part 20, 30, 40 using materials with higher magnetization or incorporate permanent magnets (see section IX for a detailed design method for magnetization profiles).

Alternatively, if the strategy is to increase the magnetic field 5, the associated cost of enhancing the field 5 may be considered. Within a certain range, increasing the magnetic field 5 is the simplest and most direct method, as it is both straightforward and rapid, without necessitating changes to the existing design. However, once the magnetic field strength reaches a certain threshold, the required field 5 may exceed the capabilities of the employed magnetic field generator 100, rendering this approach impractical.

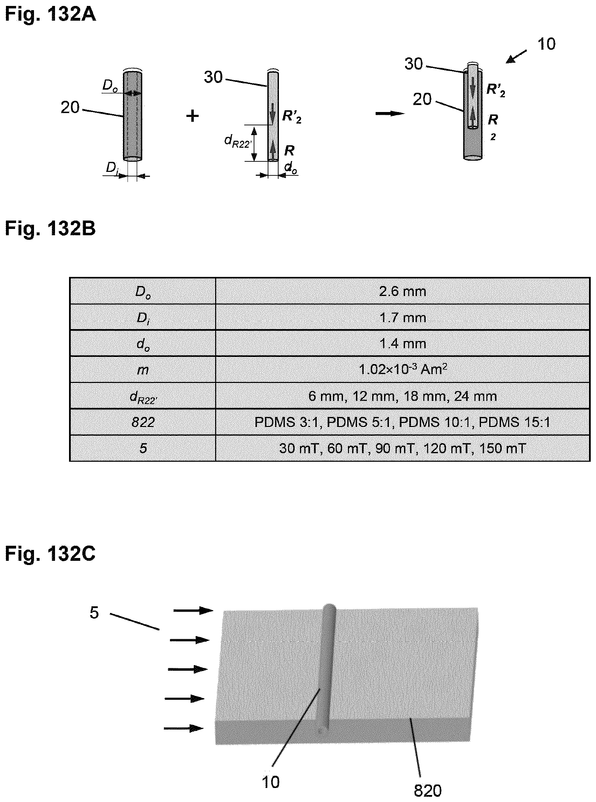

Furthermore, we have experimentally verified the effects of both the elastic modulus and the magnetic field 5 on bending of the parts 20, 30, 40 (see section III. below and Fig. 132 to 134 for further details).

### b) Increasing the area moment of inertia

Similar to the elastic modulus, the area moment of inertia is directly proportional to the critical buckling load *F_{cr}*. Increasing the area moment of inertia can effectively raise *F_{cr}*. This approach shares the same design concept as increasing the elastic modulus; by augmenting the area moment of inertia, the resistance of the parts 20, 30, 40 to bending is enhanced, which in turn necessitates an increase in the magnetic moment or magnetic field 5 to compensate (see Equation (S9-22)). For a detailed discussion, please refer to the analysis on increasing the elastic modulus above. However, unlike the elastic modulus, enhancing the critical buckling load by increasing the area moment of inertia also poses dimensional design challenges. The area moment of inertia is directly related to the part's dimensions and is given, for a tubular part, by $I = \frac{π}{4} \left({R}^{4}-{r}^{4}\right)$ (S9-23) where *R* is the outer radius of the part 20, 30, 40 and *r* is the inner radius of the part 20, 30, 40. Thus, an increase in the area moment of inertia can only be achieved by either enlarging the outer diameter or reducing the inner diameter. Nevertheless, the outer and inner diameters are critical to the overall dimensions of the part 20, 30, 40 and the fit between multiple nested parts 20, 30, 40. As a result, the possibility of adjusting the diameters is subject to various factors such as specific operational requirements and inter-part compatibility. Unlike the elastic modulus, which can be varied more freely, modifications to the part's dimensions must take all design parameters into careful consideration.

### c) Reducing part length

The square of the length of the parts 20, 30, 40 is inversely proportional to the critical buckling load. Consequently, shortening the part 20, 30, 40 yields a more pronounced increase in the critical buckling load than merely increasing the elastic modulus or the area moment of inertia. However, because the part's length is directly tied to specific operational requirements, there is no corresponding compensation strategy akin to those available for the elastic modulus and the area moment of inertia. Thus, reducing the part length may not serve as an effective post-design optimization measure; it may be applied during the design phase, aiming to minimize the length of the parts 20, 30, 40 while still meeting the operational criteria.

### 1.1.2.3 Increasing the Yield Force F_{y}

To increase the yield force, according to Equation (S9-13), one can either enhance the yield strength or augment the cross-sectional area of the parts 20, 30, 40. For yield strength, selecting materials with higher yield strengths is a viable approach. With regard to increasing the cross-sectional area, this inherently involves adjustments to the part's dimensions. The cross-sectional area is calculated by: $A = π \left({R}^{2}-{r}^{2}\right)$ (S9-24) where *R* denotes the outer radius of the part 20, 30, 40 and r the inner radius of the part20, 30, 40. According to this equation, increasing the outer radius or decreasing the inner radius will yield a larger cross-sectional area. However, as with the area moment of inertia, changes in dimensions affect the overall part size and the compatibility among nested parts 20, 30, 40. Therefore, practical operational requirements and all pertinent design parameters may be carefully considered to effectively achieve an increase in the cross-sectional area.

### 1.1.3 Case Study Analysis

To more clearly demonstrate the design solutions and optimization strategies discussed above, a case study integrates the designs and applications involved in this research as detailed by the following embodiments.

For the most common parts 20, 30, 40 used in this study, the outer part 20 is configured as a tube and has an outer diameter of 2.6 mm and an inner diameter of 1.7 mm, and is fabricated from PDMS with a base-to-curing agent mass ratio of 20:1. The inner part 30 is replaced by a rod with a diameter of 1.4 mm, made from PDMS with a base-to-curing agent mass ratio of 10:1. Regarding the calculation of the tensile force, since both parts 20,30 are made of PDMS - and as an elastomer, PDMS does not exhibit a clearly defined "yield point" as metals do (it shows nonlinear elastic behavior at large strains and often lacks a conventional yield) - an approach commonly used in other studies may be adopted. Specifically, the ultimate tensile strength rather than the traditional yield strength is used for the calculation. That is, ${σ}_{y} = {σ}_{u}$ (S9-25) where *σ_{y}* denotes the yield strength and *σᵤ* denotes the ultimate tensile strength.

Based on measurements of the elastic modulus for materials with different base-to-curing agent mass ratios, the approximate ultimate tensile strength of PDMS is about 0.4 MPa for a 20:1 ratio and about 3.2 MPa for a 10:1 ratio. Consequently, the yield force - i.e., the maximum tensile force that can be provided - can be calculated using Equations (S9-13) and (S9-25). Substituting the data into these formulas yields a maximum tensile force of 1220 mN for the tube forming the outer part 20 and 4930 mN for the rod forming the inner part 30. Fig. 116A displays the average relative motion resistance between the two parts 20, 30 under various conditions; these values are generally below 60 mN, with the maximum reaching 244 mN when multiple bends occur. Since the maximum tensile force provided by the rod far exceeds this relative motion resistance, effective relative motion between the parts 20, 30 can be achieved by pulling.

For calculating the pushing force, the critical buckling load is computed using Equation (S9-28). Since the critical buckling load depends on the effective length of the part 20, 30, 40 under load - decreasing as the length increases - while the tensile force remains independent of this length, the available pushing force becomes the key condition determining whether the operation can be successfully completed. In Fig. 116A, we measured the average friction force between the two parts 20, 30 formed by the outer tube and the inner rod in a straight configuration (0.9 mN). Based on this value, we back-calculate the maximum operational length that can be achieved under this friction force. Substituting the data into Equation (S9-28) yields a maximum operational length of 179 mm for the outer part 20 and 162.5 mm for the inner part 30 formed by the rod. To ensure safety and to account for the cumulative effect of friction as the length increases, a safety factor of 2 is applied. Under these conditions, the maximum operational length is reduced to 127 mm for the outer part 20 formed by the tube and 115 mm for the inner part 30 formed by the rod. This length is sufficient for the vast majority of linear operations. A similar calculation method can be applied for bending operations. For designs requiring a longer operational length, specific design solutions and optimization strategies are presented in section I.4.1 below.

Next, each of the three improvement measures analyzed in Section I.1.2 above are discussed.

For the strategy of reducing the relative motion resistance *Fₘ₋ₜₒₜₐₗ*, some designs may be employed both enhanced lubrication conditions and an altered part motion approach. For example, with relative motion of 1D parts as well as 2D or 3D objects, talcum powder may be applied to the surfaces of the components to reduce friction during relative motion. Additionally, relative rotational movement between the parts 20, 30, 40 may be combined with translation of the parts 20, 30, 40 to facilitate the pushing or pulling of the parts 20, 30, 40. For example, in the Application I - Contact-free object navigation - where two bends are used to overcome an obstacle 402, see, for example Fig. 55B at (iii) - a method involving rotational advancement and rotational retraction of the outer part 20 may be implemented, which significantly reduces friction and eases operation. In contrast, in other applications where friction is relatively minor (e.g., Application II- Reprogrammable cilia array 300), direct pushing and pulling may be implemented to complete the operation quickly, thereby eliminating the need for rotational motion.

In the measures for increasing the critical buckling load *F_{cr}*, some designs may employ strategies such as increasing the elastic modulus and reducing the part length. For instance, in Application II - Reprogrammable cilia array 300, the relative motion resistance *Fₘ₋ₜₒₜₐₗ* is low. Consequently, using an inner part 30 configured as a rod fabricated from PDMS with a base-to-curing agent mass ratio of 10:1 (with an elastic modulus of 1 MPa) is sufficient to achieve the desired operation. In contrast, in the Application I-Contact-free object navigation, multiple bends in 3D space are used to overcome obstacles 402 (see Figs. 69 to 71) and the increased relative motion resistance *Fₘ₋ₜₒₜₐₗ* necessitates a higher critical buckling load *F_{cr}* to ensure successful operation. In this case, a mixture 566 comprising a composite material comprising PDMS and magnetic powder, with an effective elastic modulus of 12.3 MPa, may be employed. The more than tenfold increase in the elastic modulus leads to a corresponding increase in *F_{cr}* by a similar factor. In designing the specific application, the effective part length may be minimized as much as possible while still meeting the operational requirements.

Regarding measures to increase the yield force *F_{y}*, some schemes may substitute the original material with one that has a higher yield strength. In Application I, for example, to achieve multiple bends for overcoming obstacles 402 in 3D space, pure PDMS may be replaced with a composite of PDMS and magnetic particles to fabricate the inner part 30, for example as a rod (Fig. 71). According to experiments, the ultimate tensile strength of the PDMS/magnetic particle composite (with a mass ratio of PDMS to magnetic particle of 1:3) is approximately 4.3 MPa, compared to about 3.2 MPa for PDMS with a base-to-curing agent mass ratio of 10:1. By substituting the ultimate tensile strength for the yield strength in the calculations (i.e., *σ_{y} = σᵤ*), and incorporating the data into Equation (S9-13), the computed yield force of the composite is 7.1 N, while that of pure PDMS is 5.28 N - a 35% increase in the yield force.

### 1.2. Feeding Zone

### 1.2.1 Mechanics Analysis

In the feeding zone 135, the parts 20, 30, 40 are no longer influenced by the magnetic field 5. However, they are subjected to the pushing, pulling, or torque during movement, as well as forces or torques transmitted from the parts 20, 30, 40 in the magnetic interaction zone 130. In this region, the parts 20,30, 40 either have a straight configuration or exhibit bending. Two types of motion or deformation occur in the feeding zone 135: relative motion between the parts 20, 30, 40 and bending under load.

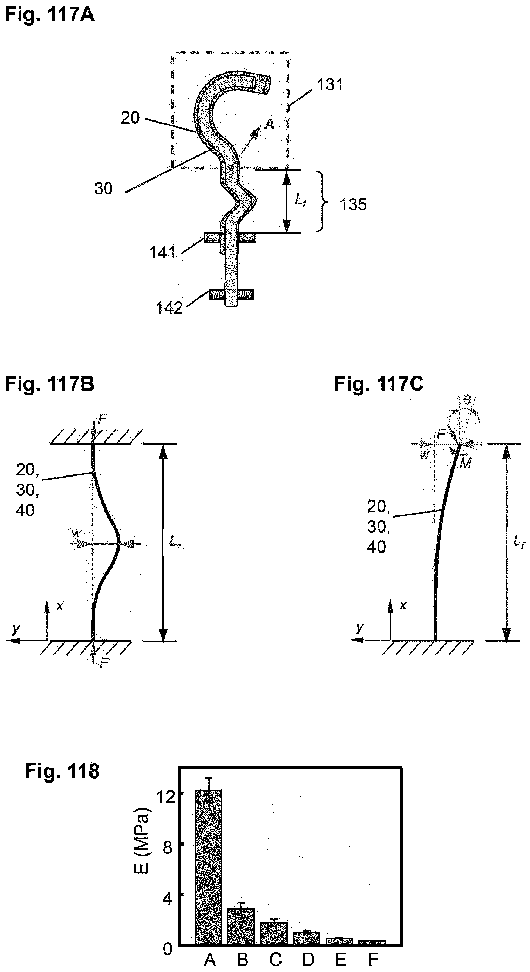

Fig. 117 depicts a force analysis of the tube forming the part 20, 30, 40 in the feeding zone 135. As shown in Fig. 117A, the tube forming the part 20, 30, 40 undergoes bending deformation under applied forces in the feeding zone 135. Point A is located at the boundary (interface) between the feeding zone 135 and the magnetic interaction zone 130. Fig. 117B depicts a compressive rod stability model. In this model, both ends of the tube forming the part 20, 30, 40 are fixed, and it exhibits bending deformation under compression. This model is employed to analyze the stability issues of the tube forming the part 20, 30, 40 under compression during the relative movement between two tubes. Fig. 117B depicts a cantilever beam model. One end of the beam is fixed, while the other end is subjected to both a force and a moment, leading to deflection. This model is used to analyze the deformation behavior of two nested tubes under load within the feeding zone 136.

For the relative motion between parts 20, 30, 40, when the two nested parts 20, 30, 40 are subjected to tensile or compressive forces, it is essential to ensure that relative movement can be achieved. The section of the part 20, 30, 40 in the feeding zone 135, which may be a second inner or outer part section of the respective part 20, 30, 40, serves as a connector between the magnetic interaction zone 130 and the actuation zone 138, transmitting forces from the actuation zone 138 to a section of the part 20, 30, 40 in the magnetic interaction zone 130. The force acting on the part 20, 30, 40 at the interface between the feeding zone 135 and the magnetic interaction zone 130 is identical to the force it transmits to the magnetic interaction zone 130 (i.e., the force at point A at the junction of the two zones 130, 135 as shown in Fig. 117A, neglecting acceleration effects), namely, ${F}_{f - total} = {F}_{m - total}$ (S9-26)

Therefore, the force acting on the part in the feeding zone 135 can be determined using the same calculations as for the part 20, 30, 40 in the magnetic interaction zone 130 described in section I.1.1 above. Similarly, due to the stability of the tension rod, to achieve relative motion in the feeding zone 135 through pulling, the following condition must be satisfied: ${F}_{f - total} < {F}_{y}$ (S9-27)

For obtaining relative motion between the parts 20, 30 through pushing, a similar analysis as in section I.1.1 above may be performed, by simplifying the situation to one in which one part 20, 30 is fixed while the other part 20, 30, 40 moves. Whether the outer part 20 is under compression or the inner part 30 is under compression, the scenario can be equivalently represented by the situation shown in Fig. 117B, where both ends of the rod are subjected to compressive forces, resulting in deflection.

Based on the working conditions of the two parts 20, 30, 40, fixed boundary conditions may be assumed for the compression rod at both ends. According to classical Euler buckling theory, the critical buckling load is given by: ${F}_{cr} = \frac{4 {π}^{2} EI}{{L}_{fl}^{2}}$ (S9-28) where E is the elastic modulus of the soft part 20, 30, 40, *I* is the area moment of inertia of the soft part 20, 30, 40, and *L_{fl}* is the feeding length of the soft part 20, 30, 40.

When the axial load applied at both ends of the soft part 20, 30, 40 exceeds the critical buckling load, the soft part 20, 30, 40 will buckle. According to the Euler-Bernoulli beam theory, we have: $EI \frac{{d}^{4} w}{d {x}^{4}} = F \frac{{d}^{2} w}{d {x}^{2}}$ (S9-29) where w represents the lateral deflection and *F* denotes the applied axial load.

Therefore, the buckling equation of the part 20, 30, 40 is given by $\frac{{d}^{4} w}{d {x}^{4}} - \frac{F}{EI} \frac{{d}^{2} w}{d {x}^{2}} = 0$ (S9-30)

By solving the characteristic equation of this differential equation, the general solution is obtained as $w \left(x\right) = A + Bx + C cos \left(kx\right) + D sin \left(kx\right)$ (S9-31)

Since the part 20, 30, 40 is assumed to be fixed at both ends, both the displacements and rotations at its ends are zero. The specific boundary conditions are: $w \left(0\right) = 0 , w \left({L}_{fl}\right) = 0$ (S9-32) $w ′ \left(0\right) = 0 , w ′ \left({L}_{fl}\right) = 0$ (S9-33)

Based on these boundary conditions, the deflection curve of the part 20, 30, 40 is given by $w \left(x\right) = A \left[1-cos\left(\frac{2 πx}{{L}_{fl}}\right)\right]$ (S9-34)

Since the maximum deflection occurs at the midpoint of the part 20, 30, 40, the maximum deflection is given by ${w}_{max} = w \left(\frac{{L}_{fl}}{2}\right) = 2 A$ (S9-35)

Here, the constant *A* is determined by the initial imperfections, the degree of overload, and the material properties. For example, for roughly processed rod elements, a typical estimate is *A* = 0.01*L_{fl}.* In this disclosure, as a conservative estimate, zero deflection is assumed, meaning that the critical buckling load is treated as the maximum compressive force the part 20, 30, 40 can withstand while remaining stable - that is, the maximum pushing force it can provide. Therefore, as long as the relative motion resistance between the parts 20, 30, 40 is less than the critical buckling load, the part 20, 30, 40 can be effectively pushed, namely, ${F}_{f - total} < {F}_{cr}$ (S9-36)

In summary, the mechanical criterion for achieving relative motion between the parts 20, 30, 40 in the feeding zone 135 is $\left\{\begin{matrix}{F}_{cr} > {F}_{f - total} \\ {F}_{y} > {F}_{f - total}\end{matrix}\right.$ (S9-37)

In the feeding zone 135, the bending of the part 20, 30, 40 is induced by a force or torque transmitted from the part 20, 30, 40 in the magnetic interaction zone 130, and the magnitude of this force or torque is predetermined. Therefore, to calculate the deformation under this loading, a simplified cantilever beam model (Fig. 117C) may be adopted to evaluate the bending deformation experienced by the two nested parts 20, 30, 40 under the applied force and moment.

As shown in Fig. 117C, the cantilever beam comprises the two parts 20,30, 40, with both a force *F* and a torque *M* applied simultaneously at the free end. Owing to the superposition principle inherent in linear elasticity, the deformations induced by the force and the toruqe can be analyzed separately. First, the deflection due to the force is calculated, followed by the deflection resulting from the torque, and finally, the two deflections are superimposed.

The displacement of the part 20, 30,40 under the applied force can be decomposed into *x*- and *y*-components. Since axial deformation along the *x* direction is negligible for small deformations, only the deformation along the *y* direction is considered. Therefore, the *y*-component of the force **F** is given by: ${F}_{y} = F ⋅ {e}_{y}$ (S9-38) where ***e_{y}*** is the unit vector in the *y*-direction. The torque distribution along the beam under the force is given by ${M}_{F} \left(x\right) = {F}_{y} \left({L}_{f}-x\right)$ (S9-39) where *L_{f}* represents the length of the part in the feeding zone 135. The fundamental bending equation for the cantilever beam is: ${E}_{e} {I}_{e} \frac{{d}^{2} y \left(x\right)}{{dx}^{2}} = M \left(x\right)$ (S9-40) where *Eₑ* is the equivalent elastic modulus of the two parts and *Iₑ* is the equivalent area moment of inertia. If the parts 20, 30, 40 possess identical elastic moduli, then the equivalent elastic modulus is equal to the original modulus, and the equivalent area moment of inertia can be determined by the following equation: ${I}_{e} = \sum {I}_{i}$ (S9-41) where *Iᵢ* represents the area moment of inertia of the *¡*^{th} part. If the parts 20, 30,40 have different elastic moduli, then we can calculate the equivalent area moment of inertia by combining the individual area moments of inertia as follows: ${E}_{e} {I}_{e} = \sum {E}_{i} {I}_{i}$ (S9-42) where *Eᵢ* is the elastic modulus of the *i*^{th} part 20, 30, 40. By substituting the moment distribution derived from the force into the cantilever beam bending equation and applying the beam's boundary conditions, integration yields the displacement at the free end as follows: ${δ}_{F} = \frac{{L}_{f}^{3}}{3 {E}_{e} {I}_{e}} F ⋅ {e}_{y}$ (S9-43)

Similarly, the displacement at the free end produced by the torque is given by ${δ}_{M} = \frac{{L}_{f}^{2}}{2 {E}_{e} {I}_{e}} M$ (S9-44)

Consequently, the total displacement of the part end along the *y*-direction is ${δ}_{total} = {δ}_{F} + {δ}_{M}$ (S9-45) ${δ}_{total} = \frac{{L}_{f}^{3}}{3 {E}_{e} {I}_{e}} F ⋅ {e}_{y} + \frac{{L}_{f}^{2}}{2 {E}_{e} {I}_{e}} M$ (S9-46)

After deformation under load, the part's end undergoes a rotation corresponding to an angle of: $θ \left(x\right) = \frac{dy}{dx}$ (S9-47)

By integrating the fundamental bending equation for a cantilever beam and applying the boundary conditions, the rotation at the free end of the part 20, 30, 40 due to the applied force is given by: ${θ}_{F} = θ \left(L\right) = \frac{{L}_{f}^{2}}{2 {E}_{e} {I}_{e}} F ⋅ {e}_{y}$ (S9-48)

Similarly, the rotation produced at the free end of the part 20, 30, 40 by the applied torque is given by ${θ}_{M} = \frac{{L}_{f}}{{E}_{e} {I}_{e}} M$ (S9-49)

Therefore, the total rotation at the free end of the part 20, 30, 40 resulting from both the applied force and torque is ${θ}_{total} = {θ}_{F} + {θ}_{M}$ (S9-50) ${θ}_{total} = \frac{{L}_{f}^{2}}{2 {E}_{e} {I}_{e}} F ⋅ {e}_{y} + \frac{{L}_{f}}{{E}_{e} {I}_{e}} M$ (S9-51)

### 1.2.2 Design and Optimization Strategies

Effective control of the part 20, 30, 40 in the magnetic interaction zone 130 may require that relative motion between the parts 20, 30, 40 is achieved in the feeding zone 135, which connects the magnetic interaction zone 130 and the actuation zone 138. According to the analysis above, two conditions must be met, as expressed in Equation (S9-37). If the relative motion resistance *F_{f-total},* becomes too high such that Equation (S9-37) is no longer satisfied, and then manipulation of the part 20, 30, 40 in the feeding zone 135 cannot be accomplished. Consequently, the following solutions or optimization strategies are proposed.

To meet the conditions outlined in the equation, three main considerations can be addressed: reducing the relative motion resistance *F_{f-total},* increasing the critical buckling load *F_{cr},* and increasing the yield force *F_{y}*. These improvement strategies are essentially the same as those discussed in section I.1.2 above, so they will not be repeated here. The primary distinction lies in the approach of shortening the part 20, 30, 40 to increase *F_{cr}*. Shortening the part 20, 30, 40 has a more pronounced effect on enhancing the critical buckling load than increasing either the elastic modulus or the area moment of inertia. However, because the length of the part 20, 30, 40 is directly tied to specific operational requirements, there is no corresponding compensation strategy for length as there is for elastic modulus and area moment of inertia. Consequently, in section I.1.2 above, reducing the length of the part 20, 30, 40 may not be considered an effective post-design optimization measure but rather a design guideline to be addressed during the initial design phase. In contrast, within the feeding zone 135, the length of the part 20, 30, 40 is not directly related to the specific operational requirements. Under appropriate conditions, reducing the length of the part 20, 30, 40 in the feeding zone 135 can effectively increase *F_{cr}*. In addition to the aforementioned methods for increasing *F_{cr},* a segmented design approach (stiffness segmentation) may be employed, as discussed in detail in section I.4.1 below.

Since the part 20, 30, 40 in the feeding zone 135 is subjected to forces and torques transmitted from the part 20, 30, 40 in the magnetic interaction zone 130, it will experience a displacement in the direction perpendicular to its longitudinal axis 2. The magnitude of this displacement and the corresponding rotation can be calculated using equations (S9-46) and (S9-51). This displacement may cumulatively affect the operation of the part 20, 30, 40 in the magnetic interaction zone 130. If the cumulative effect is negligible or does not interfere with the operation, the displacement can be ignored. However, if the displacement significantly impacts the performance of the part 20, 30, 40 in the magnetic interaction zone 130, measures may be taken to reduce it. Based on the mechanical analysis above, optimization can be approached from three aspects: increasing the equivalent elastic modulus *Eₑ,* increasing the equivalent area moment of inertia *Iₑ,* and reducing the applied force *F* and torque *M*. The strategies for increasing the elastic modulus and the area moment of inertia have already been discussed in section I.1.2 above, so those analyses need not be repeated here. In contrast, the force and torque acting on the part 20, 30, 40 are determined by the operation of the part 20, 30, 40 in the magnetic interaction zone 130 and may not be arbitrarily reduced. Therefore, enhancing the elastic modulus and the area moment of inertia is an effective method for mitigating displacement and rotational errors. Additionally, the segmented design method (yield strength segmentation design) proposed in section I.4.1 below can also effectively reduce or even eliminate the observed displacement and rotational errors.

We have discussed methods to reduce or even eliminate the displacement at the free end of the part 20, 30, 40 in the feeding zone 135, the free end connecting the part 20, 30, 40 to the magnetic interaction zone 130, thereby mitigating the impact of the displacement on the operation of the part 20, 30, 40 in the magnetic interaction zone 130. An alternative approach is to incorporate this displacement into the design of the operation for the part 20, 30, 40 in the magnetic interaction zone 130. In other words, when designing the operation for the part 20, 30, 40 in the magnetic interaction zone 130, this displacement is fully considered, and a compensation strategy is employed to account for it. This compensation method is relatively effective and practical, although it does increase the complexity of the design process.

### 1.2.3 Case Study Analysis

Below, specific embodiments derived from the study above are analyzed and the motion of the part 20,30, 40 in the feeding zone 135 is discussed, focusing on the feasibility of the operation, the effective operating distance, and the displacement error at the part's free end. For example, in Application III - Coordinated multi-instrument operation, the outer part 20 is fabricated as a tube from PDMS with a base-to-curing agent mass ratio of 20:1, having an elastic modulus of 0.33 MPa, see Fig. 118, an outer diameter of 2.6 mm, and an inner diameter of 1.7 mm. For the inner part 30, a rod is used as a substitute; it is made from PDMS with a base-to-curing agent mass ratio of 10:1, with an elastic modulus of 1 MPa, see Fig. 118, and a diameter of 1.4 mm.

Thereby, Fig. 118 depicts the elastic modulus E of different materials listed in the following table:

| **Symbol** | **Materials** |
|---|---|
| A | PDMS (polydimethylsiloxane ) with a base-to-curing agent ratio (B:C ratio) of 10:1 + Magnetic particle m_{PDMS} : m_{Magnetic particle}=3:1 |
| B | PDMS, B:C ratio 3:1 |
| C | PDMS, B:C ratio 5:1 |
| D | PDMS, B:C ratio 10:1 |
| E | PDMS, B:C ratio 15:1 |
| F | PDMS, B:C ratio 20:1 |

Tensile tests were conducted using a Universal Testing System (Model 5942, INSTRON, USA). The tensile test for each ratio was repeated three times to calculate the mean and standard deviation.

Suppose that, in the magnetic interaction zone 130, the body 10 is required to achieve a radius of curvature of 15 mm. Under these conditions, experiments measured a relative motion resistance between the parts 20, 30 of 15.2 mN (Fig. 115). By setting this 15.2 mN as the critical buckling load in Equation (S9-28), the calculated maximum operating lengths are 39.7 mm for the outer part 20 and 22.1 mm for the inner part 30 formed by the rod. Therefore, the outer part 20 and the inner part 30 can be arbitrarily pushed over ranges of 39.7 mm and 22.1 mm, respectively. For the yield force calculation, as discussed in section I.1.3 above, the ultimate tensile strength is substituted for the conventional yield strength (see Equation (S9-25)). Experimental results indicate that PDMS with a base-to-curing agent mass ratio of 20:1 has an ultimate tensile strength of approximately 0.4 MPa, whereas PDMS with a ratio of 10:1 exhibits an ultimate tensile strength of about 3.2 MPa. Using Equation (S9-13), the yield force for the outer part 20 is calculated to be 1.22 N, and the yield force for the inner part 30 formed by the rod is 4.93 N. Since both the outer part 20 and the inner part 30 possess yield forces that far exceed the relative motion resistance of 15.2 mN, pulling operations can be readily performed.

Regarding the operational errors induced by the part 20, 30, 40 in the feeding zone 135, the parts 20, 30, 40 used in Application III - Coordinated multi-instrument operation may be analyzed as an example. For the radial displacement error, only the error due to the operating force is considered. To calculate the maximum error, a conservative approach is adopted: the maximum achievable operating length is used as the part length, and the maximum relative motion resistance is taken as the operating force. Generally, the part 20, 30, 40 transmits the operating force axially, so the angle between the force and the part's axis is minimal; for conservative calculation, this angle is set to 3°. Substituting these parameters along with the elastic modulus and geometric properties into Equations (S9-41), (S9-42) and (S9-46), a lateral deflection at the part's free end of 3.59 mm and an angular displacement of 14° are calculated. In addition, using the axial deformation formula $L = \frac{FL}{EA}$ yields an axial deformation of 0.132 mm, which is much smaller than the lateral deflection. This lateral error and angular displacement are directly superimposed on the part 20, 30, 40 in the magnetic interaction zone 130.

To address this issue, two approaches can be adopted. The first approach is to incorporate this error into the design and operation of the part 20, 30, 40 in the magnetic interaction zone 130 by employing a compensation strategy to mitigate or eliminate the error originating from the feeding zone 135. The second approach is to reduce these errors using the methods described in sections I.2.1 and I.2.2 above, whereby the second approach is exemplarily adopted in the embodiments. In conjunction with the solution presented in section I.4.1 below, the inner part 30 formed by the rod in the feeding zone 135 may be replaced by a 1 mm diameter copper rod. Pure copper has an elastic modulus of approximately 115 GPa and a yield strength of around 150 MPa. After substituting these data and recalculating, the lateral deflection at the part's free end is determined to be 5.05×10⁻⁴mm, the angular displacement is 0.002°, and the axial deformation is 3.71 ×10⁻⁶mm. These errors are negligible. Moreover, substituting these values into Equations (S9-28) and (S9-13) yields a critical buckling load of 2228 N and a yield force of 117.8 N, both of which far exceed the relative motion resistance of the part 30. Therefore, pushing and pulling can effectively be used to achieve relative motion between the parts 20, 30, 40.

### 1.3. Actuation zone

### 1.3.1 Mechanics Analysis

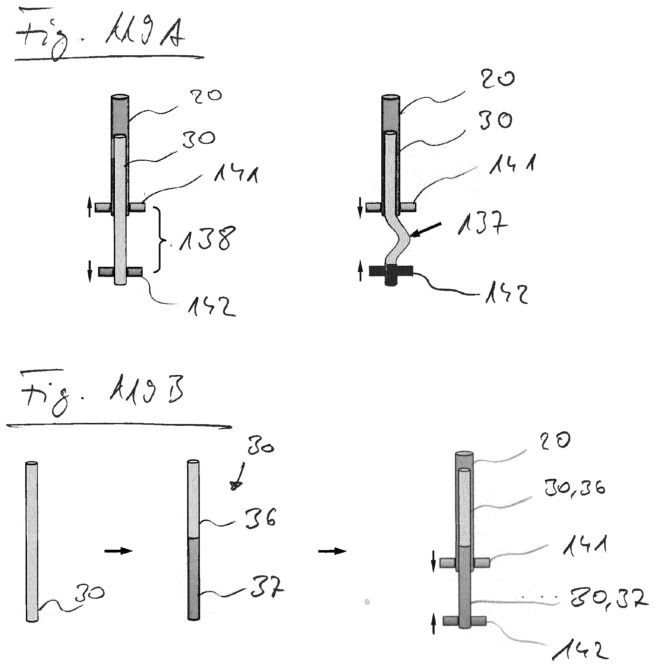

In the actuation zone 138, the two parts 20, 30 are actuated separately by the parts 141, 142 of the actuator 140 to perform the operation, as shown in Fig. 119A, which depicts bending deformation 137 of the tube formed by the outer part 20 and the inner part 30 in the actuation zone 138. Two grippers forming the parts 141, 142, of the actuator 140 independently control the two tubes forming the outer part 20 and the inner part 30, facilitating their relative motion, namely relative separation (left) and relative proximity (right). Due to the inherent characteristics of the tensile and compressive members, the tube forming the inner part 30 is prone to bending deformation 137 during relative proximity.

Thereby, only the inner part 30 undergoes tensile and compressive deformations. When pulling the inner part 30, similar to the analysis in section I.1.1 above, it is sufficient that the part's yield force exceeds the resistance to relative motion between the outer part 20 and the inner part 30. In the actuation zone 138, there is no nesting of multiple parts 20, 30, 40; therefore, this relative motion resistance is equal to the relative motion resistance in the feeding zone 135, *F_{f-total},* and must satisfy ${F}_{f - total} < {F}_{y}$ (S9-52)

For pushing the inner part 30, as analyzed in section I.2.1 above, the issue of compression rod stability arises. Consequently, it is similarly required that the critical buckling load exceeds the relative motion resistance, i.e., ${F}_{f - total} < {F}_{cr}$ (S9-53)

In summary, similar to the feeding zone 135, the mechanical criteria for achieving part manipulation in the actuation zone 138 are: $\left\{\begin{matrix}{F}_{cr} > {F}_{f - total} \\ {F}_{y} > {F}_{f - total}\end{matrix}\right.$ (S9-54)

### 1.3.2 Design and Optimization Strategies

In the actuation zone 138, only the tensile and compressive deformations of a single part, namely the inner part 30 are involved. If the part's operation does not satisfy Equation (S9-54), effective manipulation cannot be achieved. The specific design solutions and improvement strategies are identical to those in the feeding zone 135 and are therefore not repeated here. In addition, a segmented design method (stiffness segmentation design and yield strength segmentation design) as detailed in section I.4.1 below can be applied to the design of the parts 20, 30, 40 in the actuation zone 138. The detailed design is as follows.

With the segmented design method, the inner part 30 is no longer designed as a single, homogeneous element using identical materials or parameters. Instead, a second material with a higher elastic modulus and higher yield strength is used to fabricate a second inner part section 37 of the inner part 30 located in the actuation zone 138, while a first inner part section 36 located in the feeding zone 135 and in the magnetic interaction zone 130 is made from a first material having a lower elastic modulus and a lower yield strength. These two sections 36, 37 are then connected, as shown in Fig. 119B.

As shown in Fig. 119B, the inner part 30 comprises segments or sections 36, 37 with varying stiffness. The portion of the inner part 30 that is required to deform under the magnetic field 5 retains the soft tube design having the first material as the first inner part section 36, whereas the segment or second inner part section 37 located in the actuation zone is constructed from the second material with higher stiffness - employing either a semi-rigid or rigid material - to prevent the bending deformation 137.

The use of a high-modulus second material transforms the second inner part section 37 from a soft part into a semi-rigid or rigid part. As a result, in the magnetic interaction zone 130, where deformation is required, the original soft part is still employed as the first inner part section 36 to ensure the desired deformation and functionality, whereas, in the actuation zone 138, the semi-rigid or rigid second inner part section 37 substantially increases the yield force *F_{y}* and the critical buckling load *F_{cr},* thereby ensuring that Equation (S9-54) is satisfied and effective part manipulation is achieved. Quantitative improvements and corresponding operational performance are presented in section I.3.3 below.

### 1.3.3 Case Study Analysis

The method discussed in section I.3.2 above may be used to optimize the second inner part section 37 of the inner part 30 in the actuation zone 138 (with the inner part 30 replaced by a rod) in both Application I and Application III. Two different materials may be employed as the material of the second inner part section 37 for the improvement, and both yield excellent results.

The first material is a composite of PDMS and magnetic particles. In this composite, PDMS may be prepared with a base-to-curing agent mass ratio of 10:1, and the mass ratio of magnetic particles to PDMS may be 3:1, resulting in an elastic modulus of 12.3 MPa (Fig. 118). In the actuation zone 138, the actuator spacing Lₐₛ between the parts 20, 30 is exemplarily set to 10 mm, and the rod forming the inner part 30 exemplarily has a diameter of 1.4 mm. By substituting these parameters into the critical buckling load equation (Equation (S9-28)), the critical buckling load is calculated to be 920 mN. In contrast, using pure PDMS with a 10:1 mass ratio (elastic modulus of 1 MPa) yields a critical buckling load of only 75 mN. Thus, the segmented design dramatically increases the critical buckling load compared to the original design. For the yield force calculation, as discussed in section I.1.3 above, the "yield point" for elastomers is less well-defined than for metals due to their nonlinear elastic behavior under large strains. Therefore, the ultimate tensile strength is used in the calculations. Experimentally, the ultimate tensile strength of the first material (the PDMS and magnetic particle composite) is approximately 4.3 MPa, compared to about 3.2 MPa for PDMS with a 10:1 mass ratio. Substituting these values into Equation (S9-13) results in a yield force of 7.1 N for the composite, whereas pure PDMS provides a yield force of 5.28 N. Furthermore, experimental measurements of the relative motion resistance between the parts 20, 30, 40 under various radii of curvature R (Fig. 115A) show that operating a part 20, 30, 40 in the magnetic interaction zone 130 with a radius of curvature of 5 mm requires a force of 43 mN, while a radius of curvature of 2 mm demands 118 mN. Therefore, the segmented design method enables both pushing and pulling operations for parts 20, 30, 40 with a radius of curvature of 2 mm, whereas the original design would allow only pulling.

The second material is pure copper. The rod forming the inner part 30 may have a copper post, having, for example, a diameter of 1 mm, in the second inner part section 37. Pure copper exhibits an elastic modulus of approximately 115 GPa and a yield strength of around 150 MPa. Substituting these values into Equations (S9-28) and (S9-13) yields a critical buckling load of 2228 N and a yield force of 117.8 N, respectively - values that far exceed the necessary requirements. Therefore, using a copper rod instead of a PDMS rod will enable very smooth execution of the relative motion operation between the parts 20, 30, 40.

### 1.4. Other considerations

In the preceding three sections, the three zones - the magnetic interaction zone 130, the feeding zone 135, and the actuation zone 138 - have been analyzed and discussed and comprehensive design solutions and improvement strategies have been provided. However, during practical applications, several specific operational requirements arise. Although these requirements could be addressed within the scope of the aforementioned discussions and solutions, a more detailed analysis may be needed to effectively tackle these particular issues. Accordingly, the following separately analyzes and discusses long-range operation, multi-bend deformation operation, high-curvature deformation operation and multi-nested-part operation, and discloses corresponding effective embodiments as solutions.

### 1.4.1 Long-Range Operation

The operational region of the outer and inner parts 20, 30, 40 and the outer and inner parts 20, 30, 40 themselves may be divided into three zones: the magnetic interaction zone 130, the feeding zone 135, and the actuation zone 138. Only the magnetic interaction zone 130 and the feeding zone 135 contribute to the effective operational length of the outer and inner parts 20, 30, 40. Therefore, the Effective Operating Distance (*Dₑₒ*) of the outer and inner parts 20, 30, 40 is defined as the sum of the part lengths within these two zones, i.e., ${D}_{eo} = {L}_{mi} + {L}_{f}$ (S9-55) where *Lₘᵢ* represents the Magnetic Interaction Length (in the magnetic interaction zone 130) and *L_{f}* is the Feeding Length (in the feeding zone 135). In all embodiments presented in the foregoing and in the following - whether involving 1D part or 2D/3D objects derived from the 1D part - the Effective Operating Distance exemplarily remains within 100 mm. With increasing part length, additional design considerations emerge. The following discloses embodiments and design strategies tailored for long-distance operations (100 mm or longer).

Based on the analysis in the previous sections, to achieve the relative motion of the outer and inner parts 20, 30, 40, the relative motion resistance *Fₘ₋ₜₒₜₐₗ,* the critical buckling load *F_{cr}*, and the yield force *F_{y}* must satisfy Equation (S9-16). However, as the operating length increases, the relative motion resistance *Fₘ₋ₜₒₐₜₐₗ* increases while the critical buckling load *F_{cr}* decreases, making it increasingly challenging or even impossible to meet this condition. This issue may be addressed by a segmented design method that enables long-distance operations. Moreover, this segmented design method can also effectively resolve the challenges encountered in short-distance operations.

### I.4.1.1 Segmented Design Method

The segmented design method involves dividing the outer and inner parts 20, 30, 40 into several smaller segments, each with distinct physical or geometric parameters, and then assembling these segments to form a complete outer or inner part 20, 30, 40. As a result, the outer or inner part 20, 30, 40 exhibits different physical properties in each segment. In this study, based on the analysis in the previous sections, the two key parameters affecting the relative motion resistance *Fₘ₋ₜₒₜₐₗ,* the critical buckling load *F_{cr},* and the yield force *F_{y}* are the bending stiffness and yield strength.

Therefore, different stiffnesses and yield strengths are assigned to the outer and inner parts 20, 30, 40 in the magnetic interaction zone 130, the feeding zone 135, and the actuation zone 138. The segmented stiffness for the different regions or zones 130, 135, 138 is as follows: ${E}_{s} {I}_{s} = \left\{\begin{matrix}{E}_{1} {I}_{1} , & Magnetic interaction zone \\ {E}_{2} {I}_{2} , & Feeding zone \\ {E}_{3} {I}_{3} , & Actuation zone\end{matrix}\right.$ (S9-56) where *EₛIₛ* represents the segmented stiffness, and *EᵢIᵢ* (*i*=1, 2, 3) denotes the stiffness of each region or zone 130, 135, 138. Similarly, the segmented yield strengths for the different regions or zones 130, 135, 138 are given as follows ${σ}_{ys} = \left\{\begin{matrix}{σ}_{y 1} , & Magnetic interaction zone \\ {σ}_{y 2} , & Feeding zone \\ {σ}_{y 3} , & Actuation zone\end{matrix}\right.$ 57

Here, *σ_{ys}* represents the segmented yield strength, and *σ_{yi}* (*i =* 1, 2, 3) denotes the yield strength in each region or zone 130, 135, 138. Since the relative motion resistance between the parts increases from the magnetic interaction zone 130 to the feeding zone 135 and the actuation zone 138, the segmented stiffness and segmented yield strength can be set as follows: ${E}_{1} {I}_{1} < {E}_{2} {I}_{2} < {E}_{3} {I}_{3}$ (S9-58) ${σ}_{y 1} < {σ}_{y 2} < {σ}_{y 3}$ (S9-59)

Based on the above equations, three materials with increasing stiffness and yield strength can be selected to manufacture segments of the outer and inner parts 20, 30, 40 in the magnetic interaction zone 130, the feeding zone 135, and the actuation zone 138, respectively. The stiffness *EI* is the product of the elastic modulus E and the moment of inertia *I* of the cross-section. Since altering the moment of inertia *I* would require changes in dimensions that not only increase manufacturing difficulty but also affect the fit between the outer and inner parts 20, 30, 40, modifying E is the simplest and most direct method to change the stiffness of the outer and inner parts 20, 30, 40. Therefore, the segmented design of stiffness is equivalent to a segmented design of the elastic modulus, namely: ${E}_{s} = \left\{\begin{matrix}{E}_{1} , & Magnetic interaction zone \\ {E}_{2} , & Feeding zone \\ {E}_{3} , & Actuation zone\end{matrix}\right.$ (S9-60)

Because the relative motion resistance changes with the position of the outer and inner parts 20, 30, 40 within the environment, being zero at the end of the magnetic interaction zone 130 and reaching its maximum at the gripper or actuator 140 in the actuation zone 138, the design may be improved by dividing the outer and inner parts 20, 30, 40 into more segments instead of into only three segments. The detailed segmentation is as follows: $E \left(x\right) = \left\{\begin{matrix}{E}_{1} , & x ∈ \left[0, {L}_{1}\right) \\ {E}_{2} , & x ∈ \left[{L}_{1}, {L}_{2}\right) \\ ⋮ & ⋮ \\ {E}_{n} , & x ∈ \left[{L}_{n - 1}, {L}_{n}\right]\end{matrix}\right.$ (S9-61) ${E}_{1} < {E}_{2} < ⋯ < {E}_{n}$ (S9-62) $0 < {L}_{1} < {L}_{2} < ⋯ < {L}_{n} = L$ (S9-63) where *x* denotes the axial distance along the respective outer and inner part 20, 30, 40, with the starting point at the first end 24, 34, 44 of the respective outer and inner part 20, 30, 40 in the magnetic interaction zone 130, and L represents the total length of the respective outer and inner part 20, 30, 40. Naturally, if conditions permit, a design approach similar to that of "constant strength beams" may be adopted to further increase the number of segments and achieve a smooth and gradual transition. Its elastic modulus can be expressed as: $E = {E}_{0} + kx$ (S9-64) ${E}_{0} > 0 , k > 0$ (S9-65)

The yield strength of the outer and inner parts 20, 30, 40 can be designed in the same manner.

By employing the segmented design method described above, the outer and inner parts 20, 30, 40 can be configured with varying critical buckling loads *F_{cr}* and yield forces *F_{y}* along their length, with these values increasing toward the actuation zone 138. Since the relative motion resistance *Fₘ₋ₜₒₜₐₗ* between the outer and inner parts 20, 30, 40 also increases near the actuation zone 138, this design approach ensures that in each segment the critical buckling load *F_{cr}* and yield force *F_{y}* always exceed the relative motion resistance *Fₘ₋ₜₒₜₐₗ*, thereby enabling long-range operation.

### 1.4.1.2 Design and Optimization Strategies

According to the segmented design concept described above, the parts 20, 30, 40 may be divided into individual segments.

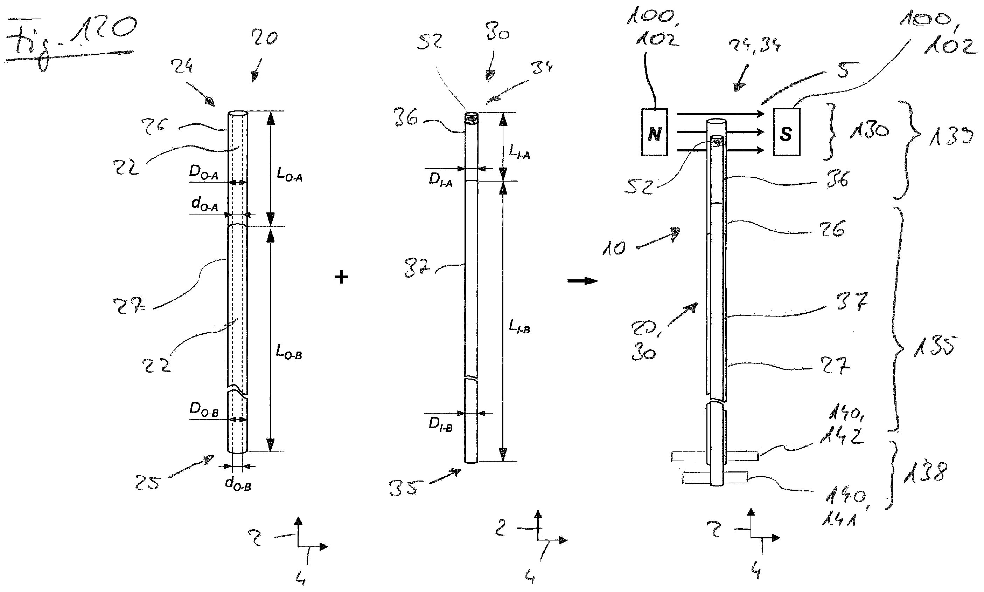

Fig. 120 exemplarily depicts a first variant of the first configuration of the body 10 shown in Fig. 3. With the first variant, the outer part 20 comprises a first outer part section 26 that is configured to be located next to the magnetic unit 52 guided within the channel 22 of the outer part 20 and a second outer part section 27 configured to be placed away from the magnetic unit 52. Furthermore, the inner part 30 of the first variant comprises a first inner part section 36 that is configured to be located next to the magnetic unit 52 and a second inner part section 37 joining the first outer part section 36, whereby the second inner part section 37 is configured to be placed away from the magnetic unit 52.

With the exemplary embodiment, the first inner part section 36 is configured to be placed next to the magnetic unit 52 by attaching the magnetic unit 52 to one end of the first inner part section 36 and the second inner part section 37 is configured to be placed away from the magnetic unit 52 by attaching the second inner part section 37 to an opposite end of the first inner part section 36. Furthermore, the first outer part section 26 is configured to be placed next to the magnetic unit 52 by locating the magnetic unit 52 within the channel 22 in the region of the first outer part section 26 and the second outer part section 27 is configured to be placed away from the magnetic unit 52 by locating the magnetic unit 52 at a distance from the second outer part section 27 during use of the soft magnetic robot 1.

The first inner part section 36 is made from a first inner part material that has a higher stiffness than a second inner part material of the second inner part section 37. Likewise, the first outer part section 26 is made from a first outer part material that has a higher stiffness than a second outer part material of the second outer part section 27.

The second inner part section 37 is fixed to the first part 141 of the actuator 140 at its end facing away from the first inner part section 36. The second outer part section 27 is fixed to the second part 142 of the actuator 140 at its end facing away from the first inner part section 36. The first part 141 and the second part 142 of the actuator 140 are movable with respect to each other to change the relative position of the inner part 20 and the outer part 30.

As it is depicted to the right in Fig. 120, the first outer part section 26 and the first inner part section 36 are configured to be placed within the interaction zone 130 that is filled with the magnetic field 5. A vibrating sample magnetometer (VSM) may be used to generate the magnetic field 5. Due to the action of the magnetic field 5 on the magnetic unit 52 located at the first ends 24, 34 of the outer part 20 and the inner part 30, the flexible first inner part section 36 and the flexible first outer part section 26 deform under the action of the magnetic field 5. The first inner part section 36 thereby is located within a bending zone 139 of the channel 22 of the outer part 20 and of the first outer part section 26.

The second ends 25, 35 of the outer part 20 and the inner part 30 are located within the actuation zone 138 in which the outer part 20 and the inner part 30 are connected to the actuator 140. The feeding zone 135 is located in between the bending zone 139 and the actuation zone 138 and the second outer part 27 is located in the feeding zone 135 and connects the first outer part 26 to the actuator 140 in the feeding zone 135. Likewise, the second inner part 37 is located within the feeding zone 135 and connects the first inner part 26 to the actuator 140 in the feeding zone 135.

The feeding zone 135 lies in between the interaction zone 130 and the actuation zone 138.

With other embodiments, the inner part 30 may comprise further inner part sections 38 adjoining, one after the other, the second inner part section 37 towards the second end 35. The further inner part sections 38 may have differing stiffnesses among each other. For example, the stiffnesses of the further inner part sections 38 may increase with increasing distance from the second inner part section 37. Additionally or alternatively, the outer part 20 may comprise further outer part sections 28 adjoining, one after the other, the second outer part section 27 towards the second end 25. The further outer part sections 28 may have differing stiffnesses among each other. For example, the stiffnesses of the further outer part sections 28 may increase with increasing distance from the second outer part section 27.

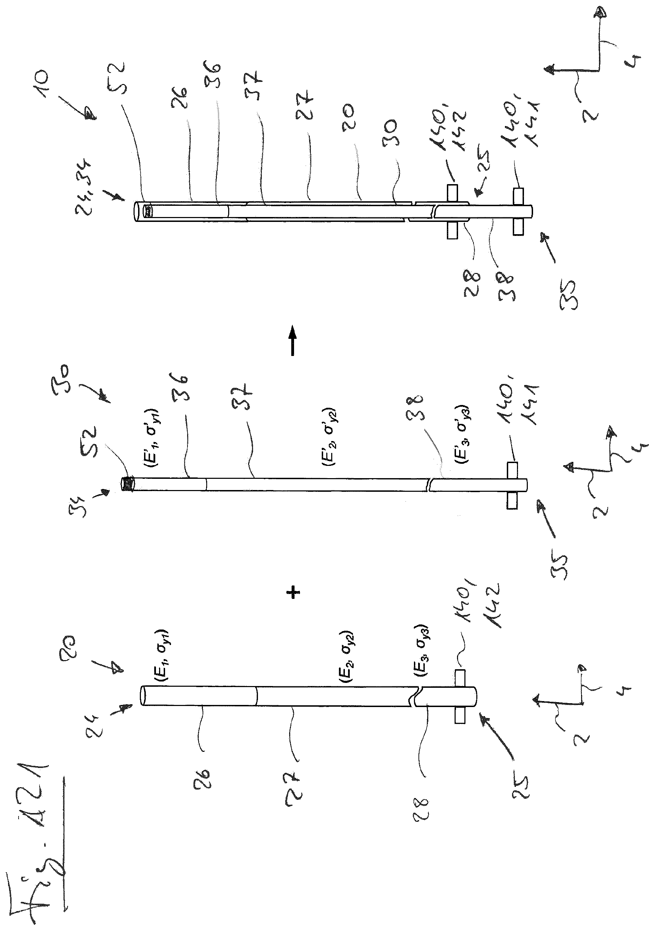

Fig. 121 shows a variant of the body 10 shown in Fig. 120, wherein the outer part 20 has one further outer part section 28 located in between the second outer part section 27 and the actuator 140 and the inner part 30 has one further inner part section 38 located in between the second inner part section 37 and the actuator 140.

With the embodiment in Fig, 121, both the outer part 20 exemplarily configured as an outer tube and the inner part 30 exemplarily formed by an inner tube are divided into three segments or sections 26, 27, 28, 36, 37, 38, each featuring distinct elastic moduli Eᵢ and yield strengths σᵢ that progressively increase from the distal first ends 24, 34 towards the gripper-ends formed by the second ends 25, 35. For both the outer part 20 and the inner part 30, the respective three segmented sections 26, 27, 28, 36, 37, 38 are secured together to form a respective composite tube, which tubes then are nested for assembly.

As shown in Fig. 121, for the outer part 20, the corresponding elastic modulus and yield strength of the first, second and further outer part sections 26, 27, 28 are *Eᵢ* and *σ_{yi}* (i = 1, 2, 3), respectively, with their values increasing sequentially. For the inner part 30, the corresponding elastic modulus and yield strength of the first, second and further inner part sections 36, 37, 38 are *E'ᵢ* and *σ*'*_{yi}* (i = 1, 2, 3), which also increase respectively. In the same region, the elastic modulus and yield strength of adjacent outer part sections 26, 27, 38 and inner part sections 36, 37, 38 may be adapted to the dimensions of the inner and outer parts 20, 30, 40, for example to ensure that the inner and outer parts 20, 30, 40 exhibit the same critical buckling load and/or yield force. Under appropriate conditions, for outer part sections 26, 27, 38 and inner part sections 36, 37, 38 located within the same zone, a straightforward approach can be adopted by setting them to have the same elastic modulus and/or yield strength, for example, by fabricating them using the same material.

For example, the elastic modulus and/or yield strength of the first outer part section 26 may be adapted to, such as equal to, the elastic modulus and/or yield strength of the first inner part section 36. The elastic modulus and/or yield strength of the second outer part section 27 may be adapted to, such as equal to, the elastic modulus and/or yield strength of the second inner part section 37. Furthermore, the elastic modulus and/or yield strength of each further outer part section 28 may be adapted to, such as equal to, the elastic modulus and/or yield strength of a corresponding further inner part section 38 located adjacent to the respective further outer part section 28 in the outer direction 4.

In fact, there is no standard for dividing the outer and inner parts 20, 30,40 into segments or sections as long as the required performance criteria are met, although the gradual increase method described above (Equation (S9-64)) may be optimal. For example, in Applications I and III, a two-segment design is adopted where the second outer and inner part sections 27, 37 in the actuation zone 138 are adjusted in terms of their elastic modulus and yield strength by providing a second material, while the first outer and inner part sections 26, 36 in the other zones 130, 135 continued to use the original first material. The first material may be a composite of the matrix material, such as PDMS or Ecoflex, and a magnetic powder, and the second material may be pure copper. With the first material being PDMS, the part's critical buckling load increased from 75 mN to 920 mN, and the yield force rose from 5.28 N to 7.1 N. With the second material, the part's critical buckling load increased from 75 mN to 2228 N, and the yield force increased from 5.28 N to 117.8 N. A detailed analysis is presented in section I.3.3 above.

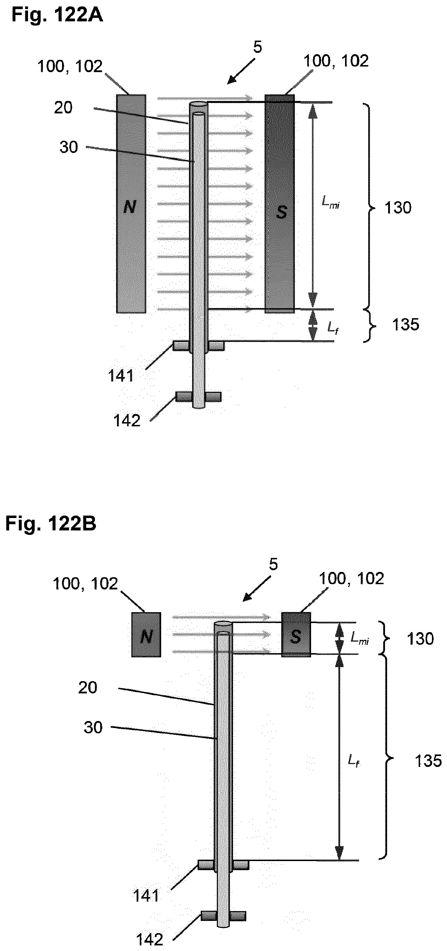

For long-range operation, according to the definition given in Equation (S9-55), the Effective Operating Distance comprises, such as consists of, the Magnetic Interaction Length *Lₘᵢ* and the Feeding Length *L_{f}.* Since the Effective Operating Distance may be determined by these two parameters, long-range operation can be categorized into three distinct modes based on their relative contributions: long-range operation with a large Magnetic Interaction Length, long-range operation with a large Feeding Length, and long-range operation with large Magnetic Interaction Length and large Feeding Length (see Fig. 122). The following sections provide a detailed explanation of these three modes.

Fig. 122 depicts different modes of long-range operation. Fig. 122A shows long-range operation with a large Magnetic Interaction Length. This operating mode features an extensive magnetic field region - that is, a relatively large or long magnetic interaction zone 130 paired with a comparatively small or short feeding zone 135. Accordingly, it is characterized by a large magnetic interaction length Lₘᵢ and a small Feeding Length L_{f}. Fig. 122B shows long-range operation with a large feeding length L_{f}. This operating mode, in contrast to the previous one, features a small or short magnetic interaction zone 130 paired with a larger or longer feeding zone 135.

### (1) Long-range operation with a large Magnetic Interaction Length

When significant deformation or complex operations are required, it may be necessary to increase the magnetic interaction length Lₘᵢ. In such cases, the soft robot 1 features an enlarged magnetic interaction zone 130 while the feeding zone 135 remains relatively small, as shown in Fig. 122A. Due to the relatively small size of the feeding zone 135, if it is negligible compared to the magnetic interaction zone 130, the outer and inner part sections 27, 28, 37, 38 in the feeding zone 135 and their corresponding feeding length Lt may be disregarded. Outer and inner part located in the actuation zone 138, such as the second outer and inner part sections 27, 37, may be fabricated using materials with high stiffness and yield strength (see section I.3 for details). Meanwhile, the outer and inner part sections located within the enlarged magnetic interaction zone 130, such as the first outer and inner part sections 26, 36, may be modified to accommodate long-range operation.

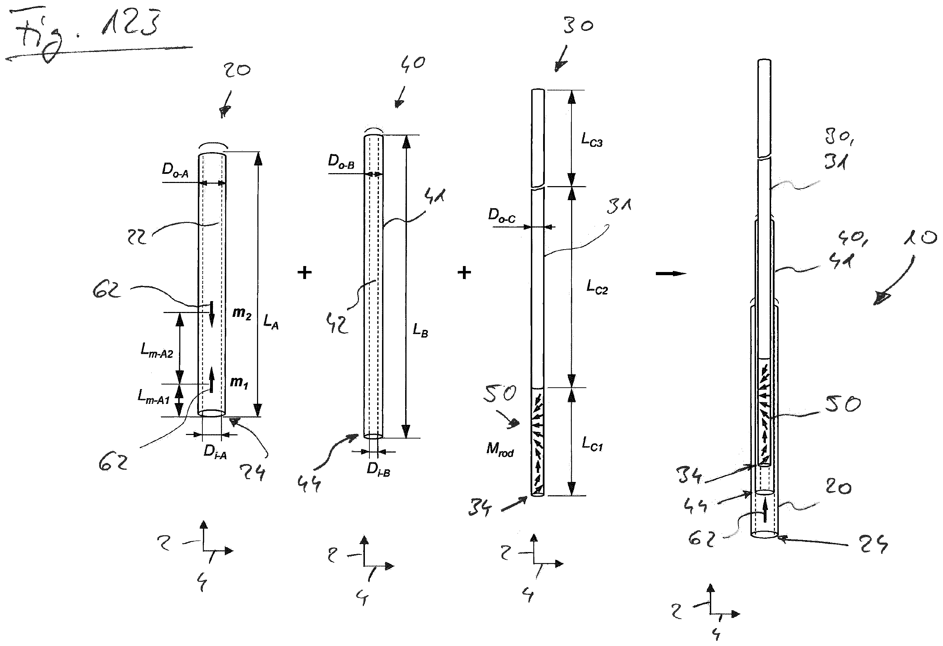

For example, an embodiment adapted for "Long-range Operation with a Large Magnetic Interaction Length," as shown in Fig. 123, may comprise a body 10 with an outer part 20, an inner part 30 and a further inner part 40. As far as no differences are disclosed in the description and the Figures, the body 10 is configured as it is disclosed for the body 10 of the sixth configuration of the cylindrical body 10 shown in Fig. 23 and vice versa. The inner part 30 may be fabricated as an inner rod or, alternatively, as a tubular member. The inner part 30 has the magnetic element 50 at the tip formed by the first end 34 of the inner part 30, which magnetic element 50 may have a continuous magnetization profile. The magnetization profile of the magnetic element 50 may be identical to the magnetization profile of the magnetic element 50 for the 3D single-obstacle traversal shown in Fig. 71.

The outer part 20 may have a first further magnetic unit 62 located at the first end 24 of the outer part 20. Thereby, the first further magnetic unit 62 may be fixed at a first distance L_{m-A1} from the first end 24. Furthermore, the outer part 20 may have a second further magnetic unit 62, which may be fixed at a second distance L_{m-A2} from the first further magnetic unit 62. The magnetic moments of the first and second further magnetic units 62 may have opposite directions and/or equal magnitudes. The further inner part 40 may be free from magnetic units.

The following table lists exemplary parameter values of the embodiment shown in Fig. 123A.

| | | |
|---|---|---|
| outer diameter of outer part 20 | *D_{o-A}* | 4.8 mm |
| inner diameter of outer part 20 | *D_{i-A}* | 3.8 mm |
| length of outer part 20 | *L_{A}* | 155 mm |
| first distance | *L_{m-A1}* | 52 mm |
| second distance | *L_{m-A2}* | 47 mm |
| outer diameter of further inner part 40 | *D_{o-B}* | 2.8 mm |
| inner diameter of further inner part 40 | *D_{i-B}* | 2 mm |
| length of further inner part30 | *L_{B}* | 315 mm |
| outer diameter of inner part 30 | *D_{o-C}* | 1.4 mm |
| length of magnetic element 50 | *L_{C1}* | 53 mm |
| first length of flexible part 31 | *L_{C2}* | 300 mm |
| second length of flexible part 31 | *L_{C3}* | 85 mm |
| magnetic moment of first further magnetic unit 62 | *m₁* | 2.4×10⁻² Am² |
| magnetic moment of second further magnetic unit 62 | *m₂* | 2.4×10⁻² Am² |
| magnetization of magnetic element 50 | *M_{rod}* | 188716 A/m |

A first method involves segmenting the outer and inner parts 20, 30, 40 within the magnetic interaction zone 130. Since the outer and inner parts 20, 30, 40 have a relatively long length within the magnetic interaction zone 130, the relative motion resistance *Fₘ₋ₜₒₜₐₗ* increases with length. To satisfy the conditions for relative motion (Equation (S9-16)), it may be necessary to both reduce the relative motion resistance *Fₘ₋ₜₒₜₐₗ* and enhance the critical buckling load *F_{cr}* and yield force *F_{y}*. The approach outlined in section I.1.2 above may be applied to reduce *Fₘ₋ₜₒₜₐₗ.* However, relying solely on this strategy may prove insufficient when the operating length increases substantially, as the mechanical conditions for relative motion might no longer be met. In contrast, increasing *F_{cr}* and *F_{y}* is more effective. Therefore, based on the analysis in section I.4.1.1 above, improvements may be achieved by enhancing the stiffness and yield strength of the outer and inner parts 20, 30,40.

With the embodiment shown in Fig. 123, the first end 24 of the outer part 20 is configured to be positioned in the magnetic interaction zone 130 on the side towards the actuation zone 138, while the first end 44 of the further inner part 40 extends out of the first end 24 of the outer part 20 and exemplarily farther into the magnetic interaction zone 130.

The outer part 20 may be fabricated using PDMS with a base-to-curing agent mass ratio of 10:1, yielding an elastic modulus of 1 MPa. The further inner part 40 may be made using PDMS with a mass ratio of 15:1, resulting in an elastic modulus of 0.55 MPa. Since the outer part 20 is nearer to the actuation zone 138, its relative motion resistance is higher. This design provides a larger critical buckling load of the outer part 20, enabling it to overcome the relative motion resistance between the parts 20, 40. Additionally, the design may also provide for multiple bending deformations under the same magnetic field 5. The design is presented as an example for reference.

A second method involves designing the sections of the parts 20, 30, 40 in the magnetic interaction zone 130, such as the first inner and outer sections 27, 37, as a whole using materials with high stiffness and high yield strength. This approach is relatively straightforward, as it directly employs the same material for the sections of the parts 20, 30, 40 in the magnetic interaction zone 130. For example, with the embodiment for "Long-range Operation with a Large Magnetic Interaction Length" shown in Fig. 123, the inner part 30 is fabricated using a composite of PDMS and magnetic particles. The PDMS is prepared with a base-to-curing agent mass ratio of 10:1, and the magnetic particle are mixed with PDMS at a mass ratio of 3:1, resulting in a composite elastic modulus of 12.3 MPa. In comparison, PDMS with a base-to-curing agent mass ratio of 10:1 has an elastic modulus of 1 MPa. This design increases the critical buckling load of the inner part 30 by a factor of 11.3.

With the embodiment of Fig. 123, the outer part 20 and the further inner part 40 are fabricated according to the first method, and the inner part 30 is fabricated according to the second method. The parts 20, 30, 40 were then assembled and operated within a large magnetic field region or workspace 131. The magnetic interaction length may be, for example, Lₘᵢ = 305 mm. Including the length in the feeding zone 135, the total Effective Operating Distance can be expressed as ${D}_{eo} = {L}_{f} + 305 mm$ (S9-66)

A magnetic interaction length of Lₘᵢ = 305 mm is only an example. Naturally, with increased part stiffness and yield strength, this length could be further extended. However, an increase in stiffness may require parts 20, 30, 40 to have a larger magnetic moment or to be exposed to a stronger magnetic field 5 to achieve the desired deformation. A detailed analysis is presented in section I.1.2.2 above.

### (2) Long-range operation with a large Feeding Length

If the magnetic interaction zone 130 is located far from the actuation zone 138, it may be necessary to increase the size of the feeding zone 135 - that is, to extend the Feeding Length - to achieve long-range operation. In this case, the feeding zone 135 may be significantly larger than the magnetic interaction zone 130, see Fig. 122B. To ensure that the relative movability of the outer and inner parts 20, 30, 40 can be maintained within this substantially enlarged feeding zone 135, the relative motion resistance *F_{f-total}* between the outer and inner parts 20, 30, 40 may be reduced and the critical buckling load *F_{cr}* and the yield force *F_{y}* may be increased. The reduction in *F_{f-total}* can be achieved using the method described in section I.1.2, while the enhancement of *F_{cr}* and *F_{y}* can be accomplished by directly employing materials with high stiffness and high yield strength.

Since the sections of the outer and inner parts 20, 30, 40 in the feeding zone 135, such as the second inner and outer sections 27, 37, are not within the magnetic field 5 and do not undergo deformation, they solely function to control the sections in the magnetic interaction zone 130. Therefore, the sections in the feeding zone 135 can be fabricated using materials with arbitrarily high stiffness and yield strength. In theory, as long as the material's stiffness and yield strength are sufficiently high, the critical buckling load *F_{cr}* and yield force *F_{y}* will also be high, thereby allowing for an extended feeding length. This approach could theoretically enable operation over any distance.

For example, in the embodiment suited for "Long-range operation with a large Feeding Length" shown in Fig. 120, the second outer part section 27 and the second inner part section 37, both located in the feeding zone 135, are formed by catheters of a commercial catheter set. The commercial catheter set (WAIN-FBK-6SD110, ASAHI INTECC Co. LTD., JP) contains two catheters: an outer catheter with an outer diameter of 2.2 mm and an inner diameter of 1.8 mm, and an inner catheter with an outer diameter of 2.7 mm and an inner diameter of 2.28 mm. Both catheters have a length of 1100 mm. The outer catheter forms the second outer part section 27 and the inner catheter forms the second inner part section 37.

After nesting these two catheters, they are connected with soft parts forming the first outer part section 26 and the first inner part section 36. The first outer part section 26 and the first inner part section 36 are placed in the magnetic field 5, and by manipulating the ends of the commercial catheters, the first outer part section 26 and the first inner part section 36 are controlled. If the section of the body 10 in the magnetic interaction zone 130 has a length *Lₘᵢ*, then the Effective Operating Distance of the body 10 in this experiment is ${D}_{eo} = {L}_{mi} + 1100 mm$ (S9-67)

The soft part of the body 10 comprising the first outer part section 26 and the first inner part section 36 may have a length of 140 mm, so the total Effective Operating Distance may be 1240 mm, whereby the feeding length of 1100 mm corresponds to the length of the commercial catheter used. As discussed above, by selecting materials with higher stiffness and yield strength, an even larger feeding length can be achieved.

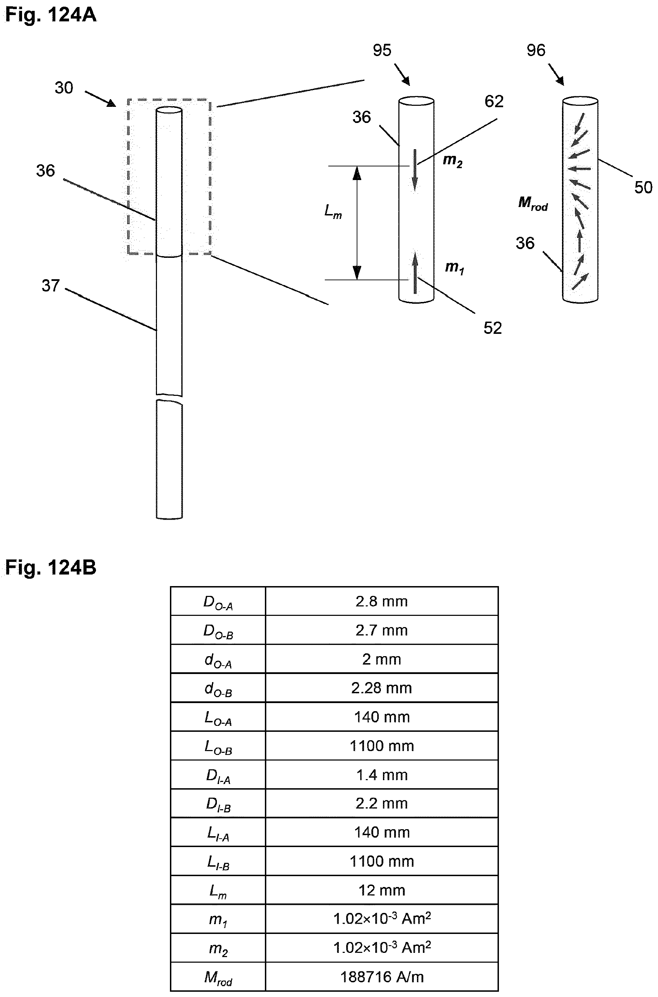

Fig. 124A depicts two different alternative magnetization profiles of the first inner part 36, which may be formed by the soft rod connected to the inner catheter of the catheter set shown in Fig. 120. A first profile 95 comprises a magnetic unit 52 and a further magnetic unit 62 located at a distance Lₘ from each other. The magnetic unit 52 and the further magnetic unit 62 thereby have magnetic moments with equal magnitude and opposite direction. A second profile 96 comprises a magnetic element 50 that has a magnetization M_{rod} that continuously changes from a first end 54 to a second end 55 of the magnetic element 50. The second magnetization profile is identical to the magnetization profile shown in Fig. 71 for 3D single-obstacle traversal. The first profile 95 exhibits deformation confined to a plane, whereas the second profile 96 enables 3D deformation.

Fig. 124B lists exemplary parameter values of the body 10 having inner and outer parts 20, 30 with two sections 26, 27, 36, 37 shown in Figures 120, 124A.

### (3) Long-range operation with large Magnetic Interaction Length and large Feeding Length

For operations in which both the magnetic interaction zone 130 and the feeding zone 135 are large or long, the two aforementioned schemes may be combined. In each zone 130, 135, 138, the corresponding method can be applied, and the total Effective Operating Distance is the sum of the Magnetic Interaction Length and the Feeding Length for the respective zones.

### 1.4.1.3 Magnetic Field Design Scheme

When the magnetic interaction zone 130 occupies a large space, the requirements for the magnetic field generation device become more stringent. For example, a Vibrating Sample Magnetometer (VSM) (EZ7, MicroSense, USA) may be employed as the magnetic field generator 100, which may provide a uniform magnetic field region of, for example, approximately 45 mm × 45 mm × 45 mm. For embodiments involving a large magnetic interaction zone 130, the conventional approach is to design a large-scale magnetic field generator 100 to obtain the required magnetic field 5. Although this method is straightforward, such large-scale devices significantly increase capital investment and can, to some extent, limit the application and dissemination of magnetically controlled technologies. To address this issue, a multi-magnetic field control method may employ a spatial combination of multiple magnetic fields 5 to replace a large-scale magnetic field device for controlling the body 10.

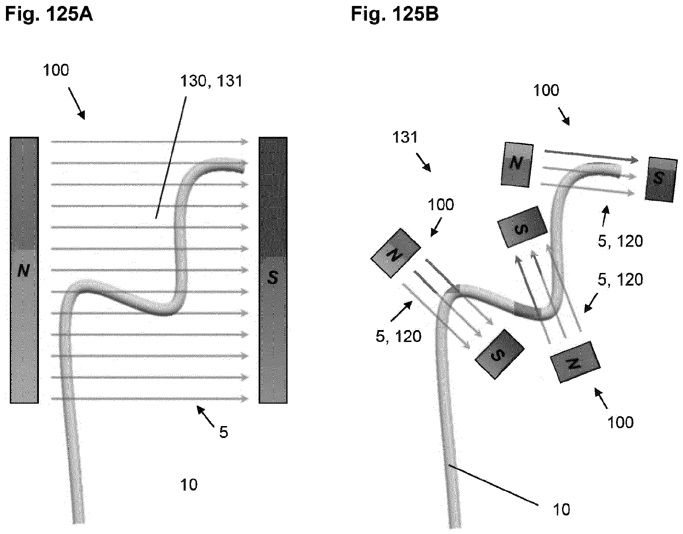

As shown in Fig. 125A, a large-scale magnetic field generator 100 may be used to create the workspace 131 required by the body 10. A schematic of a multi-magnetic field control method is presented in Fig. 125B. Based on the specific operational requirements of the body 10, magnetic fields 5, such as multiple small-scale magnetic fields 5, are designed and arranged in regions where deformation is needed. In regions where the body 10 remains undeformed, the magnetic fields can be omitted. This approach employs a spatial distribution of magnetic fields 5 to control different sections of the body 10. In concert, these fields 5 can achieve the desired deformation of the body 10, effectively replacing the need for a large-scale magnetic field generator 100.

Fig. 125A thereby illustrates operation of the soft robot 1 within a large magnetic interaction zone 130, whereby the body 10 operates within an extensive magnetic field region and a large-scale magnetic field generator 100 is used to create the magnetic field 5 that covers the entire workspace 131 of the body 10. Fig. 125B shows an alternative multi-magnetic field control method. By superimposing multiple small-scale magnetic fields 5 in space, different regions of the body 10 can be independently controlled, thereby substituting for a large-scale magnetic field 5 of Fig. 124A.

The setup shown in Fig. 125B comprises at least two magnetic field generators 100, whereby each magnetic field generator 100 generates a respective magnetic field 5. The magnetic field generators 100 are located spaced apart from each other along a path. Each magnetic field generator 100 provides a respective working zone 120 along the path. The body 10 of the soft robot 1 is configured to travel along the path through the individual working zones 120. Within each working zone 120, different sections of the body 10 interact with the magnetic field 5 generated by the magnetic field generator 100 placed at the respective working zone 120.

For example, for performing long-range operation of the embodiment shown in Fig. 123, the magnetic interaction length may be extended to 305 mm. Generating a uniform magnetic field 5 over a large enough area to accommodate a body 10 of that length could be extremely challenging. Therefore, a combination of two magnetic field generators 100 may be used. The magnetic field generators 100 may, for example, be configured as Halbach arrays.

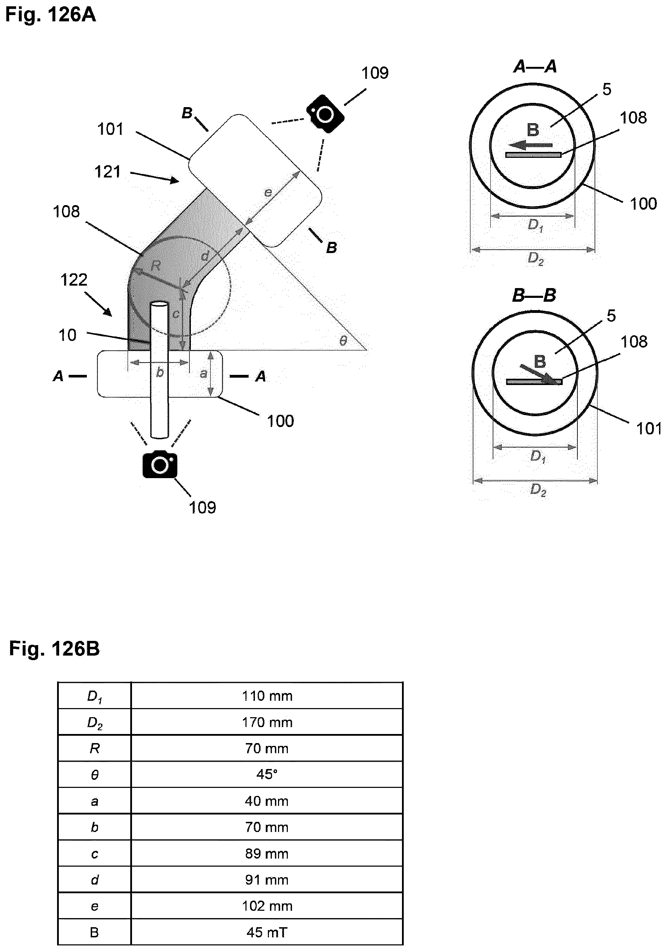

Fig. 126 details long-range operation with a large magnetic interaction length, preformed with a soft robot 1 according to the present disclosure. Fig. 126A comprises a schematic top view of an experimental platform and cross-sectional views within planes A-A and B-B. A magnetic field generator 100 is placed in plane A-A and a further magnetic field generator 101 is placed in plane B-B. Each magnetic field generator 100, 101 is exemplarily configured as a Halbach array. The magnetic field generators 100, 101 are arranged at an angle, such as 45°, creating two separate magnetic fields 5. A support 108, such as a support plate, is positioned in an intermediate region between the two magnetic field generators 100, 101 to provide support for the body 10. Cameras 109 are installed on the exterior sides of the magnetic field generators 100 to record the real-time operation of the body 10. The body 10 enters the setup at the magnetic field generator 100.

Fig. 126B lists exemplary parameter values of the setup in Fig. 126A.

The embodiment shown in Fig. 123 enables operation of the body 10 over a distance of 305 mm. Thereby, the body undergoes 2D bending in the magnetic field 5 of the magnetic field generator 100, whereby the magnetic field 5 interacts with the further magnetic units 62 of the outer part 20, and achieves multiple 2D or 3D bending deformations in the magnetic field 5 of the further magnetic field generator 101. Thereby, the magnetic field 5 of the further magnetic field generator 101 interacts with the magnetic element 50 of the inner part 30.

With the embodiment shown in Fig. 123, the first end 24 of the outer part 20 with the further magnetic units 62 may be located within the magnetic field generator 100 so that interaction of the magnetic field 5 of the magnetic field generator 100 with the further magnetic units 62 leads to a bending deformation of the outer part 20. The inner part 30 and the further inner part 40 may protrude from the first end 24 of the outer part 20 into the magnetic field 5 of the further magnetic field generator 101. The magnetic element 50 of the inner parts 20 then may interact with the magnetic field 5 of the further magnetic field generator 101 and perform a bending deformation within that magnetic field 5. The bending deformation of the outer part 20 may direct the inner part 30 and the further inner part 40 towards the second magnetic field generator 101. The outer part 20 thereby bends by the angle *θ*.

With the embodiment shown in Fig. 123, the inner part 30 and the further inner part 40 may first be moved through the magnetic field generator 100 with the magnetic field 5 of the magnetic field generator 100 set to zero. The first end 24 of the outer part 20 with the further magnetic units 62 may be placed within the magnetic interaction zone of the first magnetic field generator 100 and the first ends 34, 44 of the inner parts 30, 40 with the magnetic element 50 may protrude from the first end 24 of the outer part 20 and may be placed outside the magnetic interaction zone of the first magnetic field generator 100. The magnetic field 5 of the first magnetic field generator 100 then may be ramped up and the further magnetic units 62 may interact with the magnetic field 5 of the magnetic field generator 100, which causes a bending deformation of the outer part 20. This may direct the inner parts 30, 40 towards the magnetic interaction zone of the further magnetic field generator 101. The inner parts 30, 40 then may be further advanced into the magnetic interaction zone of the further magnetic field generator 101 while keeping the outer part 20 stationary. Within the magnetic interaction zone of the further magnetic field generator 101, the magnetic element 50 of the inner part 30 may interact with the magnetic field 5 of the further magnetic field generator 101 to cause a bending deformation of the inner part 30. Subsequently, the further inner part 40 may be further advanced while keeping the inner part 30 and the outer part 20 stationary.

When arranging multiple small-scale magnetic fields 5, mutual interference may occur, and the superposition of several fields 5 in certain areas may generate undesired magnetic fields at specific spatial locations. To address these issues, a detailed analysis and discussion using simulation methods is provided in section X. below in order to provide specific design guidance.

### 1.4.2 Multi-Bend Deformation Operation

In some applications, the body 10 may perform multiple bending deformations. The conventional approach is to design an appropriate magnetization profile to achieve these multiple bends. By designing various magnetization profiles, the foregoing demonstrated two planar bends as well as multiple spatial bends within a single magnetic field 5. However, as the number of bends increases, the complexity of the magnetization profile design also increases. To reduce the design complexity and facilitate the possibility of incorporating more bending deformations, a multi-bend deformation design method may be applied. The following sections explain the principles behind this multi-bend deformation design method and analyze specific design cases.

### I.4.2.1 Multi-Bend Deformation Design Method

Assume that the body 10 has a total length *L* and is bent into a certain arc. By dividing the body 10 along its arc into *n* segments, each segment *sᵢ* has a corresponding length *Lᵢ*. For the *i*^{th} segment, the magnetic moment is **m*ᵢ***. Suppose the body 10 is placed in a magnetic field of strength **B**; then, the curvature function for the *i*^{th} segment can be expressed as: ${κ}_{i} \left(s\right) = Γ \left({m}_{i}, B, χ\right)$ (S9-68)

Here, Γ represents a function that incorporates the effects of the magnetic field 5, magnetic moment, and the physical and geometric parameters of the body 10, while χ denotes the physical and geometric parameters of the body 10. Integrating the curvature with respect to the arc length yields the bending angle for that segment of the body 10, i.e., ${α}_{i} = {\int }_{0}^{{L}_{i}} {κ}_{i} \left(s\right) ds$ (S9-69)

At the same time, the rotation of the local coordinate system due to the bending is described by employing a rigid transformation. The local rigid transformation corresponding to the bending of the *i*^{th} segment is given by: ${R}_{i} = exp \left({α}_{i}{\hat{U}}_{i}\right)$ (S9-70) where *Ûᵢ* denotes the generator of rotation. Consequently, the final pose of the *m*^{th} body segment can be expressed as: ${R}_{m} = {R}_{m} {R}_{m - 1} ⋯ {R}_{1}$ (S9-71) ${α}_{m} = {\sum }_{i = 1}^{m} {α}_{i}$ (S9-72)

The final pose of the body 10 is determined by the rigid transformation *Rₘ* and the bending angle *αₘ*, both of which depend on the magnetic field 5, magnetic moment, and the physical and geometric parameters of the body 10 (Equation (S9-68)). When a body 10 with specific physical and geometric parameters is subjected to a magnetic field **B**, its deformation is dictated by the magnetization profile ${\left\{{m}_{i}\right\}}_{1}^{n}$. For complex deformations, such as those involving multiple bends, the magnetization profile becomes correspondingly more intricate.

With the multi-bend deformation design method, multiple bending deformations are decoupled for separate designs. In this approach, different magnetic fields 5 are used to control distinct regions of the body 10. Each region corresponds to a specific magnetization profile and a particular magnetic field 5, thereby achieving a defined bending deformation. By combining these individual bending deformations, the desired multi-bending deformation can be realized. The collection of magnetic moments **m*ᵢ*** is denoted as **m**(***s***). Under a single magnetic field 5, the corresponding curvature function of the body 10 is given by: $κ \left(s\right) = Γ \left(m\left(s\right),B,χ\right)$ (S9-73)

When multiple magnetic fields 5 are employed to control different regions, for clarity, the collection of magnetic moments (i.e., the magnetization profile) of the body 10 under the *j*^{th} magnetic field is denoted as: $m {\left(s\right)}_{j} = \left\{\left.{m}_{i}\right|i=a,a+1,⋯b\right\}$ (S9-74) where *a* and *b* denote the indices of the first and last magnetic moments, respectively, under the *j*^{th} magnetic field. The body 10 is partitioned into regions and multiple magnetic fields are employed, each corresponding to a specific magnetization profile. Consequently, the curvature function of the body 10 is given by: $κ \left(s\right) = \left\{\begin{matrix}Γ \left(m{\left(s\right)}_{1},{B}_{1},χ\right) , \\ Γ \left(m{\left(s\right)}_{2},{B}_{2},χ\right) , \\ ⋮ \\ Γ \left(m{\left(s\right)}_{k},{B}_{k},χ\right) ,\end{matrix} \begin{matrix}x ∈ \left.\left[0, {L}_{p}\right.\right) \\ x ∈ \left.\left[{L}_{p}, {L}_{q}\right.\right) \\ ⋮ \\ x ∈ \left[{L}_{l}, {L}_{n}\right]\end{matrix}\right.$ (S9-75) where **m**(***s***)***ₖ*** denotes the collection of magnetic moments (i.e., the magnetization profile) under the *k*^{th} magnetic field, while *p, q,* and *l* represent the indices of the segments of the body 10 in different regions.

By comparing equations (S9-73) and (S9-75), it can be seen that when multiple bends are required, the magnetization profile **m**(***s***) designed for a single magnetic field 5 is considerably more complex than the magnetization profiles **m**(***s***)***ₖ*** designed for multiple magnetic fields 5. Specifically, |**m**(***s***)***ₖ***| < |**m**(***s***)| and in some cases, **m**(***s***)***ₖ*** is even a subset of **m**(***s***). Furthermore, as the number of regions of the body 10 increases, the number of magnetic moments in **m**(***s***)***ₖ*** becomes increasingly smaller compared to that in **m**(***s***). Therefore, the complexity and design difficulty of **m**(***s***)***ₖ*** are significantly lower than those of **m**(***s***), indicating that the proposed multi-bend deformation design method greatly reduces the design complexity of the magnetization profile.

When applying the multi-bend deformation design method for magnetizing the body 10, the specific magnetization design procedure for a single bend can be found in section IX. below. Additionally, since this method requires multiple magnetic fields 5 to control different regions of the body 10, the magnetic field design scheme described in section I.4.1.3 can be utilized to arrange several magnetic fields 5 appropriately, thereby creating distinct magnetic field regions (see Fig. 125B). For detailed analysis of the magnetic field layout and interference, please refer to section X. below.

### 1.4.2.2 Design and Optimization Strategies

The magnetization design cases (presented in the foregoing in see Figs. 2 to 49 were all developed based on a single magnetic field 5, covering common modes of operation. For example, obstacle traversal in a plane using a single bend may be achieved (Figs. 59A and 65), obstacle traversal in a plane via two bends (Fig. 59C), and obstacle traversal in 3D space through multiple bends (Fig. 69 to 72). These strategies were implemented by designing various magnetization profiles. To achieve multiple bends with multiple magnetic fields 5, two Halbach arrays to generate two uniform magnetic fields 5 may be arranged at an angle, such as a 45° angle (detailed parameters are provided in Figs. 125 and 126). Thereby, two types of hollow members, formed by the outer part 20 and the further inner part 40, and one rod, formed by the inner part 30, are manufactured and after nesting them, the assembly is placed in the aforementioned magnetic fields 5 for operation. It was observed that, under the first magnetic field 5 of the magnetic field generator 100, the nested body 10 exhibits planar bending, while under the second magnetic field 5 of the further magnetic field generator 101, the body 10 could achieve either planar or spatial bending.

With the setup of Figs. 123, 124, 126, several shortcomings may become apparent due to the Halbach array's inability to adjust the magnetic field 5 in real time. First, the body 10 begins bending before it even enters the Halbach array. This premature bending results from the presence of residual magnetic fields around the periphery of the Halbach array, which cannot be eliminated and thus cause the body 10 to bend - an effect that is undesirable in practical applications. Second, the setup does not incorporate obstacles 402 to showcase the ability of the body 10 to traverse them. Because the Halbach array cannot modulate the magnetic field strength, it does not allow for the gradual increase in the magnetic field 5 - as observed with the magnetic field 5 generated by the VSM (Figs. 59A and 65) - that facilitates smooth transitions between different deformation states.

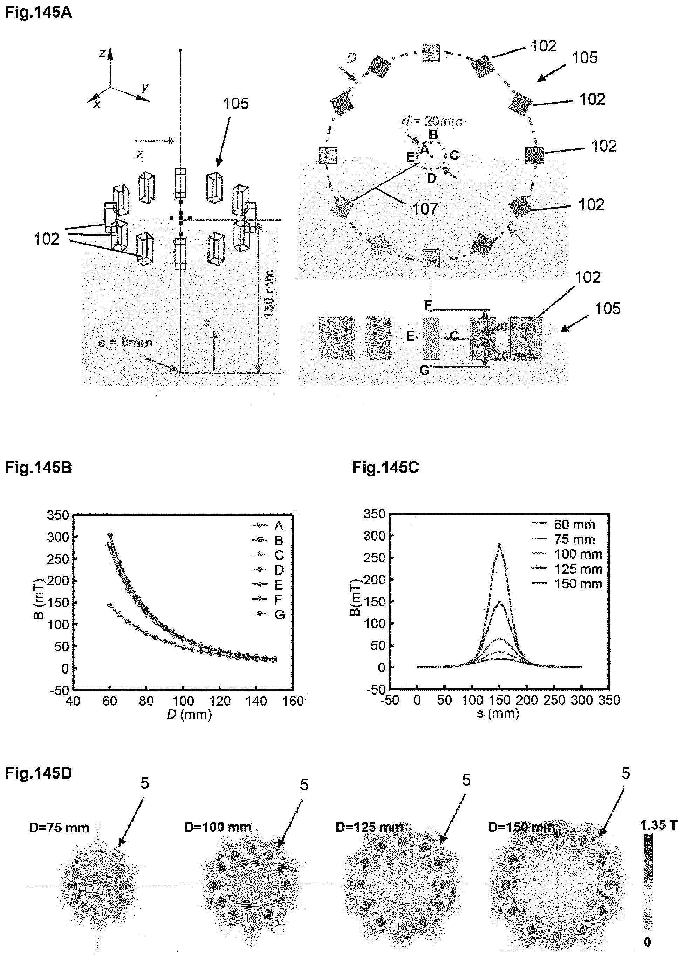

To address these shortcomings, the following methods may be implemented.
(1) The magnetic field generator 100 may be configured to adjust the diameter of a circular distribution of permanent magnets 102 in the Halbach array, thereby controlling the intensity of the magnetic field 5. By altering the diameter of the circular distribution, the strength of the magnetic field 5 can be modulated. Simulations, as shown in Fig. 145 (detailed analysis in section X. below), indicate that the uniform magnetic field 5 decreases with increasing distance or diameter, and when the circular distribution reaches a predefined diameter, such as a diameter of 150 mm, the magnetic field 5 is nearly zero. This device would enable real-time control of the magnitude of the magnetic field 5 by adjusting the diameter of the circular distribution of the permanent magnets 102, and the field direction could be controlled by rotating the Halbach array.

With the exemplary Halbach array of Fig. 145, the permanent magnets 102 form field sources for generating the magnetic field 5 and the circular distribution of the permanent magnets 102 constitutes a source arrangement 105.

In general, the magnetic field generator 100 may have other source arrangements 105 of field sources, such as elliptical or rectangular arrangements. Furthermore, the field sources may be configured different from permanent magnets 102, for example as magnetic field coils. The individual field sources may have respective distances to the magnetic interaction zone 130.

In general, the magnetic field generator 100 may comprise a source actuator, such as an electric motor, to vary the individual distances of the field sources to the magnetic interaction zone 130 and thereby alter the magnitude of the magnetic field 5.

(2) Instead of employing a Halbach array, the magnetic field 5 can be generated using electromagnetic coils. Similar to the approach with the VSM for control, coils can be designed to produce the desired magnetic field 5 in real time, enabling smooth and continuous adjustments to both its magnitude and direction. However, for applications involving larger workspaces or higher magnetic field magnitudes, the construction cost of such an electromagnetic system may be considerable.

In the multi-bend deformation design described in connection with Figs. 123, 124, 126, the magnetization profile in the first magnetic field 5 of the magnetic field generator 100 (Halbach array) employs the third configuration for 1D magnetization reprogramming, as shown in Figs. 13 to 15, while the magnetization profile in the second magnetic field 5 of the further magnetic field generator 101 is configured like the seventh configuration for 1D magnetization reprogramming (see Fig. 26). This proposed design method transforms the approach from relying on a single integrated magnetization profile for the entire body 10 to adopting a segmented, region-based magnetization profile design. The magnetization profile for each region can directly refer to the common operation paradigms provided, for example in Figs. 2 to 49. This multi-bend deformation design method not only reduces the complexity involved in designing magnetization profiles for intricate deformations but also allows for regional partitioning based on specific requirements, thereby enabling rapid implementation of various deformation designs by leveraging established paradigms.

In general, the body 10 may have a first magnetization profile in a first region and a second magnetization profile in a second region, whereby the first magnetization profile is different from the second magnetization profile. Furthermore, the first region may be configured to be placed in a first magnetic field and the second region may be configured to be placed in a second magnetic field, whereby the first magnetic field and the second magnetic field are independently controllable. With alternative embodiments, the first region and the second region may also be configured to be placed in the same magnetic field.

The first magnetization profile and/or the second magnetization profile may be selected from one of the first to seventh configurations shown in Figs. 2 to 49. The first magnetization profile and the second magnetization profile may be spaced apart from each other along the longitudinal direction 2. The first magnetization profile may be configured to change upon relative movement of a first magnetic member 16 and a first target member 15 of the body 10. Additionally or alternatively, the second magnetization profile may be configured to change upon relative movement of a second magnetic member 16 and a second target member 15 of the body 10.

The first magnetic member 16 may be identical to the second magnetic member 16 or different from the second magnetic member 16. Additionally or alternatively, the first target member 15 may be identical to the second target member 15 or different from the second target member 15.

The first magnetic member 16 and/or the second magnetic member 16 may be configured as it is disclosed in the foregoing for the magnetic member 16. Additionally alternatively, the first target member 15 and/or the second target member 15 may be configured as it is disclosed in the foregoing for the target member 15.

### I.4.3 High-Curvature Deformation Operation

Based on the analysis in section I.1.1 above, the relative motion resistance *Fₘ₋ₜₒₜₐₗ* of the outer and inner parts 20, 30, 40 is influenced by the curvature of the deformation of the outer and inner parts 20, 30, 40 under the magnetic field 5. As the deformation curvature of the outer and inner parts 20, 30, 40 gradually increases, once *Fₘ₋ₜₒₜₐₗ* exceeds the critical buckling load *F_{cr}* and yield force *F_{y}* of the outer and inner parts 20, 30, 40, it will no longer be possible to control the relative motion of the outer and inner parts 20, 30, 40. In sections I.1, I.2, and I.3 above, we presented various methods to increase *F_{cr}* and *F_{y}*, while reducing *Fₘ₋ₜₒₜₐₗ.* However, if the curvature of the deformation of the outer and inner parts 20, 30, 40 increases to a point where *Fₘ₋ₜₒₜₐₗ* surpasses a critical threshold - rendering any method incapable of satisfying the mechanical requirements for part motion control (as indicated by Equation (S9-16)) - then control over the relative motion of the outer and inner parts 20, 30, 40 may be lost.

For ease of exposition, the calculation of the relative motion resistance from section I.1.1 may be summarized using the following equation: ${F}_{m - total} = Ψ \left(κ, χ\right)$ (S9-76) where Ψ represents the mapping function that relates various parameters to the relative motion resistance *Fₘ₋ₜₒₜₐₗ, κ* denotes the curvature of the part 20, 30, 40, and χ is the part's physical and geometrical parameters. Equation (S9-68) may also be referenced as follows: $κ = Γ \left(m, B, χ\right)$ (S9-77)

In Equation (S9-77), the relationships among the parameters were analyzed in section I.1.1, indicating that the part's curvature is positively correlated with *Fₘ₋ₜₒₜₐₗ.* Furthermore, according to the analysis in section II. below, under general conditions, both the magnetic moment **m** and the magnetic field **B** are positively correlated with the curvature *κ*. Although there may be special cases - such as when switching between specific steady states - where the curvature is not positively correlated with the magnetic moment or magnetic field strength, the following focusses on the general scenario and does not address these exceptional cases.

As the curvature of the parts 20, 30, 40 increases, the relative motion resistance reaches a critical point beyond which the conditions for relative motion cannot be met. This critical point of the relative motion resistance is denoted as *F_{m-total-crit}.* Thus, the condition for controllability of the part is ${F}_{m - total} < {F}_{m - total - crit}$ (S9-78)

Let the curvature corresponding to the critical point be denoted as *κ_{crit}.* Since there is a positive correlation between curvature and relative motion resistance, the above inequality is equivalently expressed as $κ < {κ}_{crit}$ (S9-79)

During the process of achieving high-curvature deformations, when the relative motion of the parts 20, 30, 40 reaches its critical point and control is lost, the only option may be to reduce the part's relative motion resistance, and then restore control once the adjustment is complete. According to Equation (S9-76), such a reduction is accompanied by a change in the part's curvature - that is, the part's shape is altered. If this transient change in shape significantly affects the operation, then temporarily altering the part's curvature may not be a feasible solution. However, if the impact of the transient deformation is negligible or minor, then a temporary adjustment in curvature is acceptable, with the part 20, 30, 40 returning to its original shape once control is re-established. Based on Equation (S9-77), a transient reduction in the part's curvature can be achieved by modulating one of three factors: the magnetic moment, the magnetic field strength, or the part's physical and geometrical parameters. In the following sections, these approaches are discussed in detail.

### (1) Physical and geometric parameters of the part

Real-time modulation of the physical or geometric parameters of the body 10 can, to some extent, reduce its curvature. For instance, incorporating stimuli-responsive materials, such as photo-responsive or thermo-responsive materials or compounds, into the body 10 allows the elastic modulus to be adjusted in real time using an external stimulus like light or temperature. This adjustment enables control over the curvature of the body 10. Once the relative motion of the inner and outer parts 20, 30, 40 of the body 10 has terminated, the stimulus can be removed, allowing the body 10 to revert to its original curvature.

For example, at least one of the outer part 20 and the inner part 30, such as both the outer part 20 and the inner part 30 may comprise a material that is responsive to an external stimulus, such as the photo-responsive material or the thermo-responsive material.

Additionally, employing innovative designs to dynamically alter the geometric dimensions or structure of the body 10, thereby modifying the area moment of inertia, may also serve as a means to control curvature of the body 10.

### (2) Magnetization profile of the part

By reducing the effective magnetic moment of the body 10, the force or torque exerted by the magnetic field 5 is decreased, leading to a reduction in the curvature of the body 10. One approach to modify the effective magnetic moment is through the introduction of an auxiliary part, such as an auxiliary tube. For instance, if the relative motion resistance between part A, such as tube A, and part B, such as tube B, under large deformations exceeds the critical threshold - rendering effective magnetic moment adjustment via relative motion unfeasible - the motion of part C, such as tube C, can be utilized, for example to change the magnetization profile of the part A and/or the magnetization profile of the part B. The magnetic moment of part C can vectorially superimpose onto the magnetic moments of parts A and/or B, thereby lowering their effective magnetic moment and enabling the restoration of relative motion control. It should be noted, however, that the introduction of part C might interfere with other operations, which may be comprehensively considered during the design phase.

For example, part A may be a first one of the outer and inner parts 20, 30, 40, such as the outer part 20, and/or part B may be a second one of the outer and inner parts 20, 30, 40, such as the inner part 30, and/or part C may be a third one of the outer and inner parts 20, 30, 40, such as the further inner part 40.

### (3) External Magnetic Field

Reducing the external magnetic field 5 to decrease the forces or torques acting on the tube or body 10 is the simplest and most direct approach. In contrast to methods that alter the physical/geometric parameters of the tube or body 10 or modify its magnetization profile, which require more complex procedures or designs, this method is straightforward. However, it may also have certain limitations. For example, it may not be suitable in scenarios involving multiple instruments because adjusting the magnetic field 5 for one instrument may affect the performance of others. Therefore, an optimized design for multiple instruments is necessary to achieve a balance between multi-instrument operation and magnetic field reduction.

In summary, the three schemes for achieving high-curvature deformations essentially rely on temporarily reducing the deformation of the tube or body 10 to lower its relative motion resistance and the relative motion resistance of its inner and outer parts 20, 30, 40 during that time interval and thereby enable control of the relative motion before immediately restoring it to its original state. This approach assumes that such transient deformations have no adverse effect on other operations. Naturally, one may aim to avoid reaching the critical level of relative motion resistance in the designs. However, if this cannot be avoided, the methods provided above offer effective alternatives.

For example, the foregoing adopted the method of adjusting the external magnetic field 5. When using the seventh configuration for 1D magnetization reprogramming of Fig. 26 to achieve helical deformation, the tube or body 10 fabricated with Ecoflex (ECOFLEX^{™} 0050, KauPo, DE) exhibits low stiffness so that its critical buckling load is lower than the relative motion resistance between tubes or inner and outer parts 20, 30, 40. Consequently, during the control of the relative motion of the tube or inner and outer parts 20, 30, 40, the magnetic field strength may be directly reduced to 0 mT. In this manner, control of the relative motion of the tube or the inner and outer parts 20, 30, 40 is successfully achieved, whereafter the magnetic field 5 is restored. Further details, are shown in Figs. 26 to 29.

The foregoing is an example for a method that comprises setting a magnitude of the magnetic field 5 to a reduced value, such as zero, during the moving of the magnetic member 16 relative to the target member 15 and increasing the magnitude of the magnetic field 5 while the magnetic unit 52 is present in the magnetic field 5, such as after the moving of the magnetic member 16 relative to the target member 15.

### 1.4.4 Multi-Nested-Tube Operation

To reprogram the magnetization profiles, relative motion of the tubes or inner and outer parts 20, 30, 40 is used. When only a limited variety of magnetization profiles is required, a two-layer tube or part configuration may be sufficient. However, as the number of required deformation types increases, so does the variety of magnetization profiles, which in turn may necessitate an increase in the number of tubes or outer and inner parts 20, 30, 40.

Suppose there are n nested parts 20, 30, 40, which may be configured as tubes, where the *i*^{th} part has an elastic modulus *Eᵢ* and an area moment of inertia *Iᵢ*. The effective bending stiffness of the nested parts 20, 30, 40 is given by ${\left(EI\right)}_{e} = {\sum }_{i = 1}^{n} {E}_{i} {I}_{i}$ (S9-80)

Assuming the nested part 20, 30, 40 is subjected to a moment *M* in a magnetic field, the curvature produced according to Euler-Bernoulli beam theory is $κ = \frac{M}{{\sum }_{i = 1}^{n} {E}_{i} {I}_{i}}$ (S9-81)

Thus, when subjected to the same moment, the deformation of the part is directly influenced by its effective stiffness.

When altering the relative positions of the parts 20, 30, 40, the number of parts 20,30, 40 present in a given region may vary. At one moment, there might be only one part, while at another, the configuration changes such that the region contains n parts. For relatively small values of n, the effective stiffness of the nested parts changes only slightly as the positions vary.

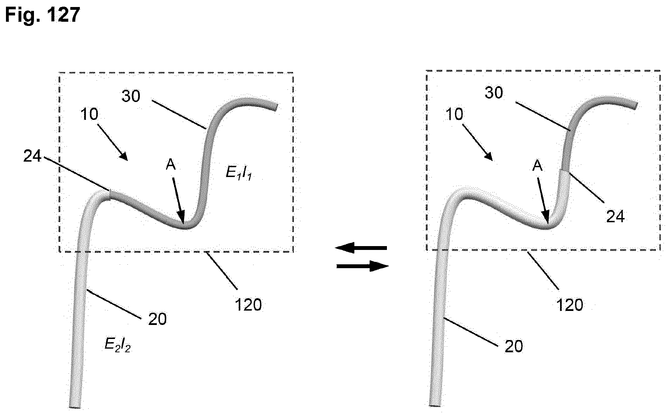

Fig. 127 illustrates stiffness variations in a multi-nested body 10, exemplarily having the outer part 20 and the inner part 30. Thereby, the inner part 30 protrudes from the free end 24 of the outer part 20. Initially, only the outer part 30 is present at a point A in a working zone 120. As the position of the outer part 20 changes with respect to the inner part 30, the body 10 comprises both the inner part 30 and the outer part 20 at point A, thereby altering the stiffness of the body 10 at point A. As the number of parts 20, 30, 40 increases, this stiffness variation becomes increasingly pronounced.

For instance, as illustrated in Fig. 127, a case of two nested parts 20, 30, such as two nested tubes, may be considered. Suppose that the relative motion of the two parts 20, 30 occurs entirely within the working zone 120. According to Equation (S9-80), at a bend at point A in Fig. 127, initially only a first part of the body 10, exemplarily the inner part 30, is present, so the effective stiffness of the body 10 is *E*₁*I*₁*.* As the first part and a second part of the body, exemplarily the outer part 20, undergo relative motion, the second part moves beyond point A, and the effective stiffness then becomes *E*₁*I*₁ *+ E*₂*I*₂*.* Similarly, when there are three tubes or parts, the effective stiffness changes from *E*₁*I*₁ to *E*₁*I*₁ *+ E*₂*I*₂ *+ E*₃I*₃*, i.e., ${\left(EI\right)}_{e} = \left\{\begin{matrix}{E}_{2} {I}_{2} , & 1 tube \\ {E}_{1} {I}_{1} + {E}_{2} {I}_{2} , & 2 tubes \\ {E}_{1} {I}_{1} + {E}_{2} {I}_{2} + {E}_{3} {I}_{3} , & 3 tubes\end{matrix}\right.$ (S9-82)

The two-layer and three-layer tube nesting configurations presented in this study can effectively achieve the required deformations. However, as the number of tubes n increases, abrupt changes in stiffness may occur in certain regions, which adversely affects the control of tube deformation.

Although Figs. 2 to 49 provide common configurations of the body 10 - including two-layer and three-layer bodies 10 - that can be used individually or in combination to satisfy typical operational requirements, the aforementioned design methods may not be adequate in scenarios requiring the nesting of a larger number of outer and inner parts, such as tubes. This issue may be addressed by a zone-based nesting design method as a feasible reference for multi-layer nesting designs.

The following discloses a zone-based nesting design method that mitigates the issue of excessive stiffness variation during operation of tubes or parts 20, 30, 40 by partitioning the nested tubes or parts into distinct working zones.

With the zone-based tube nesting design method, the body comprising the nested parts 20, 30, 40 is partitioned into zones so that each part 20, 30, 40 moves only within its designated zone. As a result, each zone contains a fixed number of parts 20, 30, 40, effectively reducing the overall stiffness variation.

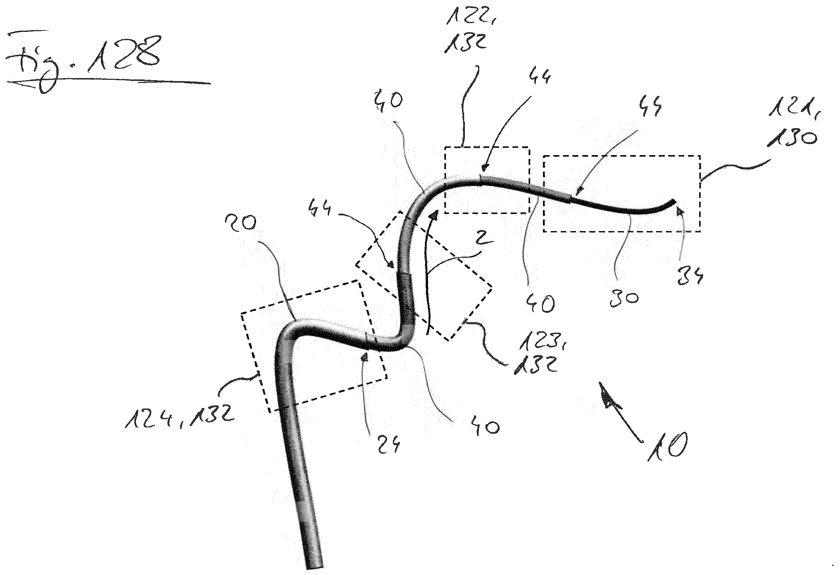

As illustrated in Fig. 128, the body 10 is divided or partitioned into several working zones 121, 122, 123, 124, so that each part 20, 30, 40, such as each free end 24, 34, 44 of the individual parts 20, 30, 40, only moves within a corresponding designated working zone 121, 122, 123, 124. As a result, each working zone 121, 122, 123, 124 contains a fixed number of parts 20, 30,40, effectively reducing the overall stiffness variation. Additionally, at each position in the longitudinal direction 2 along the body 10, a number of parts 20, 30, 40 of the body 10 present at the individual positions only changes by one within each working zone 121, 122, 123, 124 during operation of the soft robot 1.

Differently stated, the number of outer and inner parts 20, 30, 40, exemplarily formed by tubes, remains constant within each working zone 121, 122, 123, 124, and each one of the outer and inner parts 10, 30, 40 moves exclusively within its designated working zone 121, 122, 123, 124 during operation of the soft robot 1.

For example, in Fig. 128, a first working zone 120 comprises only the inner part 30 and a first further inner part 40. A second working zone 122 contains the inner part 30, the first further inner part 40, and a second further inner part 40. A third working zone 123 includes the inner part 30, the first further inner part 40, the second further inner part 40, and a third further inner part 40. A fourth working zone 124 includes the inner part 30, the first further inner part 40, the second further inner part 40, the third further inner part 40, and the outer part 20. With other embodiments, additional further inner part 40 may be nested in between the third further inner part 40 and the outer part 20.

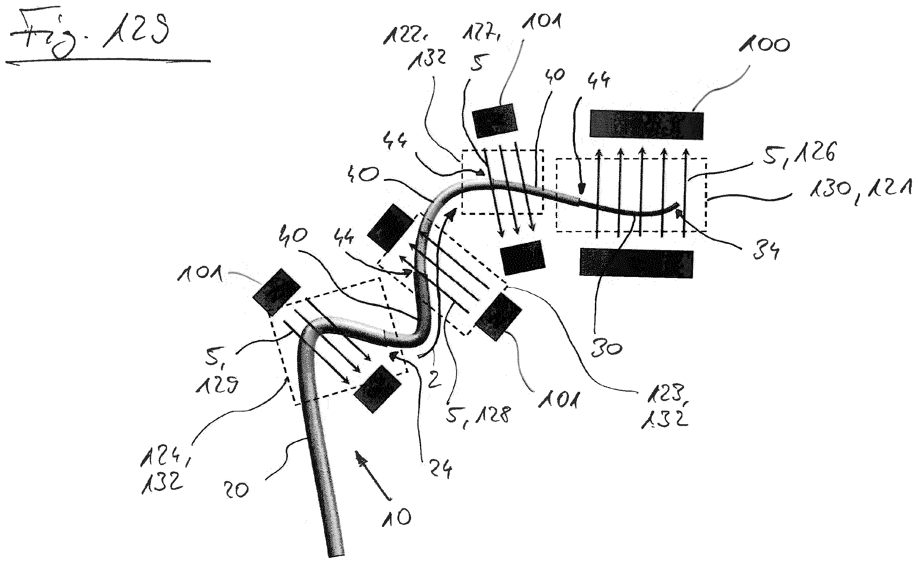

As shown in Fig. 129, the first working zone 121 comprises only the free end 44 of the first further inner part 40 and none of the free ends 24, 44 of the outer part 20 and of the remaining second and third further inner parts 40. Furthermore, the second working zone 122 comprises only the free end 44 of the second further inner part 40 and none of the free ends 24, 44 of the outer part 20 and of the remaining first and third further inner parts 40. The third working zone 123 comprises only the free end 44 of the third inner part 40 and none of the free ends 24, 44 of the outer part 20 and of the first and second further inner parts 40. Finally, the fourth working zone 124 comprises only the free end 24 of the outer part 20 and none of the free ends 44 of the first, second and third further inner parts 40. During operation of the soft robot 1 comprising the body 10, each free end 24, 44 remains within its dedicated working zone 121, 122, 123, 124.

With the layout of the body 1, each working zone 121, 122, 123, 124 only comprise a fixed number of the outer and inner parts 20, 30, 40 during operation of the soft robot 1.

Assuming that the first working zone 121, located at the distal end (i.e., far from the operational end at which the actuator 140 may be located), operates with two parts 30, 40, and that a new zone is added for each additional part 20, 40, then, due to the nested arrangement, the *i*^{th} working zone will comprise *i+1* parts 20, 30, 40. If the stiffness of the *i*^{th} part is denoted by *EᵢIᵢ,* then the maximum effective stiffness of the parts 20, 30, 40 in the *k*^{th} zone is given by ${\left(EI\right)}_{e}^{k} = {\sum }_{i = 1}^{k + 1} {E}_{i} {I}_{i}$ (S9-83)

Thus, in the *k*^{th} zone, since only the movement of the *(k+1)*^{th} part 20, 30, 40 alters the overall stiffness, the maximum stiffness variation in this zone is given by ${\left(EI\right)}_{c - max}^{k} = {E}_{k + 1} {I}_{k + 1}$ (S9-84)

As shown in Equation (S9-84), with the proposed zone-based nesting method, regardless of how many parts 20, 30, 40 are nested, the stiffness variation in the *k*^{th} working zone is solely determined by the stiffness of the *(k+1)*^{th} part 20, 30, 40. This single-part-induced stiffness change can be effectively controlled. In contrast, when the zone-based nesting design is not employed and all parts 20, 30, 40 operate their ends 24, 44 within a single working zone, the maximum stiffness variation in the *k*^{th} working zone is given by ${\left(EI\right)}_{c - max}^{k} = {\sum }_{i = 2}^{k + 1} {E}_{i} {I}_{i}$ (S9-85)

Comparing Equations (S9-84) and (S9-85) reveals that the method dramatically reduces the stiffness variation by an amount of ${\sum }_{i = 2}^{k} {E}_{i} {I}_{i}$. When only a small number of parts 20, 30, 40 are nested, the maximum stiffness variation differs only slightly between using and not using the proposed method. For example, in a two-tube configuration, both approaches yield the same stiffness variation. In a three-tube configuration, the proposed method results in a maximum stiffness variation of either *E*₂*I*₂ or *E*₃*I*₃ (depending on which is greater), whereas without the proposed method, the maximum stiffness variation is *E*₂*I*₂ *+ E*₃*I*₃. As the number of parts 20, 30, 40 increases, the difference in stiffness changes becomes more pronounced, demonstrating that the zone-based nesting design method can effectively mitigate this issue.

Moreover, as the number of nested parts 20, 30, 40 increases, corresponding adjustments to the magnetization profiles and magnetic fields 5 may become necessary for deformation control. The following discusses these modifications separately in terms of single-magnetic field control and multi-magnetic field control.

### (1) Single-Magnetic Field Control

When employing single-magnetic field control, all parts 20, 30, 40 operate under the same magnetic field 5, meaning that every nested part 20, 30, 40 is subjected to the same magnetic field strength.

With such an embodiment, each working zone 121, 122, 123, 124 constitutes a magnetic interaction zone, wherein the first working zone 121 forms the magnetic interaction zone 130 and the second, third and fourth working zones 122, 103, 124 each form a further magnetic interaction zone 132.

Since the effective stiffness of the body 10 varies across different working zones 120, 121, 122, 123, 124 and progressively increases with the number of parts 20, 30, 40 present in the respective working zone 120, 121, 122, 123, 124, the design of the magnetization profiles may likewise be adjusted so that the body 10 within each working zone 120, 121, 122, 123, 124 receives sufficient force or moment to achieve the desired deformation. Suppose the magnetic moment of the magnetic unit in the *j*^{th} zone is denoted as m(j) and then we have $m \left(j\right) = \left\{\begin{matrix}{m}_{1} , & j = 1 \\ {m}_{1} , & j = 2 \\ ⋮ & ⋮ \\ {m}_{n} , & j = n\end{matrix}\right.$ (S9-86) ${m}_{1} < {m}_{2} < ⋯ < {m}_{n}$ (S9-87) $m \left(j\right) = ℏ \left({E}_{j}{I}_{j},γ\right)$ (S9-88) where ℏ represents the mapping function from the effective stiffness (and other related parameters) of the body 10 to the magnetic moment, while *γ* denotes the dimensions and other related parameters of the body 10. Since the effective stiffness of the body 10 gradually increases with the number of nested parts 20, 30, 40, the design method described above can be employed to ensure that the magnetic moment also increases progressively with the number of parts 20, 30, 40 present within each working zone 120, 121, 122, 123, 124, thereby mitigating the significant stiffness variations induced by multi-part nesting, such as multi-tube nesting.

### (2) Multi-Magnetic Field Control

Section I.4.1.3 above discloses a multi-magnetic field control method that can be integrated with the zone-based nesting method. In this approach, a separate magnetic field 5 is applied to each working zone 120, 121, 122, 123, 124, or to every few working zones 120, 121, 122, 123, 124, such as to at least two different subsets of the working zones 120, 121, 122, 123, 124, as illustrated in Fig. 129. For instance, a first magnetic field 126 may be used to control the first working zone 121 or the first and second working zone 121, 122.

Fig. 129 depicts a combination of the zone-based nesting design method of Fig. 128 and the multi-magnetic field control method. By assigning distinct magnetic fields 126, 127, 128, 129 to different working zones 121, 122, 123, 124, this integrated approach facilitates the design of both the magnetization profile of the body 10 and the design of the external magnetic fields 126, 127, 128, 129, thereby allowing for a desired deformation.

With the embodiment shown in Fig. 129, the first working zone 121 comprises a first magnetic field 126, the second working zone 122 comprises a second magnetic field 127, the third working zone 123 comprises a third magnetic field 128 and the fourth working zone 124 comprises a fourth magnetic field 129. At least two of the magnetic fields 126, 127, 128, 129 for example each of the magnetic fields 126, 127, 128, 129 may differ among each other.

Suppose that each working zone 120, 121, 122, 123, 124 is controlled by a dedicated magnetic field 126, 127, 128, 129, with the magnetic field strength in the *j*^{th} zone denoted as *B*(*j),* then $B \left(j\right) = \left\{\begin{matrix}{B}_{1} , & j = 1 \\ {B}_{1} , & j = 2 \\ ⋮ & ⋮ \\ {B}_{n} , & j = n\end{matrix}\right.$ (S9-89) ${B}_{1} < {B}_{2} < ⋯ < {B}_{n}$ (S9-90) $B \left(j\right) = h \left({E}_{j}{I}_{j},γ\right)$ (S9-91)

Here, *h* represents the mapping function from the effective stiffness (and other related parameters) of the body 10 to the magnetic field strength. By adopting the aforementioned design - where the magnetic field strength increases gradually - it may be ensured that sufficient force or moment is generated in working zones 120, 121, 122, 123, 124 with a larger number of parts 20, 30, 40 to induce the desired deformation. In the above approach, each magnetic field 126, 127, 128, 129 is assigned to one working zone 120, 121, 122, 123, 124. However, in other embodiments, one magnetic field 126, 127, 128, 129 may be used to control two or more working zones 120, 121, 122, 123, 124, such not more than three, depending on the specific design requirements.

The single-magnetic field and multi-magnetic field control strategies address the design from the perspectives of magnetic moment and magnetic field strength, respectively, thereby ensuring that the forces/moments acting on the parts 20, 30, 40 in different zones vary appropriately. These two methods can be employed individually or in combination, with the choice of adjusting the magnetic moment, the magnetic field 126, 127, 128, 129, or both simultaneously determined by the particular application to achieve optimal control performance.

An embodiment demonstrating the feasibility of the zone-based nesting design integrates it with the multi-magnetic field control method described in section I.4.1.3 above. The specific design and experimental setup are those of Figs. 123, 124, 126. The system is divided into two working zones, a first working zone 121 and a second working zone 122, see Fig. 126A. In the first working zone 121, the inner part 30 and the further inner part 40 shown in Fig. 123 are present, while in the second working zone 122, the outer part 20, the inner part 30 and the further inner part 40 are located. These two working zones 121, 122 correspond to the two magnetic field regions generated by the magnetic field generators 100, 101, exemplarily formed by the Halbach arrays, as shown in Fig. 126A, which in turn relate to the multi-bend deformation operation described in section I.4.2. In the magnetic field generator 100 closer to the operational end with the actuator 140 (not shown), only the outer part 20 is magnetized, so that under the resulting forces it drives the embedded inner part 30 and further inner part 40 to deform. In the further magnetic field generator 101 farther from the operational end, the outer part 20 is absent, allowing the further inner part 40 and the inner part 30 to move relative to each other and achieve the desired deformation. Since this design combines several methods, additional discussions can be found in section I.4.2.2.

Furthermore, the free end 44 of the further inner part 40 is located within the first working zone 121 and the free end 24 of the outer part 20 is located within the second working zone 122. During operation of the soft robot 1 comprising the body 10 shown in Fig. 123, the free end 44 of the further inner part 40 remains within the first working zone 121 and the free end 24 of the outer part 20 remains within the second working zone 122.

### II. Modelling and mechanical analysis of soft tube deformation

The deformation of the soft tubes forming the parts 20, 30, 40 of the bodies 10, 11 in the magnetic fields 5 may be analyzed with a pseudo-rigid body method. In this approach, the soft tube or body 10 is represented as a series of rigid rods forming segments linked by hinges. The segments remain undeformed under external forces while the hinges undergo rotational motion. The elastic potential energy resulting from the deformation of the soft tube is assumed to be stored in these deforming hinges. As the soft tube deforms, the angles between the rigid rods change. By subdividing these segments, a curve composed of segments at varying angles can be constructed, thereby approximating the deformation of the soft tube. The present disclosure applies the energy method, specifically the principle of minimum potential energy, to approximate the deformation of the soft tube.

To simplify and accurately analyze the deformation of soft tubes, the present disclosure opted for suspending the tubes. It's noteworthy that similar force analysis processes apply to other placement methods, requiring only adjustments in the direction of the applied forces or the addition of corresponding forces. When a magnetic field 5 is applied to the soft tube, it is subjected to both gravity and the magnetic field 5. The total potential energy of the system is composed of magnetic potential energy, elastic potential energy, and gravitational potential energy. The total potential energy is expressed as: $V = {U}_{m} + {U}_{g} + {U}_{e}$ (S1-1) where *Uₘ* represents the magnetic potential energy of the system, *U_{g}* represents the gravitational potential energy of the system, and *Uₑ* represents the elastic potential energy of the system.

### II.1 Magnetic potential energy

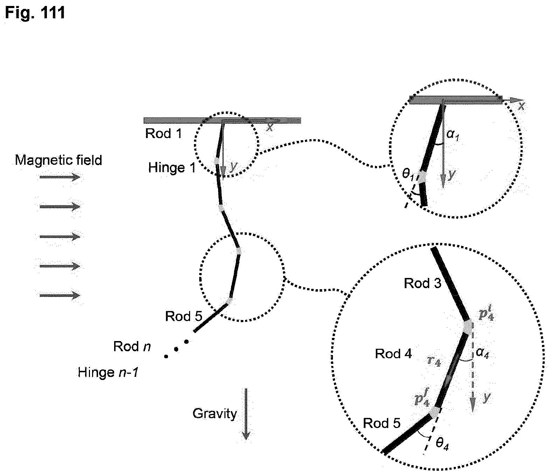

With the present disclosure, the soft tube is modeled as a structure comprised of *n* rigid rods connected by *n-1* hinges, see Fig. 111, with *m* of these rods possessing magnetic properties.

Fig. 111 thereby depicts an analysis diagram of soft tube/rod modeling. Based on the pseudo-rigid body model, the soft tube is equivalently represented as a series of connected rods and hinges. This model is vertically suspended, with its top end fixed, and is subjected to the combined effects of a magnetic field and gravity.

Within a magnetic field 5, the magnetized rigid rods experience magnetic forces, and the total magnetic potential energy of these m magnetic rods can be represented as: ${U}_{m} = - {\sum }_{j = 1}^{m} {V}_{mj}$ (S1-2)

Here, *Vₘⱼ* represents the magnetic potential energy of a single rod, which can be calculated using the following formula: ${V}_{mj} = {m}_{j} ⋅ {B}_{j}$ (S1-3) where **m*ⱼ*** denotes the magnetic moment of the *j^{th}* rod and **B*ⱼ*** represents the magnetic field experienced by the *j^{th}* rod. The magnetic moment of the *j^{th}* rod can be obtained by ${m}_{j} = {m}_{j} {r}_{j}$ (S1-4) where *mⱼ* represents the magnitude of the magnetic moment of the *j^{th}* rod and **r*ⱼ*** is the unit direction vector of the *j^{th}* rod.

As shown in Fig. 111, the present disclosure defines the horizontal rightward direction as the positive *x*-axis and the vertical downward direction as the positive *y*-axis. Assume that the angle between the *j^{th}* rod and the positive direction of the *x*-axis is *αᵢ*, and the angle between the *i+1^{th}* rod and the *i^{th}* rod is *θᵢ*. Since the deformation of the soft tube is considered to occur within the same plane, it follows that: ${θ}_{i} = {α}_{i + 1} - {α}_{i}$ (S1-5)

In the computation process, the angle *α*₁ of the first rod with the *x*-axis can be set to an initial value (for instance, 90 degrees when the soft tube is vertically suspended), then: ${α}_{i} = {α}_{1} + {\sum }_{n = 1}^{i - 1} {θ}_{n}$ (S1-6)

The unit direction vector of each rod can be represented as: ${r}_{i} = \left(cos {α}_{i},sin {α}_{i}\right)$ (S1-7)

Therefore, the total magnetic potential energy of the system can be expressed as: ${U}_{m} = - {\sum }_{j = 1}^{m} {m}_{j} {r}_{j} ⋅ {B}_{j}$ (S1-8)

### 11.2 Elastic potential energy

Since the soft tube is modeled as a combination of rigid rods and hinges, the elastic potential energy of the system is stored only in the hinges. The elastic potential energy in a hinge depends on the angle between adjacent rods, and the energy of a single hinge can be expressed as: ${V}_{e} = \frac{1}{2} {Kθ}_{i}^{2}$ (S1-9) where *K* represents the equivalent rotational stiffness of the hinge and its magnitude is given by: $K = \frac{{E}_{i} {I}_{i}}{{L}_{R}}$ (S1-10)

Here, *Eᵢ* represents the modulus of elasticity of the *i^{th}* rod;
*Iᵢ* is the moment of inertia of the cross-section of the *i^{th}* rod;
*L_{R}* is the length of the *i^{th}* rod.

Since the soft tube is evenly divided into n rigid rods, *L_{R}* can be obtained by: ${L}_{R} = \frac{L}{n}$ (S1-11)

Thus, the total elastic potential energy of the system can be calculated by: ${U}_{e} = \frac{2 n}{L} {\sum }_{i = 1}^{n} {E}_{i} {I}_{i} {θ}_{i}^{2}$ (S1-12) where *E_{í}* represents the modulus of elasticity of the *i^{th}* rod, *Iᵢ* is the moment of inertia of the cross-section of the *i^{th}* rod, and *θᵢ* is the angle between the *(i+1)^{th}* rod and the *i^{th}* rod.

### 11.3 Gravitational potential energy

To facilitate calculations, this study assigns corresponding vectors to each rod. The initial vector of the *j^{th}* rod is denoted as ${p}_{i}^{i}$, and upon the deformation of the soft tube under the influence of external forces, the final vector of the rod becomes ${p}_{i}^{f}$. Assuming that the rigid rods in the pseudo-rigid body model have a uniformly distributed mass, the center of gravity of each rod coincides with its geometric center. Assuming the mass of the *i^{lh}* rod is ${m}_{i}^{m}$, the gravitational potential energy of the system can be represented as: ${U}_{g} = - \frac{1}{2} {\sum }_{i = 1}^{n} {m}_{i}^{m} \left({p}_{i}^{f}-{p}_{i}^{i}\right) ⋅ g$ (S1-13) where g is the acceleration due to gravity.

Since the initial position of the soft tube is vertically suspended, the initial position vectors of all the rods are: ${p}_{i}^{i} = \left(0, {L}_{R}\right)$ (S1-14)

The final vectors of all rods are ${p}_{i}^{f} = {L}_{R} {r}_{i}$ (S1-15)

Assuming the mass of the *i^{th}* rod is ${m}_{i}^{m} ,$, the gravitational potential energy of the system can be represented as: ${U}_{g} = - \frac{1}{2} {\sum }_{i = 1}^{n} {m}_{i}^{m} \left({p}_{i}^{f}-{p}_{i}^{i}\right) ⋅ g$ (S1-16) where g is the acceleration due to gravity.

Through the aforementioned analysis, the magnetic potential energy, elastic potential energy, and gravitational potential energy is derived. According to Equation (1) and utilizing the principle of minimum potential energy, the static equilibrium configuration may be determined by finding the local minima of the potential energy. This allows to obtain the angles between the rods, denoted as *θᵢ*. The potential energy equation can be solved using the fmincon function in MATLAB (R2022a, The MathWorks, Inc., USA), which is a solver for finding the minimum of a constrained nonlinear multivariable function.

### 11.4 Parameter calculation

To measure the deformation of the soft tube, two parameters may be selected: the terminal bending angle 711 and the minimum radius of curvature 710 in the bending area, as described in Fig. 110, which depicts schematic diagrams of quantification of soft tube deformation.

Fig. 110A thereby shows a schematic image of the parameters. Based on the deformed contour of the soft tube forming the parts 20, 30, 40 of the body 10, a characteristic curve (indicated in red in the diagram) may be identified and the radius of curvature 710, *rᵢ,* and the terminal bending angle 711, *θ*, with respect to the *y-*axis forming the longitudinal direction 2 may be calculated. As shown in Fig. 110B, MATLAB may be used to obtain the characteristic curve and fit the radius of curvature of the characteristic curve. Circles may be utilized to approximate the characteristic curve (depicted as green circles and the red dash line in the diagram), selecting the smallest radius from among these circles as the radius of curvature 710 for the characteristic curve. This simplified approach, which involves the radius of curvature 710 and the terminal bending angle 711, provides a concise description of the deformation extent of the soft tube.

If there are multiple bending areas, then there will be multiple minimum radii of curvature.

Based on the solved data *θᵢ*, the terminal bending angle of the soft tube (i.e., the *n^{th}* rod) can be obtained through the following calculation: ${α}_{n} = {α}_{1} + {\sum }_{i = 1}^{n} {θ}_{i}$ (S1-17)

To obtain the minimum radius of curvature, first, using the solved data *θᵢ*, the initial angle *α*₁ of the first rod, and the length of the rod *L_{R}*, calculate the coordinates of both ends of the rod as well as the midpoint of the rod. Let the coordinates of the end of the *i*^{th} rod be (*xᵢ, yᵢ*), which can be obtained through the following calculation: $\left\{\begin{matrix}{x}_{i} = {x}_{i - 1} + {L}_{R} cos {α}_{i} \\ {y}_{i} = {y}_{i - 1} + {L}_{R} sin {α}_{i} \\ {α}_{i} = {α}_{1} + {\sum }_{n = 1}^{i - 1} {θ}_{n}\end{matrix}\right.$ (S1-18)

Let the geometric midpoint coordinates of the ith rod be (*x_{i,mid}, y_{i,míd}*), which can be obtained through the following calculation: $\left\{\begin{matrix}{x}_{i , mid} = {x}_{i - 1} + \frac{1}{2} {L}_{R} cos {α}_{i} \\ {y}_{i , mid} = {y}_{i - 1} + \frac{1}{2} {L}_{R} sin {α}_{i}\end{matrix}\right.$ (S1-19)

Using the method of least squares, these discrete coordinates are fitted into a curve. By dividing the fitted curve into segments and fitting these segments into circles, the radius R of the circles can be obtained. The radius of these circles represents the radius of curvature (the same method used to calculate experimental data in Fig. 110). By finding the minimum value within a bending area, the minimum radius of curvature in that area is obtained: ${R}_{min} = min \left(R\right)$ (S1-20)

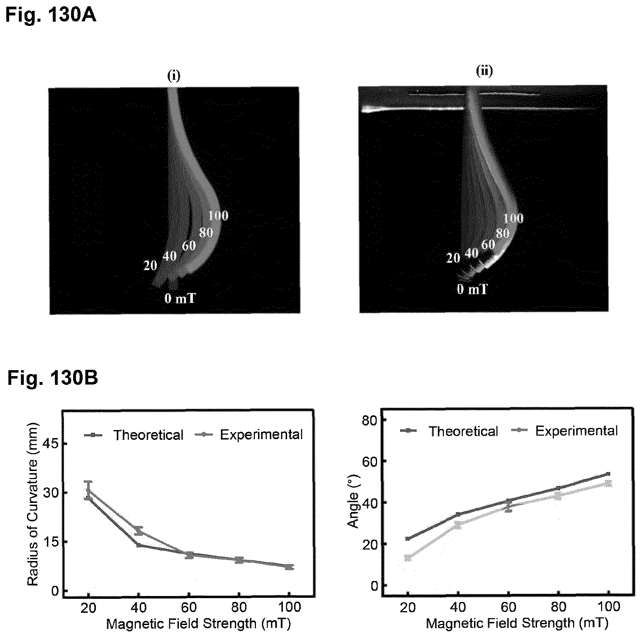

Based on the solved data *θᵢ*, the terminal bending angle 711 and minimum radius of curvature 710 may be obtained through MATLAB. More details regarding the modeling and calculations are provided below, and the theoretical results obtained from the above calculations are presented in Fig. 130, where they are compared with the experimental results.

Fig. 130 thereby depicts a comparison of theoretical and experimental values for the deformation of soft tubes in 1D magnetization reprogramming. Fig. 130A presents the scenario of the second configuration for 1D magnetization reprogramming shown in Fig. 12A with the distance 51 set to d_{R12} = 12mm under a uniform magnetic field increasing from 0 mT to 100 mT in increments of 20 mT, with the theoretical results shown at (i) and the experimental results shown at (ii). Fig. 130B presents theoretical and experimental values of soft tube deformation characterized using the two representative parameters radius of curvature 710 and terminal bending angle 711, as defined in Fig. 110.

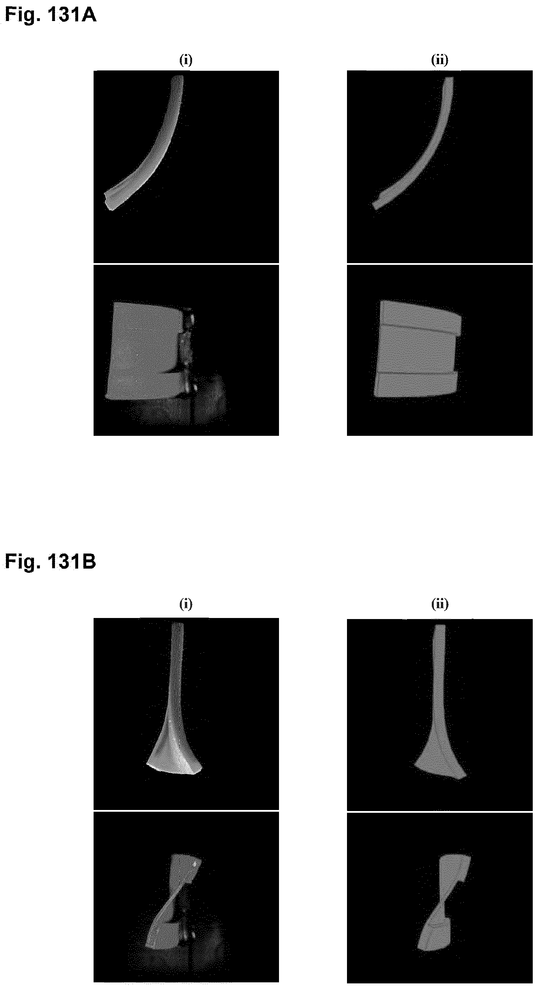

In the deformation analysis of 2D sheets 160 or 3D bodies 180, the aforementioned method of calculations using theoretical formulas becomes excessively complex. Alternatively, the present disclosure employs a finite element simulation using ABAQUS, based on the method provided in (18). According to the present disclosure, the simulation results for the 2D sheets may be obtained and compared with experimental results as detailed in Fig. 131, which shows a comparison of experimental and simulation results of the second configuration for 2D magnetization reprogramming shown in Figs. 34, 35, 51. The panels display the deformation of sheets 160 with two different configurations from Fig. 51 under a magnetic field of 20 mT directed to the right, whereby experimental results are shown at (i) and theoretical results are shown at (ii). The panels in the first row represent the front views, while those in the second row are the top views. Parameters in Fig. 131A are s1 =60, s2 =0, s3 =0, s4 =60 and parameters in Fig. 131B are s1 =0, s2 =60, s3 =0, s4 =60.

### III. Experimental Analysis of Soft Tube Deformation

The deformation of the soft tubes forming the outer and inner parts 20, 30, 40 is influenced by various factors, including the magnetic moments of the magnetic units, the number of magnetic units, the magnetic field, the elastic modulus, and the moment of inertia. The theoretical calculations for soft tube deformation are detailed in section II, where theoretical values are compared with actual experimental data (Fig. 130).

To more vividly illustrate the influence of various parameters on soft tube deformation, we conducted experiments and presented the effects from an experimental perspective. This section provides more intuitive references and specific design data for soft tube design.

The forces or torques acting on a soft tube in a magnetic field are determined by the magnetic moment and the magnetic field. For instance, the torque on the tube can be calculated as follows: $τ = m \times B$ (S2-1) where m represents the magnetic moment, and B denotes the magnetic field. The specific magnitude of the torque $\left|τ\right| = \left|m\right| \left|B\right| sin θ$ (S2-2) where *θ* is the angle between the magnetic moment vector and the magnetic field vector. We can understand that when the directions of the magnetic moment and the magnetic field remain constant, the magnitudes of the magnetic moment and the magnetic field strength have equivalent effects on the torque produced; they influence the magnitude of the magnetic moment in the same way. Similarly, the forces experienced by a soft tube in a gradient magnetic field follow a comparable trend. Therefore, when considering the effects of the magnetic moment and magnetic field strength on tube deformation, since both contribute to the generation of force or torque and exhibit similar trends in their effects, it is sufficient to consider the impact of either the magnetic moment or the magnetic field strength alone. Considering the advantages of creating and adjusting magnetic fields 5 with precision, the same magnetic units 52, 62 may be used in the manufacture of the outer and inner parts 20, 30, 40, exemplarily formed by the soft tubes, controlling variables by adjusting the magnitude of the magnetic field 5.

Based on the analysis of the effects of magnetic moment and magnetic field, five different magnetic field strengths at 30 mT, 60 mT, 90 mT, 120 mT, and 150 mT are set. For the distribution of magnetic units, the most common configuration of magnetic units discussed in this paper (two magnetic units) is used, setting the distance 51 between magnetic units 52, 62 at 6mm, 12mm, 18mm, and 24mm. To assess the impact of the elastic modulus, different materials were used to fabricate the soft tubes. Polydimethylsiloxane (PDMS) (Sylgard 184, Dow Corning, US) is used as the manufacturing material and varied the base-to-curing agent mass ratio at 3:1, 5:1, 10:1, and 15:1 to achieve tubes with different elastic moduli. For the influence of the moment of inertia, as the tubes involved in this study possess similar cross-sectional structures and the parameters do not vary significantly, the impact on deformation is relatively minor. Therefore, we chose not to conduct experiments on the effect of the moment of inertia on deformation for the time being. Following the analyses above, different soft tubes have been fabricated using the third configuration for 1D magnetization reprogramming, as outlined in Fig. 13. Specific parameters can be found in Fig.132, which depicts an experimental setup for analysis of soft tube deformation. Fig. 132A shows a configuration of the soft tube 10 used, Fig. 132B lists manufacturing and experimental parameters for the soft tube involve, including a material 822 and the magnitude of the magnetic field 5, and Fig. 132C is a diagram illustrating the placement of the soft tube 10 on a plate 820 relative to the direction of the applied magnetic field 5.

In the experiments, the soft tubes 10 were placed flat on the plate 820, and the magnetic field 5 was applied in a direction parallel to the plate and perpendicular to the tube's axis, as illustrated in Fig. 132C.

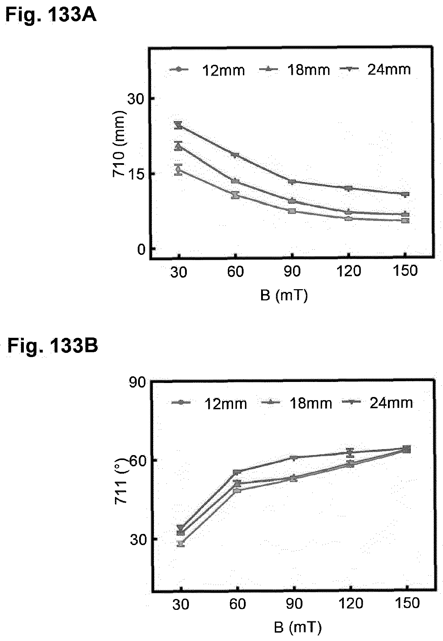

In experiments on magnetic field effects, we used tubes made from PDMS with a base-to-curing agent mass ratio of 10:1 and selected magnetic unit distances 51 of 12mm, 18mm, and 24mm. We applied uniform magnetic fields 5 of five different strengths: B = 30 mT, 60 mT, 90 mT, 120 mT, and 150 mT. For each parameter configuration, the experiment was repeated three times, and the deformation of the soft tube was recorded for each trial. We also used the radius of curvature 710 and terminal bending angle 711 (definitions provided in Fig. 110) to describe the deformation of the soft tube, with specific data shown in Fig. 133, which depicts the impact of magnetic field strength on the deformation of soft tubes with Fig. 133A showing the radius of curvature 710 and Fig. 133B showing the terminal bending angle 711. The radius of curvature 710 and terminal bending angle 711 are used to describe the deformation of soft tubes.

We observed that as the magnetic field strength B increases, the radius of curvature 710 of the soft tube with the same magnetic unit distance 51 gradually decreases, while the terminal bending angle 711 progressively increases. This indicates that the deformation of the soft tube increases with the intensity of the magnetic field 5. Soft tubes with different magnetic unit distances also exhibit the same trend.

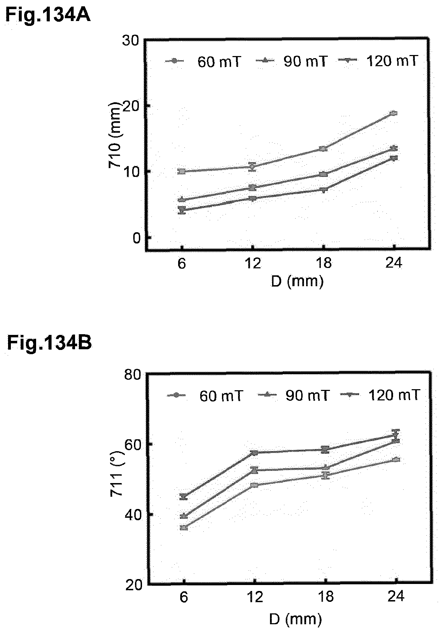

In experiments on magnetic unit distance effects, we also employed soft tubes made from PDMS with a base-to-curing agent mass ratio of 10:1, featuring magnetic unit distances 51 of 6mm, 12mm, 18mm, and 24mm. We observed the deformation of these soft tubes under magnetic field strengths of B = 60 mT, 90 mT, and 120 mT, with each parameter configuration being tested three times. The radius of curvature 710 and terminal bending angle 711 of the soft tube are documented in Fig. 134, which shows the impact of magnetic unit distance 51, denoted as D, on the deformation of soft tubes. Fig. 134A depicts the radius of curvature 710 and Fig. 134B the terminal bending angle 711.

Our findings indicate that under a consistent magnetic field 5, the radius of curvature 710 of the soft tube increases with an increase in magnetic unit distance 51, as does the terminal bending angle 711. This suggests that soft tubes with larger magnetic unit distances 51 are more likely to exhibit larger arc-shaped bends. A similar trend is observed across different magnetic field strengths B.

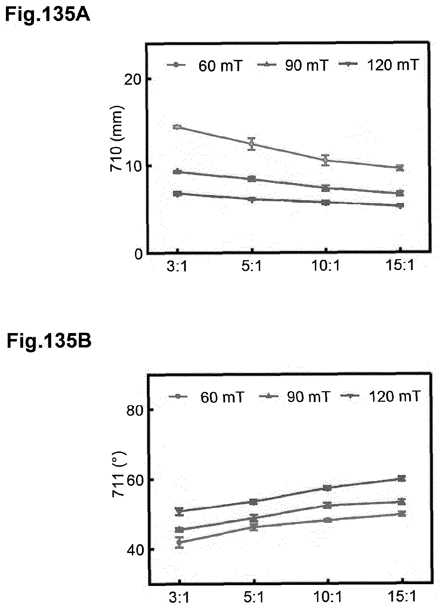

In experiments on elastic modulus effects, we set the magnetic unit distance 51 at 12mm and employed soft tubes fabricated from PDMS with varying base-to-curing agent mass ratios (3:1, 5:1, 10:1, 15:1). The deformation of these soft tubes was observed under magnetic fields of 60 mT, 90 mT, and 120 mT, with each experimental setup repeated three times. Fig. 135 shows the impact of elastic modulus on the deformation of soft tubes. Fig. 135A shows the radius of curvature 710 and Fig. 135B the terminal bending angle 711. We utilized Polydimethylsiloxane (PDMS) as the manufacturing material and varied the base-to-curing agent mass ratio at 3:1, 5:1, 10:1, and 15:1 (i.e., values of the x-axis above) to achieve soft tubes with different elastic moduli.

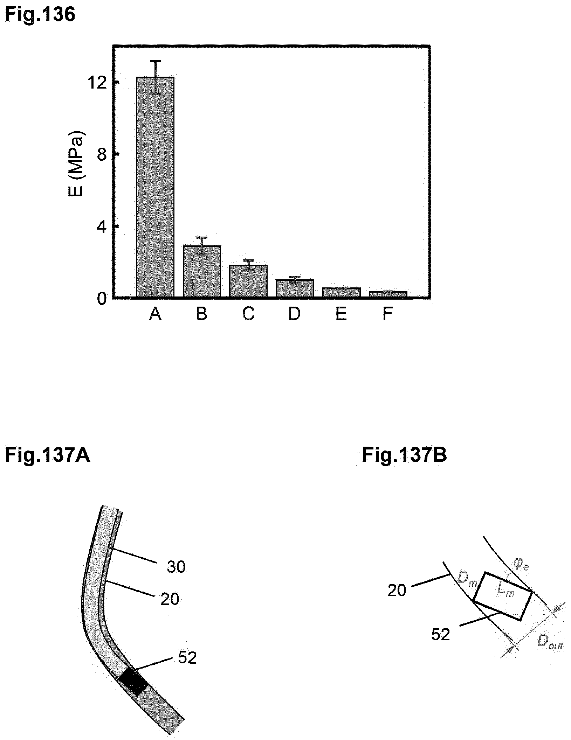

The results indicate that under the same magnetic field strength B, soft tubes with a higher base-to-curing agent mass ratio exhibit a smaller radius of curvature 710 and a larger terminal bending angle 711. This trend is consistently observed across different magnetic field strengths B. Additionally, to determine the elastic modulus of soft tubes made from PDMS with different base-to-curing agent mass ratios, we conducted tensile tests using a Universal Testing System (Model 5942, INSTRON, USA), as shown in Fig. 109B. The tensile test for each ratio was repeated three times to calculate the mean and standard deviation. The corresponding values for the elastic modulus E were obtained and are presented in Fig. 136, which depicts the elastic modulus E of different materials as listed in the following table:

| | |
|---|---|
| A | PDMS with a base-to-curing agent ratio (B:C ratio) of 10:1 + Magnetic particle m_{PDMS} : m_{Magnetic particle}=3:1 |
| B | PDMS, B:C ratio 3:1 |
| C | PDMS, B:C ratio 5:1 |
| D | PDMS, B:C ratio 10:1 |
| E | PDMS, B:C ratio 15:1 |
| F | PDMS, B:C ratio 20:1 |

The measured data indicate that higher base-to-curing agent mass ratios in PDMS result in a lower elastic modulus. Consequently, in the experiments on elastic modulus effects, the degree of soft tube bending increases as the elastic modulus of the manufacturing material increases.

We employed experimental methods to investigate the factors influencing the deformation of soft tubes, analyzing how various factors affect the radius of curvature 710 and terminal bending angle 711. This analysis provides insights for the design of soft tubes. Additionally, the specific experimental data obtained can be directly used as parameters in the design process.

### IV. Error Analysis of Magnetic Unit Position Control

The present disclosure controls the position of magnetic units 52, 62 through the relative movement of multiple tubes forming the outer and inner parts 20, 30, 40 and the magnetic member 16 and the target member 15. Given the flexibility of the tubes, the magnetic units 52, 62 may be subjected to magnetic forces or torques when exposed to an external magnetic field 5. The parts connected to the magnetic units 52, 62 may undergo deformation under the influence of these forces/torques, leading to a deviation of the magnetic units 52, 62 from their predetermined positions. Axially, the position of the magnetic units 52, 62 may be controlled by the inner parts 30, 40. Therefore, axial extension or compression of the inner parts 30, 40 affects the axial positioning of the magnetic units 52, 62, which constitutes the source of axial control error. The outer part 20 forms the main structure of the soft robot 1, with the magnetic unit 52, 62 being one of its integral parts, capable of driving deformations in the soft robot 1, resulting in its extension and compression. These deformations are inherent working characteristics of the soft robot 1 and are not considered as errors in the position control of the magnetic unit. Therefore, radially, the elastic deformation of the outer part 20 does not affect the positional accuracy of the magnetic unit 52, 62. On the other hand, in this study, due to the gap between the inner and outer parts 20, 30, 40, the magnetic unit 52, 62 cannot seamlessly fit into the outer part 20, causing a certain degree of rotation. Thus, with the present disclosure, the positional control error of the magnetic unit may be characterized by axial position error and angular error.

When calculating the axial position error, the magnetic unit 52, 62 may be subjected to the effects of a gradient magnetic field 5, a uniform magnetic field 5, or a combination of both. When a gradient magnetic field 5 is applied, the force exerted on the magnetic unit 52, 62 by the gradient magnetic field 5 can be expressed as ${F}_{i}^{G} = ∇ \left({m}_{i}⋅{B}_{i}\right)$ (S4-1) where **m*ᵢ*** represents the magnetic moment of magnetic unit *i*; **B*ᵢ*** denotes the magnetic field 5 experienced by magnetic unit *i.*

When a uniform magnetic field 5 is applied, the torque exerted on the magnetic unit 52, 62 by the uniform magnetic field 5 is ${T}_{i}^{U} = {m}_{i} \times {B}_{i}$ (S4-2)

Assuming a uniform distribution of magnetization in the magnetic unit 52, 62, the force exerted on the part 20, 30, 40 connected to it can be derived as follows: ${F}_{i}^{U} = \frac{{T}_{i}^{U}}{{L}_{m} / 2}$ (S4-3) Here, *Lₘ* denotes the axial length of the magnetic unit 52, 62.

When the magnetic unit 52, 62 is subjected to the force ${F}_{i}^{G}$ from a gradient magnetic field 5, it induces complex deformations in the inner part 30, 40, including bending, stretching, and even twisting. To obtain the maximum axial position error and simplify the calculation, we approximate the complex deformations caused by ${F}_{i}^{G}$ on the inner part 30, 40 as either stretching or compressing under axial force. Considering ${F}_{i}^{G}$ to act solely in stretching or compressing is an extreme case possible in practical situations, where the amount of stretch or compression will be maximal. It will be feasible to calculate the maximum axial position error using this extreme case scenario.

Similarly, since the outer tube surrounding the magnetic unit 52, 62 exerts a reactive force on the magnetic unit 52, 62, a portion of the force ${F}_{i}^{U}$ derived from the torque experienced by the magnetic unit 52, 62 will be counteracted by this reactive force. In other words, the force acting on the inner part 30, 40 will be less than ${F}_{i}^{U}$. However, for the sake of simplifying calculations and computing the maximum error, we neglect the external reactive force of the outer part 20 and apply the entire force ${F}_{i}^{U}$ on the inner part 30, 40. This simplification is also viable for calculating the maximum axial position error.

In various applications, each inner part 30, 40 may have multiple magnetic units 52, 62 attached, with the possibility of differing magnetization directions, leading to scenarios where the forces exerted on the inner part 30, 40 may counteract each other. To simplify the calculation process and determine the maximum error, we assume that the forces from multiple magnetic units 52, 62 are concentrated on a single magnetic unit 52, 62 for calculation purposes. Therefore, the maximum axial force can be calculated as follows: ${F}_{max} = {\sum }_{i = 1}^{n} \left({F}_{i}^{G}+{F}_{i}^{U}\right)$ (S4-4) where n represents the number of magnetic units 52, 62.

Under axial force, whether stretching or compressing, the maximum deformation of the inner part 30, 40 (i.e., the maximum axial position error of the magnetic unit 52, 62) can be expressed as: ${ε}_{a} = \frac{{F}_{max} {L}_{in}}{{E}_{in} {A}_{in}}$ (S4-5) where *Lᵢₙ* represents the length of the inner part 30, 40; *Eᵢₙ* denotes the elastic modulus of the inner part 30, 40; *Aᵢₙ* refers to the cross-sectional area of the inner part 30, 40.

In the calculation of angle errors controlled by magnetic units 52, 62, due to the existence of gaps, the magnetic units 52, 62 do not maintain perfect axial alignment with the outer part 20. To calculate the maximum angle error, as shown in Fig. 137, we assume the magnetic units 52, 62 are placed in extreme positions. Fig. 137 is a diagram for error analysis of magnetic unit position control with Fig. 137A being a diagram describing the position of the inner part 30, 40 within the outer part 20 and Fig. 137B being an illustration of the position of the magnetic unit 52, 62 in the outer part 20 under extreme conditions. This scenario is analyzed to calculate the angle error of the magnetic unit 52, 62 caused by the gap between the outer part 20 and the inner part 30, 40 in extreme cases.

Similar to the analysis for calculating axial position errors, due to the elasticity of the outer part 20, the magnetic units 52, 62 may compress the outer part 20, causing a bulge at the contact point. However, this phenomenon is a unique working characteristic of soft robots 1, and not a result of control errors of the magnetic units 52, 62. Therefore, this scenario is not considered an angle error of magnetic unit control.

Based on the geometric relationships depicted in Fig. 137, the following formula can be derived: ${L}_{m} sin {φ}_{e} + {D}_{m} cos {φ}_{e} = {D}_{out}$ (S4-6) where *Lₘ* represents the axial length of the magnetic unit 52, 62; *φₑ* is the angle between the axial direction of the magnetic unit 52, 62 and the outer part 20, which is the maximum angle error; *Dₘ* denotes the outer diameter of the magnetic unit; *Dₒᵤₜ* refers to the inner diameter of the outer part 20.

Therefore, the maximum angle error controlled by the magnetic unit 52, 62 can be expressed as: ${φ}_{e} = arcsin \frac{{D}_{out}}{\sqrt{{{L}_{m}}^{2} + {{D}_{m}}^{2}}} - arcsin \frac{{D}_{m}}{\sqrt{{{L}_{m}}^{2} + {{D}_{m}}^{2}}}$ (S4-7)

Taking the parameters used in most examples of this disclosure as examples, specifically *Dₒᵤₜ*=1.7 mm, *Dₘ*=1.4 mm, and *Lₘ*=5.6 mm, the calculations based on the given formula yielded a maximum angle error of 3.09°. Such a level of angle error is completely acceptable within soft robotics.

### V. Catheter-Blood Vessel Contact Analysis

In vascular interventional procedures, catheters/guidewires are used to navigate through blood vessels to reach target locations for diagnosis or treatment. A catheter typically consists of a catheter tip and a catheter body. The main cause of damage inflicted by the catheter tip on blood vessels is contact, leading to potential abrasions or even perforations of the vessel wall. Numerous studies have been conducted to address this challenge. However, comparatively less attention has been paid to the damage caused by the catheter body to blood vessels.

The damage inflicted by the catheter body on vessel walls is most likely to occur at sites of vessel curvature or branching. During catheter manipulation, movements involve both advancement and retraction, with the primary actions being pushing and pulling the catheter. At sites of vessel curvature, the corresponding state during catheter pushing is depicted in Fig. 138A at (i).

Fig. 138 depicts an analysis diagram of a body 10 formed as a catheter navigating through blood vessels 441. Fig. 138A is force analysis diagram of the catheter's contacts with the vessel wall during, shown at (i), pushing the catheter 10 forward and, shown at (ii), pulling the catheter 10 backward. Fig. 138B depicts a section view at sections A-A and B-B in Fig. 138A. Fig. 138C is a cross-sectional diagram of the ideal contact state between the catheter 10 and the blood vessel 441 with a section view along the plane formed by the main vessel axis and the branch vessel axis shown at (i) and a section view along section C-C of (i) shown at (ii). In the ideal contact state, the catheter 10 does not contact the sides of the vessel 441 but only has contact with the bottom of the vessel 441.

When pushing the catheter, friction occurs between the catheter body and the vessel wall. Consequently, the resultant force exerted on the vessel wall is: ${F}_{f - push} = {μF}_{n 1}$ (S5-1) where *µ* denotes the friction coefficient between the catheter body and the blood vessel, and *F*_{*n*1} represents the normal force exerted by the catheter body on the blood vessel. The normal force exerted by the catheter body on the blood vessel, i.e., the component of catheter thrust, can be expressed as ${F}_{n 1} = {F}_{push} cos θ$ (S5-2) where *Fₚᵤₛₕ* represents the thrust of the catheter, and *θ* denotes the angle between the direction of catheter movement at the point of contact with the blood vessel wall and the normal direction. Thus, the force exerted by the catheter body on the blood vessel wall during catheter propulsion can be derived as: ${F}_{f - push} = {μF}_{push} cos θ .$ (S5-3)

Similarly, during the process of pulling the catheter 10, as illustrated in Fig. 138A at (ii), the force exerted by the catheter body on the blood vessel wall can be calculated using the following formula: ${F}_{f - pull} = {μF}_{n 2}$ (S5-4) ${F}_{n 2} = {F}_{pull} cos θ$ (S5-5) ${F}_{f - pull} = {μF}_{pull} cos θ$ (S5-6) where *F_{f-pull}* is the pulling force exerted by the catheter body on the blood veseel, *F*_{*n*2} represents the normal force exerted by the catheter body on the blood vessel during catheter pulling, and *Fₚᵤₗₗ* denotes the pulling force applied to the catheter.

For the two scenarios described above regarding catheter pushing and pulling (Fig. 138A), the cross-sectional view at the point of contact is illustrated in Fig. 138B, where the forces between the catheter body and the blood vessel are independent of gravity. However, there is another scenario depicted in Fig. 138C, where the catheter 10 precisely matches the morphology of the blood vessel 441, meaning that, when viewed along the planes intersecting the main vessel axis and branch vessel axes, there is no contact between the catheter and the vessel wall. In this case, as shown in Fig. 138 at (ii) for the C-C cross-section, the frictional force resulting from the contact between the catheter body 10 and the wall of the vessel 441 during pushing or pulling is eliminated, leaving only the frictional force generated by gravity, the magnitude of which is ${F}_{f - g} = μG$ (S5-7) where *G* represents the gravitational force acting on the portion of the catheter in contact with the blood vessel wall. Due to multiple points of contact between the catheter and the vessel wall, let *N* denote the number of contact points. Therefore, the gravitational force on the catheter at these points is given by $G = mg / N$ (S5-8) where *m* represents the total mass of the catheter. Therefore, the force exerted by the catheter body 10 on the blood vessel wall at that point is given by ${F}_{f - g} = μmg / N .$ (S5-9)

Due to the flexible nature of the catheter 10, when it is pushed, it bends (forming waves) inside the blood vessel, as illustrated in Fig. 138A at (i). If the pushing force increases, the catheter 10 will bend more inside the vessel 441, and a portion of the work done by the external pushing force will be converted into the catheter's strain potential energy, unable to be entirely transmitted to the vessel wall. However, when the catheter is pulled, as depicted in Fig. 138A at (ii), it does not form waves inside the vessel 441. Therefore, the work done by the pulling force is not converted into the catheter's strain potential energy but is instead transmitted to the vessel wall. Therefore, assuming equal magnitudes of pushing and pulling forces on the catheter 10 at locations where the blood vessel 441 bends, the friction generated by the pulling force exceeds that generated by the pushing force.

We are now comparing the effects of pushing the catheter 10 and the force caused by gravity on the blood vessel wall. According to *(1),* the minimum operating force is 0.29 N, while the maximum operating force can reach up to 40 N under extreme conditions. Based on (2-5), the typical operating force ranges from 2 to 3.4 N. As a conservative estimate, we choose the minimum operating force of 2 N as the pushing force. During the process of pushing the catheter forward, as shown in Fig. 138A at (i), the angle between the catheter and the blood vessel wall axis roughly equals the angle of the blood vessel branch. Assuming the angle of the blood vessel branch is 30°, then *θ* is 60°. Assuming the friction coefficient between the catheter and the blood vessel wall is 0.2, the force exerted by the catheter 10 on the blood vessel wall is *F_{f-push} =* 0.2 × 2 × cos 60°=0.2 N.

A commonly used 7 French catheter (WAIN-FBK-7S, WASAHI INTECC CO., JP) may be used, for example, with a length of 900 mm and a weight of approximately 4.9 g. When the catheter 10 is fully adhered to the blood vessel 441, there will be numerous contact points between the catheter 10 and the vessel 441. To provide a conservative estimate, an extreme scenario is considered that assumes that the catheter 10 only has three contact points with the vessel 441: the catheter tip, tail, and a midpoint, leaving approximately 450mm of the catheter 10 suspended on each side, resulting in N=3. Thus, the force exerted by the catheter 10 on the blood vessel wall is *F_{f-g}* = 0.2 × 0.0049 × 9.8/3 =3.2×10⁻³ N.

Based on the above analysis, even under the conservative estimate using the most extreme scenario, the friction induced by the pushing force is 62.5 times greater than that induced by gravity. In other words, the frictional force caused by gravity will be significantly smaller than that caused by pulling and pushing. Furthermore, as the catheter 10 moves within the blood vessel 441, the vessel wall must counteract its weight, rendering gravity's effect unavoidable. Therefore, among the forces exerted by the catheter 10 on the vessel 441, the scenario depicted in Fig. 138C represents the most ideal condition. In this scenario, the force exerted by the catheter 10 on the vessel wall 441 is minimized. From the perspective of cross-sections along the main vessel axis and branch vessel axes, see Fig. 138C, there is no contact between the catheter 10 and the vessel walls on either side.

### VI. Experimental Analysis of Tube Manipulation

The real-time in-situ magnetization reprogramming proposed in the present disclosure is achieved by dynamically altering the relative positions of magnetic units 52, 62, a process that relies on the relative motion between magnetic members 16 and target members 15, exemplarily formed by tubes. The following presents an experimental analysis of tube manipulation, which provides empirical data to support the design and improvement discussions in section I and serves as a reference for specific design implementations when considered alongside section I. Since the manipulations in 1D, 2D, and 3D magnetization reprogramming are similar, the following analysis focusses solely on tube manipulation in the 1D case.

### VI.1. Experimental Setup

To induce relative motion between the tubes forming the outer part 20 and the inner parts 30, 40, the operation is divided into pushing and pulling actions. Achieving relative motion requires overcoming the relative motion resistance, *Fₘ₋ₜₒₜₐₗ* (for detailed analysis, please refer to section I). The factors affecting *Fₘ₋ₜₒₜₐₗ* include the tube curvature, the number of bends, the clearance between the tubes, the tube material, the mode of relative motion between the tubes, and the lubrication method. Therefore, six experiments have been designed to quantify the impact of these factors on *Fₘ₋ₜₒₜₐₗ:* Experiment I: Effect of the radius of curvature; Experiment II: Impact of the number of bends; Experiment III: Influence of the clearance between tubes; Experiment IV: Effect of the tube material's elastic modulus; Experiment V: Influence of tube motion mode; Experiment VI: Impact of the lubrication method.

In Experiment I, which assesses the impact of the radius of curvature on the relative motion resistance, the analysis due to the irregular nature of the deformations is simplified. A circular arc with a specific radius of curvature is used as an approximation.

Thereby, an integrated tube forming the body 10 was used that comprised an outer tube forming the outer part 20 and an inner rod forming the inner part 30.

As shown in Fig. 139A, which depicts an experimental setup for experiments assessing the impact of the radius of curvature on inner tube operation, the experimental model features circular cavities with various pathways. These circular cavities have a diameter of D = 2.9 mm. The model's cross-section consists of a semi-circular arc connected to two straight segments, simulating the deformation of the tube. An integrated tube forming the body 10 was used that comprised an outer tube forming the outer part 20 and an inner rod forming the inner part 30. When the integrated tube (the inner rod is positioned within the outer tube) forming the body 10 is placed into the model cavity, it assumes a specific shape, thereby simulating the deformations it would undergo in a magnetic field. To achieve various deformations, we established circular arcs with differing radii, see Fig. 139A, where "∞" indicates that the circular arc degenerates into a straight line. During the experiment, the integrated tube forming the body 10 was placed within the cavity of the model. The outer tube forming the outer part 20 was fixed to the model, which is attached to the ground. The inner rod forming the inner part 30 was then pulled along points M, N, and P. We utilized a Universal Testing System (Model 5942, INSTRON, USA) to measure the forces (Fig. 109B), with each experiment being repeated three times. The dimensional parameters of the outer and inner parts 20, 30 are identical to those of the tubes in the first configuration for 1D magnetization reprogramming, see Fig. 3. Both the tube forming the outer part 20 and the rod forming the inner part 30 were fabricated using PDMS with a base-to-curing agent ratio of 20:1.

In Experiment II, which investigates the impact of the number of bends on relative motion resistance, we designed a model featuring multiple bends. This model includes circular cavities with various pathways, each having a diameter of D= 2.9 mm. The model's cross-section consists of multiple semi-circular arcs connected to straight segments. Cross-sectional views and specific dimensional parameters are shown in Fig. 139B, which depicts an experimental setup for experiments evaluating the effect of the number of bends on inner tube operation. We set up experiments to test shapes with one, two, and three bends. For the single-bend experiment, the integrated tube was placed along the curve AFG into the model, with the outer tube fixed, and the inner rod was then pulled along points AFG; for the experiment with two bends, the integrated tube was placed along the curve BEFG, with the outer tube fixed, and the inner rod was then pulled along points BEFG; similarly, for the experiment with three bends, the integrated tube was placed along the curve CDEFG, with the outer tube fixed, and the inner rod was then pulled along points CDEFG. The forces were measured using the Universal Testing System, with each experiment conducted in triplicate. The materials and dimensional parameters of the outer tubes and inner rods are also identical to those in Experiment I.

In Experiment III, which examines the impact of the clearance between the inner and outer tubes on the relative motion resistance, we used tubes with the same parameters as those in Experiment I. The inner diameter of the outer tube was set at 1.7mm, and we used inner rods with four different outer diameters: 1.4mm, 1.5mm, 1.6mm, and 1.7mm. The model used to fix the tubes was the same as in Experiment I, featuring a cavity with a circular arc radius of Rᵢ = 20 mm. The same operational procedures from Experiment I were employed and the force measurements for each operation were recorded.

In Experiment IV, to investigate the effect of different elastic moduli of the materials on the relative motion resistance, we kept the material of the outer tube constant and fabricated the inner rod using various materials. Specifically, we prepared rods using PDMS with base-to-curing agent ratios of 20:1, 15:1, 10:1, and 3:1, respectively. Additionally, we fabricated a rod by mixing PDMS (10:1) with magnetic powder at a mass ratio of 3:1. We employed the same model as in Experiment I, selecting a cavity with a curvature radius of Rᵢ *=* 10 mm for the experiments. Using the same operational procedure as in the tube curvature influence experiment, we recorded the force values for each operation, and each experiment was repeated three times.

In Experiment V, we further employed the setup from Experiment I to assess the impact of different tube motion modes on the relative motion resistance. According to the analysis in section I above, the tube can achieve linear motion through pushing or pulling, which results in relative motion between the tubes. Additionally, rotating the tube while pushing or pulling it can reduce the relative motion resistance. Consequently, we designed two sets of experiments. The first set was identical to Experiment I, with the tube being pulled. In the second set, the tube was both pulled and rotated. The relative rotation between the tube and rod was induced by rotating the support frame that holds the tube, with a rotational speed set at 60°/s. We used the same model from Experiment I, selecting a cavity with a radius of curvature of Rᵢ = 10 mm. The outer tube remained the same as in Experiment I, while the inner rod was fabricated as described in Experiment IV using a PDMS and magnetic powder mixture. Each experimental condition was measured three times, and the force data were recorded.

In Experiment VI, to investigate the impact of different lubrication methods on the relative motion resistance, we tested four lubricants: talcum powder, 85% glycerol, 40% glycerol, and water. We used the same model as in Experiment I, specifically a cavity with a radius of curvature of Rᵢ = 10 mm. The outer tube was identical to that in Experiment I, and the inner rod was fabricated using the PDMS and magnetic particle mixture described in Experiment IV. By applying the different lubricants between the outer part 20 formed by the tube and the inner part 30 formed by the rod, we measured the force using the same methodology as in Experiment I. Each experiment was repeated three times, and the data were recorded.

### VI.2 Experimental Results and Discussion

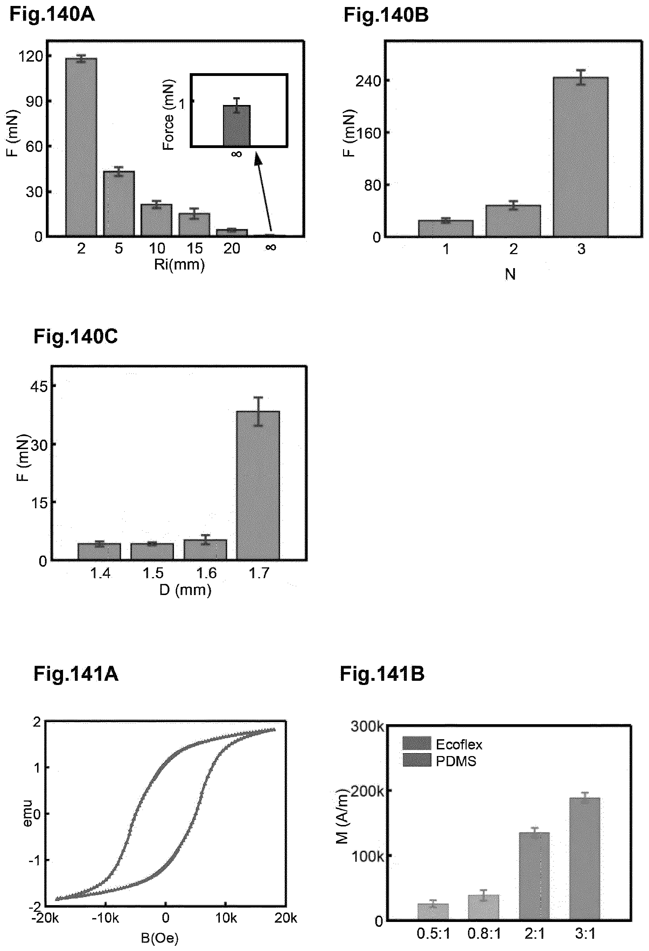

Fig. 140 depicts the effect of various parameters on the relative motion resistance involved in manipulating tubes with Fig. 140A showing the average force F for different radii of curvature Rᵢ, Fig. 140B showing the average force F for different numbers of bends, and Fig. 140C showing the average force F for different rod diameters D.

The data measured in Experiments I and II are presented in Fig. 140A and 140B. We observed that the relative motion resistance significantly decreases as the radius of curvature increases: at a radius of curvature of Rᵢ = 2mm, the average force F was 118.2 mN; at an infinite radius of curvature Rᵢ (i.e., when the tube is straight), the average force F was only 0.9 mN. The relative motion resistance significantly increases with the number of bends: with one bend, the average force was F = 24.8 mN; with three bends, it significantly increased to F = 243.9 mN. Therefore, both the radius of curvature Rᵢ and the number of bends N severely impact the manipulation of the tube forming the body 10. Simultaneously, we observed that at smaller radii of curvature Rᵢ, the forces F required to push and pull the inner rod are relatively similar. However, when the radius of curvature decreases to Rᵢ = 2 mm, it becomes impossible to push the inner rod. Consequently, pulling operations are easier than pushing: with minimal deformation, the inner rod can be moved either by pulling or pushing to adjust its position; yet, as the radius of curvature decreases and the number of bends increases, pushing becomes challenging, whereas pulling remains feasible.

The results from Experiment III are illustrated in Fig. 140C. We observed a sudden decrease in relative motion resistance as the outer diameter of the inner rod was reduced: with an outer diameter of the inner rod of D = 1.7mm (the same as the inner diameter of the outer tube), the average force is F = 38.3 mN; when the outer diameter of the inner rod is reduced to D = 1.6 mm, the average force abruptly decreases to F = 5.2 mN; with outer diameter of the inner rod smaller than D = 1.6 mm, the average force F does not significantly decrease further, but remains roughly equivalent to that observed with a D = 1.6 mm outer diameter. Our analysis suggests that when the outer diameter D of the inner part 30, exemplarily formed by the inner rod, matches the inner diameter of the channel 22 of the outer part 20, exemplarily formed by the outer tube, additional compressive forces arise, thus increasing friction; however, when the outer diameter D of the inner part 30 is less than the inner diameter of the channel 22 of the outer part 20, the compressive forces between them do not change significantly, hence the relative motion resistance remains similar. Therefore, in design, reducing relative motion resistance does not necessitate significantly increasing the clearance between the inner and outer parts, allowing more flexibility in other aspects of their design. The inner part 30 may therefore have an outer diameter D that is at most the inner diameter of the channel 20 of the outer part 20.

The data from Experiment IV, as shown in Fig. 116A, indicate that the relative motion resistance increases with an increase in the elastic modulus of the material (the elastic moduli of these materials were measured in Fig. 136). When using PDMS with a 20:1 base-to-curing agent ratio (elastic modulus of 0.33 MPa), the relative motion resistance was F = 21.3 mN; in contrast, when employing a PDMS-magnetic particle mixture (elastic modulus of 12.3 MPa), the relative motion resistance reached F = 83.6 mN. This observation is consistent with the analysis presented in section I.1 above, which states that a higher elastic modulus results in larger relative motion resistance, due to the increased elastic potential energy stored in the rod during bending.

Fig. 116B shows the results from Experiment V, and it indicates that by employing a simultaneous rotation and translation method, the relative motion resistance is significantly reduced from F = 83.6 mN to F = 27.4 mN. This reduction is attributed to the conversion of sliding friction between the tubes into rolling friction, thereby lowering the friction coefficient. Additionally, the rotation weakens the adhesive effects between the tubes by continuously rearranging and refreshing the local contact surfaces, which in turn reduces adhesive friction.

The data from Experiment VI, as shown in Fig. 116C, reveal that the relative motion resistance varies considerably with different lubricants. When using talcum powder, the relative motion resistance is F = 83.6 mN; with 40% glycerol, it increases to F = 93.75 mN; and with 85% glycerol, it reaches F = 163.75 mN. This suggests that higher-viscosity glycerol is less effective in lubricating the tubes. When water is used as the lubricant, the relative motion resistance is F = 131 mN, which is intermediate between the values obtained with talcum powder and glycerol. Given that talcum powder exhibits a relatively low friction coefficient, it is chosen as the lubricant in this study. Furthermore, in practical applications, surface treatments for the tubes, such as adding a superlubricity layer, could further reduce the relative motion resistance, thereby facilitating easier control of the tubes' relative motion.

In general, the lubricant may comprise one of talcum powder, glycerol, such as 85% glycerol, and water. Additionally or alternatively, an inner surface of the channel 22 of the outer part 20 and/or an outer surface of the inner part 30 may comprise a superlubricity layer.

The experimental results and discussion presented above offer valuable insights for the design and manipulation of the tubes, and these findings apply also to the design and operation of 2D sheets and 3D structures.

### VII. Feasibility of 3D Manipulations Without Altering External Magnetic Fields

To demonstrate the feasibility of achieving 3D manipulations of soft robots 1 without altering external magnetic fields 5 using the devices and methods of the present disclosure, the following first considers 1D tubular objects as the carriers or bodies 10, and discusses the operational scenarios for tubes with a single magnetic unit 52 and those with multiple magnetic units, 52, 62, respectively. Subsequently, the analysis is extended to 2D/3D (planar/spatial) objects. For clarity and ease of discussion, the analysis is divided into three subsections: tubes with a single magnetic unit 52, tubes with multiple magnetic units 52, 62, and 2D/3D objects.

### VII.1 Tubes with a single magnetic unit

We positioned a tube with a length of *L* and a radius of *R,* equipped with a magnetic unit 52 at the first end end, vertically along the z-axis of the coordinate system. The second end without a magnetic unit was fixed at the origin *O* of the coordinate system, and a magnetic field **B** was applied. The magnetization of the magnetic unit 52 can be expressed as $M = M a$ (S7-1) where M is the magnitude of the magnetization, and ***a*** is the unit direction vector of magnetization. Since **a** can be adjusted in direction on the unit sphere, it can be represented using spherical coordinates as $a = \left(sin θ cos φ, sin θ sin φ, cos θ\right)$ (S7-2) where *θ* is the angle between the magnetization unit vector of the magnetic unit 52 and the z-axis, and *φ* means the angle between the projection of the magnetization unit vector of the magnetic unit in the *xoy* plane and the *x*-axis.

Under the applied magnetic field 5, the magnetic unit 52 experiences a force or torque, with the force density given by $f = {μ}_{0} \left(M⋅∇\right) B \left(r\right)$ (S7-3) where *µ*₀ is the permeability of free space, and **B**(**r**) is the magnetic field strength at position r. Assuming the volume of the magnetic unit 52 is *V,* the force exerted on the magnetic unit is given by: ${F}_{V} = {\int }_{V} fd V$ (S7-4)

The magnetic moment **m** of the magnetic unit 52 can be obtained using the following formula: $m = {\int }_{V} M d V$ (S7-5)

The torque exerted on the magnetic unit 52 can be expressed as ${τ}_{V} = m \times B$ (S7-6)

If the applied magnetic field 5 is uniform and the magnetization M of the magnetic unit 52 is assumed to be uniformly distributed throughout its volume, then, since *∇***B** = 0 in a uniform magnetic field 5, the force exerted on the magnetic unit 52, according to Equations (S7-3) and (S7-4), is given by $f = {μ}_{0} \left(M⋅∇\right) B \left(r\right) = 0$ (S7-7) ${F}_{V} = {\int }_{V} fd V = 0$ (S7-8)

This indicates that no net force acts on the magnetic unit 52 in a uniform magnetic field 5. According to Equations (S7-1), (S7-5), and (S7-6), the torque exerted on the magnetic unit 52 can be expressed as ${τ}_{V} = MV \left(a\times B\right)$ (S7-9)

If the applied magnetic field 5 is a gradient field, the force exerted on the magnetic unit 52 can be expressed as ${F}_{V} = {μ}_{0} {\int }_{V} \left(M⋅∇\right) B \left(r\right) d V$ (S7-10)

Since ∇**B** ≠ 0 in a gradient field, a net force acts on the magnetic unit 52. Furthermore, the exerted force induces a torque, which is given by ${τ}_{V} = {\int }_{V} r \times fd V$ (S7-11)

Substituting the equation for force density, we obtained: ${τ}_{V} = {μ}_{0} {\int }_{V} r \times \left[\left(M⋅∇\right)B\left(r\right)\right] d V$ (S7-12)

Under the influence of forces and torques, the tube undergoes deformation in the form of stretching, bending, and twisting. Since the effect of magnetic forces on stretching deformation is limited, bending deformation is more commonly utilized in the field of robotics for 1D tubes to achieve various functions and applications. Therefore, we will focus our analysis on bending deformation in the following discussion.

Assuming that one end of the tube is fixed while the other end is free, we can use the cantilever beam model to calculate its deformation under torque. The deflection of the tube under the applied torque is given by: $\frac{{d}^{2} w \left(r\right)}{d {z}^{2}} = - \frac{τ \left(r\right)}{EI}$ (S7-13) $w \left(r\right) = \left({w}_{x}\left(r\right),{w}_{y}\left(r\right),{w}_{z}\left(r\right)\right)$ (S7-14) where, *w*(*r*) represents the deflection at position r along the tube, where the magnetic unit 52 is located, **τ**(*r*) denotes the torque applied at position r (i.e., on the magnetic unit), *E* is the elastic modulus of the tube, and *I* is the second moment of area (area moment of inertia) of the tube's cross-section.

Based on the above analysis, under a uniform magnetic field 5, substituting the torque expression in a uniform magnetic field 5 into the deflection equation, the deflection of the tube is given by: $\frac{{d}^{2} w \left(r\right)}{d {z}^{2}} = - \frac{MV \left(a\times B\right)}{EI}$ (S7-15)

Similarly, under a gradient magnetic field, the deflection of the tube due to the torque generated by the field gradient can be written as: $\frac{{d}^{2} w \left(r\right)}{d {z}^{2}} = - \frac{{μ}_{0} M {\int }_{V} r \times \left[\left(a⋅∇\right)B\left(r\right)\right] d V}{EI}$ (S7-16)

Since one end of the tube is fixed, boundary conditions apply at the fixed end are: $w \left(0\right) = 0$ (S7-17) ${\left.\frac{d w}{d z}\right|}_{z = 0} = 0$ (S7-18)

Substituting this into the deflection equation allows us to obtain the deflection of the tube at the position of the magnetic unit. Given that the unit vector a is expressed as a = (sin *θ* cos *φ ,* sin *θ* sin *φ ,*cos *θ*), it is possible to achieve different magnetization directions by adjusting the angles *θ* and *φ*.

Furthermore, the devices and methods according to the present disclosure include the adjustment of both the magnetization strength of each magnetic unit 52, 62 (by varying the effective volume V) and the position of the magnetic units 52, 62. By substituting different orientations of a or different values of V into the deflection equations derived above, the deflection of the body 10 along the *x, y,* and *z* directions can be obtained under a constant magnetic field 5, whether in a uniform or gradient magnetic field. This enables controlled three-dimensional deformation of the body 10, exemplarily formed by the tube. By incorporating variations in the positions of the magnetic units 52, 62, a wider range of deformations can be achieved. This further demonstrates the feasibility of realizing 3D manipulation under a constant magnetic field 5.

### VII.2 Tubes with multiple magnetic units

We extend the number of magnetic units 52, 62 in the body 10, exemplarily formed by a tube, from one to N. While keeping the magnetic field 5 constant, we will demonstrate the feasibility of 3D manipulation. Similar to the previous analysis of a single magnetic unit 52, when the tube contains N magnetic units 52, 62, the magnetization strength of the *i*-th magnetic unit 52, 62 is expressed as: ${M}_{i} = {M}_{i} {a}_{i}$ (S7-19) where *Mᵢ* is the magnetization strength of the i-th magnetic unit 52, 62, and **a*ᵢ*** means the unit direction vector of the *i*-th magnetic unit 52, 62, which can be expressed in spherical coordinates as: ${a}_{i} = \left({sinθ}_{i} {cosφ}_{i},{sinθ}_{i} {sinφ}_{i},{cosθ}_{i}\right)$ (S7-20) where *θᵢ* represents the angle between the magnetization unit vector of the i-th magnetic unit 52, 62 and the z-axis, and *φ_{í}* is the angle between the projection of the magnetization unit vector of the i-th magnetic unit 52, 62 in the *xoy* plane and the *x*-axis.

Under a uniform magnetic field, the torque experienced by the i-th magnetic unit 52, 62 is given by ${τ}_{Vi} = {M}_{i} {V}_{i} \left({a}_{i}\times B\right)$ (S7-21) where **B** represents the strength of the uniform magnetic field 5. The total torque acting on the entire tube is then determined by: ${τ}_{total} = {\sum }_{1}^{N} {M}_{i} {V}_{i} \left({a}_{i}\times B\right)$ (S7-22)

Substituting this into the deflection equation, the deflection at the end of the tube is given by: $\frac{{d}^{2} w \left(r\right)}{d {z}^{2}} = - \frac{1}{EI} {\sum }_{1}^{N} {M}_{i} {V}_{i} \left({a}_{i}\times B\right)$ (S7-23)

We can conclude that by adjusting the angles *θᵢ* and *φ_{í}* of each magnetic unit 52, 62, or by modifying the magnetization strength of each magnetic unit 52, 62 (i.e., by changing the effective volume), it is possible to achieve different deflections at the tube's end in various directions without altering the applied magnetic field 5. This is analogous to the case of a single magnetic unit 52. Furthermore, the present disclosure includes modifications to the relative positions of individual magnetic units 52, 62. Since the deformation of the tube depends not only on the magnitude of the net torque but also on the spatial distribution of the magnetic units 52, 62, a broader range of deflections with varying magnitudes and directions can also be achieved.

We only demonstrated the case under a uniform magnetic field 5. Similar to the case of a single magnetic unit 52, 62, in a gradient field, the total torque is also the superposition of individual torques. Since this conclusion holds for the gradient field as well, we did not provide further proof.

### VII.3 2D/3D objects

The following extends the above analysis of a 1D structure to 2D and 3D cases. We begin by analyzing the 2D case, taking a sheet 160 as a representative example. The sheet 160 has a length L, a width B, and a thickness t. For simplification, we assume the thickness is negligible, i.e., $t ≪ L , B$ (S7-24)

Magnetic units 52, 62 are randomly distributed on the surface of the sheet 160. Similar to the deformation analysis of a tube with multiple magnetic units 52, 62, the magnetization and unit direction vector of each magnetic unit 52, 62 are given by: ${M}_{i} = {M}_{i} {a}_{i}$ (S7-25) ${a}_{i} = \left({sinθ}_{i} {cosφ}_{i},{sinθ}_{i} {sinφ}_{i},{cosθ}_{i}\right)$ (S7-26) where *Mᵢ* represents the magnitude of the magnetization of the *i*-th magnetic unit 52, 62, **a*ᵢ*** means the magnetization unit vector of the i-th magnetic unit 52, 62, *θᵢ* represents the angle between the magnetization unit vector of the *i*-th magnetic unit 52, 62 and the z-axis, and *φ_{í}* is the angle between the projection of the magnetization unit vector of the *i*-th magnetic unit 52, 62 in the *xoy* plane and the *x*-axis.

The position of the *i*-th magnetic unit 52, 62 can be expressed as ${r}_{i} = \left({x}_{i}, {y}_{i}, {z}_{i}\right)$ (S7-27)

Under a uniform magnetic field 5, the torque acting on the *i*-th magnetic unit 52, 62 is given by: ${τ}_{Vi} = {M}_{i} {V}_{i} \left({a}_{i}\times B\right)$ (S7-28)

In the Kirchhoff-Love thin plate theory, only the torque components perpendicular to the plate directly contribute to its bending deformation. Therefore, we defined the equivalent external load induced by the distribution of magnetic torques as: $q \left(r\right) = {\sum }_{i = 1}^{N} \left({τ}_{Vi}⋅t\right) δ \left(r-{r}_{i}\right)$ (S7-29) where t is the unit vector in the thickness direction of the sheet, *δ*(**r** - **r*ᵢ***) means the 2D Dirac delta function, representing the localized application of torque at position **r*ᵢ***.

Substituting the magnetic torque of each magnetic unit 52, 62 into the above equation, we obtain: $q \left(r\right) = {\sum }_{i = 1}^{N} \left[{M}_{i}{V}_{i}\left({a}_{i}\times B\right)⋅t\right] δ \left(r-{r}_{i}\right)$ (S7-30)

Neglecting the effects of gravity, external pressure, and other influences, and under the small deflection and linear elasticity assumptions of Kirchhoff-Love thin plate theory, the equilibrium equation of the sheet is given by: $D {∇}^{4} w \left(r\right) = q \left(r\right)$ (S7-31) $D = \frac{{Et}^{3}}{12 \left(1-{ν}^{2}\right)}$ (S7-32) where D is the bending stiffness of the sheet 160, w(r) represents the deflection of the sheet in the thickness direction, and v is the Poisson's ratio.

The equilibrium equation above can be transformed from the spatial domain to the frequency domain to facilitate the solution. First, applying the Fourier transform, solving the equation in the frequency domain, and then performing the inverse Fourier transform, we obtain the solution in the spatial domain. The deflection corresponding to the i-th magnetic unit 52, 62 can be expressed as: ${w}_{i} \left(r\right) = \frac{{M}_{i} {V}_{i} \left({a}_{i}\times B\right) ⋅ t}{D} G \left(r, {r}_{i}\right)$ (S7-33) where *G*(**r**,**r*ᵢ***) is the biharmonic Green's function, which can be obtained using the following equation: $G \left(r, {r}_{i}\right) = \frac{1}{8 π} {\left|r-{r}_{i}\right|}^{2} ln \left|r-{r}_{i}\right|$ (S7-34)

When the sheet contains only a single magnetic unit 52, 62, the deflection induced by this unit is given by Equation (S7-33). Since the unit magnetization direction is expressed as **a*****ᵢ** =* (sin *θᵢ* cos *φᵢ*, sin *θᵢ* sin *φᵢ*, cos *θᵢ*), it is possible to achieve different magnetization orientations by adjusting the angles *θᵢ* and *φᵢ*. Additionally, the effective volume *Vᵢ* of the magnetic unit 52, 62 can be modified to control the magnetization strength.

By substituting different unit magnetization directions **a*ᵢ*** or different magnetization strengths (via varying *Vᵢ*) into the deflection equation while keeping the magnetic field 5 constant, different deflections can be obtained. This results in 3D deformation of the sheet 160, thereby demonstrating the feasibility of achieving 3D manipulation under a constant magnetic field 5. Furthermore, the present disclosure also allows for the adjustment of the position r*ᵢ* of the magnetic units 52, 62. Changing the position of the magnetic units 52, 62 affects the location where the torque is applied, leading to different deformation patterns.

When the **sheet 160** contains ***N*** magnetic units 52, 62, the total torque can be obtained by summing the torques of all magnetic units 52, 62. Substituting this total torque into the **deflection equation,** the deflection of the sheet is given by: $w \left(r\right) = \frac{1}{D} {\sum }_{i = 1}^{N} \left[{M}_{i}{V}_{i}\left({a}_{i}\times B\right)⋅t\right] G \left(r, {r}_{i}\right)$ (S7-35)

In the equations (S7-35), similar to the analysis of a sheet 160 with a single magnetic unit 52, 62, a sheet 160 with multiple magnetic units 52, 62 can also achieve 3D deflection variations by adjusting the parameters, all while keeping the magnetic field 5 constant.

So far, we only considered a uniform magnetic field 5. Similar to the 1D tube with a single magnetic unit 52, 62, the total torque is also the superposition of individual torques in a gradient field. Since this conclusion also holds for the gradient field, we did not provide further proof.

Similarly, the manipulation of a 2D sheet can be extended to 3D objects, and the same conclusion applies. One of the simplest examples is stacking two sheets 160 in a non-parallel configuration, forming a 3D structure. Since each sheet 160 can independently achieve 3D deformation under a constant external magnetic field 5, the combined 3D object can also exhibit 3D deformation under the same conditions. Therefore, we did not further discuss the case of 3D objects.

The above analysis demonstrates the feasibility of 3D operation without altering external magnetic fields 5. Furthermore, the external magnetic field 5 may be varied, which enables even more complex 3D deformations. Section *VIII below,* shows that the force-torque space generated by the devices and methods according to the present disclosure, when combined with a variable magnetic field 5, fully encompasses the force-torque space achievable by existing methods. This implies that existing methods are a subset of the method according to the present disclosure. This is illustrated in Fig. 142, which illustrates the force-torque space generated by different methods and theoretically compares the force-torque space A generated by existing methods, which only change the external magnetic field 5, the force-torque space B generated by the method according to the present disclosure under a static magnetic field 5, and the force-torque space C generated by the method according to the present disclosure under a tunable magnetic field 5.

### VIII. Force-Torque Space Analysis

To compare the manipulation capabilities of the device and methods according to the present disclosure with those of the existing method for soft robotics, we analyzed their respective force-torque spaces, thereby theoretically demonstrating their manoeuvrability. The existing devices and methods - wherein only the external magnetic field 5 is altered while the robot's magnetization profile remains unchanged - is designated as Method 1 in the following. The devices and methods according to the present disclosure that employ a static magnetic field 5 (i.e., where no alteration of the magnetic field 5 occurs during the operation), with only the robot's magnetization curve being modified, are designated as Method 2. The devices and methods according to the present disclosure that employ a tunable magnetic field 5 (i.e., one that can be adjusted as required), are referred to as Method 3, whereby both the external magnetic field 5 and the robot's magnetization profile can be modified. To simplify the analysis, we initially examined the scenario under a uniform magnetic field 5, and subsequently extended the analysis to the case of a general magnetic field 5.

### VIII.1 Spatial representation

We assume that the magnetic soft robot 1 is situated in the 3D Euclidean space ${ℝ}^{3}$ and is composed of n magnetic units 52, 62, where n ≥ 1. Each magnetic unit 52, 62 possesses a magnetic moment, denoted by **m*ᵢ***. When the robot 1 is subjected to a uniform magnetic field, the *i^{th}* magnetic unit 52, 62 experiences only a torque, given by ${τ}_{i} = {m}_{i} \times B$ (S8-1) where **B** represents the magnetic field 5. Since the magnetic robot 1 comprises n magnetic units 52, 62, its torques can be expressed as $\left({τ}_{1},{τ}_{2},…,{τ}_{n}\right)$ (S8-2)

Thus, the overall torque of the robot is distributed in the space ${ℝ}^{3 n}$.

For Method 1, since the magnetization profile (i.e., **m*ᵢ***) is maintained constant while the external magnetic field (i.e., $B ∈ {ℝ}^{3}$) is varied freely, the torque experienced by the robot 1 is computed using equation (S8-1). Consequently, the set of torque distributions (torque space) is given by ${S}_{1} = \left\{\left.\left({τ}_{1},…,{τ}_{n}\right)\right|∃B∈{ℝ}^{3},{τ}_{i}={m}_{i}\times B\left(∀i\right)\right\}$ (S8-3)

The torque distribution space S₁ contains only those torque combinations that can be generated by some magnetic field **B**, and it does not span the entire space ${ℝ}^{3 n}$. We define a mapping $F : {ℝ}^{3} \to {ℝ}^{3 n} , F \left(B\right) = \left({m}_{1}\times B,…,{m}_{n}\times B\right)$ (S8-4)

Since **m₁** × **B** is linear, the mapping *F* is also linear and its image is given by ${S}_{1} = Im \left(F\right)$ (S8-5)

Here, S₁ represents the set of all possible vectors that *F* can output. Because the magnetic field **B** has only three independent components, it follows that $dim \left(Im \left(F\right)\right) \leq 3$ (S8-6)

Thus, the torque space generated by Method 1 can realize all torque distributions within S₁ in the space ${ℝ}^{3 n}$, but it is at most a 3D subspace. The specific dimension depends on the linear independence of the magnetic moments **m*ᵢ***.

For Method 2, since the robot's magnetization profile are varied while the external magnetic field 5 remains constant, the torques acting on the robot 1 are computed using Equation (S8-1). Thus, the torque distribution set (torque space) is defined as ${S}_{2} = \left\{\left.\left({τ}_{1},…,{τ}_{n}\right)\right|∃\left({m}_{1}^{′},…,{m}_{n}^{′}\right)∈{\left({ℝ}^{3}\right)}^{n},{τ}_{i}={m}_{i}^{′}\times {B}_{0}\left(∀i\right)\right\}$ (S8-7)

In this formula, the robot's magnetic moment **m*ᵢ*** can be arbitrarily adjusted in both magnitude and direction, and is denoted by ${m}_{i}^{′}$. The fixed, nonzero magnetic field 5 is represented by **B₀**, where ${B}_{0} ∈ {ℝ}^{3} ∖ \left\{0\right\}$. Although any ${m}_{i}^{′}$ can be selected to achieve a desired torque combination, the nature of the cross product imposes constraints such that *S*₂ does not span the entire ${ℝ}^{3 n}$ space.

Since the cross product of two vectors always yields a vector perpendicular to both, the torque experienced by each magnetic unit 52, 62 must be orthogonal to the magnetic field 5. This condition is expressed as ${τ}_{i} ⋅ {B}_{0} = \left({m}_{i}^{′}\times {B}_{0}\right) ⋅ {B}_{0} = 0$ (S8-8)

Thus, the torque space S₂ can be formulated as: ${S}_{2} = \left\{\left({τ}_{1},…,{τ}_{n}\right)\left|{τ}_{i}⋅{B}_{0}=0 \left(∀i\right)\right.\right\}$ (S8-9)

In 3D space, if the magnetic field **B₀** is fixed, then every torque **τ*ᵢ*** must lie within the 2D plane perpendicular to **B₀**. In other words, we can independently adjust each magnetic moment ${m}_{i}^{′}$ to control the corresponding component of **τ*ᵢ*** within that plane. Consequently, each **τ*ᵢ*** has two independent degrees of freedom, and with n independent magnetic units, the robot can generate n independent torques. Thus, the overall torque space S₂ forms a 2n-dimensional linear subspace, i.e., $dim \left({S}_{2}\right) = 2 n$ (S8-10)

For Method 3, where both the robot's magnetic moments and the external magnetic field are variable, the torque acting on the robot 1 is again computed using Equation (S8-1). Therefore, the torque distribution set (torque space) is defined as ${S}_{3} = \left\{\left({τ}_{1},…,{τ}_{n}\right)\left|∃B∈{ℝ}^{3},∃\left({m}_{1}^{′},…,{m}_{n}^{′}\right),{τ}_{i}={m}_{i}^{′}\times B, ∀i\right.\right\}$ (S8-11)

Here, we can arbitrarily choose the magnetic field **B** and the magnetic moment ${m}_{i}^{′}$ to achieve any desired combination of torques. However, due to the inherent properties of the cross product, S₃ does not span the entire ${ℝ}^{3 n}$ space. Since each ${m}_{i}^{′}$ can be independently selected, yet the resulting torque **τ*ᵢ*** is always orthogonal to **B,** the torque for each magnetic unit 52, 62 is confined to a fixed 2D plane. Therefore, S₃ can be equivalently expressed as ${S}_{3} = \left\{\left({τ}_{1},…,{τ}_{n}\right)\left|∃B\neq 0,{τ}_{i}⋅B=0 \left(∀i\right)\right.\right\}$ (S8-12)

Because the magnetic field **B** can be varied as needed, different 2D planes can be obtained by selecting different magnetic fields 5. For any two torque vectors **τ*ₘ*** and **τₙ** in *S*₃ (**τ*ₘ*** E S₃, **τ*ₙ*** E S₃), which are generated under magnetic fields **Bₘ** and **Bₙ** respectively, the fact that **τ*ₘ*** and **τ*ₙ*** lie on two distinct 2D planes implies that their linear combination will generally not lie in any single plane, i.e., $α {τ}_{m} + β {τ}_{m} ∉ {S}_{3}$ (S8-13)

Thus, S₃ is not a linear subspace but rather the union of all 2D planes corresponding to all possible orientations of the magnetic field **B.**

### VIII.2 Spatial relationships

Regarding the relationships among the torque spaces generated by the three methods, we first compare Methods 1 and 2, and then examine their relationship with Method 3. For the torque space S₁ generated by Method 1, based on the analysis above, since the torques are produced under the same magnetic field **B,** S₁ is at most three-dimensional - thus representing a low-dimensional subspace in ${ℝ}^{3 n}$. In contrast, the torque space S₂ generated by Method 2 is a 2*n*-dimensional subspace. When *n* ≥ 2, we have dim(*S₂*) ≥ 4, while dim(*S*₁) ≤ 3.

### VIII.2.1 Relationship between the torque spaces generated by methods 1 and 2

For the intersection of the torque spaces corresponding to Methods 1 and 2, based on the analysis above, we define the following set: ${S}_{1} ∩ {S}_{2} = \left\{\left({τ}_{1},…,{τ}_{n}\right)\left|∃B, {τ}_{i}={m}_{i}\times B,{τ}_{i}⋅{B}_{0}=0, ∀i\right.\right\}$ (S8-14)

This set is equivalent to ${S}_{1} ∩ {S}_{2} = \left\{\left({m}_{1}\times B,…,{m}_{n}\times B\right)\left|B∈{ℝ}^{3},\left({m}_{i}\times B\right)⋅{B}_{0}=0,∀i\right.\right\}$ (S8-15)

The intersection *S*₁ n *S*₂ comprises those torque distributions that can be generated by a magnetic field **B** while also being required to be orthogonal to **B₀**. According to the properties of vector operations: $\left({m}_{i}\times B\right) ⋅ {B}_{0} = \left({B}_{0}\times {m}_{i}\right) ⋅ B$ (S8-16)

Therefore, the magnetic field **B** must lie within span (**B₀** × **m*ᵢ***).

When dim(span(**B₀** × **m*ᵢ***)) ≤ 2, the vector **B₀** × ***mᵢ*** spans at most a 2D plane. In such a 2D space, one can always choose a magnetic field **B** that is perpendicular to this plane, thereby satisfying (**B₀** × **m*ᵢ***) · **B** = 0. Under this condition, the intersection *S*₁ n *S*₂ is nonempty, i.e., *S*₁ ∩ *S*₂ ≠ Ø.

In contrast, when dim(span(**B₀** × **m*ᵢ***)) ≥ 3-for instance, when it equals 3-the vector **B₀** × **m*ᵢ*** spans a 3D space. In that case, **B** would have to be orthogonal to every direction in this 3D space, which is only possible if **B** = 0; hence, *S*₁ ∩ *S*₂ = {0}.

From the analysis above, it follows that *S*₁ and *S*₂ have a nontrivial (nonzero) intersection under certain conditions. We now proceed to discuss their inclusion relationship. Let us first assume that *S*₁ ⊆ *S*₂, that is, every torque distribution generated by Method 1 can also be achieved by Method 2. The corresponding mathematical description is: $∀ B , \left({m}_{1}\times B,…,{m}_{n}\times B\right) ∈ {S}_{2}$ (S8-17)

Since *S*₂ is the set of all torque distributions that are perpendicular to the fixed magnetic field **B₀**, every **m*ᵢ*** × **B** must be orthogonal to **B₀**. Hence, we have: $\left({m}_{i}\times B\right) ⋅ {B}_{0} = 0 , ∀ i$ (S8-18)

Based on the properties of vector operations, the above equation can be rewritten as: $\left({m}_{i}\times B\right) ⋅ {B}_{0} = \left({B}_{0}\times {m}_{i}\right) ⋅ B$ (S8-19)

According to equations (S8-18) and (S8-19), in order for the condition to hold for all magnetic fields B, it is necessary that: ${B}_{0} \times {m}_{i} = 0$ (S8-20)

Thus, it follows that either **m*ᵢ*** ∥ **B₀** or **m*ᵢ*** = 0. Since a magnetic soft robot 1 must be magnetic, not all **m*ᵢ*** can be zero simultaneously. Based on the above analysis, for *S*₁ ⊆ *S*₂ to hold, it is required that: $\left.{m}_{i}\right‖ {B}_{0} , ∀ i$ (S8-21)

In other words, *S*₁ ⊆ *S*₂ occurs if and only if all ***mᵢ*** are parallel to **B₀**. If at least one ***mᵢ*** is not parallel to **B₀**, then some torque distributions generated by Method 1 will not lie in *S*₂, i.e., *S*₁ ⊈ *S*₂.

Similarly, let us assume that *S*₂ ⊆ *S*₁, i.e., every torque distribution generated by Method 2 can also be achieved by Method 1. According to the spatial description and discussion in the previous section, Method 1 generates torque distributions by altering the external magnetic field, and its dimension is $dim \left({S}_{1}\right) \leq 3$ (S8-22) whereas the torque space generated by Method 2 has dimension $dim \left({S}_{2}\right) = 2 n$ (S8-23)

In order for *S*₂ ⊆ *S*₁ to hold, it is necessary that $dim \left({S}_{2}\right) \leq dim \left({S}_{1}\right)$ (S8-24) i.e., $2 n \leq 3$ (S8-25)

Since n is a positive integer (representing the number of magnetic units 52, 62), the inequality 2*n* ≤ 3 can only be satisfied when *n* = 1. Therefore, *S*₂ ⊆ *S*₁ holds only for n = 1. When *n* ≥ 2, the dimension of *S*₂ exceeds that of *S*₁, which implies that *S*₂ ⊈ *S*₁.

Based on the above analysis of the intersection and inclusion relationships between *S*₁ and *S*₂, we conclude that *S*₁ ∩ *S*₂ ≠ Ø, *S*₁ ⊈ *S*₂,and *S*₂ ⊈ *S*₁.Thus, the relationship between *S*₁ and *S*₂ is that they partially overlap without one being a subset of the other.

### VIII.2.2 Relationship between the torque spaces generated by method 3 and those generated by methods 1 and 2

According to the description of the torque spaces generated by the three methods in section VIII.1 above, the torque space generated by Method 1 is ${S}_{1} = \left\{\left.\left({τ}_{1},…,{τ}_{n}\right)\right|∃B∈{ℝ}^{3},{τ}_{i}={m}_{i}\times B \left(∀i\right)\right\}$ (S8-26)

The torque space generated by Method 2 is ${S}_{2} = \left\{\left.\left({τ}_{1},…,{τ}_{n}\right)\right|∃\left({m}_{1}^{′},…,{m}_{n}^{′}\right)∈{\left({ℝ}^{3}\right)}^{n},{τ}_{i}={m}_{i}^{′}\times {B}_{0} \left(∀i\right)\right\}$ (S8-27)

The torque space generated by Method 2 can be expressed as ${S}_{3} = \left\{\left.\left({τ}_{1},…,{τ}_{n}\right)\right|∃B∈{ℝ}^{3},∃\left({m}_{1}^{′},…,{m}_{n}^{′}\right),{τ}_{i}={m}_{i}^{′}\times B, ∀i\right\}$ (S8-28)

For the relationship between the torque spaces *S*₁ and *S*₃, we first consider and analyze *S₃*. In the set *S*₃ (see Equation (S8-28)), since the magnetic field **B** can take any value in ${ℝ}^{3}$, we choose it to be the same variable magnetic field as in *S*₁. Simultaneously, we set ${m}_{i}^{′} = {m}_{i}$, and then, within *S*₃ we have ${τ}_{i} = {m}_{i}^{′} \times B = {m}_{i} \times B , ∀ i$ (S8-29)

This demonstrates that every torque distribution generated by Method 1 can also be produced by Method 3, i.e., *S*₁ ⊆ *S*_{3∘}

Similarly, for the relationship between the torque spaces *S*₂ and *S*₃, we again consider and analyze *S₃*. In the set *S*₃, since the magnetic field **B** can assume any value in ${ℝ}^{3}$, we set it to the fixed magnetic field **B₀** used in *S*₂. Again, let ${m}_{i}^{′} = {m}_{i}$ and then, within *S*₃ we obtain ${τ}_{i} = {m}_{i}^{′} \times B = {m}_{i} \times {B}_{0} , ∀ i$ (S8-30)

This shows that every torque distribution generated by Method 2 can also be achieved by Method 3, i.e., *S*₂ ⊆ *S*₃.

In summary, the torque spaces generated by Methods 1 and 2 can both be produced by Method 3, i.e., *S*₁ ⊆ *S*₃, *S*₂ ⊆ *S*₃. When the variable magnetic field in *S*₃ is set to be the same as the variable magnetic field used in *S*₁, and the variable magnetic moments in *S*₃ are fixed to be identical to those in *S*₁, then *S*₃ degenerates into *S*₁. Similarly, when the variable magnetic field in *S*₃ is set to the fixed magnetic field used in *S*₂, and the variable magnetic moments in *S*₃ are chosen to be identical to those in *S*₂, then *S*₃ degenerates into *S*₂. The relationship between them can be expressed as follows: ${S}_{1} ⊆ {S}_{3} , {\left.{S}_{3}\right|}_{{m}_{i}^{′} = {m}_{i}} = {S}_{1}$ (S8-31) ${S}_{2} ⊆ {S}_{3} , {\left.{S}_{3}\right|}_{B = {B}_{0}} = {S}_{2}$ (S8-32)

### VIII.3 Generalization

The analysis above examined the relationships among the force-torque spaces generated by the three methods under a uniform magnetic field 5. We now extend the discussion to the case of a general (non-uniform) magnetic field 5. Similarly, we assume that the robot 1 is equipped with *n* magnetic units 52, 62, where the *i-*th magnetic unit 52, 62 is located at ${r}_{i} ∈ {ℝ}^{3}$ and possesses a magnetic moment ${m}_{i} ∈ {ℝ}^{3}$. Because a non-uniform magnetic field 5 is influenced not only by external control but also by spatial position, we define the magnetic field 5 at a given point as **B**(**r*ᵢ***, Ψ), where Ψ denotes the set of externally adjustable parameters that modulate the magnitude and direction of the magnetic field 5. Here, $Ψ ∈ {ℝ}^{k}$, with *k* representing the number of parameters for adjusting the external magnetic field 5 (which may include, for example, current, the number of coil turns, etc.). Under a non-uniform magnetic field 5, the robot 1 experiences both forces and torques. Therefore, the force acting on the *i*-th magnetic unit is given by: ${F}_{i} = \left({m}_{i}⋅∇\right) B \left({r}_{i}, Ψ\right)$ (S8-33) ${τ}_{i} = {m}_{i} \times B \left({r}_{i}, Ψ\right)$ (S8-34)

Thus, the force-torque distribution for the *i*-th magnetic unit 52, 62 is given by $\left({F}_{i}, {τ}_{i}\right) ∈ {ℝ}^{6}$ (S8-35)

The overall force-torque distribution for a soft robot 1 comprising n magnetic units 52, 62 is denoted by $\left({F}_{1},{τ}_{1},…,{F}_{n},{τ}_{n}\right) ∈ {ℝ}^{6 n}$ (S8-36)

For Method 1, where only the external magnetic field **B** is varied (i.e., by altering the parameter Ψ), and analogous to the analysis for a uniform magnetic field 5 above, we obtain that the force-torque space generated by Method 1 is given by ${S}_{1}^{′} = \left\{\left.\left({F}_{1},{τ}_{1},…,{F}_{n},{τ}_{n}\right)\right|∃Ψ∈{ℝ}^{k},{F}_{i}=\left({m}_{i}⋅∇\right)B\left({r}_{i}, Ψ\right),{τ}_{i}={m}_{i}\times B\left({r}_{i}, Ψ\right), ∀i\right\}$ (S8-37)

By contrast, in Method 2, the magnetic field **B** is held constant (i.e., **B**(**r*ᵢ***,Ψ₀)) while the magnetic moments ${m}_{i}^{′}$ of each magnetic unit 52, 62 are varied, yielding a force-torque space given by ${S}_{2}^{′} = \left\{\left({F}_{1},{τ}_{1},…,{F}_{n},{τ}_{n}\right)\left|\begin{matrix}∃ \left({m}_{1}^{′},…,{m}_{n}^{′}\right) ∈ {\left({ℝ}^{3}\right)}^{n} , \\ {F}_{i} = \left({m}_{i}^{′}⋅∇\right) B \left({r}_{i}, {Ψ}_{0}\right) , {τ}_{i} = {m}_{i}^{′} \times B \left({r}_{i}, {Ψ}_{0}\right) , ∀ i\end{matrix}\right.\right\}$ (S8-38)

Similarly, in Method 3, both the external magnetic field **B** and the magnetic moments ${m}_{i}^{′}$ are adjusted, which leads to a force-torque space represented as ${S}_{3}^{′} = \left\{\left({F}_{1},{τ}_{1},…,{F}_{n},{τ}_{n}\right)\left|\begin{matrix}∃ Ψ ∈ {ℝ}^{k} , ∃ \left({m}_{1}^{′},…,{m}_{n}^{′}\right) ∈ {\left({ℝ}^{3}\right)}^{n} \\ {F}_{i} = \left({m}_{i}^{′}⋅∇\right) B \left({r}_{i}, Ψ\right) , {τ}_{i} = {m}_{i}^{′} \times B \left({r}_{i}, Ψ\right) , ∀ i\end{matrix}\right.\right\}$ (S8-39)

Based on Equations (S8-37) and (S8-38), the intersection of the force-torque spaces generated by Methods 1 and 2 must simultaneously satisfy the following conditions: $\left({m}_{i}⋅∇\right) B \left({r}_{i}, Ψ\right) = \left({m}_{i}^{′}⋅∇\right) B \left({r}_{i}, {Ψ}_{0}\right)$ (S8-40) ${m}_{i} \times B \left({r}_{i}, Ψ\right) = {m}_{i}^{′} \times B \left({r}_{i}, {Ψ}_{0}\right)$ (S8-41)

In the equations above, **m*ᵢ*** is fixed, ${m}_{i}^{′}$ is adjustable, **B**(**r*ᵢ***,Ψ) is variable, and (**r*ᵢ***,Ψ₀) is fixed.

Since **B**(***rᵢ***, Ψ) and **B**(***rᵢ***, Ψ₀) can represent any magnetic field 5, to prove whether ${S}_{1}^{′}$ and ${S}_{2}^{′}$ have a nonempty intersection, it suffices to show that for a particular choice of **B**(**r*ᵢ***,Ψ) and **B**(**r*ᵢ***,Ψ₀) the intersection ${S}_{1}^{′} ∩ {S}_{2}^{′}$ is nonempty. Assume that both **B**(***rᵢ***, Ψ) and **B**(**r*ᵢ***,Ψ₀) are uniform magnetic fields 5. In this case, the two methods degenerate to the scenario discussed in section VIII.2.1 above. As we proved in VIII.2.1 that Methods 1 and 2 have a nonempty intersection under a uniform magnetic field 5, it follows that ${S}_{1}^{′} ∩ {S}_{2}^{′} \neq ∅$.

Regarding the inclusion relationship between ${S}_{1}^{′}$ and ${S}_{2}^{′}$, we first assume that ${S}_{1}^{′} ⊆ {S}_{2}^{′}$. This assumption implies that every force and torque in ${S}_{1}^{′}$ has a corresponding force and torque in ${S}_{2}^{′}$. In other words, the following conditions must be simultaneously satisfied: $∀ Ψ , ∀ i , \left({m}_{i}⋅∇\right) B \left({r}_{i}, Ψ\right) ∈ {S}_{2}^{′}$ (S8-42) $∀ Ψ , ∀ i , {m}_{i} \times B \left({r}_{i}, Ψ\right) ∈ {S}_{2}^{′}$ (S8-43)

If either of these conditions fails, then it necessarily follows that ${S}_{1}^{′} ⊈ {S}_{2}^{′}$. Because the magnetic field 5 can be chosen arbitrarily, we set it as a uniform magnetic field 5. Under this assumption, the above requirement reduces to the sole condition given by Equation (S8-43). In section VIII.2.1, we demonstrated that under a uniform magnetic field (i.e., when only considering Equation (S8-43)), the torque space generated by Method 1 is not a subset of that generated by Method 2. Therefore, even when considering only Equation (S8-43), it holds that ${S}_{1}^{′} ⊈ {S}_{2}^{′}$, and consequently, when both Equations (S8-42) and (S8-43) are taken into account, we must have ${S}_{1}^{′} ⊈ {S}_{2}^{′}$.

Similarly, using section VIII.2.1 as a special case, we can also prove that ${S}_{2}^{′} ⊈ {S}_{1}^{′}$. In summary, given that ${S}_{1}^{′} ∩ {S}_{2}^{′} \neq ∅ , {S}_{1}^{′} ⊈ {S}_{2}^{′}$, and ${S}_{2}^{′} ⊈ {S}_{1}^{′}$, we conclude that the relationship between ${S}_{1}^{′}$ and ${S}_{2}^{′}$ is characterized by partial overlap, with neither space being a subset of the other.

Regarding the relationship between ${S}_{1}^{′}$ and ${S}_{3}^{′}$, we prove as follows. In Method 1, $\left({F}_{1},{τ}_{1},…,{F}_{n},{τ}_{n}\right) ∈ {S}_{1}^{′}$, since ${S}_{1}^{′}$ is achieved by adjusting the external magnetic field 5, there exists a Ψ* such that for all magnetic units, ${F}_{i} = \left({m}_{i}⋅∇\right) B \left({r}_{i}, {Ψ}^{∗}\right)$ (S8-44) ${τ}_{i} = {m}_{i} \times B \left({r}_{i}, {Ψ}^{∗}\right)$ (S8-45)

In Method 3, since the magnetic field 5 can be chosen arbitrarily, we select the same varying magnetic field as in Method 1, i.e., Ψ = Ψ*; moreover, because the magnetic moments in Method 3 are also adjustable, we choose them to be identical to those in Method 1, i.e., ${m}_{i}^{′} = {m}_{i}$. Under these selections, all forces and torques achievable by Method 1 (i.e., Equations (S8-44) and (S8-45)) can also be produced by Method 3. The force-torque space ${S}_{1}^{′}$ obtained by Method 1 is simply a special case of the force-torque space ${S}_{3}^{′}$ produced by Method 3, and we conclude that ${S}_{1}^{′} ⊆ {S}_{3}^{′}$.

Similarly, Method 3 can be configured by choosing the same fixed magnetic field as used in Method 2, and by selecting the variable magnetic moments to be identical to those in Method 2. In this case, the force-torque space obtained by Method 2 is simply a special case of that generated by Method 3, i.e., ${S}_{2}^{′} ⊆ {S}_{3}^{′}$.

In summary, similar to the case of a uniform magnetic field 5, under a general magnetic field 5, the force-torque spaces generated by Methods 1 and 2 partially overlap and neither is contained within the other; moreover, the force-torque spaces produced by Methods 1 and 2 are both subsets of the force-torque space generated by Method 3; and under certain conditions, Method 3 can degenerate into either Method 1 or Method 2. Specifically, the details are as follows: ${S}_{1}^{′} ⊆ {S}_{3}^{′} , {\left.{S}_{3}^{′}\right|}_{{m}_{i}^{′} = {m}_{i}} = {S}_{1}^{′}$ (S8-46) ${S}_{2}^{′} ⊆ {S}_{3}^{′} , {\left.{S}_{3}^{′}\right|}_{B = {B}_{0}} = {S}_{2}^{′}$ (S8-47)

The relationships among the force-torque spaces produced by the three methods are illustrated in an Euler diagram, as shown in Fig. 142.

### IX. Design of Magnetization Profile

The deformation of soft robots 1 under a magnetic field 5 depends on both the external magnetic field 5 and the intrinsic magnetization profile. Common configurations and deformations of 1D, 2D, and 3D structures are presented in Figs. 2 to 48, covering widely used paradigms that can accommodate most application needs. However, to establish a more general design approach, the following provides three design methods as references for broader design applications: Forward Design Method, Inverse Design Method, and Template-Based Design Method. Since the deformation of a 1D tube serves as the foundation for 2D and 3D structures, the following analysis focusses on the deformation of a 1D soft tube, while the deformation of 2D and 3D structures can be extended based on the 1D case.

### IX.1 Forward Design Method

For relatively simple deformation designs, empirical knowledge or an exhaustive search approach can be used. Once the design is completed, theoretical analysis or simulations are employed before fabrication or testing to validate and refine the design, ensuring feasibility. At this stage, the specific magnetization profile of the body 10 formed as a soft tube is determined, allowing the Forward Design Method to be applied. By utilizing the known magnetization profile, applied magnetic field 5, and other given parameters, the potential deformations can be predicted.

The detailed computational method is provided in section II above. In this approach, the pseudo-rigid-body model is adopted, approximating the soft tube as a system composed of multiple rigid rods. Gravitational potential energy, magnetic potential energy, and elastic potential energy are considered in formulating the total potential energy function. If additional external factors are present, they can also be incorporated into the analysis. The total potential energy function can be expressed as: $V = {U}_{m} + {U}_{g} + {U}_{e} + {U}_{o}$ (S10-1)
where *Uₘ* represents the magnetic potential energy of the system;
*U_{g}* means the gravitational potential energy of the system;
*Uₑ* represents the elastic potential energy of the system;
*Uₒ* is the potential energy contributed by other possible external factors.

Based on the principle of minimum potential energy, the fmincon function in MATLAB can be used to determine the joint angles *θ* between rigid rods, thereby obtaining the final deformation of the soft tube. Alternatively, the modelling approach described above can be employed, using ABAQUS to simulate the deformation of soft tubes, 2D sheets, or 3D bodies. The Forward Design Method is used to solve the forward problem, where the computation is relatively simpler compared to the Inverse Design Method, and the solution is generally unique.

### IX.2 Inverse Design Method

Compared to the Forward Design Method, the Inverse Design Method requires less prior experience from the designer but poses greater mathematical challenges. The following section introduces the approach for determining the magnetization profile of a soft tube forming the body 10 using the Inverse Design Method.

When solving the forward problem, the pseudo-rigid-body model is employed, and the principle of minimum potential energy is used to determine the joint angles between rigid rods. A similar approach can be applied to solving the inverse problem. The body 10 is assumed to consist of *n* rigid rods, each containing a magnetic unit with a variable magnitude and direction. The direction of each magnetic unit is influenced by the position of its corresponding rod. Consequently, the magnetic moment of each unit can be expressed as: ${m}_{i} = {m}_{i} \left(χ, θ\right)$

Here, *χ* represents the magnetization parameter of the magnetic units, defined as *χ =* (*χ₁, ... , χₙ*), which are the unknown parameters to be identified. *θ* denotes the joint angles between the rigid rods, given by *θ* = (*θ₁,* ... , *θₙ*). Based on the analysis in section II, the total magnetic potential energy of the soft tube can be directly obtained as: ${U}_{m} \left(χ, θ\right) = - {\sum }_{i = 1}^{m} {m}_{i} \left(χ, θ\right) ⋅ {B}_{i}$ (S10-3) where **B*ᵢ*** represents the magnetic field acting on the *i*^{th} magnetic unit. Similarly, the elastic potential energy and gravitational potential energy of the soft tube can be expressed as: ${U}_{e} \left(θ\right) = \frac{2 n}{L} {\sum }_{i = 1}^{n} {E}_{i} {I}_{i} {θ}_{i}^{2}$ (S10-4) ${U}_{g} \left(θ\right) = - \frac{1}{2} {\sum }_{i = 1}^{n} {m}_{i}^{m} \left({p}_{i}^{f}-{p}_{i}^{i}\right) ⋅ g$ (S10-5)
where *E_{¡}* represents the modulus of elasticity of the *i*^{th} rod;
*Iᵢ* is the moment of inertia of the cross-section of the *i*^{th} rod;
${m}_{i}^{m}$ represents the weight of the *i*^{th} magnetic unit;
${p}_{i}^{f}$ means the initial position vectors of the *i*^{th} rod;
${p}_{i}^{i}$ is the final position vectors of the *i*^{th} rod;
***g*** is the acceleration due to gravity.
${p}_{i}^{i}$ and ${p}_{i}^{f}$ can be determined based on the joint angles between the rigid rods. For detailed calculations, please refer to section II.

If the body 10 is influenced by additional factors that may affect either the magnetization of the magnetic units or the joint angles between the rigid rods, these effects are denoted as *Uₒ*(*χ,θ*). Thus, the total potential energy of the soft tube system can be expressed as: $V \left(χ, θ\right) = {U}_{m} \left(χ, θ\right) + {U}_{g} \left(θ\right) + {U}_{e} \left(θ\right) + {U}_{o} \left(χ, θ\right)$ (S10-6)

A specific magnetization parameter *χ* is first assigned to the equation, and a search is conducted within the *θ* space to determine *θ**(*χ*), which minimizes the total potential energy. *θ**(*χ*) can be expressed as: $θ * \left(χ\right) = arg {min}_{θ} V \left(χ, θ\right)$ (S10-7)

By solving the above equation, the magnetization parameter *χ* is mapped to the equilibrium deformation parameter *θ**(*χ*). In the pseudo-rigid-body model, the deformation of the body 10 is governed by the joint angles *θ* between the rigid rods. To ensure that the obtained deformation matches the target deformation, functions are defined as: $Ω \left(χ\right) = {\left‖θ*\left(χ\right)-{θ}^{target}\right‖}^{2}$ (S10-8) $χ * = arg {min}_{χ} Ω \left(χ\right)$ (S10-9)
where *θ^{target}* represents the set of joint angles in the target deformation;
*χ** denotes the magnetization parameter that minimizes Ω(*χ*).

In the above computation, the smaller Ω(*χ*) is, the better. As it approaches zero, the obtained solution θ*(*χ*) becomes increasingly close to the target value *θ^{target}.* However, in practical applications, achieving an exact zero is unnecessary as long as the deformation meets the application requirements. A smaller Ω(*χ*) leads to higher deformation control accuracy but significantly increases computational cost. Therefore, instead of minimizing Ω(*χ*) indefinitely, it is sufficient to ensure that it meets a predefined threshold *δ*, given by: $Ω \left(χ\right) \leq δ$ (S10-10) where *δ* represents the threshold for the error between the actual deformation and the target deformation, ensuring that it meets application requirements.

We set Equation (S10-7) as the inner function while defining Equation (S10-8) as the outer function. A numerical iteration method is used to solve the equations iteratively. In the inner function, a search is conducted to determine *θ**(χ), while in the outer function, Ω(*χ*) is optimized. For each iteration, the computed joint angles are compared with the desired angles, and the distribution of the magnetization profile is adjusted accordingly. After multiple iterations, the joint angles of all rods gradually converge to the expected values, ultimately yielding a magnetization profile that meets the required criteria.

During the optimization process, the magnetization parameter *χ* may experience abrupt variations, leading to an unsmooth magnetization distribution or numerically extreme solutions. To obtain a feasible solution, regularization can be applied to the outer optimization function. In this case, Equation (S10-8) can be extended as: ${Ω}_{reg} \left(χ\right) = Ω \left(χ\right) + μρ \left(χ\right)$ (S10-11) where *µ* is the coefficient of the regularization term, and *ρ*(*χ*) represents the regularization function.

The coefficient µ is used to adjust the weight of the regularization term, while *ρ*(*χ*) is introduced to constrain excessive variations in the magnetization parameter *χ.* The specific form of *ρ*(*χ*) can be determined based on practical requirements. For instance, to prevent excessive fluctuations in magnetization, Tikhonov Regularization can be introduced. By penalizing the sum of the squared magnetization parameters, the optimization process becomes more stable and avoids excessively large numerical values. This can be expressed as: $ρ \left(χ\right) = {\left‖χ\right‖}^{2}$ (S10-12)

Another example is that, due to manufacturing constraints, the maximum magnetization magnitude of certain substrate materials may be significantly limited, requiring an upper bound on the magnetization magnitude. This can be enforced using Physical Constraints to restrict its maximum value. The corresponding regularization term can be expressed as: $ρ \left(χ\right) = \sum max {\left(0,\left|{χ}_{j}\right|-{χ}_{max}\right)}^{2}$ (S10-13)

Where *χₘₐₓ* represents the maximum allowable value of the magnetization parameter. Other regularization methods, such as Total Variation Regularization, can also be introduced to impose different types of constraints on the function.

The bi-level optimization approach described above is relatively simple and easy to implement, making it suitable for problems of smaller scale. For extensive computations, a more efficient approach is to optimize *θ* and *χ* simultaneously, avoiding the need for nested iterations. When solving the Inverse Design Method, obtaining a valid solution is not always guaranteed, and the solution may not be unique. The approach outlined above provides a fundamental framework for implementing the Inverse Design Method. However, numerous studies have been dedicated to addressing inverse problems (*6, 7*), and these methods continue to evolve. Existing advancements in inverse problem-solving techniques can be referenced for further improvements.

### IX.3 Template-Based Design Method

### IX.3.1 Design Steps

Among the previously discussed design methods, both the Forward Design Method and the Inverse Design Method require accounting for the interactions among various parameters, such as the magnetization profile, magnetic field, elasticity, and gravity, which can be rather cumbersome. A simpler alternative is the Template-Based Design Method, which employs a template, such as the jig 810, to facilitate the design process, thereby directly bypassing the interdependent constraints among the parameters.

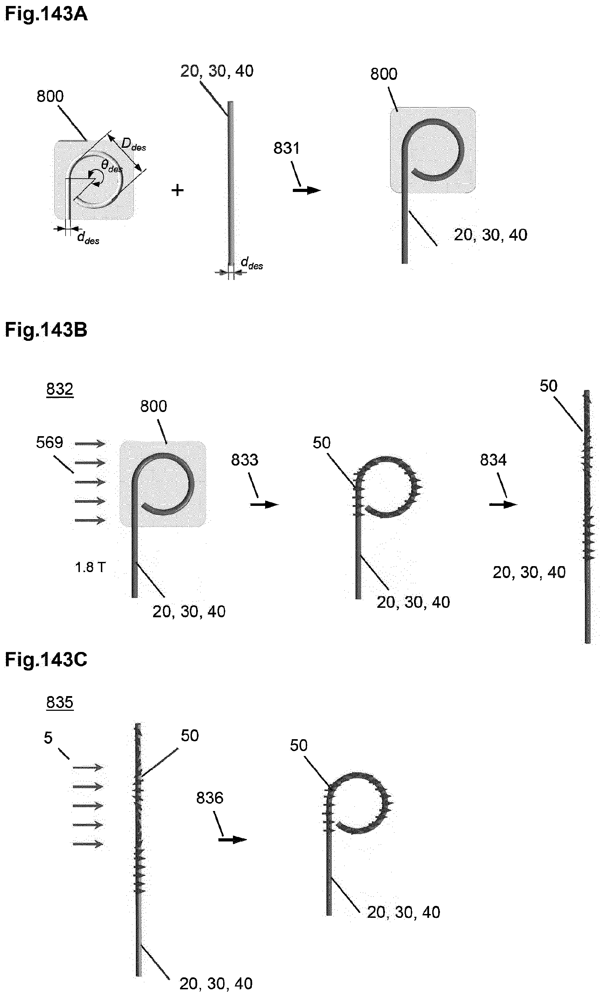

Fig. 143 depicts the template-based magnetization profile design method. As shown in Fig. 124A, according to the requirements, a template 800 and an outer or inner part 20, 30, 40, configured, for example, as a tube or a rod, are fabricated with predetermined parameters and the part 20, 30, 40 is mounted (831) onto the template 800 to fix its shape. In Fig. 143B, the part 20, 30, 40 assembled within the template 800 is then magnetized (832), for example by placing it in a magnetization field 569 for magnetization; after magnetization 832, the template 800 is removed (833) to yield the part 20, 30, 40 with a predefined magnetization profile given by a magnetic element 50, which elastically recovers after removing the template 800. In Fig. 143C, the part 20, 30, 40 with the predefined magnetization profile of the magnetic element 50 is placed (835) in a uniform magnetic field 5, where it deforms (836) magnetically driven in a manner determined by the template 800.

The specific design steps for the Template-Based Design Method are as follows:

### (1) Tube fabrication

Fabricate the part 20, 30, 40, such as the outer part 20, the inner part 30 or one of the further inner parts 40, for example as a tube or rod with the desired diameter *d_{des}* and length *L_{des}* according to the specific requirements. The part 20, 30, 40, may be made from a composite of a soft material and magnetic particles, with the soft material (e.g., PDMS or Ecoflex) serving as the substrate material. The amount of magnetic particles can be adjusted based on the required magnetization magnitude; typical mixing ratios and corresponding magnetization magnitudes are shown in Fig. 141. The fabrication methods for the tube (or rod) are illustrated in Figs. 106 and 107.

### (2) Template fabrication

Create an appropriate template 800, such as the jig 810, based on the specific application requirements. For instance, if a circular deformation is desired, design the template 800 using parameters of the circular shape, including the desired median diameter *D_{des},* spanning angle *θ_{des},* and tube diameter *d_{des}* (see Fig. 143A).

### (3) Magnetization process

Mount (831) the part 20, 30, 40 within the template 800 and magnetize (832) it, for example by placing it in a strong magnetization field 569 for magnetization. The part 20, 30, 40 is forced to conform to the shape defined by the template 800 (e.g., circular), and during magnetization 832, the magnetic units 52, 62 within the curved part 20, 30, 40 align parallel to the magnetization field 569 (see Fig. 143B).

### (4) Template removal and profile acquisition

Remove (833) the part 20, 30, 40 from the template to obtain the desired magnetization profile. After the template 800 is removed, the part 20, 30, 40, under its own elastic recovery, recovers (834) to a straight configuration while retaining a specific magnetization profile that varies with the axial distance (see Fig. 143B).

The magnetized part 20, 30, 40 is then placed (835) in a uniform magnetic field 5 and it deforms (836) to a shape determined by the template 800, for example it may become a ring (Fig. 143C).

In this design process, one approach is to mix magnetic particles forming magnetic units 52, 62 with the fluid 589 forming a substrate material and subsequently magnetize the composite to obtain the magnetization profile. Alternatively, permanent magnets may be employed. Using magnetic particles for magnetization is relatively straightforward, although its magnetization magnitude tends to be lower. For instance, PDMS may be used as the substrate material, for example with a mass ratio of 3:1 (magnetic particles to PDMS). Experimental observations and empirical evidence indicate that a mass ratio of 3:1 (magnetic particles to PDMS) offers a good compromise between material pliability and a relatively high magnetization magnitude. Under these conditions, the average magnetization magnitude is approximately 1.88×10⁵A/m, see Fig. 141. Alternatively, N50 or N52 permanent magnets mey be used. N50 or N52 permanent magnets can easily achieve a magnetization magnitude on the order of 10⁶A/m. Additionally, alternative substrate materials 589 may be used, such as the Ecoflex series. The proportion of magnetic particles 52, 62 that can be incorporated then may be lower, resulting in a magnetization magnitude significantly below 1.88x10⁵A/m.

The magnetic particles may be replaced with permanent magnets as magnetic units 52, 63. This can yield a considerably higher magnetization magnitude, which either enables larger deformations or reduces the requirement for high external magnetic field strengths. However, this approach may introduce increased design complexity. When employing permanent magnets instead of magnetic particles, a process similar to that depicted in Fig. 143 is followed: a fabricated part 20, 30, 40, devoid of magnetic particles, is installed in the template 800 and then magnetized within the magnetization field 569. The key difference is that the part 20, 30, 40 has pre-designated position for mounting the permanent magnets serving as the magnetic units 52, 62. In this case, only a small magnetization field 569 (generally below 20 mT) may be used to reorient the permanent magnets, and the magnetization field 569 is maintained until the material securing the permanent magnets cures.

### IX.3.2 Parameter Tuning Methods

The parameter tuning method enables the design of templates 800 with arbitrary deformation shapes according to specific deformation requirements, thereby achieving the desired deformation. For example, in Figs. 26 to 28, a helical deformation is obtained by employing a cylindrical template 800 featuring spiral grooves. This approach is simple and practical, requiring minimal theoretical or design expertise.

However, it is noted that the deformations achieved by this method are inherently gradual. For instance, a square template may be used to produce a square deformation. Then, even though the magnetization directions align with a square configuration, the part 20, 30, 40 may not exhibit a perfect square deformation when being subjected to the magnetization field 569. This discrepancy may occur because the magnetization profile undergoes abrupt changes at the square's corners, while the bending deformation of the part 20, 30, 40 transitions gradually, leading to a mismatch. The primary drawback of this method may be the need for post-process tuning. Even if the magnetization profile is gradual, factors such as gravity and the inherent elasticity of the part 20, 30, 40 may cause the resulting deformation to deviate from the template shape, necessitating adjustments based on the specific design. For example, in Figs. 26 to 28, the template 800 formed by the jig 810 has a helical diameter 812 of Dₘ = 12 mm and a pitch 814 of P = 0.3 mm, yet the deformation achieved with this template 800 is consistently larger than these dimensions. The following outlines tuning strategies, categorized by external influencing factors, using helical deformation as an illustrative example.

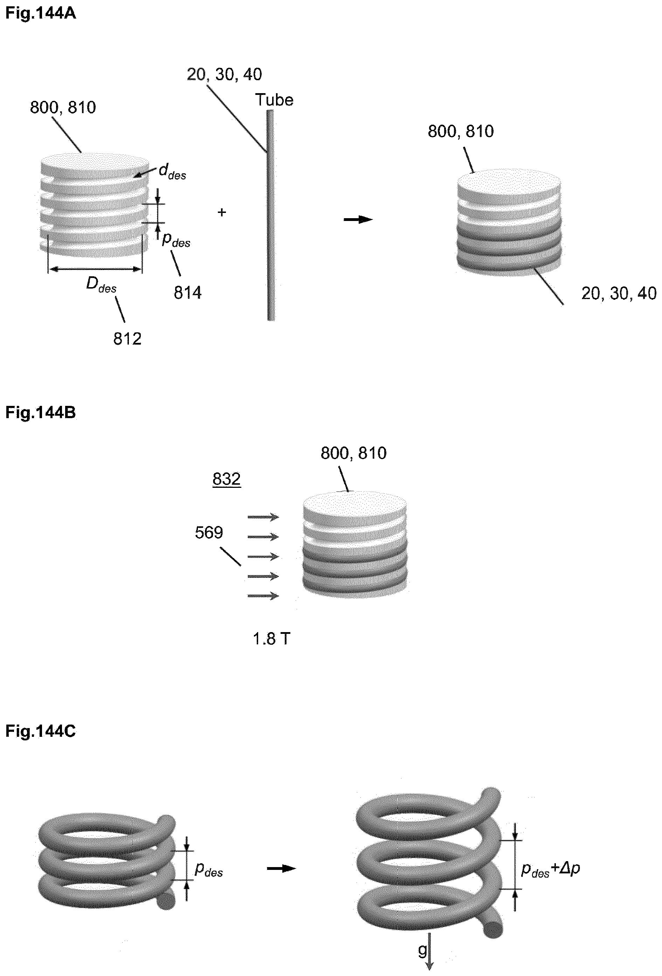

Fig. 144 depicts magnetization and analysis of helical elongated parts 20, 30, 40, such as tubes. According to Fig. 144A, a helical magnetization profile of the part 20, 30, 40 is obtained using a template 800, such as the jig 810 of Fig. 27. The template 800 and the part 20, 30, 40 are first fabricated with predetermined parameters, and the part 20, 30, 40 is wound and mounted (831) onto the template 800. In Fig. 144B, the part 20, 30, 40, mounted to the template 800, is then magnetized (832), exemplarily by placing it in a strong magnetization field 569 for magnetization to generate the desired magnetization profile. In Fig. 144C, under the influence of gravity g and the magnetic field 5, the part 20, 30, 40 deforms into a helical shape having an increase in pitch p_{des} + Δp compare to the pitch p_{des} of the template 800.

### (1) Effects of Gravity

As shown in Fig. 144, a template 800 with a helical diameter *D_{des},* a pitch *p_{des},* and a cross-sectional diameter *d_{des}* of a helical groove on its outer surface may be used to magnetize the part 20, 30, 40. Here, *D_{des}, p_{des}*, and *d_{des}* represent the desired helical outer diameter, pitch, and groove cross-sectional diameter, respectively. The part 20, 30, 40 may be oriented vertically along the helical axis during operation (as illustrated in Fig. 144C). Then, the effect of gravity g induces a moment **M^{g}** that causes the helical tube's pitch to change from *p_{des}* to *p_{des}* + Δ*p,* wherein Δ*p* denotes the deviation from the desired pitch and can be expressed as $Δ p = ω \left(\sum {M}_{i}^{g}\right)$ (S10-14) where *ω* is the function describing the relationship between the soft tube's pitch variation and the bending moment (which depends on the physical properties and geometric characteristics of the soft tube), and ${M}_{i}^{g}$ is the gravitational moment acting on the *i*^{th} magnetic unit.

To reduce or eliminate this pitch error *Δp,* the following three methods can be adopted.

### (i) Increasing the magnetic field strength

Enhance the magnetic field 5 from the originally specified value **B** to **B** + Δ**B** so that the magnetic moment acting on the part 20, 30, 40 is augmented, thereby compensating for the deformation induced by the gravitational moment. For a uniform magnetic field 5, when the magnetic field strength is increased from its predetermined value, the pitch error is given by: $Δ p = ω \left[\sum {M}_{i}^{g}-\sum \left({m}_{i}\times ΔB\right)\right]$ (S10-15) where **m*ᵢ*** represents the magnetic moment of the *i*^{th} magnetic unit. As the magnetic field strength increases, the pitch error decreases directly. It is noted that, in the above equation, even if the magnetic field-generated moment exceeds the gravitational moment, the pitch error will not become negative. This is because the magnetization profile designed by the Template-Based Design Method progressively approaches the shape defined by the template 800 (Fig. 144B) as the magnetic field 5 increases, rather than inverting to a reversed helix.

This method is the simplest and most direct; however, it may have drawbacks. Increasing the magnetic field 5 may be constrained by the external magnetic field generation system and specific application conditions, so the magnetic field strength may not be increased indefinitely. Moreover, gravity also exerts a stretching effect on the part 20, 30, 40 along the direction of gravity g, which may not be compensated by the magnetic field 5. If error tolerances are not particularly stringent or if there are no strict requirements on the magnetic field strength, this method can be employed to achieve the desired deformation in a simple and rapid manner.

### (ii) Increasing magnetization magnitude

This approach is analogous to increasing the magnetic field strength 5. Since the force or moment generated by a magnetic field 5 depends on both the magnetization and the magnetic field 5, the magnetization and/or the magnetic field 5 may be enhanced. Enhancement of either parameter can counteract gravitational deformation by introducing a larger force or moment to balance gravity. The magnetic moment of an individual magnetic unit 52, 62 may be increased from **m*ᵢ*** to **m*ᵢ*** + Δ**m*ᵢ***. Then, the pitch error can be expressed as: $Δ p = ω \left[\sum {M}_{i}^{g}-\sum \left({Δm}_{i}\times B\right)\right]$ (S10-16)

During the fabrication of the part 20, 30, 40, the magnetic particle content may be increased. This can elevate the magnetization magnitude, as a higher proportion of magnetic particles directly results in greater magnetization magnitude (see Fig. 141B). However, it is important to note that a higher magnetic particle content also may lead to a significant increase in the tube's weight due to the high density of the particle. This creates a trade-off that may be carefully balanced based on the specific geometric parameters. To address the additional weight introduced by increasing the number of magnetic particles, a magnetic material with a high magnetization-to-weight ratio may be employed (i.e., a material where the increase in magnetization magnitude outweighs the added weight). Permanent magnets may offer an excellent solution in this regard. However, as previously discussed, their use amy introduce additional design complexity and challenges. Ultimately, magnetic particles, permanent magnets, or a combination of both may be employed as magnetic units 52, 62 according the present disclosure and a specific a choice may be determined by the specific application requirements.

### (iii) Reverse pitch compensation design

With the reverse pitch compensation design, the template's pitch P_{des} is intentionally designed to be smaller than the desired pitch - that is, reduced from *p_{des}* to *p_{des}* - Δ*p.* During operation, gravity g induces an additional pitch change of *p_{g}.* By setting the reverse compensation value Δ*p* equal to *p_{g},* the resulting pitch becomes: ${p}_{f} = {p}_{des} + {p}_{g}$ (S10-17) ${p}_{f} = {p}_{des} - Δ p + Δ p = {p}_{des}$ (S10-18)

As shown by the above equation, the final pitch will be *p_{des},* precisely meeting the operational requirements. This design leverages the elastic recovery 834 of the part 20, 30, 40 to counteract the gravitational effect, thereby achieving the desired deformation. Although this method is relatively simple and straightforward, it requires multiple tuning iterations to reliably attain the expected pitch *p_{des}.*

The three methods described above pertain to the specific tuning procedures when the part 20, 30, 40 is oriented vertically. In practical applications, the part 20, 30, 40 can be positioned arbitrarily, and similar tuning strategies based on the aforementioned principles can be applied accordingly.

### (2) Effects of Elasticity

In the Template-Based Design Method, the part 20, 30, 40 is initially deformed into various shapes by the template 800. Upon removal of the template 800, the principle of minimum potential energy drives the part 20, 30, 40 to gradually revert toward its original configuration (for instance, if the part 20, 30, 40 is fabricated using the dip coating method, it will naturally tend to straighten). Consequently, the greater the deviation of the designed deformation from the original shape, the higher the associated elastic potential energy. When the part 20, 30, 40 is subsequently placed in the magnetic field 5 to induce deformation, a corresponding force or moment is required to overcome this elastic resistance.

Taking helical deformation as an example, a part 20, 30, 40 may be produced via the dip coating method and may be magnetized using a template 800 with a designed outer diameter *D_{des},* pitch *p_{des},* and groove cross-sectional diameter *d_{des}.* Due to the elasticity of the part 20, 30, 40, both the pitch and the outer diameter will deviate from their designed values, changing from *p_{des}* to *p_{des}* + Δ*p* and from *D_{des}* to *D_{des}* + Δ*D,* respectively. To compensate for the increased pitch error Δ*p* and outer diameter error Δ*D,* the same three methods previously discussed for reducing gravitational effects can be employed. Specifically, the pitch error can be reduced by increasing the magnetic field strength, increasing the magnetization magnitude, or implementing a reverse compensation design for the pitch. In terms of addressing the outer diameter error, besides enhancing the magnetic field strength and magnetization magnitude, a reverse compensation strategy analogous to that used for the pitch - namely, a reverse compensation design for the helical diameter - can be applied.

In the reverse compensation design for the helical diameter, the designed helical diameter is reduced from *D_{des}* to *D_{des}* - Δ*D.* Similarly, when the part 20, 30, 40 is placed in the magnetic field 5, its inherent elasticity causes its diameter to spontaneously increase, thereby compensating for the diameter error Δ*D.* However, this compensation may not always be feasible because, as the helical diameter decreases from *D_{des},* the elastic potential energy rises rapidly. Beyond a certain threshold, the elastic potential energy may exceed the compensatory capacity of the magnetic force or moment. For example, with the present disclosure, a template 800, 810 with an outer diameter of 4 mm, a pitch of 2 mm, and a groove cross-sectional diameter of 1.9 mm was used to magnetize a soft tube fabricated from PDMS (exemplarily using a 10:1 PDMS formulation as the fluid 589 providing the substrate material, with magnetic particle mixed in a 3:1 ratio by weight, yielding a part 20, 30, 40 with a diameter of 1.9 mm, configured as a straight tube). However, even when employing a magnetic field several times stronger than the originally specified value (300 mT), the desired deformation could not be achieved. This failure is attributed to the fact that transforming the part 20, 30, 40 from its initial configuration to the preset helical shape requires storing an excessive amount of elastic potential energy, which the magnetic field 5 cannot provide sufficient work to overcome.

In such cases, an alternative approach is to reduce the elastic potential energy. The elastic potential energy *Uₑ* is positively correlated with both the elastic modulus *E* and the strain *s,* as expressed in the following equation: ${U}_{e} ∝ E$ (S10-19) ${U}_{e} ∝ {s}^{2}$ (S10-20)

Based on these relationships, the elastic modulus of the part 20, 30, 40 may be reduced, thereby lowering its elastic potential energy. An alternative strategy is to reduce the deformation magnitude. The part 20, 30, 40 may be fabricated with an initial shape that has a predetermined shape differing from straight shape, such as a helical shape or an approximately helical shape. Then, the relative deformation during the final actuation will be smaller, thus reducing the required elastic potential energy to overcome, and enabling the desired deformation. However, this approach results in an initial configuration that is deformed, such as helical (or near-helical), rather than straight, so whether it satisfies specific operational requirements may be carefully evaluated through detailed design.

### (3) Effects of Other Factors

The previous discussion specifically addressed design strategies to mitigate the effects of gravity g and inherent elasticity. In practical applications, additional influencing factors may also be present. If these factors are static, similar compensation strategies - akin to those used for gravity and elasticity - can be incorporated during the design stage. By accounting for their impact on the deformation magnitude in the preliminary design, these static influences can be readily eliminated. Conversely, if the influencing factors are dynamic, pre-design compensation may be insufficient. In such cases, a method may include determining at least one predetermined adjustment parameter of the body 10, such as specific adjustment parameters, based on the dynamic environment of the body 10, and to perform a real-time adjustment of the magnetic field 5 and/or magnetization of the body 10 to set the predetermined adjustment parameter of the body 10.

Several applications presented in this study involve similar dynamic operations, albeit not strictly falling under this category. These examples are mentioned here to illustrate the concept of dynamic compensation. For instance, in Application I - contact-free object navigation, as the body 10 formed as a soft tube enters and exits a blood vessel 441, its relative position with respect to the vessel 441 changes. Consequently, the radius of curvature of the body 10 may form the adjustment parameter that is adjusted in real time to accommodate these positional variations. This may be achieved by dynamically altering the magnetic field 5 (Fig. 65). Similarly, in Application II - reprogrammable cilia array 300, the bending amplitude of the cilia 310 may form the adjustment parameter that may be adjusted to meet operational demands and the real-time adjustment may comprise altering the magnetization profile of the cilia 310, for example by moving the magnetic member 16 formed by the inner part 30 (Fig. 82).

The methods described above for compensating for various influencing factors provide relatively simple and direct tuning strategies that enable the rapid attainment of the desired deformation. However, if higher precision is required, more accurate tuning may become necessary. In such cases, one approach is to combine the Template-Based Design Method with the Forward Design Method outlined in section IX.1, thereby allowing for precise calculations of these influencing factors and achieving the desired deformation with higher accuracy.

### IX.4. Discussion

By employing the Forward Design Method, the Inverse Design Method, and the Template-Based Design Method, the magnetization profile required for a specific deformation can be obtained. For different functional requirements, multiple deformations are designed, namely, ${F}_{i} = g \left({D}_{i}\right) , i ∈ \left\{1,2,3,…\right\}$ (S10-21) where *Fᵢ* corresponds to distinct functional requirements, *g* is the mapping function between functionality and deformation, and *Dᵢ* represents different deformations. Each deformation can be realized through a unique magnetization profile: ${D}_{i} = h \left({MP}_{i}\right) , i ∈ \left\{1,2,3,…\right\}$ (S10-22) where *h* is the mapping function from the magnetization profile to the resulting deformation, and *MPᵢ* denotes different magnetization profiles. With the present disclosure, the magnetization profile of the body 1, which may be a soft tube, can be modified in real time to obtain various *MPᵢ.* To achieve different deformations *Dᵢ* by dynamically altering the magnetization profiles *MPᵢ,* it may be essential to consider how these different profiles can be implemented through the nesting of multiple parts 20, 30, 40, each of which may be configured as a soft tube, and variations in their positions, as well as the transitions between various deformations. The primary challenge may lie in the design of the body 10 and its parts 20, 30, 40, which may depend on design experience. This may require the empirical development of diverse magnetization profiles, various configuration schemes, and switching mechanisms to enable transitions among the desired deformations.

However, it may be difficult to provide a comprehensive, systematic summary of such design experience. Instead, numerous design cases, as shown in Figs. 2 to 49 are presented, covering the current widely used paradigms, which are believed to encompass most practical application scenarios. Each case includes detailed design strategies and experimental demonstrations of specific deformations, thereby serving as both a reference for practical applications and an inspiration for future design endeavours.

### X. Analysis of Multi-Magnetic Field Interference

For operations requiring a large working space within a Magnetic Interaction Zone, one approach involves designing large-scale magnetic field generators 100 to achieve the desired magnetic fields. However, such large-scale magnetic field generators 100 may significantly increase equipment costs, thus restricting the broader application and promotion of magnetically controlled technologies. To address this issue, the multi-magnetic field control method proposed in section I may be implemented, which involves spatially combining multiple small-scale magnetic fields 435, 436, 437, 438 to replace a single large-scale magnetic field 5 for controlling soft robots 1. When utilizing multiple small-scale magnetic fields 435, 436, 437, 438 to control different regions of soft robots 1, these smaller fields 435, 436, 437, 438 may be strategically arranged in space. This spatial arrangement may lead to interference among the fields 435, 436, 437, 438, with overlapping regions potentially producing undesired magnetic fields at certain locations. Therefore, we conducted simulation analyses on the interference among multiple magnetic fields 435, 436, 437, 438 to provide practical references for implementing the multi-magnetic field control method. In our application involving long-range operations with a large Magnetic Interaction Length (as shown in Fig. 126), we employed magnetic field generators 100, 101 configured as Halbach arrays to generate the required magnetic fields. Accordingly, we selected the Halbach array as our simulation model and carried out simulations using COMSOL Multiphysics software (version 6.2, COMSOL Inc., Sweden).

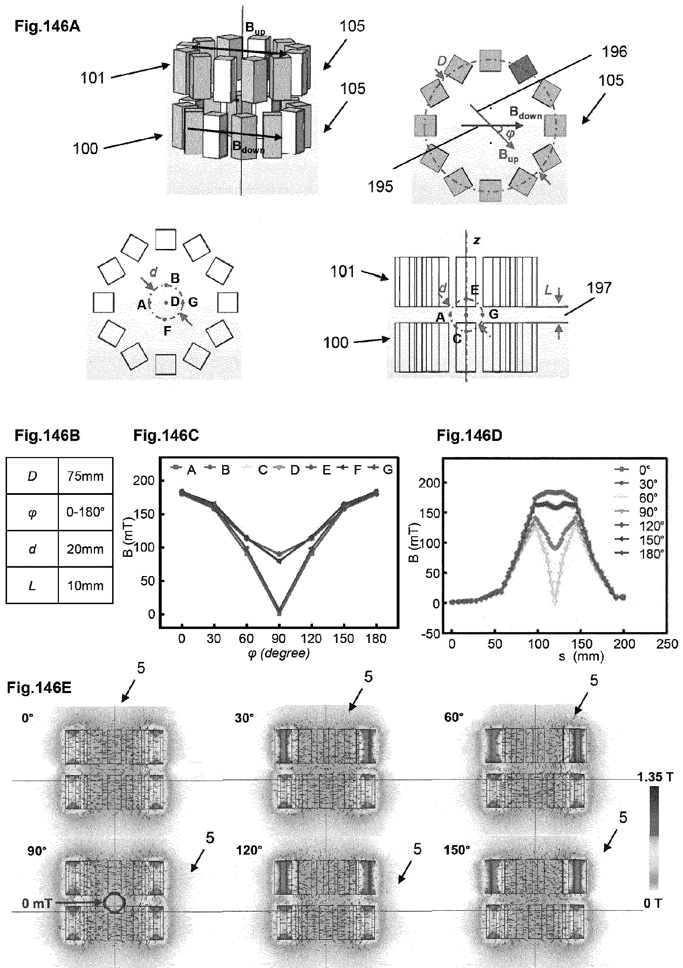

As shown in Figs. 146 to 149, the magnetic field generators 100, 101 may have permanent magnets as field sources, such as neodymium permanent magnet, like N52-grade neodymium permanent magnets. For example, the magnetic field generators 100, 101 each may have twelve field sources. The field sources may be arranged in a circular configuration having a diameter. Each permanent magnet may be rectangular in shape and, for example, measure 12 mm × 12 mm × 30 mm. For example, the permanent magnets may be arranged in a Halbach configuration.

Two magnetic field generators 100, 101 may be employed to generate two magnetic fields. The magnetic field generators 100, 101 may be identical. The field sources of a first magnetic field generator 100 may be centered in a first field plane and the field sources of a second magnetic field generator 101 may be centered in a second field plane. The field sources of the first magnetic field generator 100 form first field sources and the field sources of the second magnetic field generator 101 form second field sources.

The magnetic field of the first magnetic field generator 100 may have a first direction 195 in the first field plane and the magnetic field of the second magnetic field generator 101 may have a second direction 196 in the second field plane.

The first field plane and the second field plane may be spaced apart from each other by a magnetic field spacing 197 in a direction perpendicular to the field planes. The first and second directions 195, 196 of the magnetic fields in the first and second field plane may have an angle *φ* with respect to each other. The angle *φ* may be different from zero.

The first field plane and the second field plane may be parallel to each other. With other embodiments, the first field plane and the second field plane may have a magnetic field plane angle *α* with respect to each other. The magnetic field plane angle *α* may be different from zero.

The following embodiment comprises twelve N52-grade neodymium permanent magnets, each rectangular in shape and measuring 12 mm × 12 mm × 30 mm. The magnets are arranged in a magnet arrangement, which is a circular configuration following the Halbach array pattern, and the magnet arrangement has a distribution circle with a diameter D. The diameter D defines the distance of the individual field sources formed by the permanent magnets from the magnetic interaction zone at the central region of the arrangement.

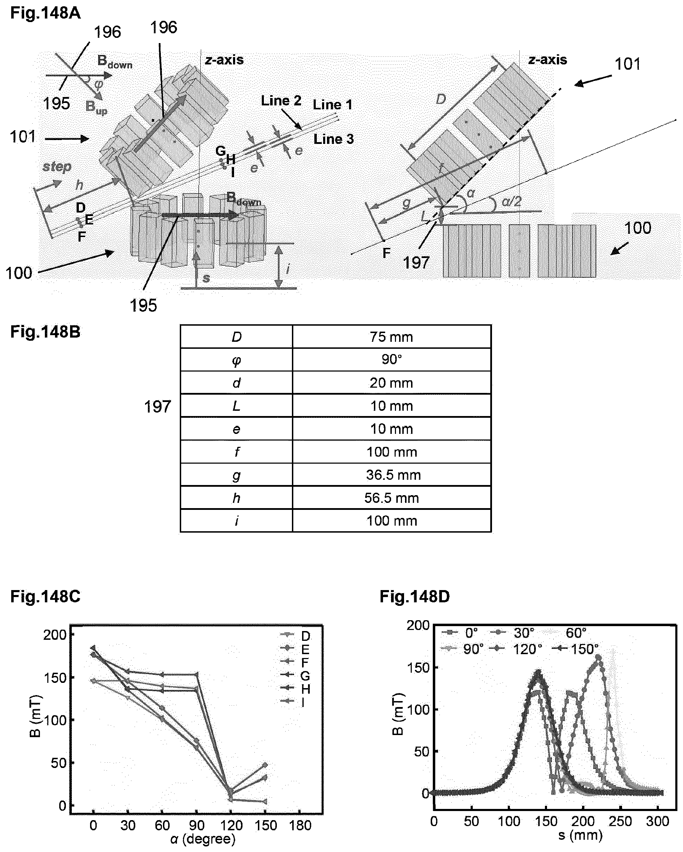

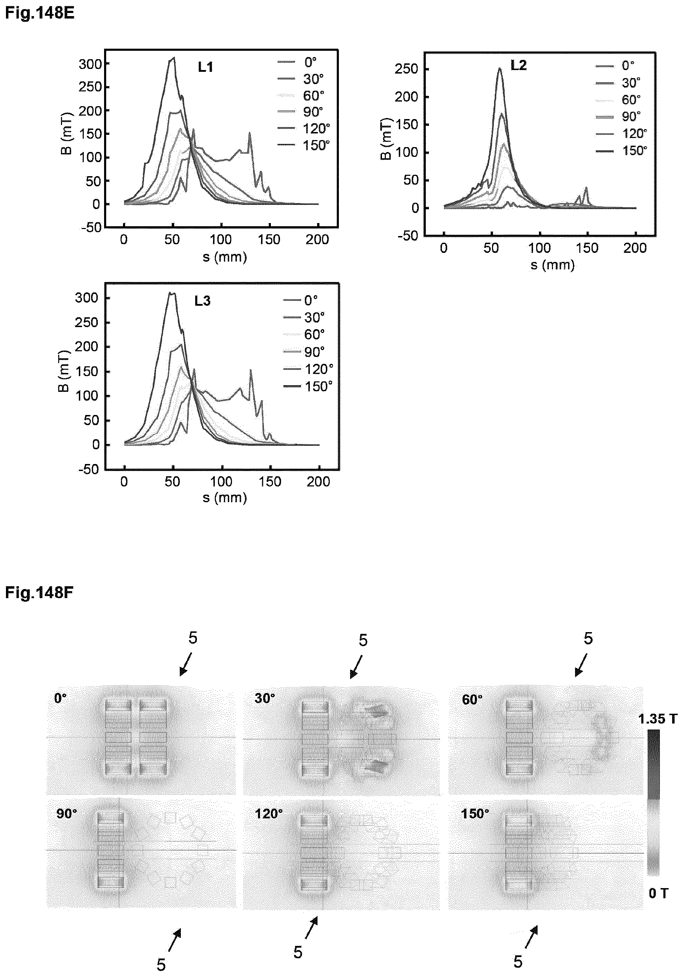

Two identical Halbach arrays were employed to generate two magnetic fields, designated as the upper magnetic field (**B***ᵤₚ*) and lower magnetic field (**B***_{down}*), respectively, as depicted in Fig. 145A. The angle between **B***ᵤₚ* and **B***_{down}* is denoted as *φ,* and the distance between the end faces of the two Halbach arrays is denoted as L (defined here as the magnetic field spacing). While the lower Halbach array remains fixed, the upper Halbach may be rotated around an axis perpendicular to the plane of the paper, with an origin located at a specific quadrant point on its end face. The angle formed between the end faces of these two Halbach arrays during this rotation is defined as the magnetic field plane angle (*α*), as illustrated in Fig. 148.

Fig. 145 depicts the influence of the diameter D of the magnet distribution circle of the magnet arrangement 105 on magnetic field intensity in different regions. Fig. 145A is a schematic diagram showing dimensions of the magnet arrangement 105. The magnet arrangement 105 comprises a multitude of permanent magnets 102. As shown, the magnet arrangement 105 may be configured as a Halbach array. The illustration includes specific dimensions of the magnet arrangement 105 and the positions of sampling points. Fig. 145B depicts a variation of the magnitude B of the magnetic field 5 at the different sampling points with varying diameter D of the magnet distribution circle in the Halbach array. Fig. 145C depicts a variation of the magnitude B of the magnetic field along the z-axis for different diameter D of the magnet distribution circle in the Halbach array. Fig. 145D depicts the magnitude B of the magnetic field in the z = 150 mm plane for different diameter D.

Fig. 145 shows, for a single magnetic field, how the diameter of the magnet distribution circle D influences the magnetic field strength given by the magnitude B of the magnetic field 5 generated by the magnetic field generator 100 comprising the magnet arrangement 105. The diameter D was increased incrementally from an initial value of 60 mm, with increments of 5 mm, until the magnetic field intensity in the central region approached approximately 0 mT. Seven sampling points (labeled A-G) and one sampling line (along the z-axis) were selected, with their specific positions illustrated in Fig. 145A. As depicted in Fig. 145B-144C, the magnetic field intensity B gradually decreases as the diameter D of the magnet distribution circle increases. When the diameter *D* of the magnet distribution circle reaches 150 mm, the magnetic field intensity in the central region approaches nearly zero.

Subsection I.4.2.2 above discussed the possibility of designing an adjustable device capable of varying the diameter D of the magnet distribution circle within the Halbach array, thereby enabling control over the magnets and consequently altering the magnitude of the magnetic field. The simulation results provide theoretical support for this approach. Therefore, the soft robot 1 may employ such an adjustable device to achieve effective control of the magnetic field strength.

Fig. 145D presents magnetic field intensity distributions for various diameters D of the magnet distribution circle using magnetic flux lines. It is evident that a smaller diameter D of the magnet distribution circle corresponds to denser magnetic flux lines, thus indicating a stronger magnetic field 5. Additionally, Fig. 145C illustrates the magnetic field's influence on surrounding regions. Along the z-axis, at distances greater than 50 mm from the Halbach array center, the magnetic field intensity effectively reduces to 0 mT. This distance may serve as a reference for the safe separation required to prevent interference from a single Halbach array with non-magnetic regions, and as a valuable parameter for future design considerations.

Fig. 146 depicts the impact of the magnetic field angle on the magnetic field in different regions. Fig. 146A is a schematic of the two magnetic field generators 100, 101, exemplarily configured as two Halbach arrays, and sampling points. Two magnetic fields are generated by the two Halbach arrays, whose end faces are parallel. The magnetic field generated by the upper Halbach array is denoted as Bᵤₚ, and that of the lower Halbach array as B_{down}. The angle φ represents the angle between Bᵤₚ and B_{down}. Fig. 146B depicts numerical values of the relevant parameters in Fig. 146A. Fig. 146C depicts the magnitude B of the magnetic field at various sampling points A, B, C, D, E, F, G in Fig. 146A for different magnetic field angles φ. Fig. 146D depicts the magnitude B of the magnetic field 5 along the z-axis for different magnetic field angles *φ* within a sampling interval along the z-axis in Fig. 145A. In Fig. 145A, the starting point s = 0 mm of the sampling interval is located at z = -150 mm downward along the central axis from the center of the lower Halbach array, and the starting point used in Fig. 146D is at z = -120 mm. Fig. 146E depicts the magnetic field distribution under different magnetic field angles φ.

To explore how varying the magnetic field angle *φ* affects the magnetic field 5 in different regions, *φ* may be set from 0° to 180° in increments of 30°. The diameter of the magnet distribution circles *D* in both Halbach arrays was set to 75 mm, and the magnetic field spacing *L* between the two arrays was fixed at 10 mm. Additionally, the magnetic field plane angle *α* was set to 0°, meaning the two end faces were parallel. Seven sampling points (points A-G) and one sampling line (along the z-axis) were chosen, with their specific positions detailed in Fig. 146A. According to the simulation results (Figs. 145C-145D), as *φ* increased from 0° to 90°, the magnetic field intensity, given by the magnitude B of the magnetic field 5, gradually decreased. conversely, as *φ* increased from 90° to 180°, the intensity progressively increased. When the magnetic field angle reached 90°, the magnetic field intensity in the central region achieved its minimum, with an area exhibiting zero magnetic intensity. As illustrated by the magnetic flux lines in Fig. 146E, at *φ* = 90°, no magnetic flux lines appear in a certain central region, indicating a magnetic field strength of approximately 0 mT. Therefore, the two magnetic fields may be arranged spatially such that their angle is close to 90°, such as 90°. This minimizes the field intensity in transitional regions between them and effectively reduces interference with non-target control areas. Moreover, as depicted in Fig. 146D, regardless of the magnetic field angle *φ,* the magnetic field intensity drops significantly to a minimal value (approximately 20 mT) at distances greater than 60 mm from the Halbach array center. At distances exceeding 80 mm, the magnetic intensity approaches nearly zero and can thus be considered negligible. This distance can serve as a reference "safe distance" beyond which external regions remain unaffected by the Halbach arrays.

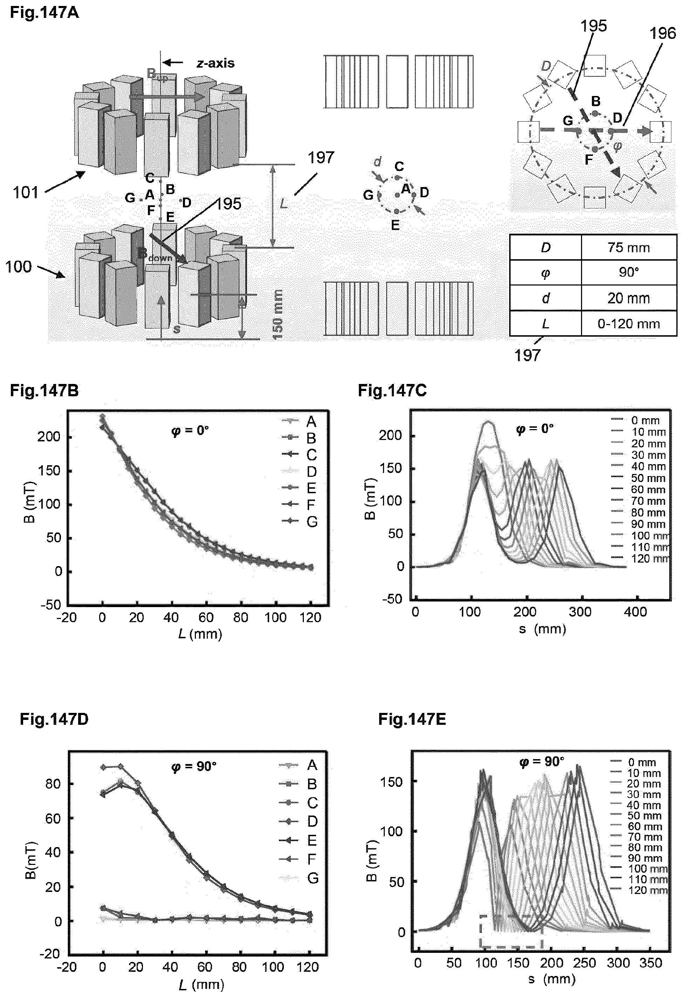

Fig. 147 depicts the impact of the magnetic field spacing L of the two magnetic field generators 100, 101 on the magnetic field in different regions. Fig. 147A is a schematic of the arrangement of the two magnetic field generators 100, 101, exemplarily configured as Halbach arrays, and positions of the sampling points. Two magnetic fields are generated by the two Halbach arrays, whose end faces are parallel. The magnetic field spacing L represents the distance between the end faces of the two Halbach arrays (magnetic field spacing). φ represents the angle between Bᵤₚ and B_{down}, that is, between the central field planes of the magnetic field 5 generators 100, 101. Fig. 147B depicts the magnitude B of the magnetic field 5 as a function of the magnetic field spacing L at various sampling points shown in Fig. 147A if φ is 0°. Fig. 147C depicts the magnitude B of the magnetic field 5 along the z-axis if φ is 0° for different values of the magnetic field spacing L (color coded). Here, s denotes a distance from the starting point of the sampling interval upward along the z-axis in Fig. 147A. Fig. 147D depicts the magnitude B of the magnetic field 5 as a function of the magnetic field spacing L at various sampling points when φ is 90°. Fig. 147E depicts the magnitude B of the magnetic field 5 along the z-axis when φ is 90° for different values of the magnetic field spacing L (color coded).

We examined the influence of varying the magnetic field spacing *L* (distance between the two Halbach array end-faces) on the magnetic field across different regions. The magnetic field spacing *L* was increased incrementally, starting from 0 mm and increasing by increments of 10 mm, until the resulting magnetic field intensity approached approximately 0 mT. The diameter *D* of the magnet distribution circles for both Halbach arrays was fixed at 75 mm. The magnetic field plane angle *α* was set to 0°, meaning parallel end faces of the two Halbach arrays. The magnetic field angle *φ* was tested at two values: 0° and 90°. Sampling points and a sampling line (along the z-axis) used in the analysis are shown in Fig. 147A. As demonstrated in Fig. 147B, simulation results indicate that when the magnetic field angle *φ* is 0°, magnetic field intensity at all points in the central region decreases with increasing magnetic field spacing *L,* reaching approximately 0 mT for all central points at an *L* of 120 mm. Regarding magnetic field intensity variation along the z-axis (Fig. 147C), at smaller magnetic field spacings (e.g., 0 mm or 10 mm), there is only a single peak, indicating the continuous presence of a magnetic field between the two Halbach arrays; the magnetic field intensity reduces to zero outside the arrays only at distances greater than 70 mm from the center. With increasing magnetic field spacing *L,* two peaks start to emerge, implying the appearance of a region with reduced magnetic intensity between the arrays. When the magnetic field spacing *L* reaches 110 mm, a magnetic-field-free region appears in the central area. In contrast, as illustrated in Fig. 147D, when the magnetic field angle *φ* is set at 90°, several points in the central region continuously exhibit a near-zero magnetic field intensity, regardless of the magnetic field spacing *L.* Correspondingly, Fig. 147E shows that along the *z*-axis, all curves consistently exhibit two distinct peaks separated by valleys with magnetic intensities approaching zero (as indicated by the red boxes in Fig. 147E), irrespective of changes in *L.*

Therefore, when arranging magnetic fields, the magnetic field spacing *L* can be suitably increased within functional constraints to reduce interference in the central region. However, this reduction has limitations. As a more effective strategy, the magnetic field angle *φ* may be set to 90°. Similar to the earlier analysis of magnetic field angle *φ* on magnetic field, setting *φ* to 90° maintains the magnetic field intensity at nearly zero in the central region. This characteristic can thus serve as a critical parameter to minimize mutual interference between multiple magnetic fields.

Fig. 148 depicts the impact of the magnetic field plane angle *α* on the magnetic field in different regions. Fig. 148A is a schematic showing the arrangement of the two magnetic field generators 100, 101, exemplarily configured as Halbach arrays, and positions of the sampling points. The two magnetic fields are generated by the Halbach arrays, with the angle between their magnetic field planes (i.e., the end faces of the arrays) being α. Fig. 148B shows numerical values of the relevant parameters in Fig. 148A. Fig. 148C depicts the magnitude B of the magnetic field 5 at various sampling points for different magnetic field plane angles α. Fig. 148D depicts the magnitude B of the magnetic field 5 along the z-axis as a distance s from the start of the sampling interval at z = -100 mm for different magnetic field plane angles *α.*

Fig. 148E depicts the magnitude B of the magnetic field 5 along Lines 1, 2, and 3 in Fig. 148A. Here, s denotes the distance from the starting point of a sampling interval along the axis of Lines 1, 2, and 3 in Fig. 148A. Fig. 148F depicts the magnitude B of the magnetic field 5 for different magnetic field plane angles α.

Fig. 148 depicts the effects of varying the magnetic field plane angle *α* on the magnetic field 5 in different regions. The diameter *D* of the magnet distribution circles for both Halbach arrays was set at 75 mm, the magnetic field angle *φ* was fixed at 90°, and the magnetic field spacing *L* was fixed at 10 mm. The magnetic field plane angle *α* was varied from 0° to 150° in increments of 30°. Six sampling points (points D-I) and four sampling lines (*z*-axis, lines 1, 2, and 3) were selected, with their locations and parameters shown in Figs. 147A and 147B. As shown in Fig. 148C, for sampling points located on the inner side (points G, H, I) and outer side (points D, E, F) of the Halbach arrays, the magnetic field intensity remains relatively high at smaller magnetic field plane angles *α.* However, when *α* exceeds approximately 60° or 90°, a sharp decrease in magnetic intensity occurs. The variation of magnetic intensity along the *z*-axis (Fig. 148D) also follows this pattern, with the intensity profile transitioning from two distinct peaks to a single peak as *α* increases. Fig. 148E presents the magnetic intensity variations along lines 1, 2, and 3. We observed that the magnetic intensity reaches its maximum near the Halbach array end-face plane (i.e., near the rotation origin of the upper Halbach array), due to the proximity of this location to the magnets in both arrays. On line 2, regardless of the changes in the magnetic field plane angle, magnetic intensities remain consistently low on both the inner and outer sides of the Halbach arrays and approach approximately zero beyond a short distance of about 35 mm. On lines 1 and 3, when the magnetic field plane angle *α* exceeds 90°, magnetic intensities on both sides of the arrays behave similarly to line 2. In contrast, when *α* is smaller than 90°, the magnetic field intensity is essentially zero on the outer side but remains high on the inner side of the arrays. These simulation results illustrate how different magnetic field plane angles *α* influence the magnetic field in various spatial regions. In practical applications, a larger magnetic field plane angle *α* may be selected. This may be advisable to reduce the magnetic field's interference in both inner and outer regions. Nevertheless, even at smaller magnetic field plane angles *α*, it is possible to identify specific regions with relatively low magnetic interference to satisfy particular application requirements.

In summary, the foregoing analyzed the effect of the diameter *D* of the magnet distribution circle on the magnetic field intensity for a single magnetic field. Additionally, the foregoing evaluated the influence of magnetic field angle *φ,* magnetic field spacing *L,* and magnetic field plane angle *α* on the magnetic field intensity across various regions. Although the analysis is not exhaustive - only selected sampling points and sampling lines were studied, rather than all possible regions - the investigated areas are representative of typical application scenarios. Furthermore, the associated parameters and observed trends provided herein can serve as practical references for specific magnetic field configurations. These results are readily applicable to designs where high control precision is not strictly required. For scenarios demanding greater accuracy, more detailed simulation studies can be conducted following the methodologies and insights established in this analysis. Additionally, the simulations focused solely on the interference between two magnetic fields. Due to space constraints, the foregoing did not conduct a detailed analysis of interference among multiple magnetic fields. However, using the methodology outlined in the present disclosure, further simulations can be conducted as needed to investigate the interactions of multiple magnetic fields.

The multi-magnetic field control method proposed in section I, as well as the foregoing study of interference of magnetic fields generated by two magnetic field generators 100, 101 is not limited to the use of magnetic field generators 100, 101 configured as Halbach arrays. According to the present disclosure, the magnetic field generators 100, 101 may also comprise other field sources, such as electromagnetic coils. Since the long-range operation with a large Magnetic Interaction Length setup presented in this disclosure exemplarily utilizes Halbach arrays, the foregoing simulations were conducted exclusively on this configuration. Nevertheless, the same approach can be applied to analyze the interference of magnetic fields generated by other field sources, such as electromagnetic coils.

### References

1. M. Li, A. Pal, A. Aghakhani, A. Pena-Francesch, M. Sitti, Soft actuators for real-world applications. Nat Rev Mater 7, 235-249 (2022).
2. W. Hu, G. Z. Lum, M. Mastrangeli, M. Sitti, Small-scale soft-bodied robot with multimodal locomotion. Nature 554, 81-85 (2018).
3. M. Cianchetti, C. Laschi, A. Menciassi, P. Dario, Biomedical applications of soft robotics. Nat Rev Mater 3, 143-153 (2018).
4. B. J. Nelson, I. K. Kaliakatsos, J. J. Abbott, Microrobots for Minimally Invasive Medicine. Annual Review of Biomedical Engineering 12, 55-85 (2010).
5. J. Burgner-Kahrs, D. C. Rucker, H. Choset, Continuum Robots for Medical Applications: A Survey. IEEE Transactions on Robotics 31, 1261-1280 (2015).
6. M. Sitti, H. Ceylan, W. Hu, J. Giltinan, M. Turan, S. Yim, E. Diller, Biomedical Applications of Untethered Mobile Milli/Microrobots. Proceedings of the IEEE 103, 206-224 (2015).
7. T. Ranzani, M. Cianchetti, G. Gerboni, I. D. Falco, A. Menciassi, A Soft Modular Manipulator for Minimally Invasive Surgery: Design and Characterization of a Single Module. IEEE Transactions on Robotics 32, 187-200 (2016).
8. A. Sadeghi, A. Mondini, B. Mazzolai, Toward Self-Growing Soft Robots Inspired by Plant Roots and Based on Additive Manufacturing Technologies. Soft Robotics 4, 211-223 (2017).
9. J. Rossiter, H. Hauser, Soft Robotics - The Next Industrial Revolution? [Industrial Activities]. IEEE Robotics & Automation Magazine 23, 17-20 (2016).
10. G. Li, X. Chen, F. Zhou, Y. Liang, Y. Xiao, X. Cao, Z. Zhang, M. Zhang, B. Wu, S. Yin, Y. Xu, H. Fan, Z. Chen, W. Song, W. Yang, B. Pan, J. Hou, W. Zou, S. He, X. Yang, G. Mao, Z. Jia, H. Zhou, T. Li, S. Qu, Z. Xu, Z. Huang, Y. Luo, T. Xie, J. Gu, S. Zhu, W. Yang, Self-powered soft robot in the Mariana Trench. Nature 591, 66-71 (2021).
11. S. Aracri, F. Giorgio-Serchi, G. Suaria, M. E. Sayed, M. P. Nemitz, S. Mahon, A. A. Stokes, Soft Robots for Ocean Exploration and Offshore Operations: A Perspective. Soft Robotics 8, 625-639 (2021).
12. P. A. der Maur, B. Djambazi, Y. Haberthür, P. Hörmann, A. Kübler, M. Lustenberger, S. Sigrist, O. Vigen, J. Förster, F. Achermann, E. Hampp, R. K. Katzschmann, R. Siegwart, "RoBoa: Construction and Evaluation of a Steerable Vine Robot for Search and Rescue Applications" in 2021 IEEE 4th International Conference on Soft Robotics (RoboSoft) (2021), pp. 15-20.
13. C. Li, G. C. Lau, H. Yuan, A. Aggarwal, V. L. Dominguez, S. Liu, H. Sai, L. C. Palmer, N. A. Sather, T. J. Pearson, D. E. Freedman, P. K. Amiri, M. O. de la Cruz, S. I. Stupp, Fast and programmable locomotion of hydrogel-metal hybrids under light and magnetic fields. Science Robotics 5, eabb9822 (2020).
14. S. Wang, Y. Gao, A. Wei, P. Xiao, Y. Liang, W. Lu, C. Chen, C. Zhang, G. Yang, H. Yao, T. Chen, Asymmetric elastoplasticity of stacked graphene assembly actualizes programmable untethered soft robotics. Nat Commun 11, 4359 (2020).
15. B. Jin, H. Song, R. Jiang, J. Song, Q. Zhao, T. Xie, Programming a crystalline shape memory polymer network with thermo- and photo-reversible bonds toward a single-component soft robot. Science Advances 4, eaao3865 (2018).
16. T. Chen, O. R. Bilal, K. Shea, C. Daraio, Harnessing bistability for directional propulsion of soft, untethered robots. Proceedings of the National Academy of Sciences 115, 5698-5702 (2018).
17. M. Kaynak, P. Dirix, M. S. Sakar, Addressable Acoustic Actuation of 3D Printed Soft Robotic Microsystems. Advanced Science 7, 2001120 (2020).
18. Y. Kim, H. Yuk, R. Zhao, S. A. Chester, X. Zhao, Printing ferromagnetic domains for untethered fast-transforming soft materials. Nature 558, 274-279 (2018).
19. Z. Ren, W. Hu, X. Dong, M. Sitti, Multi-functional soft-bodied jellyfish-like swimming. Nat Commun 10, 2703 (2019).
20. B. Gorissen, D. Melancon, N. Vasios, M. Torbati, K. Bertoldi, Inflatable soft jumper inspired by shell snapping. Science Robotics 5, eabb1967 (2020).
21. S. Li, D. M. Vogt, D. Rus, R. J. Wood, Fluid-driven origami-inspired artificial muscles. Proc. Natl. Acad. Sci. U.S.A. 114, 13132-13137 (2017).
22. M. Duduta, E. Hajiesmaili, H. Zhao, R. J. Wood, D. R. Clarke, Realizing the potential of dielectric elastomer artificial muscles. Proceedings of the National Academy of Sciences 116, 2476-2481 (2019).
23. Z. S. Davidson, H. Shahsavan, A. Aghakhani, Y. Guo, L. Hines, Y. Xia, S. Yang, M. Sitti, Monolithic shape-programmable dielectric liquid crystal elastomer actuators. Science Advances 5, eaay0855 (2019).
24. R. Pelrine, R. Kornbluh, Q. Pei, J. Joseph, High-Speed Electrically Actuated Elastomers with Strain Greater Than 100%. Science 287, 836-839 (2000).
25. V. Vikas, E. Cohen, R. Grassi, C. Sözer, B. Trimmer, Design and Locomotion Control of a Soft Robot Using Friction Manipulation and Motor-Tendon Actuation. IEEE Transactions on Robotics 32, 949-959 (2016).
26. Y. Kim, G. A. Parada, S. Liu, X. Zhao, Ferromagnetic soft continuum robots. Sci Robot 4, eaax7329 (2019).
27. G. Z. Lum, Z. Ye, X. Dong, H. Marvi, O. Erin, W. Hu, M. Sitti, Shape-programmable magnetic soft matter. Proceedings of the National Academy of Sciences 113, E6007-E6015 (2016).
28. J. Zhang, Z. Ren, W. Hu, R. H. Soon, I. C. Yasa, Z. Liu, M. Sitti, Voxelated three-dimensional miniature magnetic soft machines via multimaterial heterogeneous assembly. Sci Robot 6, eabf0112 (2021).
29. Y. Alapan, A. C. Karacakol, S. N. Guzelhan, I. Isik, M. Sitti, Reprogrammable shape morphing of magnetic soft machines. Science Advances 6, eabc6414 (2020).
30. X. Kuang, S. Wu, Q. Ze, L. Yue, Y. Jin, S. M. Montgomery, F. Yang, H. J. Qi, R. Zhao, Magnetic Dynamic Polymers for Modular Assembling and Reconfigurable Morphing Architectures. Advanced Materials 33, 2102113 (2021).
31. H. Wen, Y. Sun, R. Liu, L. Li, Q. Cao, Reprogrammable magnetization pattern and shape morphing of phase-change magnetic soft composites. Compos Commun 40, 101618 (2023).
32. H. Song, H. Lee, J. Lee, J. K. Choe, S. Lee, J. Y. Yi, S. Park, J.-W. Yoo, M. S. Kwon, J. Kim, Reprogrammable Ferromagnetic Domains for Reconfigurable Soft Magnetic Actuators. Nano Lett 20, 5185-5192 (2020).
33. Q. Ze, X. Kuang, S. Wu, J. Wong, S. M. Montgomery, R. Zhang, J. M. Kovitz, F. Yang, H. J. Qi, R. Zhao, Magnetic Shape Memory Polymers with Integrated Multifunctional Shape Manipulation. Adv Mater 32, 1906657 (2020).
34. H. Deng, K. Sattari, Y. Xie, P. Liao, Z. Yan, J. Lin, Laser reprogramming magnetic anisotropy in soft composites for reconfigurable 3D shaping. Nat Commun 11, 6325 (2020).
35. J. Cui, T.-Y. Huang, Z. Luo, P. Testa, H. Gu, X.-Z. Chen, B. J. Nelson, L. J. Heyderman, Nanomagnetic encoding of shape-morphing micromachines. Nature 575, 164-168 (2019).
36. M. R. Clary, S. N. Cantu, J. A.-C. Liu, E. E. Evans, J. B. Tracy, Magnetic Reprogramming of Self-Assembled Hard-Magnetic Cilia. Adv Mater Technol-us 9, 2302243 (2024).
37. H. Rafii-Tari, C. J. Payne, G.-Z. Yang, Current and Emerging Robot-Assisted Endovascular Catheterization Technologies: A Review. Ann Biomed Eng 42, 697-715 (2014).
38. M. J. Mack, Minimally Invasive and Robotic Surgery. JAMA 285, 568 (2001).
39. M. Runciman, A. Darzi, G. P. Mylonas, Soft Robotics in Minimally Invasive Surgery. Soft Robot 6, 423-443 (2019).
40. J. Jayender, R. V. Patel, S. Nikumb, Robot-assisted Active Catheter Insertion: Algorithms and Experiments. The International Journal of Robotics Research 28, 1101-1117 (2009).
41. H.-J. Cha, H.-S. Yoon, K. Y. Jung, B.-J. Yi, S. Lee, J. Y. Won, "A robotic system for percutaneous coronary intervention equipped with a steerable catheter and force feedback function" in 2016 IEEE/RSJ International Conference on Intelligent Robots and Systems (IROS) (IEEE, 2016), pp. 1151-1156.
42. X. Bao, S. Guo, N. Xiao, Y. Li, C. Yang, Y. Jiang, A cooperation of catheters and guidewires-based novel remote-controlled vascular interventional robot. Biomed Microdevices 20, 20 (2018).
43. X. Bao, S. Guo, N. Xiao, Y. Li, C. Yang, R. Shen, J. Cui, Y. Jiang, X. Liu, K. Liu, Operation evaluation inhuman of a novel remote-controlled vascular interventional robot. Biomed Microdevices 20, 34 (2018).
44. X. Bao, S. Guo, Y. Guo, C. Yang, L. Shi, Y. Li, Y. Jiang, Multilevel Operation Strategy of a Vascular Interventional Robot System for Surgical Safety in Teleoperation. IEEE T Robot 38, 2238-2250 (2022).
45. X. Bao, S. Guo, C. Yang, L. Zheng, Haptic Interface With Force and Torque Feedback for Robot-Assisted Endovascular Catheterization. IEEE/ASME Trans. Mechatron., 1-15 (2023).
46. Y.-H. Kim, Y.-J. Park, H. In, C. W. Jeong, K.-J. Cho, Design Concept of Hybrid Instrument for Laparoscopic Surgery and Its Verification Using Scale Model Test. IEEE/ASME Trans. Mechatron., 1-1 (2015).
47. G. A. Fontanelli, M. Selvaggio, L. R. Buonocore, F. Ficuciello, L. Villani, B. Siciliano, A New Laparoscopic Tool With In-Hand Rolling Capabilities for Needle Reorientation. Ieee Robot Autom Let 3, 2354-2361 (2018).
48. H. Liu, M. Selvaggio, P. Ferrentino, R. Moccia, S. Pirozzi, U. Bracale, F. Ficuciello, The MUSHA Hand II: A Multi-Functional Hand for Robot-Assisted Laparoscopic Surgery. IEEE/ASME Trans. Mechatron., 1-1 (2020).
49. K.-W. Kwok, K. Hung Tsoi, V. Vitiello, J. Clark, G. C. T. Chow, W. Luk, G.-Z. Yang, Dimensionality Reduction in Controlling Articulated Snake Robot for Endoscopy Under Dynamic Active Constraints. IEEE T Robot 29, 15-31 (2013).
50. J. M. Prendergast, G. A. Formosa, M. J. Fulton, C. R. Heckman, M. E. Rentschler, A Real-Time State Dependent Region Estimator for Autonomous Endoscope Navigation. IEEE T Robot 37, 918-934 (2021).
51. W. Wei, R. E. Goldman, H. F. Fine, S. Chang, N. Simaan, Performance Evaluation for Multi-arm Manipulation of Hollow Suspended Organs. IEEE T Robot 25, 147-157 (2009).
52. A. Ebrahimi, M. G. Urias, N. Patel, R. H. Taylor, P. Gehlbach, I. lordachita, Adaptive Control Improves Sclera Force Safety in Robot-Assisted Eye Surgery: A Clinical Study. IEEE T Bio-med Eng 68, 3356-3365 (2021).
53. H. G. Shin, I. Park, K. Kim, H. K. Kim, W. K. Chung, Corneal Suturing Robot Capable of Producing Sutures With Desired Shape for Corneal Transplantation Surgery. Ieee T Robot 37, 304-312 (2021).
54. D. Kundrat, G. Dagnino, T. M. Y. Kwok, M. E. M. K. Abdelaziz, W. Chi, A. Nguyen, C. Riga, G.-Z. Yang, An MR-Safe Endovascular Robotic Platform: Design, Control, and Ex-Vivo Evaluation. Ieee T Bio-med Eng 68, 3110-3121 (2021).
55. F. Bechet, K. Ogawa, E. Sariyildiz, K. Ohnishi, Electrohydraulic Transmission System for Minimally Invasive Robotics. IEEE T Ind Electron 62, 7643-7654 (2015).
56. M. P. Armacost, J. Adair, T. Munger, R. R. Viswanathan, F. M. Creighton, D. T. Curd, R. Sehra, Accurate and Reproducible Target Navigation with the Stereotaxis Niobe® Magnetic Navigation System. J Cardiovasc Electr 18 (2007).
57. F. Qi, F. Ju, D. Bai, Y. Wang, B. Chen, Kinematic analysis and navigation method of a cable-driven continuum robot used for minimally invasive surgery. Robotics Computer Surgery 15, e2007 (2019).
58. H. Rafii-Tari, C. V. Riga, C. J. Payne, M. S. Hamady, N. J. W. Cheshire, C. D. Bicknell, G.-Z. Yang, Reducing contact forces in the arch and supra-aortic vessels using the Magellan robot. J Vasc Surg 64, 1422-1432 (2016).
59. J. Jayender, R. V. Patel, S. Nikumb, "Robot-assisted catheter insertion using hybrid impedance control" in Proceedings 2006 IEEE International Conference on Robotics and Automation, 2006. ICRA 2006. (IEEE, Orlando, FL, USA, 2006), pp. 607-612.
60. ASAHI INTECC CO., LTD, Core proprietary technologies, ASAHI INTECC MEDICAL PRODUCTS. https://medical.asahi-intecc.com/en/technologies.
61. Y. Cao, Z. Yang, B. Hao, X. Wang, M. Cai, Z. Qi, B. Sun, Q. Wang, L. Zhang, Magnetic Continuum Robot with Intraoperative Magnetic Moment Programming. Soft Robot 10, 1209-1223 (2023).
62. P. Yang, L. Mao, C. Tian, X. Meng, H. Xie, A Cooperative and Multifunctional Magnetic Continuum Robot for Noninteractive Access, Dexterous Navigation, and Versatile Manipulation. Adv Funct Mater 35, 2412543 (2025).
63. L. Mao, P. Yang, C. Tian, X. Shen, F. Wang, H. Zhang, X. Meng, H. Xie, Magnetic steering continuum robot for transluminal procedures with programmable shape and functionalities. Nat Commun 15, 3759 (2024).
64. R. Faubel, C. Westendorf, E. Bodenschatz, G. Eichele, Cilia-based flow network in the brain ventricles. Science 353, 176-178 (2016).
65. M. A. Sleigh, J. R. Blake, N. Liron, The Propulsion of Mucus by Cilia. Am Rev Respir Dis 137, 726-741 (1988).
66. R. A. Lyons, E. Saridogan, O. Djahanbakhch, The reproductive significance of human Fallopian tube cilia. Hum Reprod Update 12, 363-372 (2006).
67. J. R. Blake, M. A. Sleigh, Mechanics of Ciliary Locomotion. Biol Rev 49, 85-125 (1974).
68. W. Gilpin, V. N. Prakash, M. Prakash, Vortex arrays and ciliary tangles underlie the feeding-swimming trade-off in starfish larvae. Nat Phys 13, 380-386 (2017).
69. M. G. Stafford-Smith, R. F. G. Ormond, Sediment-rejection mechanisms of 42 species of Australian scleractinian corals. Mar Freshwater Res 43, 683-705 (1992).
70. X. Zhang, J. Guo, X. Fu, D. Zhang, Y. Zhao, Tailoring Flexible Arrays for Artificial Cilia Actuators. Adv Intell Syst-ger 3, 2000225 (2021).
71. T. ul Islam, Y. Wang, I. Aggarwal, Z. Cui, H. E. Amirabadi, H. Garg, R. Kooi, B. B. Venkataramanachar, T. Wang, S. Zhang, P. R. Onck, J. M. J. den Toonder, Microscopic artificial cilia - a review. Lab on a Chip 22, 1650-1679 (2022).
72. S. Peerlinck, E. Milana, E. De Smet, M. De Volder, D. Reynaerts, B. Gorissen, Artificial Cilia-Bridging the Gap with Nature. Adv Funct Mater 33, 2300856 (2023).
73. W. Wang, Q. Liu, I. Tanasijevic, M. F. Reynolds, A. J. Cortese, M. Z. Miskin, M. C. Cao, D. A. Muller, A. C. Molnar, E. Lauga, P. L. McEuen, I. Cohen, Cilia metasurfaces for electronically programmable microfluidic manipulation. Nature 605, 681-686 (2022).
74. Z. Ren, M. Zhang, S. Song, Z. Liu, C. Hong, T. Wang, X. Dong, W. Hu, M. Sitti, Soft-robotic ciliated epidermis for reconfigurable coordinated fluid manipulation. Sci Adv 8, eabq2345 (2022).
75. S. Li, M. M. Lerch, J. T. Waters, B. Deng, R. S. Martens, Y. Yao, D. Y. Kim, K. Bertoldi, A. Grinthal, A. C. Balazs, J. Aizenberg, Self-regulated non-reciprocal motions in single-material microstructures. Nature 605, 76-83 (2022).
76. J. Han, X. Dong, Z. Yin, S. Zhang, M. Li, Z. Zheng, M. C. Ugurlu, W. Jiang, H. Liu, M. Sitti, Actuation-enhanced multifunctional sensing and information recognition by magnetic artificial cilia arrays. Proceedings of the National Academy of Sciences 120, e2308301120 (2023).
77. S. Zhang, X. Hu, M. Li, U. Bozuyuk, R. Zhang, E. Suadiye, J. Han, F. Wang, P. Onck, M. Sitti, 3D-printed micrometer-scale wireless magnetic cilia with metachronal programmability. Sci Adv 9, eadf9462 (2023).
78. X. Dong, G. Z. Lum, W. Hu, R. Zhang, Z. Ren, P. R. Onck, M. Sitti, Bioinspired cilia arrays with programmable nonreciprocal motion and metachronal coordination. Sci Adv 6, eabc9323 (2020).
79. Z. Cui, Y. Wang, S. Zhang, T. Wang, J. M. J. den Toonder, Miniaturized metachronal magnetic artificial cilia. Proceedings of the National Academy of Sciences 120, e2304519120 (2023).
80. C. Freschi, V. Ferrari, F. Melfi, M. Ferrari, F. Mosca, A. Cuschieri, Technical review of the da Vinci surgical telemanipulator. The International Journal of Medical Robotics and Computer Assisted Surgery 9, 396-406 (2013).
81. Y. Kim, E. Genevriere, P. Harker, J. Choe, M. Balicki, R. W. Regenhardt, J. E. Vranic, A. A. Dmytriw, A. B. Patel, X. Zhao, Telerobotic neurovascular interventions with magnetic manipulation. Sci Robot 7, eabg9907 (2022).
82. E. Diller, J. Giltinan, M. Sitti, Independent control of multiple magnetic microrobots in three dimensions. The International Journal of Robotics Research 32, 614-631 (2013).
83. F. Chen, W. Xu, H. Zhang, Y. Wang, J. Cao, M. Y. Wang, H. Ren, J. Zhu, Y. F. Zhang, Topology Optimized Design, Fabrication, and Characterization of a Soft Cable-Driven Gripper. Ieee Robot Autom Let 3, 2463-2470 (2018).
84. Y. Liu, Q. Bi, Y. Li, "Development of a Bio-inspired Soft Robotic Gripper based on Tensegrity Structures" in 2021 IEEE/RSJ International Conference on Intelligent Robots and Systems (IROS) (2021), pp. 7398-7403.
85. Z. Xiang, L. Hongwei, D. Bingxiao, C. Lu, C. Xiaoqian, H. Yiyong, "Design and experimental validation of a cable-driven continuum manipulator and soft gripper" in 2019 IEEE International Conference on Robotics and Biomimetics (ROBIO) (2019), pp. 1965-1968.
86. Y. Li, Y. Chen, Y. Li, Pre-Charged Pneumatic Soft Gripper With Closed-Loop Control. Ieee Robot Autom Let 4, 1402-1408 (2019).
87. Z. Zhang, X. Ni, H. Wu, M. Sun, G. Bao, H. Wu, S. Jiang, Pneumatically Actuated Soft Gripper with Bistable Structures. Soft Robot 9, 57-71 (2022).
88. R. Deimel, O. Brock, A novel type of compliant and underactuated robotic hand for dexterous grasping. The International Journal of Robotics Research 35, 161-185 (2016).
89. W. Thongking, A. Wiranata, A. Minaminosono, Z. Mao, S. Maeda, Soft Robotic Gripper Based on MultiLayers of Dielectric Elastomer Actuators. J Robot Mechatron 33, 968-974 (2021).
90. G.-K. Lau, K.-R. Heng, A. S. Ahmed, M. Shrestha, Dielectric elastomer fingers for versatile grasping and nimble pinching. Appl Phys Lett 110, 182906 (2017).
91. S. Shian, K. Bertoldi, D. R. Clarke, Dielectric Elastomer Based "Grippers" for Soft Robotics. Adv Mater 27, 6814-6819 (2015).
92. W. Wang, S.-H. Ahn, Shape Memory Alloy-Based Soft Gripper with Variable Stiffness for Compliant and Effective Grasping. Soft Robot 4, 379-389 (2017).
93. Y. She, C. Li, J. Cleary, H.-J. Su, Design and Fabrication of a Soft Robotic Hand With Embedded Actuators and Sensors. Journal of Mechanisms and Robotics 7 (2015).
94. H. Rodrigue, W. Wang, D.-R. Kim, S.-H. Ahn, Curved shape memory alloy-based soft actuators and application to soft gripper. Compos Struct 176, 398-406 (2017).

In general, the present disclosure is directed at the following embodiments of a soft robot, a system comprising the soft robot, an arrangement of a first soft robot according to the present disclosure and a second soft robot according to the present disclosure, and a method for operating the soft robot according to the present disclosure.
1. Soft robot (1) comprising a body (10):
   wherein the body (10) has an outer part (20), an inner part (30), and a magnetic unit (52),
   wherein the outer part (20) is flexible and comprises a flexible channel (22),
   wherein the inner part (30) is movably arranged within the channel (22),
   wherein one of the inner part (30) and the outer part (20) forms a magnetic member (16) comprising the magnetic unit (52) and wherein the other one of the inner part (30) and the outer part (20) forms a flexible target member (15),
   wherein the magnetic unit (52) is arranged inside the channel (22) or adjacent to the channel (22) in an outer direction (4) perpendicular to the channel (22),
   wherein the outer part (20), the inner part (30), and the magnetic unit (52) are configured to be placed within a magnetic field (5) so that the magnetic field (5) exerts a magnetic force on the magnetic unit (52),
   wherein the magnetic member (16) and the target member (15) are configured to move relative to each other to allow for a change of a magnetization profile of the target member (15) and for a change of a response of the target member (15) to the magnetic field (5).
2. The soft robot (1) of embodiment 1,
   wherein the movement of the magnetic unit (52) comprises a translation of the magnetic unit (52) with respect to the target member (15) and/or a rotation of the magnetic unit (52) with respect to the target member (15).
3. The soft robot (1) of one of the preceding embodiments,
   wherein a direction of a magnetic moment (51) of the magnetic unit (52) is parallel to the channel (22).
4. The soft robot (1) of one of the preceding embodiments,
   wherein the inner part (30) forms the magnetic member (16) comprising the magnetic unit (52) so that the magnetic unit (52) is movably arranged within the channel (22),
   wherein the magnetic unit (52) is configured to be actuated through the channel (22).
5. The soft robot (1) of embodiment 4,
   wherein movement of the magnetic unit (52) is limited to an interior of the channel (22).
6. The soft robot (1) of one of the preceding embodiments,
   wherein the inner part (30) comprises a flexible part (31),
   wherein the flexible part (31) is movably arranged with respect to the outer part (20).
7. The soft robot (1) of embodiment 6,
   wherein the flexible part (31) and/or the magnetic unit (52) are configured as solid figures, such as filled cylinders.
8. The soft robot (1) of embodiment 6,
   wherein the flexible part (31) and/or the magnetic unit (52) are hollow,
   wherein, for example, the flexible part (31) and/or the magnetic unit (52) are configured as hollow cylinders.
9. The soft robot (1) of embodiment 4 and one of embodiments 6 to 8,
   wherein the magnetic unit (52) is fixedly attached to the flexible part (31),
   wherein the flexible part (31) forms a manipulator (110) for actuating the magnetic unit (52).
10. The soft robot (1) of embodiment 9,
   wherein the manipulator (110) mechanically connects the magnetic unit (52) to an actuator (140),
   wherein the manipulator (110) is configured to transfer an actuation force from the actuator (140) to the magnetic unit (52).
11. The soft robot (1) of one of embodiments 9 and 10,
   wherein the magnetic unit (52) is attached to an end of the manipulator (110).
12. The soft robot (1) of one of embodiments 9 and 10,
   wherein the magnetic unit (52) is attached to the manipulator (110) at a location spaced apart from an end of the manipulator (110).
13. The soft robot (1) of one of the preceding embodiments,
   wherein the body (10) comprises a further magnetic unit (62),
   wherein the further magnetic unit (62) is configured to be placed within the magnetic field (5) so that the magnetic field (5) exerts a magnetic force on the further magnetic unit (62),
   wherein the further magnetic unit (62) is arranged inside the channel (22) or adjacent to the channel (22) in an outer direction (4) perpendicular to the channel (22),
   wherein the magnetic unit (52) and the further magnetic unit (62) are configured for being placed at a distance (53) from each other along the channel (22).
14. The soft robot (1) of embodiment 13,
   wherein a magnetic moment (51) of the magnetic unit (52) differs from a magnetic moment (63) of the further magnetic unit (62).
15. The soft robot (1) of one of embodiments 13 and 14,
   wherein the magnetic unit (52) and the further magnetic unit (62) are configured for being placed at the distance (53) from each other with no additional magnetic unit being placed in between the magnetic unit (52) and the further magnetic unit (62).
16. The soft robot (1) of one of embodiments 13 to 15,
   wherein the further magnetic unit (62) is movably arranged with respect to the target member (15) to allow for a change of the magnetization profile of the target member (15) within the external magnetic field (5) and a change of a response of the target member (15) to the magnetic field (5).
17. The soft robot (1) of one of embodiments 13 to 16,
   wherein the further magnetic unit (62) is longitudinally fixed with respect to the magnetic unit (52).
18. The soft robot (1) of one of embodiments 13 to 16,
   wherein the further magnetic unit (62) is movable with respect to the magnetic unit (52),
   wherein, for example, the further magnetic unit (62) is fixed to the target member (15).
19. The soft robot (1) of embodiment 18,
   wherein the magnetic unit (52) and the further magnetic unit (62) are configured to be placed adjacent to each other, such as adjoining each other in a longitudinal direction (2) or nested around each other in the outer direction (3).
20. The soft robot (1) of one of embodiments 13 to 19,
   wherein one of the magnetic unit (52) and the further magnetic unit (62) is placed within the channel (22) and the other one of the magnetic unit (52) and the further magnetic unit (62) is placed at least partially around the channel (22).
21. The soft robot (1) of one of embodiments 13 to 19,
   wherein a direction of a magnetic moment (51) of the further magnetic unit (62) is opposite to a direction of a magnetic moment (63) of the magnetic unit (52).
22. The soft robot (1) of one of embodiments 13 to 21,
   wherein a magnitude of the magnetic moment (63) of the further magnetic unit (62) equals a magnitude of the magnetic moment (51) of the magnetic unit (52).
23. The soft robot (1) of one of embodiments 13 to 21,
   wherein a magnitude of the magnetic moment (63) of the further magnetic unit (62) is essentially twice or half a magnitude of the magnetic moment (51) of the magnetic unit (52), such as twice or half the magnitude of the magnetic moment (51) of the magnetic unit (52).
24. The soft robot (1) of embodiment 23,
   wherein the magnetic unit (52) is configured to be placed adjacent to the further magnetic unit (62) by placing, in a first configuration, a manipulator (110) connected to one of the magnetic unit (52) and the further magnetic unit (62) within the channel (22),
   wherein the magnetic unit (52) is configured to be placed, in a second configuration, at the distance (53) from the further magnetic unit (62) by removing the manipulator (110) from the channel (22),
   wherein, in the first configuration, the outer part (20) and the manipulator (110) placed within the channel (22) have a first stiffness against transverse deflection and, in the second configuration, the outer part (20) without the manipulator (110) has a second stiffness against transverse deflection, wherein the magnitude of the magnetic moment (51) of the magnetic unit (52) deviates from twice or half the magnitude of the magnetic moment (63) of the further magnetic unit (62) by a margin magnetic moment (66),
   wherein a magnetic force acting on the margin magnetic moment (66) in the magnetic field (5) compensates for a difference between the first stiffness and the second stiffness so that a deflection amplitude of the outer part (20) within the magnetic field (5) is essentially equal in the first configuration and in the second configuration.
25. The soft robot (1) of one of the preceding embodiments,
   comprising an additional body (11) having an additional outer part (220), an additional inner part (230), and an additional magnetic unit (252),
   wherein the additional outer part (220) is flexible and comprises a flexible additional channel (222),
   wherein the additional inner part (230) is movably arranged within the additional channel (222),
   wherein one of the additional inner part (230) and the additional outer part (220) forms an additional magnetic member (16) comprising the additional magnetic unit (252) and wherein the other one of the additional inner part (230) and the additional outer part (220) forms a flexible additional target member (15),
   wherein the additional magnetic unit (252) is arranged inside the additional channel (222) or adjacent to the additional channel (222) in an outer direction (4) perpendicular to the additional channel (222),
   wherein the additional outer part (220), the additional inner part (230), and the additional magnetic unit (252) are configured to be placed within the magnetic field (5) so that the magnetic field (5) exerts a magnetic force on the additional magnetic unit (252),
   wherein the additional magnetic member (16) is configured to move relative to the additional target member (15) to allow for a change of a magnetization profile of the additional target member (15) and for a change of a response of the additional target member (15) to the magnetic field (5),
   wherein the outer part (20) and the additional outer part (220) are deformable independently from each other,
   wherein the magnetic unit (52) and the additional magnetic unit (252) are movable independently from each other.
26. The soft robot (1) of embodiment 25,
   wherein the outer part (20) and the additional outer part (220) are fixed to a common support (12).
27. The soft robot (1) of one of embodiments 25 and 26,
   wherein the outer part (20) and the additional outer part (20) are placed next to each other.
28. The soft robot (1) of one of the preceding embodiments,
   wherein the outer part (20) is configured as an actor for moving a target (80), such as a fluid or a solid object.
29. The soft robot (1) of one of the preceding embodiments,
   wherein the body (10) comprises a further inner part (40),
   wherein the further inner part (40) is flexible and comprises a further channel (42),
   wherein the further inner part (40) is stacked in the outer direction (4) in between the outer part (20) and the inner part (30) so that the further inner part (40) is located within the channel (22) of the outer part (20) and the inner part (30) is located within the further channel (42) of the further inner part (40),
   wherein the inner part (30) and the further inner part (40) are movable with respect to each other.
30. The soft robot (1) of embodiment 29,
   wherein the further inner part (40) is configured to extend through an end of the channel (22) of the outer part (20) further into the magnetic field (5).
31. The soft robot (1) of one of embodiments 29 and 30 and one of embodiments 13 to 24,
   wherein the further inner part (40) comprises the further magnetic unit (62) fixed to the further inner part (40).
32. The soft robot (1) of one of embodiments 29 to 31,
   wherein a stiffness of the outer part (20) is larger than a stiffness of the further inner part (40).
33. The soft robot (1) of one of embodiments 29 to 32,
   wherein a stiffness of the inner part (30) is lower than the stiffness of the further inner part (40) and, optionally, than the stiffness of the outer part (20).
34. The soft robot (1) of one of the preceding embodiments,
   comprising a set of further inner parts (40) with at least one further inner part (40),
   wherein each of the further inner parts (40) is flexible and comprises a further channel (42), wherein the further inner parts (40) of the set are stacked one into the other in between the outer part (20) and the inner part (30),
   wherein, for example, at least one of the further inner parts (40), such as a multitude of the further inner parts (40), like all further inner parts (40), each comprise a respective further magnetic unit (62) attached thereon.
35. The soft robot (1) of embodiment 34,
   wherein a stiffness of the inner part (30), respective stiffnesses of the further inner parts (40), and a stiffness of the outer part (20) decrease in the outer direction (4) from the inner part (30) to the outer part (20).
36. The soft robot (1) of one of embodiments 34 and 35,
   wherein the inner part (30), further inner parts (40) and the outer part (20) are configured to be axially arranged with respect to each other with respective ends (24, 44) of the further inner parts (40) and of the outer part (20) being sequentially located along a length of the channel (22) in a set of working zones (120, 121, 122, 123, 124) so that each one of the working zones (120, 121, 122, 123, 124) only comprises one of the respective ends (24, 44),
   wherein at least one of the working zones (120, 121, 122, 123, 124) is filled with the magnetic field (5),
   wherein the working zones (120, 121, 122, 123, 124) of the set do not overlap with each other,
   wherein the inner part (30), the further inner parts (40) and the outer part (20) are configured to axially translate with respect to each other with each of the respective ends (24, 44) of the further inner parts (40) and the outer part (20) remaining within its respective working zone (120, 121, 122, 123, 124) during actuation of the soft robot (1).
37. The soft robot (1) of one of embodiment 36,
   wherein each of the working zones (120, 121, 122, 123, 124) is filled with a respective magnetic field (5),
   wherein the magnetic fields (5) of at least two of the working zones (120, 121, 122, 123, 124) differ from each other.
38. The soft robot (1) of one of embodiments 34 to 37,
   further including a set of magnetic units (52, 62, 72), the magnetic unit (52) being part of the set,
   wherein to each one of the further inner parts (40) a respective one of the magnetic units (52, 62, 72) of the set is attached,
   wherein respective magnetic fields (5) within the individual interaction zones (130, 132) and magnetic moments (53, 63, 73) of respective magnetic units (52, 62, 72) located within the individual interaction zones (130, 132) are configured to generate magnetic forces on the individual magnetic units (52, 62, 72),
   wherein the magnetic forces on the individual magnetic units (52, 62, 72) increase with a number of parts of the body (10) present in the interaction zone (130, 132) comprising the respective magnetic unit (52, 62, 72).
39. The soft robot (1) of one of the preceding embodiments,
   wherein the inner part (30) comprises a first inner part section (36) that is configured to be located next to the magnetic unit (52) and a second inner part section (37) adjoining the first inner part section (36), the second inner part section (37) being configured to be placed away from the magnetic unit (52),
   wherein the second inner part section (37) is configured to connect the first inner part section (36) to an actuator (140) to actuate a movement of the inner part (30) relative to the outer part (20),
   wherein the first inner part section (36) is made from a first inner part material and the second inner part section (37) is made from a second inner part material,
   wherein the second inner part material has a different stiffness than the first inner part material, such as a higher stiffness than the first inner part material.
40. The soft robot (1) of one of embodiments 6 to 12 and embodiment 39,
   wherein the flexible part (31) forms the first inner part section (36).
41. The soft robot (1) of one of embodiments 39 and 40,
   wherein the second inner part section (37) is at least partially located within a feeding zone (135),
   wherein the feeding zone (135) is located in between an interaction zone (130, 132) and an actuation zone (138) for actuation of the relative movement between the magnetic member (16) and the target member (15),
   wherein the magnetic unit (52) is configured to interact with the magnetic field (5) within the interaction zone (130, 132), such as only within the interaction zone (130, 132).
42. The soft robot (1) of one of embodiments 39 to 41,
   wherein the second inner part section (37) is directly connected to an actuator (140).
43. The soft robot (1) of one of embodiments 39 to 42,
   wherein the second inner part section (37) is located outside a bending zone (139) of the channel (22) for all positions that the magnetic member (16) is configured to be located at relative to the target member (15) during operation of the soft robot (1),
   wherein the bending zone (139) comprises a region in which the shape of the channel (22) changes due to the magnetic force on the magnetic unit (52).
44. The soft robot (1) of one of the preceding embodiments,
   wherein the outer part (20) comprises a first outer part section (26) that is configured to be located next to the magnetic unit (52) and a second outer part section (27) adjoining the first outer part section (26), the second outer part section (27) being configured to be placed away from the magnetic unit (52),
   wherein the second outer part section (27) is configured to connect the first outer part section (26) to an actuator (140) to actuate a movement of the inner part (30),
   wherein the first outer part section (26) is made from a first outer part material and the second outer part section (27) is made from a second outer part material,
   wherein the second outer part material has a different stiffness than the first outer part material, such as a higher stiffness than the first outer part material.
45. The soft robot (1) of one of the preceding embodiments,
   wherein the magnetic unit (52) forms a part of a magnetic element (50),
   wherein the magnetic element (50) extends in a longitudinal direction (2) from a first end (54) of the magnetic element (50) to a second end (55) of the magnetic element (50),
   wherein a magnetic moment (51) of the magnetic element (50), such as a direction of the magnetic moment (51) of the magnetic element (50), continuously changes from a first magnetic moment (51) at the first end (54) to a second magnetic moment (56) at the second end (55), the second magnetic moment (56) being different from the first magnetic moment (51).
46. The soft robot (1) of embodiment 45,
   wherein the magnetic element (50) is flexible.
47. The soft robot (1) of one of embodiments 45 and 46,
   wherein the magnetic element (50) is configured as an elongated cylinder, such as an elongated hollow cylinder or an elongated solid cylinder.
48. The soft robot (1) of one of embodiments 45 to 47,
   wherein a projection of the direction of the magnetic moment (51) of the magnetic element (50) in a direction along the channel (22) continuously rotates from a first direction of the first magnetic moment (51) to a second direction of the second magnetic moment (56).
49. The soft robot (1) of one of embodiments 45 to 48,
   wherein the magnetic moment (51) of the magnetic element (50) comprises a non-zero component that is perpendicular to the plane parallel to the channel (22).
50. The soft robot (1) of one of the preceding embodiments,
   wherein a material of the channel (22) and/or a material of the inner part (30) comprises a surface tension diffuser.
51. The soft robot (1) of one of the preceding embodiments,
   wherein the soft robot (1) comprises a lubricant in between the channel (22) and the inner part (30).
52. The soft robot (1) of one of the preceding embodiments,
   further comprising an actuator (140),
   wherein the actuator (140) is configured to generate an actuation force that induces a movement of the magnetic unit (52) relative to the target member (15).
53. The soft robot (1) of embodiment 52,
   wherein the actuator (140) is configured to deliver the actuation force to the magnetic unit (52) via the channel (22),
   wherein the actuator (140), for example, delivers the force on the magnetic unit (52) by a mechanical link or a medium, such as a gas or a liquid, present within the channel (22).
54. The soft robot (1) of one of the preceding embodiments,
   wherein the magnetic unit (52) is configured to remain longitudinally stationary with respect to a surrounding of the soft robot (1) while the target member (15) translates longitudinally with respect to the magnetic unit (52).
55. The soft robot (1) of one of the preceding embodiments,
   wherein the outer part (20) is configured as an elongated cylinder, for example as a flexible tube or a flexible rod.
56. The soft robot (1) of embodiment 55,
   wherein the body (10) forms part of a medical instrument (400), such as a catheter or an endoscope for medical use.
57. The soft robot (1) of one of the preceding embodiments,
   wherein the soft robot (1) comprises a support (12),
   wherein the outer part (20) extends in a longitudinal direction (2) between a first end (24) and a second end (25),
   wherein the second end (25) is fixed to the support (12) and the first end (24) is configured as a free end,
   wherein the channel (22) runs from the second end (25) to the first end (24),
   wherein the outer part (20) forms the target member (15) and the inner part (30) forms the magnetic member (16).
58. The soft robot (1) of embodiments 55 and 57,
   configured as an artificial cilia array (300),
   wherein the body (10) forms an artificial cilium (310) placed in the artificial cilia array (300), the artificial cilia array (300) comprising a multitude of other bodies (11) forming other artificial cilia (310),
   wherein the other artificial cilia (310) each comprise an outer part (20), an inner part (30), and a magnetic unit (52),
   wherein the outer part (20) of each of the other artificial cilia (310) is flexible and comprises a flexible channel (22),
   wherein the inner part (30) of each of the other artificial cilia (310) is movably arranged within the channel (22) of the respective one of the other artificial cilia (310),
   wherein the inner part (30) of each of the other artificial cilia (310) forms a magnetic member (16) comprising the magnetic unit (52) of the respective one of the other artificial cilia (310) and wherein the outer part (20) of each of the other artificial cilia (310) forms a flexible target member (15) of the respective one of the other artificial cilia (310),
   wherein the magnetic unit (52) of each of the other artificial cilia (310) is arranged inside the channel (22) of the respective one of the other artificial cilia (310),
   wherein the outer part (20) of each of the other artificial cilia (310), the inner part (30) of each of the other artificial cilia (310), and the magnetic unit (52) of each of the other artificial cilia (310) are configured to be placed within the magnetic field (5) so that the magnetic field (5) exerts a magnetic force on the magnetic units (52) of each of the other artificial cilia (310),
   wherein the magnetic unit (52) of each of the other artificial cilia (310) is configured to move relative to the outer part (20) of the respective one of the other artificial cilia (310) to allow for a change of a magnetization profile of the outer part (20) of the respective one of the other artificial cilia (310) and for a change of a response of the outer part (20) of the respective one of the other artificial cilia (310) to the magnetic field (5),
   wherein the magnetic unit (52) of the artificial cilium (310) and the magnetic units (52) of the other artificial cilia (310) are movable independently from each other within the common magnetic field (5).
59. The soft robot (1) of embodiment 57,
   wherein the soft robot (1) is configured as a soft gripper (350),
   wherein the body (10) forms a flexible finger (352) of the soft gripper (350).
60. The soft robot (1) of one of embodiments 1 to 54 or of embodiment 59,
   wherein the outer part (20) is configured as a flat sheet (160).
61. The soft robot (1) of one of embodiments 1 to 54 or of embodiment 60,
   wherein the outer part (20) comprises at least two walls (170) arranged at an angle with respect to each other and adjoining each other at an edge (164, 165) of the outer part (20),
   wherein, for example, the at least two walls (170) each are configured as a flat sheet (160).
62. The soft robot (1) of embodiment 61,
   wherein the channel (22) is located at the edge (164, 165),
   wherein, for example, the channel (22) runs along the edge (164, 165).
63. The soft robot (1) of one of embodiments 61 and 62,
   wherein the outer part (20) is configured as a box (180) having sidewalls (181, 182) adjoining each other at edges (164, 165) of the box (180),
   wherein each of the two walls (170) forms one of the sidewalls (181, 182).
64. The soft robot (1) of embodiment 63,
   wherein the channel (22) forms an edge channel (166) located at one of the edges (164, 165),
   wherein the outer part (20) comprises further edge channels (166) located at each of the remaining ones of the edges (164, 165) of the box (180),
   wherein each of the edge channels (166) has a respective magnetic unit (52) movably arranged therein.
65. The soft robot (1) of embodiment 64,
   wherein the outer part (20) comprises a first sidewall (181) and a second sidewall (182), the second sidewall (182) being located opposite the first sidewall (181),
   wherein magnetic moments (53) of magnetic units (52) movably arranged within the edge channels (166) of the first sidewall (181) have a first common direction (260),
   wherein magnetic moments (53) of magnetic units (52) movably arranged within the edge channels (166) of the second sidewall (182) have a second common direction (262),
   wherein the first common direction (160) is opposite to the second common direction (162).
66. The soft robot (1) of one of embodiments 1 to 54 or of one of embodiments 60 to 65,
   wherein the outer part (20) forms the target member (15) and the inner part (30) forms the magnetic member (16),
   wherein the magnetic unit (52) is arranged inside the channel (22),
   wherein the outer part (20) comprises a second channel (211),
   wherein a second magnetic unit (212) is movably arranged within the second channel (21),
   wherein the second magnetic unit (212) is configured to be placed within the magnetic field (5) so that the magnetic field (5) exerts a magnetic force on the second magnetic unit (212),
   wherein the second magnetic unit (212) is configured to move relative to the outer part (20) to allow for a change of a magnetization profile of the outer part (20) and for a change of a response of the outer part (20) to the magnetic field (5).
67. The soft robot (1) of embodiment 66,
   wherein the channel (22) and the second channel (211) run parallel to each other.
68. The soft robot (1) of one of embodiments 66 and 67,
   wherein the channel (22) and the second channel (211) are adjacent to each other.
69. The soft robot (1) of one of embodiments 66 to 68,
   wherein a magnetic moment (51) of the magnetic unit (52) and a magnetic moment (51) of the second magnetic unit (212) are opposite with respect to each other.
70. The soft robot (1) of one of embodiments 66 to 69,
   wherein the outer part (20) is configured as a flat sheet (160) extending in a length direction (7) and in a width direction (8),
   wherein the flat sheet (160) has a height (163) in a height direction (9) that is smaller than a length (161) of the flat sheet (160) in the length direction (2) and a width (162) of the flat sheet (160) in the width direction (8),
   wherein the channel (22) and the second channel (211) are located at the same edge (164, 165) of the flat sheet (160) in the width direction (8).
71. The soft robot (1) of one of embodiments 66 to 69,
   wherein the outer part (20) is configured as a flat sheet (160) extending in a length direction (7) and in a width direction (8),
   wherein the flat sheet (160) has a thickness in a height direction (9) that is smaller than a length of the flat sheet (160) in the length direction (2) and a width of the flat sheet (160) in the width direction (8),
   wherein the channel (22) and the second channel (211) are located at opposite edges (164, 165) of the flat sheet (160) in the width direction (8).
72. The soft robot (1) of embodiment 71,
   wherein the outer part (20) comprises a third channel (213) and a third magnetic unit (214) movably arranged within the third channel (213),
   wherein the third magnetic unit (214) is configured to be placed within the magnetic field (5) so that the magnetic field (5) exerts a magnetic force on the third magnetic unit (214),
   wherein the third magnetic unit (214) is configured to move relative to the outer part (20) to allow for a change of a magnetization profile of the outer part (20) and for a change of a response of the outer part (20) to the magnetic field (5).
73. The soft robot (1) of embodiment 72,
   wherein the third channel (213) is located in between the channel (22) and the second channel (211) in the width direction (8).
74. The soft robot (1) of embodiment 73,
   wherein the third channel (213) is located at a first distance (201) from the channel (22) in the width direction (8) and at a second distance (202) from the second channel (22) in the width direction (8),
   wherein a ratio of the first distance (201) to the second distance (202) is at least 0.5 and at most 1.5.
75. The soft robot (1) of one of embodiments 72 and 73,
   wherein the channel (22) and the third channel (213) are located adjacent to each other at the same edge (164, 165) of the flat sheet (160) in the width direction (8).
76. The soft robot (1) of embodiment 75,
   wherein the outer part (20) comprises a fourth channel (215) and a fourth magnetic unit (216) movably arranged within the fourth channel (215),
   wherein the fourth magnetic unit (216) is configured to be placed within the magnetic field (5) so that the magnetic field (5) exerts a magnetic force on the fourth magnetic unit (216),
   wherein the fourth magnetic unit (216) is configured to move relative to the outer part (20) to allow for a change of a magnetization profile of the outer part (20) and for a change of a response of the outer part (20) to the magnetic field (5).
77. The soft robot (1) of embodiment 76,
   wherein the second channel (212) and the fourth channel (215) are located adjacent to each other at the same edge (164, 165) of the flat sheet (160) in the width direction (8).
78. The soft robot (1) of one of embodiments 76 and 77,
   wherein a direction of a magnetic moment (51) of the magnetic unit (52) and a direction of a magnetic moment (51) of the third magnetic unit (214) are opposite with respect to each other,
   wherein a direction of a magnetic moment (51) of the second magnetic unit (212) and a direction of a magnetic moment (51) of the fourth magnetic unit (216) are opposite with respect to each other,
   wherein the direction of the magnetic moment (51) of the magnetic unit (52) and the direction of the magnetic moment (51) of the second magnetic unit (212) are parallel to each other.
79. The soft robot (1) of one of embodiments 76 and 78,
   wherein a magnitude of a magnetic moment (51) of the magnetic unit (52) and a magnitude of a magnetic moment (51) of the third magnetic unit (214) are equal,
   wherein a magnitude of a magnetic moment (51) of the second magnetic unit (212) and a magnitude of a magnetic moment (51) of the fourth magnetic unit (216) are equal.
80. The soft robot (1) of one of embodiment 79,
   wherein the magnitude of the magnetic moment (51) of the magnetic unit (52) and the magnitude of the magnetic moment (51) of the second magnetic unit (212) are equal.
81. The soft robot (1) of one of embodiments 78 to 80,
   comprising an additional magnetic unit (252), an additional second magnetic unit (254), an additional third magnetic unit (256), and an additional fourth magnetic unit (258),
   wherein the magnetic unit (52) and the additional magnetic unit (252) are serially connected to each other and spaced apart from each other,
   wherein the second magnetic unit (112) and the additional second magnetic unit (254) are serially connected to each other and spaced apart from each other,
   wherein the third magnetic unit (114) and the additional third magnetic unit (256) are serially connected to each other and are spaced apart from each other,
   wherein the fourth magnetic unit (116) and the additional fourth magnetic unit (258) are serially connected to each other and are spaced apart from each other.
82. The soft robot (1) of one of embodiments 78 to 81,
   wherein a direction of a magnetic moment (51) of the magnetic unit (52) and a direction of a magnetic moment (253) of the additional magnetic unit (252) are opposite to each other, and/or
   wherein a direction of a magnetic moment (51) of the second magnetic unit (112) and a direction of a magnetic moment (253) of the additional second magnetic unit (254) are opposite to each other, and/or
   wherein a direction of a magnetic moment (51) of the third magnetic unit (114) and a direction of a magnetic moment (255) of the additional third magnetic unit (256) are opposite to each other, and/or
   wherein a direction of a magnetic moment (51) of the fourth magnetic unit (116) and a direction of a magnetic moment (257) of the additional fourth magnetic unit (258) are opposite to each other,
83. The soft robot (1) of one of embodiments 81 and 82,
   wherein a magnitude of a magnetic moment (51) of the magnetic unit (52) and a direction of a magnetic moment (253) of the additional magnetic unit (252) are equal, and/or
   wherein a magnitude of a magnetic moment (51) of the second magnetic unit (112) and a direction of a magnetic moment (51) of the additional second magnetic unit (254) are equal, and/or
   wherein a magnitude of a magnetic moment (51) of the third magnetic unit (114) and a direction of a magnetic moment (51) of the additional third magnetic unit (256) are equal, and/or
   wherein a magnitude of a magnetic moment (51) of the fourth magnetic unit (116) and a direction of a magnetic moment (51) of the additional fourth magnetic unit (258) are equal.
84. The soft robot (1) of one of embodiments 66 to 83,
   wherein the body (10) comprises a set of extra channels (218) located in between the channel (22) and the second channel (212),
   wherein, for each of the extra channels (218), a respective extra magnetic unit (119) from a set of extra magnetic units (219) is placed within the respective extra channel (118).
85. The soft robot (1) of embodiment 84,
   wherein the extra channels (218) are pairwise located adjacent to each other to form pairs of adjacent extra channels (218),
   wherein the pairs of adjacent extra channels (218) are spaced apart from each other, from the channel (22), and from the second channel (112).
86. The soft robot (1) of embodiment 85,
   wherein directions of magnetic moments (53) of the respective extra magnetic units (219) of each of the pairs of adjacent extra channels (218) are opposite to each other.
87. The soft robot (1) of one of embodiments 85 and 86,
   wherein magnitudes of magnetic moments (53) of the respective extra magnetic units (219) of each of the pairs of adjacent extra channels (218) are equal.
88. A system (650) comprising the soft robot (1) of one of the preceding embodiments and a magnetic field generator (100, 101),
   wherein the magnetic field generator (100, 101) is configured to generate the magnetic field (5) within an interaction zone (130, 132), and
   wherein the outer part (20), the inner part (30), and the magnetic unit (52) are configured to be placed within the magnetic field (5) within the interaction zone (130, 132).
89. The system (650) of embodiment 88,
   wherein the magnetic field (5) is a time-varying magnetic field,
   wherein, for example, a direction and/or magnitude of the magnetic field (5) is variable in time,
   wherein, for example, the direction of the time-varying magnetic field rotates.
90. The system (650) of one of embodiments 88 and 89,
   wherein the magnetic field generator (100, 101) comprises a field source, such as a permanent magnet (102), and a source actuator (104),
   wherein the source actuator (104) is configured to vary a distance (107) of the field source from the interaction zone (130, 132) to modify the magnitude of the magnetic field (5).
91. The system (650) of one of embodiments 88 and 89,
   wherein the magnetic field generator (100, 101) comprises a multitude of field sources, such as permanent magnets (102), and a source actuator (140),
   wherein the field sources are provided in a source arrangement (105) with respect to the interaction zone (130, 132),
   wherein the source actuator (140) is configured to vary respective distances (107) of the field sources to the interaction zone (130, 132),
   wherein, for example, the source arrangement (105) is a circular arrangement of the field sources around the interaction zone (130, 132) and the actuator (140) is configured to vary a diameter of the circular arrangement.
92. The system (650) of one of embodiments 88 to 91,
   wherein the magnetic field (5) is essentially homogeneous, such as homogeneous, within the interaction zone (130, 132).
93. The system (650) of one of embodiments 88 to 92 and of embodiment 52,
   wherein the actuator (140) and the magnetic field (5) generator are configured to move the magnetic unit (52) within the interaction zone (130, 132) with a magnitude of the magnetic field (5) set to a reduced value, such as zero, and to subsequently increase the magnitude of the electric field while the magnetic unit (52) is present in the interaction zone (130, 132).
94. An arrangement (600) of a first soft robot (1) according to one of embodiments 1 to 87 and a second soft robot (1) according to one of embodiments 1 to 87,
   wherein the first soft robot (1) and the second soft robot (1) are independently actuatable within the magnetic field (5) to cooperate with each other.
95. A method (500) for operating the soft robot (1) of one of embodiments 1 to 87,
   the method (500) comprising the steps of:
   - generating (505) the external magnetic field (5) in an interaction zone (130, 132) by a magnetic field generator (100, 101),
   - placing (510) the outer part (20), the inner part (30), and the magnetic unit (52) within the magnetic field (5) in the interaction zone (130, 132) so that the magnetic field (5) exerts a magnetic force on the magnetic unit (52),
   - moving (515) the magnetic member (16) relative to the target member (15) by an actuator (140) to change the magnetization profile of the target member (15) and to change the response of the target member (15) to the magnetic field (5).
96. The method (500) of embodiment 95,
   further comprising the steps of:
   - setting (520) a magnitude of the magnetic field (5) to a reduced value, such as zero, during the moving (515) of the magnetic member (16) relative to the target member (15) and
   - increasing (525) the magnitude of the magnetic field (5) while the magnetic unit (52) is present in the magnetic field (5), such as after the moving (515) of the magnetic member (16) relative to the target member (15).
97. The method (500) of one of embodiments 95 and 96,
   further comprising the steps of:
   - placing (512) an outer part (20), an inner part (30), and a magnetic unit (52) of a further soft robot (1) according to one of embodiments 1 to 87 within the magnetic field (5) in the interaction zone (130, 132) so that the magnetic field (5) exerts a magnetic force on the magnetic unit (52) of the further robot (1),
   - actuating the soft robot (1) and the further soft robot (1) independently from each other to cooperate with each other within the magnetic field (5).
98. The method (500) of one of embodiments 95 to 97,
   wherein the soft robot (1) comprises a further magnetic unit (62) movably arranged with respect to target member (15),
   wherein the further magnetic unit (62) is movable independently from the magnetic unit (52) with respect to the target member (15),
   wherein the method (500) comprises the further steps of:
      - placing (530) the magnetic unit (52) at a first location and placing the further magnetic unit (62) at a second location, the first location being at a distance (53) from the second location so that the force on the target member (15) at the first location is given by the magnetic moment (51) of the magnetic unit (52);
      - placing (535) the further magnetic unit (62) next to the magnetic unit (52) at the first location so that the force on the target member (15) in the vicinity of the magnetic unit (52) is given by a superposition of the magnetic moment (51) of the magnetic unit (52) and a magnetic moment (63) of the further magnetic unit (62).
99. The method (500) of one of embodiments 95 to 98,
   further comprising:
   - rotating (540) a manipulator (110) of the magnetic member (16) while moving it along the channel (22).

The present disclosure is also directed at the following embodiments of a soft robot configured as an elongated cylindrical tube:
1. A soft robot (1) comprising a body (10):
   wherein the body (10) has an outer part (20), an inner part (30), and a magnetic unit (52),
   wherein the outer part (20) is configured as an elongated cylindrical tube comprising a flexible channel (22),
   wherein the inner part (30) has a magnetic member (16) comprising the magnetic unit (52) and a flexible part (31) attached to the magnetic unit (52),
   wherein the inner part (30) with the magnetic unit (52) and the flexible part (31) is movably arranged within the channel (22),
   wherein the flexible part (31) forms a manipulator (110) for moving the magnetic unit (52),
   wherein the outer part (20), the inner part (30), and the magnetic unit (52) are configured to be placed within a magnetic field (5) so that the magnetic field (5) exerts a magnetic force on the magnetic unit (52),
   wherein the inner part (30) and the outer part (20) are configured to move relative to each other to allow for a change of a magnetization profile of the outer part (20) and for a change of a response of the outer part (20) to the magnetic field (5),
   wherein the inner part (30) with the magnetic unit (52) is configured to remain longitudinally stationary with respect to a surrounding while the outer part (20) translates longitudinally in a longitudinal direction (2) of the channel (22) with respect to the inner part (30) and the magnetic unit (52).
2. The soft robot (1) of embodiment 1,
   wherein movement of the magnetic unit (52) is limited to an interior of the channel (22).
3. The soft robot (1) of one of the preceding embodiments,
   wherein the inner part (30) forming the magnetic member (16) comprises a further magnetic unit (62),
   wherein the magnetic unit (52) and the further magnetic unit (62) are fixed to each other,
   wherein the magnetic unit (52) and the further magnetic unit (62) are placed at a distance (53) from each other.
4. The soft robot (1) of embodiment 3,
   wherein a magnetic moment (51) of the magnetic unit (52) differs from a magnetic moment (63) of the further magnetic unit (62).
5. The soft robot (1) of one of embodiments 3 and 4,
   wherein the inner part (30) forming the magnetic member (16) comprises a third magnetic unit (72),
   wherein the third magnetic unit (72) is longitudinally fixed with respect to the magnetic unit (52) and the further magnetic unit (62) at respective distances (73, 74) from the magnetic unit (52) and the further magnetic unit (62).

The present disclosure is also directed at the following embodiments of an artificial cilia array:
1. A soft robot (1) configured as an artificial cilia array (300),
   wherein the soft robot (1) comprises a support (12) and a multitude of bodies (10, 11) forming artificial cilia (310),
   wherein each artificial cilium (310) comprises an outer part (20), an inner part (30), and a magnetic unit (52),
   wherein, for each artificial cilium (310), the outer part (20) is configured as an elongated flexible tube comprising a flexible tubular channel (22),
   wherein, for each artificial cilium (310), the outer part (20) extends in a longitudinal direction (2) between a first end (24) and a second end (25) and the channel (22) runs from the second end (25) to the first end (24),
   wherein, for each artificial cilium (310), the second end (25) is fixed to the support (12) and the first end (24) is configured as a free end,
   wherein, for each artificial cilium (310), the inner part (30) is movably arranged within the channel (22),
   wherein, for each artificial cilium (310), the inner part (30) forms a magnetic member (16) comprising the magnetic unit (52) and the outer part (20) forms a flexible target member (15),
   wherein, for each artificial cilium (310), the magnetic unit (52) is arranged inside the channel (22),
   wherein, for each artificial cilium (310), the outer part (20), the inner part (30), and the magnetic unit (52) are configured to be placed within a magnetic field (5) so that the magnetic field (5) exerts a magnetic force on the magnetic unit (52),
   wherein, for each artificial cilium (310), the magnetic unit (52) is configured to move relative to the outer part (20) to allow for a change of a magnetization profile of the outer part (20) and for a change of a response of the outer part (20) to the magnetic field (5),
   wherein the magnetic units (52) of the individual cilia (310) are configured to independently move with respect to each other.
2. The soft robot (1) of embodiment 1,
   wherein, for each artificial cilium (310), the magnetic unit (52) is configured to be moved between a first position (151) located near the first end (24) and a second position (152) located away from the first end (24), for example located at the second end (25).
3. The soft robot (1) of embodiment 2,
   wherein, for each artificial cilium (310), moving the magnetic unit (52) from the first position (151) to the second position (152) changes an amplitude of the response of the outer part (20) of the respective artificial cilium (310) to the magnetic field (5).
4. The soft robot (1) of embodiment 3,
   wherein, for each artificial cilium (310), moving the magnetic unit (52) to one of the first position (151) and the second position (152) reduces the amplitude of the response of the outer part (20) of the respective artificial cilium (310) to the magnetic field (5) to essentially zero, such as zero.
5. The soft robot (1) of one of embodiments 2 and 4,
   wherein, for each artificial cilium (310), moving the magnetic unit (52) from the first position (151) to the second position (152) changes a direction of the response of the outer part (20) of the respective artificial cilium (310) to the magnetic field (5), for example, reverses the direction of the response of the outer part (20) of the respective artificial cilium (310) to the magnetic field (5).
6. The soft robot (1) of one of the preceding embodiments,
   wherein each artificial cilium (310) comprises a further magnetic unit (62),
   wherein, for each artificial cilium (310), the further magnetic unit (62) is fixed to the flexible part (31), for example at the first end (24).
7. The soft robot (1) of embodiment 6,
   wherein, for each artificial cilium (310), the magnetic unit (52) is configured to be placed next to the further magnetic unit (62).
8. The soft robot (1) of one of embodiments 6 and 7,
   wherein, for each artificial cilium (310), a direction of a magnetic moment of the further magnetic unit (62) differs from a direction of a magnetic moment (51) of the magnetic unit (52),
   wherein, for example, the direction of the magnetic moment of the further magnetic unit (62) is opposite to the direction of the magnetic moment (51) of the magnetic unit (52).
9. The soft robot (1) of one of embodiments 6 to 8,
   wherein a magnitude of the magnetic moment (253) of the further magnetic unit (62) equals a magnitude of the magnetic moment (51) of the magnetic unit (52).
10. The soft robot (1) of one of embodiments 6 to 8,
   wherein a magnitude of the magnetic moment (253) of further magnetic unit (62) is essentially twice or half a magnitude of the magnetic moment (51) of the magnetic unit (52).
11. The soft robot (1) of one of the preceding embodiments,
   wherein the artificial cilia (310) are arranged in a regular pattern, such as a row or a two-dimensional matrix.
12. The soft robot (1) of one of the preceding embodiments,
   wherein the artificial cilia (310) are configured as an actuator for moving a target (80), such as a fluid or a solid object.

The present disclosure is also directed at the following embodiments of a soft gripper:
1. A soft robot (1) configured as a soft gripper (350),
   comprising a body (10), a counterpart (354), and a support (12),
   wherein the body (10) comprises an outer part (20), an inner part (30), a magnetic unit (52),
   wherein the outer part (20) is flexible and forms a flexible finger (352) of the soft gripper (350),
   wherein the outer part (20) extends in a longitudinal direction (2) between a first end (24) and a second end (25) and a flexible channel (22) runs from the second end (25) to the first end (24) within the outer part (20),
   wherein the second end (25) is fixed to the support (12) and the first end (24) is configured as a free end,
   wherein the inner part (30) comprises the magnetic unit (52) and is movably arranged within the flexible channel (22),
   wherein the outer part (20), the inner part (30), and the magnetic unit (52) are configured to be placed within a magnetic field (5) so that the magnetic field (5) exerts a magnetic force on the magnetic unit (52),
   wherein the magnetic unit (52) and the outer part (20) are configured to move relative to each other to allow for a change of a magnetization profile of the outer part (20) and for a change of a response of the outer part (20) to the magnetic field (5),
   wherein the magnetic unit (52) is configured to be moved between a first position (151) with respect to the outer part (20) and a second position (152) with respect to the outer part (20),
   wherein the first end (24) of the outer part (20) is placed from the counterpart (354) at a first distance (355) in a bending direction (3) when the magnetic unit (52) is at the first position (151) and the first end (24) of the outer part (20) is placed from the counterpart (354) at a second distance (356) in the bending direction (3) when the magnetic unit (52) is at the second position (152),
   wherein the second distance (356) is smaller than the first distance (355) so that a gripper part (360) comprising the flexible finger (352) and the counterpart (354) is in an open state when the magnetic unit (52) is at the first position (151) and in a closed state when the magnetic unit (52) is at the second position (152).
2. The soft robot (1) of embodiment 1,
   wherein the outer part (20) is configured as a flat sheet (160) extending in the longitudinal direction (2) and in a width direction (8),
   wherein the flat sheet (160) has a thickness in a transverse direction (4) that is smaller than a length of the flat sheet (160) in the longitudinal direction (2) and a width of the flat sheet (160) in the width direction (8).
3. The soft robot (1) of one of the preceding embodiments,
   wherein the outer part (20) comprises a further channel (23),
   wherein a further magnetic unit (62) is movably arranged within the further channel (23),
   wherein the further magnetic unit (62) is configured to be placed within the magnetic field (5) so that the magnetic field (5) exerts a magnetic force on the further magnetic unit (62),
   wherein the further magnetic unit (62) is configured to move relative to the outer part (20) to allow for a change of a magnetization profile of the outer part (20) and for a change of a response of the outer part (20) to the magnetic field (5).
4. The soft robot (1) of embodiment 3,
   wherein the channel (22) and the further channel (23) run parallel to each other.
5. The soft robot (1) of one of embodiments 3 and 4,
   wherein a direction of a magnetic moment (51) of the magnetic unit (52) and a direction of a magnetic moment (63) of the further magnetic unit (62) are opposite with respect to each other.
6. The soft robot (1) of one of embodiments 3 to 5,
   wherein a magnitude of the magnetic moment (51) of the further magnetic unit (62) equals a magnitude of the magnetic moment (63) of the magnetic unit (52).
7. The soft robot (1) of one of embodiments 3 to 5,
   wherein a magnitude of the magnetic moment (63) of the further magnetic unit (62) is essentially twice or half a magnitude of the magnetic moment (51) of the magnetic unit (52), such as twice or half the magnitude of the magnetic moment (51) of the magnetic unit (52).
8. The soft robot (1) of one of the preceding embodiments,
   comprising an additional body (11) with an additional outer part (220), an additional inner part, and an additional magnetic unit,
   wherein the additional outer part (220) is flexible and forms a flexible additional finger (353) of the soft gripper (350),
   wherein the additional outer part (220) extends in the longitudinal direction (2) between a first end (224) and a second end (225) of the additional outer part (220) and a flexible additional channel (222) runs from the second end (225) to the first end (224) within the additional outer part (220),
   wherein the second end (225) of the additional outer part (220) is fixed to the support (12) and the first end (224) of the additional outer part (220) is configured as a free end,
   wherein the additional inner part comprises the additional magnetic unit and is movably arranged within the flexible additional channel (222),
   wherein the additional outer part (220), the additional inner part, and the additional magnetic unit are configured to be placed within the magnetic field (5) so that the magnetic field (5) exerts a magnetic force on the additional magnetic unit,
   wherein the additional magnetic unit and the additional outer part (220) are configured to move relative to each other to allow for a change of a magnetization profile of the additional outer part (220) and for a change of a response of the additional outer part (220) to the magnetic field (5),
   wherein the additional magnetic unit is configured to be moved between a first position with respect to the additional outer part (220) and a second position with respect to the additional outer part (220),
   wherein the additional outer part (220) assumes a first shape within the magnetic field (5) with the additional magnetic unit at the first position and the additional outer part (220) assumes a second shape within the magnetic field (5) with the additional magnetic unit at the second position, the first shape being different from the second shape,
   wherein the outer part (20) and the additional outer part (220) are deformable independently from each other.
9. The soft robot (1) of embodiment 8,
   wherein the magnetic unit (52) and the additional magnetic unit are movable independently from each other.
10. The soft robot (1) of one of embodiments 8 and 9,
   wherein the soft robot (1) comprises an additional counterpart (357),
   wherein the first end (224) of the additional outer part (220) is placed from the additional counterpart (357) at a first distance (358) in a bending direction (3) when the additional magnetic unit (252) is at the first position (151) and the first end (24) of the additional outer part (220) is placed from the additional counterpart (357) at a second distance (359) in the bending direction (3) when the additional magnetic unit (252) is at the second position (152),
   wherein the second distance (359) is smaller than the first distance (358) so that an additional gripper part (362) comprising the flexible additional finger (353) and the additional counterpart (357) is in an open state when the additional magnetic unit (252) is at the first position (151) and in a closed state when the additional magnetic unit (252) is at the second position (152).
11. The soft robot (1) of embodiment 10,
   wherein the additional outer part (220) forms the counterpart (354) and the outer part (20) forms the additional counterpart (357).
12. The soft robot (1) of one of embodiments 8 to 10,
   wherein the outer part (20) and the additional outer part (220) are placed next to each other in a width direction (8).

### Reference numeral list

- 1: soft robot
- 2: longitudinal direction
- 3: bending direction
- 4: outer direction
- 5: magnetic field
- 6: further magnetic field
- 7: length direction
- 8: width direction
- 9: height direction
- 10: body
- 11: additional body
- 12: support
- 15: target member
- 16: magnetic member
- 19: flexible part
- 20: outer part
- 21: fixed magnetic unit
- 22: channel
- 23: further channel
- 24: first end
- 25: second end
- 26: first outer part section
- 27: second outer part section
- 28: further outer part section
- 29: distance
- 30: inner part
- 31: flexible part
- 32: channel
- 34: first end
- 35: second end
- 36: first inner part section
- 37: second inner part section
- 38: further inner part section
- 40: further inner part
- 41: further flexible part
- 42: further channel
- 44: first end
- 45: second end
- 46: first section
- 47: second section
- 48: further section
- 49: distance
- 50: magnetic element
- 51: distance
- 52: magnetic unit
- 53: magnetic moment
- 54: first end
- 55: second end
- 56: further magnetic moment
- 57: distance
- 60: further magnetic element
- 62: further magnetic unit
- 63: magnetic moment
- 64: first end
- 65: second end
- 66: margin magnetic moment
- 67: further magnetic moment
- 68: distance
- 72: third magnetic unit
- 73: first distance
- 74: second distance
- 80: target
- 85: control body
- 86: control inner part
- 90: superposition
- 95: first profile
- 96: second profile
- 100: magnetic field generator
- 101: further magnetic field generator
- 102: field source
- 104: source actuator
- 105: source arrangement
- 106: magnitude
- 107: distance
- 108: support
- 110: manipulator
- 120: working zone
- 121: first working zone
- 122: second working zone
- 123: third working zone
- 124: fourth working zone
- 126: first magnetic field
- 127: second magnetic field
- 128: third magnetic field
- 129: fourth magnetic field
- 130: interaction zone
- 131: workspace
- 132: further interaction zones
- 133: overlapping region
- 135: feeding zone
- 137: bending deformation
- 138: actuation zone
- 139: bending zone
- 140: actuator
- 141: first part
- 142: second part
- 144: translational motion
- 145: rotational motion
- 151: first position
- 152: second position
- 160: flat sheet
- 161: length
- 162: width
- 163: height
- 164: first edge
- 165: second edge
- 166: edge channel
- 170: wall
- 180: box
- 181: first sidewall
- 182: second sidewall
- 190: state
- 191: distance
- 192: distance
- 195: first direction
- 196: second direction
- 197: magnetic field spacing
- 201: first distance
- 202: second distance
- 203: first inner part
- 204: first magnetic unit
- 205: second inner part
- 206: third inner part
- 207: fourth inner part
- 208: extra inner part
- 210: first channel
- 211: second channel
- 212: second magnetic unit
- 213: third channel
- 214: third magnetic unit
- 215: fourth channel
- 216: fourth magnetic unit
- 218: extra channel
- 219: extra magnetic unit
- 220: additional outer part
- 222: additional channel
- 224: first end
- 225: second end
- 230: additional inner part
- 241: distance
- 242: distance
- 251: additional magnetic unit
- 252: additional first magnetic unit
- 254: additional second magnetic unit
- 256: additional third magnetic unit
- 258: additional fourth magnetic unit
- 260: first common direction
- 262: second common direction
- 300: artificial cilia array
- 301: fluid
- 302: ink
- 303: further ink
- 305: paddle
- 310: artificial cilium
- 311: first cilium
- 312: second cilium
- 313: third cilium
- 314: path
- 315: row
- 316: trajectory
- 317: fourth cilium
- 318: first flow direction
- 319: second flow direction
- 320: first object
- 322: second object
- 323: third object
- 350: soft gripper
- 352: flexible finger
- 353: flexible additional finger
- 354: counterpart
- 355: first distance
- 356: second distance
- 357: additional counterpart
- 358: first distance
- 359: second distance
- 360: gripper part
- 362: additional gripper part
- 365: inactive outer part
- 366: active outer part
- 400: medical instrument
- 401: prior art instrument
- 402: obstacle
- 403: vessel
- 404: target
- 405: further target
- 406: starting point
- 407: further path
- 408: path
- 409: heart
- 410: additional further path
- 411: step
- 412: step
- 413: step
- 414: step
- 415: step
- 416: step
- 417: step
- 418: step
- 419: position
- 421: first state
- 422: second state
- 431: time
- 435: first magnetic field
- 436: second magnetic field
- 437: third magnetic field
- 438: third magnetic field
- 440: vascular system
- 441: blood vessel
- 443: heart
- 445: ultrasonic probe
- 451: first soft robot
- 452: second soft robot
- 453: third soft robot
- 455: obstruction
- 500: method
- 505: generating an external magnetic field
- 510: placing an outer part in an interaction zone
- 512: placing an outer part in an interaction zone
- 515: moving a magnetic member relative to a target member
- 520: setting a magnitude of an magnetic field to a reduced value
- 525: increasing the magnitude of the magnetic field
- 530: placing a magnetic unit at a first location and placing a further magnetic unit at a second location
- 535: placing a further magnetic unit next to a magnetic unit
- 540: rotating manipulator
- 550: producing positive mold
- 552: placing into a container with material
- 554: suspending vertically
- 556: curing
- 558: removing from mold
- 559: connecting
- 560: positive mold
- 561: plate
- 562: pin
- 563: part material
- 564: mold material
- 565: negative mold
- 566: mixture
- 567: spacer material
- 568: magnetic tube
- 569: magnetization field
- 570: prdoucing positive mold
- 571: immersing in part material
- 572: curing
- 573: removing from positive mold
- 574: providing magnetic particle mixture
- 575: filling into mold
- 576: curing
- 577: removing from negative mold
- 578: removing from positive mold
- 579: magnetizing
- 580: providing positive mold
- 581: placing into mold material
- 582: curing
- 583: removing from positive mold
- 585: providing magnetic particle mixture
- 586: filling
- 587: curing
- 588: removing from negative mold
- 589: substrate material
- 590: providing parts
- 591: assembling mold and filling with material
- 592: curing
- 593: removing mold
- 594: provide sidewalls
- 595: assemble sidewalls
- 596: base
- 597: inner mold part
- 598: mold
- 600: arrangement
- 650: system
- 701: first shape
- 702: second shape
- 703: third shape
- 705: deformation
- 710: radius of curvature
- 711: terminal bending angle
- 712: first radius of curvature
- 713: first terminal bending angle
- 714: second radius of curvature
- 715: second terminal bending angle
- 800: template
- 810: jig
- 812: diameter
- 814: pitch
- 820: plate
- 822: material
- 831: mounting
- 832: magnetizing
- 833: removing template
- 834: elastically recovering
- 835: placing in magnetic field
- 836: deforming
- 900: scale bar
- 910: sunflower
- 912: auxin
- 914: auxin transporter
- 916: deformation
- 918: sunlight

## Claims

1. Soft robot (1) comprising a body (10):
wherein the body (10) has an outer part (20), an inner part (30), and a magnetic unit (52),
wherein the outer part (20) is flexible and comprises a flexible channel (22),
wherein the inner part (30) is movably arranged within the channel (22),
wherein one of the inner part (30) and the outer part (20) forms a magnetic member (16) comprising the magnetic unit (52) and wherein the other one of the inner part (30) and the outer part (20) forms a flexible target member (15),
wherein the magnetic unit (52) is arranged inside the channel (22) or adjacent to the channel (22) in an outer direction (4) perpendicular to the channel (22),
wherein the outer part (20), the inner part (30), and the magnetic unit (52) are configured to be placed within a magnetic field (5) so that the magnetic field (5) exerts a magnetic force on the magnetic unit (52),
wherein the magnetic member (16) and the target member (15) are configured to move relative to each other to allow for a change of a magnetization profile of the target member (15) and for a change of a response of the target member (15) to the magnetic field (5).

2. The soft robot (1) of claim 1,
wherein the inner part (30) forms the magnetic member (16) comprising the magnetic unit (52) so that the magnetic unit (52) is movably arranged within the channel (22),
wherein the magnetic unit (52) is configured to be actuated through the channel (22),
wherein, for example, movement of the magnetic unit (52) is limited to an interior of the channel (22),
wherein, for example, the inner part (30) comprises a flexible part (31), the flexible part (31) is movably arranged with respect to the outer part (20), the magnetic unit (52) is fixedly attached to the flexible part (31), and the flexible part (31) forms a manipulator (110) for actuating the magnetic unit (52).

3. The soft robot (1) of one of the preceding claims,
wherein the body (10) comprises a further magnetic unit (62),
wherein the further magnetic unit (62) is configured to be placed within the magnetic field (5) so that the magnetic field (5) exerts a magnetic force on the further magnetic unit (62),
wherein the further magnetic unit (62) is arranged inside the channel (22) or adjacent to the channel (22) in an outer direction (4) perpendicular to the channel (22),
wherein the magnetic unit (52) and the further magnetic unit (62) are configured for being placed at a distance (53) from each other along the channel (22).

4. The soft robot (1) of one of claims 3,
wherein the further magnetic unit (62) is movable with respect to the magnetic unit (52),
wherein, for example, the further magnetic unit (62) is fixed to the target member (15),
wherein, for example, the magnetic unit (52) and the further magnetic unit (62) are configured to be placed adjacent to each other, such as adjoining each other in a longitudinal direction (2) or nested around each other in the outer direction (3).

5. The soft robot (1) of one of claims 3 and 4,
wherein a direction of a magnetic moment (51) of the further magnetic unit (62) is opposite to a direction of a magnetic moment (63) of the magnetic unit (52),
wherein a magnitude of the magnetic moment (63) of the further magnetic unit (62) equals a magnitude of the magnetic moment (51) of the magnetic unit (52) or the magnitude of the magnetic moment (63) of the further magnetic unit (62) is essentially twice or half a magnitude of the magnetic moment (51) of the magnetic unit (52), such as twice or half the magnitude of the magnetic moment (51) of the magnetic unit (52).

6. The soft robot (1) of one of the preceding claims,
wherein the magnetic unit (52) is configured to remain longitudinally stationary with respect to a surrounding of the soft robot (1) while the target member (15) translates longitudinally with respect to the magnetic unit (52).

7. The soft robot (1) of one of the preceding claims,
comprising a set of further inner parts (40) with at least one further inner part (40),
wherein each of the further inner parts (40) is flexible and comprises a further channel (42),
wherein the further inner parts (40) of the set are stacked one into the other in between the outer part (20) and the inner part (30),
wherein, for example, at least one of the further inner parts (40), such as a multitude of the further inner parts (40), like all further inner parts (40), each comprise a respective further magnetic unit (62) attached thereon,
wherein, for example, a stiffness of the inner part (30), respective stiffnesses of the further inner parts (40), and a stiffness of the outer part (20) decrease in the outer direction (4) from the inner part (30) to the outer part (20).

8. The soft robot (1) of one of the preceding claims,
wherein the inner part (30) comprises a first inner part section (36) that is configured to be located next to the magnetic unit (52) and a second inner part section (37) adjoining the first inner part section (36), the second inner part section (37) being configured to be placed away from the magnetic unit (52), the second inner part section (37) being configured to connect the first inner part section (36) to an actuator (140) to actuate a movement of the inner part (30) relative to the outer part (20), the first inner part section (36) being made from a first inner part material and the second inner part section (37) being made from a second inner part material, the second inner part material having a different stiffness than the first inner part material, such as a higher stiffness than the first inner part material, and/or
wherein the outer part (20) comprises a first outer part section (26) that is configured to be located next to the magnetic unit (52) and a second outer part section (27) adjoining the first outer part section (26), the second outer part section (27) being configured to be placed away from the magnetic unit (52), the second outer part section (27) being configured to connect the first outer part section (26) to an actuator (140) to actuate a movement of the inner part (30) with respect to the outer part (20), the first outer part section (26) being made from a first outer part material and the second outer part section (27) being made from a second outer part material, the second outer part material having a different stiffness than the first outer part material, such as a higher stiffness than the first outer part material.

9. The soft robot (1) of one of the preceding claims,
comprising an additional body (11) having an additional outer part (220), an additional inner part (230), and an additional magnetic unit (252),
wherein the additional outer part (220) is flexible and comprises a flexible additional channel (222),
wherein the additional inner part (230) is movably arranged within the additional channel (222),
wherein one of the additional inner part (230) and the additional outer part (220) forms an additional magnetic member (16) comprising the additional magnetic unit (252) and wherein the other one of the additional inner part (230) and the additional outer part (220) forms a flexible additional target member (15),
wherein the additional magnetic unit (252) is arranged inside the additional channel (222) or adjacent to the additional channel (222) in an outer direction (4) perpendicular to the additional channel (222),
wherein the additional outer part (220), the additional inner part (230), and the additional magnetic unit (252) are configured to be placed within the magnetic field (5) so that the magnetic field (5) exerts a magnetic force on the additional magnetic unit (252),
wherein the additional magnetic member (16) is configured to move relative to the additional target member (15) to allow for a change of a magnetization profile of the additional target member (15) and for a change of a response of the additional target member (15) to the magnetic field (5),
wherein the outer part (20) and the additional outer part (220) are deformable independently from each other,
wherein the magnetic unit (52) and the additional magnetic unit (252) are movable independently from each other,
wherein, for example, the outer part (20) and the additional outer part (220) are fixed to a common support (12),
wherein, for example, the outer part (20) and the additional outer part (20) are placed next to each other.

10. The soft robot (1) of one of the preceding claims,
wherein the outer part (20) forms the target member (15) and the inner part (30) forms the magnetic member (16),
wherein the magnetic unit (52) is arranged inside the channel (22),
wherein the outer part (20) comprises a second channel (211),
wherein a second magnetic unit (212) is movably arranged within the second channel (211),
wherein the second magnetic unit (212) is configured to be placed within the magnetic field (5) so that the magnetic field (5) exerts a magnetic force on the second magnetic unit (212),
wherein the second magnetic unit (212) is configured to move relative to the outer part (20) to allow for a change of a magnetization profile of the outer part (20) and for a change of a response of the outer part (20) to the magnetic field (5),
wherein, for example, the channel (22) and the second channel (211) run parallel to each other and/or the channel (22) and the second channel (211) are adjacent to each other and/or a magnetic moment (51) of the magnetic unit (52) and a magnetic moment (51) of the second magnetic unit (212) are opposite with respect to each other.

11. The soft robot (1) of one of the preceding claims,
further comprising an artificial cilia array (300),
wherein the outer part (20) forms an artificial cilium (310) placed in the artificial cilia array (300), the artificial cilia array (300) comprising a multitude of other artificial cilia (310),
wherein the artificial cilia array (300) comprises a multitude of other artificial cilia (310),
wherein the artificial cilium (310) and the other artificial cilia (310) each extend in a longitudinal direction (2) between a first end (24) and a second end (25),
wherein the respective second ends (25) are fixed to a common support (12) and the respective first ends (24) are configured as free ends,
wherein the magnetic unit (52) of the artificial cilium (310) and magnetic units (52) of the other artificial cilia (310) are movable independently from each other within the magnetic field (5) to independently modify a response of the artificial cilium (310) and the other artificial cilia (310) to the magnetic field (5).

12. The soft robot (1) of one of the preceding claims,
wherein the outer part (20) is configured as an elongated cylinder, for example as a flexible tube or a flexible rod,
wherein, for example, the body (10) forms part of a medical instrument (400), such as a catheter or an endoscope for medical use.

13. The soft robot (1) of one of claims 1 to 11,
wherein the outer part (20) is configured as a flat sheet (160) and/or
wherein the outer part (20) comprises at least two walls (170) arranged at an angle with respect to each other and adjoining each other at an edge (164, 165) of the outer part (20), the channel (22) being located at the edge (164, 165), such as running along the edge (164, 165) and/or
wherein the outer part (20) is configured as a box (180) having sidewalls (181, 182) adjoining each other at edges (164, 165) of the box (180), the channel (22) being located at one of the edges (164, 165), such as running along one of the edges (164, 165).

14. The soft robot (1) of one of the preceding claims,
wherein the body (10) is configured as a soft gripper (350),
wherein the outer part (20) forms a flexible finger (352) of the soft gripper (350).

15. A method (500) for operating the soft robot (1) of one of the preceding claims,
the method (500) comprising the steps of:
- generating (505) the external magnetic field (5) in an interaction zone (130, 132) by a magnetic field generator (100, 101),
- placing (510) the outer part (20), the inner part (30), and the magnetic unit (52) within the magnetic field (5) in the interaction zone (130, 132) so that the magnetic field (5) exerts a magnetic force on the magnetic unit (52),
- moving (515) the magnetic member (16) relative to the target member (15) by an actuator (140) to change the magnetization profile of the target member (15) and to change the response of the target member (15) to the magnetic field (5).
